# EUROPEAN PATENT APPLICATION

(11) **EP 1 188 448 A2**
(43) Date of publication of application: **20.03.2002**
(21) Application number: 01125266.5
(22) Date of filing: 15.03.1995
(51) Int. Cl.: A61K 47/48, A61K 48/00, A61K 41/00, C12N 15/62

(54) **Heparin-binding growth factors for gene therapy and anterior eye disorders**

(30) Priority: 15.03.1994 US 213446; 15.03.1994 US 213447
(62) Divisional of application: 95916103.5
(71) Applicant: PRIZM PHARMACEUTICALS, INC., San Diego, CA 92121-1204 (US)
(72) Inventor: The designation of the inventor has not yet been filed
(74) Representative: Gowshall, Jonathan Vallance

(57) **Abstract**

Preparations of conjugates of a heparin-binding growth factor and a targeted agent and compositions containing such preparations are provided. The conjugates contain a polypeptide that is reactive with an FGF receptor, such as bFGF, or another heparin-binding growth factor coupled to a targeted agent through a linker. The linker is selected to increase the specificity, toxicity, solubility, serum stability, and/or intracellular availability of the targeted moiety. Several linkers may be included in order to take advantage of desired properties of each linker. Pharmaceutical compositions containing these conjugates of FGF and a targeted agent and methods for prevention of recurrence of pterygii, closure of trabeculectomy and corneal hazing following excimer laser surgery are provided. The methods entail contacting the area of the eye that has been surgically treated with the composition during or immediately after surgery. Compositions of conjugates of a heparin-binding growth factor and a nucleic acid binding domain are provided. The conjugates bind nucleic acid molecules through the nucleic acid binding domain. These conjugates may be used to deliver nucleic acid encoding a cytotoxic protein or an antisense nucleic acid and the like to cells expressing receptors for the heparin-binding growth factor.

## Description

### Technical Field

The present invention relates generally to the treatment of diseases, and more specifically, to the preparation and use of heparin-binding growth factor conjugates to alter the function, gene expression, or viability of a cell in a therapeutic manner.

### Background of the Invention

### Ocular Disorders, Treatments and Complications

### Glaucoma

Glaucoma, which is the leading cause of blindness in the United States, is a group of diseases characterized by progressive atrophy of the optic nerve head leading to visual field loss, and, ultimately, blindness. Glaucoma is generally associated with elevated intraocular pressure, which is an important risk factor for visual field loss because it causes further damage to optic nerve fibers. There are several types of glaucoma, including open and closed angle glaucoma. The most prevalent type is primary open angle glaucoma in which the aqueous humor has free access to the irridocorneal angle, but aqueous humor drainage is impaired. In contrast, in closed angle glaucoma, the irridocorneal angle is closed by the peripheral iris. The angle block can usually be corrected by surgery. Less prevalent types of glaucoma include secondary glaucomas related to inflammation, trauma and hemorrhage.

The aqueous humor keeps the eyeball inflated, supplies the nutritional needs of the vascular lens and cornea and washes away metabolites and toxic substances within the eye. Aqueous humor enters posterior chamber by three means: (1) active secretion by nonpigmented epithelial cells of the ciliary process; (2) ultrafiltration of blood plasma; and (3) diffusion. Newly formed aqueous humor flows from the posterial chamber around the lens and through the pupil into the anterior chamber; aqueous humor leaves the eye by passive bulk flow at the irridocorneal angle and uveoscleral outflow. Intraocular pressure is a function of the difference between the rate at which aqueous humor enters and leaves eye.

Most treatments for glaucoma focus on reducing intraocular pressure. One problem in treating glaucoma is the difficulty in devising means to generate pharmacologically effective intraocular concentrations and to prevent extraocular side effects elicited by systemic administration. Many of the currently used drugs are administered locally. However, the amount of a drug that gets into the eye is only a small percentage of the topically applied dose because the tissues of the eye are protected from such substances by numerous mechanisms, including tear turnover, blinking, conjunctival absorption into systemic circulation, and a highly selective corneal barrier.

In addition, patients who have glaucoma are always at risk for developing an intolerance to medical therapy or laser therapy and may eventually require a filtration operation for control of their intraocular pressure. Present surgical techniques to lower intraocular pressure include procedures that permit fluid to drain from within the eye to extraocular sites. The most common operations for glaucoma are glaucoma filtering operations, particularly trabeculectomy. These operations involve creation of a fistula between the subconjunctival space and the anterior chamber. In order for the surgery to be effective, the fistula must remain substantially unobstructed. However, these drainage or filtering procedures often fail by virtue of their closure of the passageway resulting from the healing of the very wound created for gaining access to the surgical site. The surgery fails immediately in at least 15% of patients, and fails long term in a much higher percentage. Most frequently, failures result from scarring at the site of the incisions in the conjunctiva and the tenon's capsule. Presently, this consequence of trabeculectomy is treated with 5-fluorouracil and mitomycin C. These drugs, however, are highly toxic and have undesirable side effects, including scleral melting. Therefore, less toxic treatments to prevent closure are needed.

### Refractive surgery and complications therefrom

Until recently, surgical operations on the cornea were most commonly carried out using diamond or steel knives or razors. Corneal surgery, particularly with mechanical instruments, has often been less than satisfactory because the basement membrane upon which the epithelium attaches to the corneal proper is destroyed or damaged so that epithelial cells cannot regrow and form a continuous protective layer over the surface of the eye. Recently, new laser surgical techniques have been developed to ablate or otherwise treat corneal defects without mechanical abrasion. These techniques include photorefractive keratectomy ("PRK") and phototherapeutic keratectomy ("PTK") in which laser radiation is applied to the cornea with minimal heating effects to ablate or smooth refractive aberrations. Use of the laser achieves a predetermined refractive correction by volumetric removal of corneal tissue.

One technique for corneal reshaping involves the use of a pulsed laser photoablation apparatus to ablate very thin layers of corneal tissue with greater precision than can typically be achieved with mechanical means (*see,* Trokel et al., *Am. J. Ophthalmol*. *96*:710-715, 1983).

These laser corneal reprofiling operations, also referred to as photorefractive keratectomy ("PRK") or laser refractive keratoplasties (LRK), are performed with a high energy excimer laser (a laser based on the excited state of a halogen atom combining with the ground state of a rare gas such as krypton or xenon), which emits ultraviolet (UV) laser radiation and which ablates biological tissues without thermal damage to surrounding tissue (*see, e.g*., Marshall et al., "Photo-ablative Reprofiling Of The Cornea Using An Excimer Laser: Photorefractive Keratectomy," *Lasers in Ophthalmology*, *1*:21-48, 1986; U.S. Patent Nos. 5,133,708, 4,856,513 and 4,941,093; and U.S. Patent Nos. 4,665,913 and 4,732,148, which describe various procedures for correcting eye disorders attributable to abnormal curvature of the cornea). These laser keratectomies are used to correct astigmatisms; remove corneal scar tissue; and excise corneal tissue for accommodation of corneas in corneal transplants. In addition, procedures involving lasers can be used to perform incisions, including incisions for refractive effects such as radial keratotomy.

The use of excimer lasers for ophthalmic surgery is increasingly common since corneal transplants and keratotomies may be more precisely performed (*see, e.g.,* U.S. Patent No. 4,665,913). Even with the improved surgical methods using UV and non-UV emitting lasers, such as CO₂ and most lasers emitting in the visible spectrum, a condition known as "corneal haze" or "corneal clouding" an opacification of the cornea, often develops following use of these lasers. The opacification from laser surgery is seen in different parts of the cornea, but particularly in the stroma. The development of corneal haze is of potentially greater concern in those procedures affecting a large surface of the cornea versus procedures involving laser incisions and appears to result from exposure of the cornea to laser irradiation during ophthalmic surgery.

With the increasing use of lasers in ophthalmic surgery, particularly UV, CO₂, and most lasers emitting in the visible spectrum, there is a need for prevention of the corneal haze which results during ophthalmic procedures involving the use of lasers.

### Pterygia

Ptergyii are triangular fibrovascular growths on the surface of the eye that originate in the bulbar conjunctiva. They grow progressively over the cornea reducing vision by causing irregular astigmatism. In more severe stages, they grow across the visual axis causing blindness. Ultraviolet radiation exposure of mammalian eyes has been associated with the growth of the eye disease pterygia and the promotion of the conversion of pinguecula to pterygia (American Academy of Ophthalmology, Basic and Clinical Science Course, Cornea Section 4, Retina Vitress 1987-1988 Edition, pp. 72-73). Pterygii are often treated by surgical removal, but they are difficult to manage because of a 40%-50% recurrence rate. Recurrence is presently treated by radiation, mitomycin C drops and conjunctival autografting. Because radiation and mitomycin can cause scleral melting and loss of the eye, and autografting is expensive, delicate and often ineffective, there is a need to develop alternative treatments to prevent recurrence of pterygii.

In order to prevent recurrence of pterygii, to ensure the success of glaucoma filtering surgeries and to prevent corneal hazing following excimer laser surgeries of the eye, there is, thus, a need to develop methods and compositions for safe and effective treatments.

### Gene therapy

Genetic therapy for treatment of acquired and inherited diseases is a recent and highly promising addition to the repertoire of treatments for such diseases. It is expected that many congenital genetic abnormalities and acquired diseases will be amenable to treatment by genetic therapy. Diseases that are candidates for such treatment include those that are caused by a missing or defective gene that normally encodes an enzyme, hormone, or other protein. Examples of such diseases include: a severe combined immunodeficiency disorder, which is caused by a defect in the DNA that encodes adenosine deaminase (ADA) (*see, e.g*., Kredich et al. (1983), p. 1157, in *The Metabolic Basis of Inherited Disease* (5th ed.), eds. Stanbury, et al., McGraw-Hill, New York); Lesch Nyhan disease, which is caused by a defect in the enzyme hypoxanthine-guanine phosphoribosyl transferase (HGPRT); cystic fibrosis and Duchenne muscular dystrophy for which the respective defective genes have recently been identified; Tay sachs disease; and hemoglobin disorders, such as β-thalassemia. In addition, genetic therapy has been proposed as a means to deliver therapeutic products, such as tumor necrosis factor (TNF) for the treatment of cancers and CD4 receptor protein for the treatment of AIDS (*see, e.g.,* PCT International Application No. WO 90/01870). Recently, a gene encoding a transplantation antigen, HLA-B7, was introduced via liposomes by injection into malignant melanomas of several patients.

Genetic therapy involves introducing DNA into at least some cells of a host organism in a manner such that the products encoded by the DNA are expressed in the host. Upon introduction into the host cell, the DNA may be integrated into the genome of the host cells or it may be maintained and replicated as part of an episomal element. The DNA may encode products that replace or supplement the product of a defective or absent gene, a gene expressed at low levels, or therapeutic products that are effective for treating a disease. The DNA is typically operatively linked to a promoter and other transcriptional and translational regulatory elements that are recognized by host cell effector molecules, such as RNA polymerase II so that it can be expressed in the host cell. As the understanding of the underlying genetic bases for disease increases, it will be possible to refine the methods of genetic therapy so that regulatory controls that operate at the level of gene transcription or translation or that rely on mechanisms, such as feedback inhibition, to control expression of gene products can also be provided to the host cells. For example, the DNA may also mediate or encode RNA or protein products that mediate expression of a host cell gene or biochemical process. Expression of the DNA can thereby be fine-tuned to the needs of the afflicted host.

Genetic therapy is presently effected by removing selected target cells, from an afflicted individual, introducing DNA that encodes a therapeutically effective product into the cells and returning the modified cells to the individual.

At the present time, recombinant viral vectors, which are derived from viruses that infect eukaryotic cells, provide the most promising means for introducing DNA into cells. Generally, upon infection of a eukaryotic host, a virus commandeers the transcriptional and translational machinery of the host cell. In order to do so, viral regulatory signals, such as promoters, particularly those recognized early in infection, tend to be highly efficient so that any DNA that is in operative linkage with such promoters and regulatory signals is efficiently expressed at high levels. Eukaryotic viruses have, therefore, been used as vectors for cloning and expression of DNA in eukaryotic cells. There is, however, a risk, that the eukaryotic viruses, including the retroviruses presently in use, may recombine with host DNA to produce infectious virus. In addition, because retroviral viruses are often inactivated by the complement system, use *in vivo* is limited. Thus, there is a need to develop means to introduce DNA into targeted host cells without the use of recombinant viruses.

### Heparin-binding growth factors

In ocular disorders and in gene therapy, specificity of delivery of a cytotoxic agent or nucleic acid will enhance the effectiveness of the therapy by minimizing damage to normal cells or inappropriate and undesirable expression of products in nontarget cells. Growth factors, such as FGF, which specifically bind to receptors on target cells, have been conjugated directly to saporin-6 to produce the mitotoxin FGF-SAP (*see, e.g.,* U.S. Patent No. 5,191,067 to Lappi et al.; and Lappi et al., *Biochem. and Biophys. Res. Comm. 160*:917-923, 1989).

The chemistry of conjugation, however, gives rise to various structures, resulting in a heterogeneous population of products that are difficult to separate from each other and form aggregates as well. Because of the difficulties encountered in separating the resulting conjugates with different structures, heterogeneous mixtures are often used in experiments and even therapeutic applications.

Another limitation in the therapeutic use of cytotoxic conjugates for treatment of ocular disorders is the relatively low ratio of therapeutic to toxic dosage. Additionally, it is difficult to direct sufficient concentrations of the targeted agent into the cytoplasm and intracellular compartments in which the agent can exert its desired activity. This may be an especially important consideration in gene therapy. Upon binding to a receptor, such as the FGF receptor, it is believed that the conjugate is internalized via a pathway that directs a portion of the conjugate to the endosome, where, if cleaved, the targeted agent can be released into the cytoplasm. The conjugate is then trafficked to the lysosome, where it is degraded. It would be desirable to modify the conjugates so that, upon, internalization, a larger percentage of internalized conjugate is directed to the endosome or is cleaved in the endosome, whereby the effect of the linked agent may be realized. Since the cleavage is generally necessary for the linked agent to exert its effects, it would be desirable to increase the percentage of internalized conjugates that are cleaved upon internalization. It would also be desirable to render the conjugate more selective for the targeted cells, so that more of the conjugate or cleaved targeted agent reaches the cytoplasm of targeted cells, such as tumor cells, than the cytoplasm of non-targeted cells, so that lower concentrations may be administered.

In view of the problems associated with treatment of ocular disorders and gene therapy, there is a compelling need for improved treatments which are more effective and are not associated with such disadvantages. The present invention exploits the use of heparin-binding growth factor conjugates which have increased specificity and deliver higher amounts of agents, such as cytotoxins and nucleic acids to targeted cells, while providing other related advantages.

### Summary of the Invention

Preparations of conjugates and compositions containing preparations of conjugates are provided. The conjugates contain a polypeptide that is reactive with an FGF receptor (also referred to herein as an FGF protein), such as bFGF, linked to a targeted agent. In preferred embodiments the compositions are substantially monogenous. Conjugates and preparations of conjugates with enhanced specificity and/or activity are also provided.

The specificity, activity, and/or intracellular availability of conjugates of FGF and a targeted agent has been altered by including a linker or modifying the linkage between the FGF portion of the conjugate and the targeted moiety and/or by modifying the FGF portion of the conjugate, and, also, where advantageous modifying the targeted agent.

The conjugates provided herein may be represented by the formula:

FGF-(L)_{q}-targeted agent in which FGF refers to a polypeptide that is reactive with an FGF receptor (also referred to herein as an FGF protein), such as bFGF, L refers to a linker, q is 1 or more, generally 1 to 4, and the targeted agent is any agent, such as a cytotoxic agent or a nucleic acid, or a drug, such as methotrexate, intended for internalization by a cell that expresses an FGF receptor. More than one linker may be present; as many linkers as desired may be present as long as the resulting conjugate retains the requisite ability to bind to an FGF receptor and internalize the linked agent, which upon internalization retains its activity. The FGF may be linked through it N-terminus, C-terminus or elsewhere in the polypeptide to the targeted agent or linker.

Polypeptides that are reactive with an FGF receptor (FGF proteins) include any molecule that reacts with FGF receptors on cells that bear FGF receptors and results in internalization of the linked targeted agent. Particularly preferred polypeptides that are reactive with an FGF receptor include members of the FGF family of polypeptides, muteins of these polypetides, and chimeric or hybrid molecules that contain portions of any of these family members. Any member of the FGF family or any portion thereof that binds to FGF receptors and internalizes a linked agent may be used.

The linker is selected to increase the specificity, toxicity, solubility, serum stability, and/or intracellular availability targeted moiety. More preferred linkers are those that can be incorporated in fusion proteins and expressed in a host cell, such as *E. coli*. Such linkers include: enzyme substrates, such as cathepsin B substrate, cathepsin D substrate, trypsin substrate, thrombin substrate, subtilisin substrate, factor Xa substrate, and enterokinase substrate; linkers that increase solubility, flexibility, and/or intracellular cleavability, such as (glyₘser)ₙ and (serₘgly)ₙ, in which n is 1 to 6, preferably 1 to 4, more preferably 2 to 4, and m is 1 to 6, preferably 1 to 4, more preferably 2 to 4. Preferred among such linkers, are those, such as cathepsin D substrate, that are preferentially cleaved in the endosome or cytoplasm following internalization of the conjugate linker; other such linkers, such as (glyₘser)ₙ and (serₘgly)ₙ, increase the serum stability and/or solubility of the conjugate or the availability of the region joining the FGF and targeted agent for cleavage. In some embodiments, several linkers may be included in order to take advantage of desired properties of each linker.

Other linkers include, acid cleavable linkers, such as bismaleimideothoxy propane, acid labile-transferrin conjugates and adipic acid diihydrazide, that would be cleaved in more acidic intracellular compartments; photocleavable cross linkers that are cleaved by visible or UV light. For treatment of eye disorders, photocleavable linkers are of particular interest.

The targeted agents or moieties include any molecule that, when internalized, alter the metabolism or gene expression in the cell. Such agents include cytotoxic agents, such as ribosome inactivating proteins, nucleic acids and nucleic acids encoding cytotoxins, that result in inhibition of growth or cell death. Other such agents also include antisense RNA, DNA, and truncated proteins that alter gene expression via interactions with the DNA, or co-suppression or other mechanism. The conjugates herein may also be used to deliver DNA and thereby serve as agents for gene therapy or to deliver agents that, upon, transcription and/or translation thereof, result in cell death. Cytotoxic agents include, but are not limited to, ribosome inactivating proteins, inhibitors of DNA, RNA and/or protein synthesis, including antisense nucleic acids, and other metabolic inhibitors. In certain embodiments, the cytotoxic agent is a ribosome-inactivating protein (RIP), such as, for example, saporin, although other cytotoxic agents can also be advantageously used.

In preferred embodiments, substantially all of the conjugates in a preparation contain the same ratio of the polypeptide that is reactive with an FGF receptor to targeted agent. Such preparations are referred to as monogenous preparations. In preferred embodiments, all of the conjugates contain the same ratio of molecule of FGF protein and targeted agent per mole of conjugate so that the resulting preparation is a substantially monogenous.

Methods for the preparation of the conjugates, cytotoxic agents, such as a RIPs, including SAP, and the FGF polypeptides and monogenous preparation of cytotoxic conjugates that contains a defined molar ratio of each of the constituents are provided. These methods include chemical conjugation methods and methods that rely on recombinant production of the conjugates.

The resulting conjugates provided herein can be used in pharmaceutical compositions to treat FGF-mediated pathophysiological conditions by specifically targeting to cells having FGF receptors and inhibiting proliferation of or causing death of the cells. Such pathophysiological conditions include, for example, tumor development, restenosis, Dupuytren's Contracture, certain complications of diabetes such as proliferative diabetic retinopathies, rheumatoid arthritis, and certain ophthalmic disorders, such as secondary lens clouding following extracapsular cataract surgery and corneal clouding following laser surgery, such as photorefractive kerectomy (PRK), and dermatological disorders, such as psoriasis and Karposi's sarcoma. Treatment is effected by administering a therapeutically effective amount of the FGF conjugate in a physiologically acceptable excipient. Additionally, the conjugate can be used to target cytotoxic agents into cells having FGF receptors, and to inhibit the proliferation of such cells.

Methods and compositions for the treatment of complications following laser surgery and glaucoma surgery and for prevention and treatment of pterygii are provided. The methods entail contacting the affected portion of the eye with a composition containing conjugates of a polypeptide that is reactive with an fibroblast growth factor (FGF) receptor (also referred to herein as an FGF protein or FGF polypeptide) and a targeted cytotoxic agent.

Pharmaceutical compositions for use in the methods herein are also provided.

The resulting conjugates provided herein also can be used in pharmaceutical compositions to deliver nucleic acids to cells in order to alter the transcription translation of a particular gene product, to bind to a selected site on an intracellular protein or an extracellular protein, via an autocrine mechanism, or to effect genetic therapy. Methods for genetic therapy are also provided. The methods entail linking a nucleic acid encoding a therapeutic agent or encoding a gene that replaces a defective gene or provides an absent gene to a protein reactive with an FGF receptor and administering the resulting conjugate.

Conjugates of a heparin-binding growth factor protein and a nucleic acid binding domain bound to a nucleic acid molecule are provided. The nucleic acid binding domain may be bound to a specific sequence or bind nonspecifically. The growth factor is a member of the FGF, VEGF, or HBEGF family or fragment thereof. The nucleic acid molecule preferably encodes a protein capable of killing a cell or rendering the cell susceptible to killing. Further, the conjugate linkage may contain a linker that increases the serum stability or intracellular availability of the nucleic acid binding domain. A preferred embodiment is FGF conjugated to poly-L-lysine and the nucleic acid encodes saporin.

### Detailed Description

The disclosures of United States Application Serial No. 08/213,446, U.S. Application Serial No. 08/213,447, United States Application Serial No. 08/145,829, United States Application Serial No. 08/099,924, International PCT Application Serial No. PCT/US93/05702, United States Application Serial No. 07/901,718, U.S. Application Serial No. 08/024,682. U.S. Application Serial No. 08/030,218, International Application WO 92/04918, U.S. Application Serial No. 07/585,319 and U.S. Patent No. 5,191,067, to Lappi et al., are incorporated herein in their entirety by reference thereto.

### Definitions

Unless defined otherwise, all technical and scientific terms used herein have the same meaning as is commonly understood by one of skill in the art to which the subject matter herein belongs. All U.S. patents and all publications mentioned herein are incorporated in their entirety by reference thereto.

As used herein, "corneal haze" or "clouding" refers to the clouding of the cornea that results from exposure of the cornea to laser radiation during eye surgery, particularly LRK. The haze or clouding appears to result from fibroblastic keratocyte proliferation in the subepithelial zone following photoablation of the cornea.

As used herein, the term "cytotoxic agent" refers to a molecule capable of inhibiting cell function. The agent may inhibit proliferation or may be toxic to cells. The term includes agents whose toxic effects are mediated only when transported into the cell and also those whose toxic effect is mediated at the cell surface. A variety of cytotoxic agents can be used and include those that inhibit protein synthesis and those that inhibit expression of certain genes essential for cellular growth or survival. Cytotoxic agents include those that result in cell death and those that inhibit cell growth, proliferation and/or differentiation.

Cytotoxic agents include saporin, the ricins, abrin and other RIPs, *Pseudomonas exotoxin*, inhibitors of DNA, RNA or protein synthesis or other metabolic inhibitors that are known to those of skill in this art. Saporin is preferred, but other suitable RIPs and toxins include, but are not limited to, ricin, ricin A chain, maize RIP, gelonin, diphtheria toxin, diphtheria toxin A chain, trichosanthin, tritin, pokeweed antiviral protein (PAP), mirabilis antiviral protein (MAP), Dianthins 32 and 30, abrin, monordin, bryodin, shiga, a catalytic inhibitor of protein biosynthesis from cucumber seeds (*see, e.g.,* WO 93/24620, pseudomonas endotoxin and others known to those of skill in this art. The term RIP is used herein to broadly include such cytotoxins, as well as other cytotoxic molecules that inhibit cellular metabolic process, including transcription, translation, biosynthetic or degradative pathways, DNA synthesis and other such process, or that kill cells.

As used herein, "saporin" (abbreviated herein as, SAP) refers to polypeptides having amino acid sequences found in the natural plant host *Saponaria officinalis*, as well as modified sequences, having amino acid substitutions, deletions, insertions or additions, which still express substantial ribosome-inactivating activity. Purified preparations of saporin are frequently observed to include several molecular isoforms of the protein. It is understood that differences in amino acid sequences can occur in saporin from different species as well as between saporin molecules from individual organisms of the same species.

As used herein, "N-terminal extension" refers to a peptide region that is linked to the amino terminus of a biologically active portion of a cytotoxic agent, such as a saporin polypeptide, or another protein, such as a DNA binding domain. N-terminal extensions having as few as 2 amino acids, and up to many amino acids, are provided. The upper limit is determined empirically. The length of the N-terminal extension is not important as long as the resulting conjugate or fusion protein binds to cell surface receptors and is internalized. (*See* WO 93/25688, the disclosure of which is incorporated in its entirety herein.)

As used herein, a "linker" is an N-terminal extension that links the heparin-binding growth factor or fragment thereof and the targeted moiety, a second protein or nucleic acid. The linkers provided herein confer specificity, enhance intracellular availability, serum stability and/or solubility on the conjugate.

The linkers provided herein confer specificity on the cytotoxic conjugate by, for example, conferring specificity for certain proteases, particularly proteases that are present in only certain subcellular compartments or that are present at higher levels in tumor cells than normal cells. The linkers may also include sorting signals that direct the conjugate to particular intracellular loci or compartments. The linkers may also serve as spacers to reduce steric hindrance between the growth factor and other protein or linked nucleic acid.

A modification that is effected substantially near the N-terminus of a cytotoxic agent, such as saporin, is generally effected within the first about ten residues of the protein. Such modifications, include the addition or deletion of residues, such as the addition of a cysteine to facilitate conjugation between the polypeptide reactive with an FGF receptor or fragment of the polypeptide and the cytotoxic moiety portion to form cytotoxic agents that contain a defined molar ratio, preferably a ratio of 1:1, of cytotoxic agent and polypeptide reactive with an FGF receptor or fragment of the polypeptide.

As used herein, a "mitotoxin" is a cytotoxic molecule targeted to specific cells by a mitogen.

As used herein, "ligand" refers to any polypeptide that is capable of binding to a cell-surface protein and is capable of facilitating the internalization of a ligand-containing fusion protein into the cell. Such ligands include growth factors, antibodies or fragments thereof, hormones, and other types of proteins.

As used herein, the term "polypeptide reactive with an FGF receptor" refers to any polypeptide that specifically interacts with an FGF receptor, preferably the high-affinity FGF receptor, and that is transported into the cell by virtue of its interaction with the FGF receptor. Polypeptides reactive with an FGF receptor are also referred to herein as FGF proteins. FGF proteins include members of the FGF family of peptides, including FGF-1 through FGF-9, chimeras or hybrids of any of FGF-1 through FGF-9, or FGFs that have deletions (*see*, *e.g*., Published International Application No. WO 90/02800 and national stage applications and patents based thereon) or insertions of amino acids, as long as the resulting peptide or protein specifically interacts with an FGF receptor and is internalized by virtue of this interaction.

As used herein, "FGF" refers to polypeptides having amino acid sequences of native FGF proteins. as well as modified sequences, having amino acid substitutions, deletions. insertions or additions in the native protein but retaining the ability to bind to FGF receptors and to be internalized. Such polypeptides include, but are not limited to, FGF-1 - FGF-9. For example, bFGF should be generally understood to refer to polypeptides having substantially the same amino acid sequences and receptor-targeting activity as that of bovine bFGF or human bFGF. It is understood that differences in amino acid sequences can occur among FGFs of different species as well as among FGFs from individual organisms or species.

Reference to FGFs is also intended to encompass proteins isolated from natural sources as well as those made synthetically, as by recombinant means or possibly by chemical synthesis. FGF also encompasses muteins of FGF that possess the ability to target to FGF-receptor expressing cells. Such muteins include, but are not limited to, those produced by replacing one or more of the cysteines with serine as herein or that have any other amino acids deleted or replaced as long as the resulting protein has the ability to bind to FGF-receptor bearing cells and internalized the linked targeted agent. Typically, such muteins will have conservative amino acid changes, such as those set forth below in Table 1. DNA encoding such muteins will, unless modified by replacement of degenerate codons, hybridize under conditions of at least low stringency to DNA encoding bFGF (SEQ ID NOS. 12 and 13) or DNA encoding any of the FGF's set forth in SEQ ID. NOS. 24-32.

As used herein, "DNA encoding an FGF peptide or polypeptide reactive with an FGF receptor" refers to any of the DNA fragments set forth herein as coding such peptide and to any such DNA fragments known to those of skill in the art. Any such DNA molecule may be isolated from a human cell library using any of the preceding DNA fragments as a probe. It includes any DNA fragment that encodes any of the FGF peptides set forth in SEQ ID NOS. 24-32 (such DNA sequences are available in publicly accessible databases; see, also U.S. Patent No. 4,956,455, U.S. Patent No. 5,126,323, U.S. Patent No. 5,155,217, U.S. Patent No. 4,868.113, published International Application WO 90/08771 (and the corresponding U.S. patent, upon its issuance), which is based on U.S. Application Serial No. 07/304,281, filed January 31, 1989, and Miyamoto et al., *Mol. Cell. Biol. 13*:4251-4259, 1993), and any DNA fragment that may be produced from any of the preceding DNA fragments by substitution of degenerate codons. It is understood that once the complete amino acid sequence of a peptide, such as an FGF peptide, and the DNA fragment encoding such peptide are available to those of skill in this art, it is routine to substitute degenerate codons and produce any of the possible DNA fragments that encode such peptide. It is also generally possible to synthesize DNA encoding such peptide based on the amino acid sequence.

As used herein, "FGF receptors" refer to receptors that specifically interact with a member of the FGF family of proteins and transport it into the cell. Included among these are the receptors described in International Application No. WO 91/00916, which is based on U.S. Patent Application Serial No. 07/377,033; International Application No. WO 92/00999, which is based on U.S. Patent Application Serial No. 07/549,587; International Application No. WO 90/05522; and International Application No. WO 92/12948; *see, also*, Imamura, *Biochem. Biophys. Res. Comm. 155*:583-590, 1988; Partanen et al., *EMBO J. 10*:1347-1354, 1991; and Moscatelli. *J. Cell. Physiol*. *131*:123-130, 1987.

As used herein, the term "VEGF" refers to any polypeptide that specifically, either as a monomer or dimer, interacts with a VEGF receptor and is transported into the cell by virtue of its interaction with the receptor. In particular, as used herein, VEGF refers to peptides having amino acid sequences of native VEGF polypeptide monomers, as well as modified VEGF polypeptides, having amino acid substitutions, deletions, insertions or additions in the native protein, but when dimerized, retaining the ability to bind to a VEGF receptor and to be internalized in a cell bearing such receptor. Such polypeptides include, but are not limited to human VEGF₁₂₁, human VEGF₁₆₅, human VEGF₁₈₉, human VEGF₂₀₆, bovine VEGF₁₂₀, bovine VEGF₁₆₄, bovine VEGF₁₈₈, bovine VEGF₂₀₅, and homodimers and heterodimers of any VEGF monomer or monomers. It is understood that differences in amino acid sequences can occur among VEGFs of different species as well as among VEGFs from individual organisms or species and that such minor allelic variations or variations among species are intended to be encompassed by reference to VEGF herein.

Reference to VEGFs is intended to encompass proteins isolated from natural sources as well as those made synthetically, as by recombinant means or possibly by chemical synthesis. VEGF also encompasses muteins of VEGF that possess the ability to target a linked targeted agent to VEGF-receptor bearing cells. Such muteins include, but are not limited to, those produced by replacing one or more of the cysteines with serine as herein or those that have any other amino acids deleted or replaced, with the proviso that the resulting protein has the ability, either as a monomer or as a dimer, to bind to VEGF-receptor bearing cells and to be internalized upon such binding or to internalize a linked targeted agent. Typically, such muteins will have conservative amino acid changes, such as those set forth below in Table 1. DNA encoding such muteins will, unless modified by replacement of degenerate codons, hybridize under conditions of at least low stringency to DNA encoding a VEGF (SEQ ID NOS. 87-90) or an exon thereof (SEQ ID NOS. 78-86).

As used herein, a portion of a VEGF refers to a fragment or piece of VEGF that is sufficient, either alone or as a dimer with another fragment or a VEGF monomer, to bind to a receptor to which VEGF dimers bind and internalize a linked targeted agent.

As used herein, "DNA encoding a VEGF peptide or "polypeptide" refers to any of the DNA fragments set forth herein as coding such peptides, to any such DNA fragments known to those of skill in the art, any DNA fragment that encodes a VEGF that binds to a VEGF receptor and is internalized thereby and may be isolated from a human cell library using any of the preceding DNA fragments as a probe or any DNA fragment that encodes any of the VEGF peptides set forth in SEQ ID NOS. 87-90 and any DNA fragment that may be produced from any of the preceding DNA fragments by substitution of degenerate codons. It is understood that once the complete amino acid sequence of a peptide, such as a VEGF peptide, and one DNA fragment encoding such peptide are available to those of skill in this art, it is routine to substitute degenerate codons and produce any of the possible DNA fragments that encode such peptide. It is also generally possible to synthesize DNA encoding such peptide based on the amino acid sequence.

As used herein, "VEGF-mediated pathophysiological condition" refers to a deleterious condition characterized by or caused by proliferation of cells that are sensitive to VEGF mitogenic stimulation.

As used herein, "VEGF receptors" refer to receptors that specifically interact with a naturally-occurring member of the VEGF family of proteins and transport it into a cell bearing such receptors. Included among these are the fms-like tyrosine kinase receptor (FLT) and the kinase insert domain-containing receptor (KDR) (*see, e.g.,* International Application WO 92/14748, which is based on U.S. Applications Serial No. 08/657,236, de Vries et al., *Science 255*:989-91, 1992; Terman et al., *Biochem. Biophys. Res. Commun. 187*:1579-1586, 1992; Kendall et al., *Proc. Natl. Acad. Sci. USA 90*:10705-10709, 1993; and Peters et al., *Proc. Natl*. *Acad. Sci. USA 90*:8915-8919, 1993).

As used herein, "heparin-binding epidermal growth factor-like growth factor (HBEGF) polypeptides" refer to any polypeptide that specifically interacts with a HBEGF receptor, a receptor to which native human HBEGF polypeptide binds and which transports the HBEGF intracellularly, that has a heparin-binding domain, and that is transported into the cell by virtue of its interaction with the receptor. In particular, as used herein, HBEGF refers to polypeptides having amino acid sequences of a native HBEGF polypeptide, as well as variants, having amino acid substitutions, deletions, insertions or additions in the native protein but retaining the ability to bind to a HBEGF receptor and to be internalized in a cell bearing such receptor. Such HBEGF polypeptides peptides include, but are not limited to human HBEGF (SEQ ID NO. 92), monkey HBEGF (SEQ ID NO. 94) and rat HBEGF (SEQ ID NO. 95). HBEGF polypeptides include those having SEQ ID NOS. 91-95, N-terminally or C-terminally shortened versions thereof, including mature HBEGFs, and also including, modified versions of HBEGF thereof that retain the ability to bind to HBEGF receptors and internalize linked targeted agents.

Reference to HBEGFs is intended to encompass HBEGF polypeptides isolated from natural sources as well as those made synthetically, as by recombinant means or by chemical synthesis. This term also encompasses the precursor forms, such as those set forth in SEQ ID NOS. 91, 92 and 94-96 and mature forms, such as that set forth in SEQ ID NO. 93. HBEGF also encompasses muteins of HBEGF that possess the ability to target a targeted agent, such as a cytotoxic agent, including but not limited to ribosome- inactivating proteins, such as saporin, light activated porphyrin, and antisense nucleic acids, to HBEGF-receptor expressing cells. Such muteins include, but are not limited to, those produced by replacing one or more of the cysteines with serine as described hereinafter or that have any other amino acids deleted or replaced as long as the resulting protein has the ability to hind to HBEGF-receptor bearing cells and internalize the linked targeted agent. Typically, such muteins will have conservative amino acid changes, such as those set forth below in Table 1. DNA encoding such muteins will, unless modified by replacement of degenerate codons, hybridize under conditions of at least low stringency to DNA encoding native HBEGF (*e.g*., SEQ ID NO. 91) and encode an HBEGF polypeptide, as defined herein.

As used herein, "mature HBEGF" refers to processed HBEGFs. It has been found that various isoforms of mature HBEGF have variable N-termini, and include, but are not limited to, those having N-termini corresponding to amino acid positions 63, 73, 74, 77 and 82 of the precursor protein (*see, e.g*., SEQ ID NOS. 91-93, *see, also*, SEQ ID NOS. 94 and 95).

As used herein, a "portion of a HBEGF" refers to a fragment or piece of HBEGF that is sufficient to bind to a receptor to which native HBEGF binds and internalize a linked targeted agent.

As used herein, an "amino acid residue of HBEGF" is non-essential if a HBEGF polypeptide that has been modified by deletion of the residue possesses substantially the same ability to bind to a HBEGF receptor and internalize a linked agent that the unmodified HBEGF has.

As used herein, "DNA encoding an HBEGF peptide or polypeptide" refers to any DNA fragment encoding an HBEGF, as defined above. Exemplary DNA fragments include: any such DNA fragments known to those of skill in the art; any DNA fragment that encodes an HBEGF that binds to an HBEGF receptor and is internalized thereby and may be isolated from a human cell library using any of the preceding DNA fragments as a probe; and any DNA fragment that encodes any of the HBEGF polypeptides set forth in SEQ ID NOS. 92-95. Such DNA sequences encoding HBEGF fragments are available from publicly accessible databases, such as: DNA* July 1993 release from DNASTAR, Inc. Madison, WI, and GENBANK Accession Nos. M93012 (monkey) and M60278 (human); the plasmid pMTN-HBEGF (ATCC #40900) and pAX-HBEGF (ATCC #40899) described in published International Application WO/92/06705 (*see, also,* the corresponding U.S. Patent upon its issuance); and Abraham et al., *Biochem. Biophys. Res. Comm. 190*:125-133, 1993). DNA encoding HBEGF polypeptides will, unless modified by replacement of degenerate codons, hybridize under conditions of at least low stringency to DNA encoding a native HBEGF (*e.g.*, SEQ ID NO. 91). In addition, any DNA fragment that may be produced from any of the preceding DNA fragments by substitution of degenerate codons is also contemplated for use herein. It is understood that since the complete amino acid sequence of HBEGF polypeptides, and DNA fragments encoding such peptides, are available to those of skill in this art, it is routine to substitute degenerate codons and produce any of the possible DNA fragments that encode such HBEGF polypeptides. It is also generally possible to synthesize DNA encoding such peptides based on the amino acid sequence.

As used herein, the "HBEGF receptor (HBEGF-R)" refers to receptors that specifically interact with members of the HBEGF family of proteins and that are able to transport HBEGF into the cell, *e.g.,* by receptor-mediated endocytosis. For example, HBEGF polypeptides interact with the high affinity EGF receptors (EGF-R) on bovine aortic smooth muscle cells and A431 epidermoid carcinoma cells (*see* Higashiyama et al., *Science 251*:936-939, 1991; Higashiyama et al., *J. Biol. Chem. 267*:6205-6212, 1992). Thus, EGF-receptors are also HBEGF-Rs. Included among these are the EGF receptors described in U.S. Patent Nos. 5,183,884 and 5,218,090; and Ullrich et al., *Nature 309*:418-425, 1984. The EGF-Rs described herein include those encoded by the erbB gene family.

"Heparin-binding growth factor" refers to any member of a family of heparin-binding growth factor proteins, in which at least one member of the family binds heparin. Preferred growth factors in this regard include FGF, VEGF, and HBEGF. Such growth factors encompass isoforms, peptide fragments derived from a family member, splice variants, and single or multiple exons, some forms of which may not bind heparin.

"Nucleic acid binding domain (NABD)" refers to a protein, polypeptide, or peptide that binds nucleic acids, such as DNA or RNA, under physiological salt conditions. Such NABD may bind to a specific DNA sequence or bind irrespective of the sequence.

As used herein, to "target" a targeted agent, such as a cytotoxic agent, means to direct it to a cell that expresses a selected receptor by linking the agent to a polypeptide reactive with an FGF receptor or other heparin-binding growth factor to produce a conjugate. Upon binding to the receptor the conjugate is internalized by the cell and the conjugate is trafficked through the cell via the endosomal compartment, where cleavage of the conjugate can occur.

As used herein, "preparations of monogenous conjugates" are preparations of conjugates in which each conjugate has the same, generally 1:1, though not necessarily, molar ratio of targeting molecule to targeted agent. Monogenous conjugates are substantially identical in that they possess indistinguishable chemical and physical properties and generally preparations of such conjugates contain only one species of conjugate. It is, of course understood, that some variability among the species may be present and will be tolerated to the extent that the activity of each member of the conjugate is substantially the same. For example, saporin that is expressed in bacterial hosts as provided herein may contain a mixture of species that differ at their N-terminus. Such recombinantly produced saporin, however, is suitable for use to produce chemically conjugated conjugates by the methods herein. The resulting preparation is monogenous as defined herein in that each conjugate contains the same molar ratio of FGF protein to targeted agent, but each conjugate is not necessarily identical, but is substantially identical in that each conjugate has substantially the same biological activity.

As used herein, a "homogeneous population" or composition of conjugates means that the constituent members of the population or composition are monogenous and further do not form aggregates.

As used herein, "secretion signal" refers to a peptide region within the precursor protein that directs secretion of the precursor protein from the cytoplasm of the host into the periplasmic space or into the extracellular growth medium. Such signals may be either at the amino terminus or carboxyl terminus of the precursor protein. The preferred secretion signal is linked to the amino terminus of the N-terminal extension region.

As used herein, a "nuclear translocation or targeting sequence" (NTS) is a sequence of amino acids in a protein that are required for translocation of the protein into a cell nucleus. Examples of NTS are set forth in Table I, below. Comparison with known NTSs, and if necessary testing of candidate sequences, should permit those of skill in the art to readily identify other amino acid sequences that function as NTSs. A heterologous NTS refers to an NTS that is different from the NTS that occurs in the wild-type peptide, polypeptide, or protein. For example, the NTS may be derived from another polypeptide, it may be synthesized, or it may be derived from another region in the same polypeptide.

As used herein, "nucleic acids" describe any nucleic acids used in the context of the invention that modify gene transcription or translation. This term also includes nucleic acids and methods that provide nucleic acids that bind to sites on proteins and to receptors. It includes, but is not limited to, the following types of nucleic acids: nucleic acids encoding a protein, antisense mRNA, DNA intended to form triplex molecules, extracellular protein binding oligonucleotides, and small nucleotide molecules.

Antisense nucleic acids are single-stranded nucleic acids construct that specifically bind to mRNA by way of complementary sequences, thereby preventing. translation of the mRNA (*see, e.g*., U.S. Patent No. 5,168,053 to Altman et al., U.S. Patent No. 5,190,931 to Inouye, U.S. Patent No. 5,135,917 to Burch, and U.S. Patent No. 5,087,617 to Smith). Antisense nucleic also include double-stranded cyclic oligonucleotides, such as hammerhead or dumbbell oligonucleotides, which have been shown to specifically inhibit RNA synthesis (*see, e.g*., Clusel et al., *Nucl. Acids Res. 21*:3405-3411, 1993).

Triplex molecules refer to single DNA strands that target duplex DNA, forming colinear triplexes by binding to the major groove, and thereby prevent or alter transcription (*see, e.g*., U.S. Patent No. 5,176,996 to Hogan et al.). Triplex DNA has been designed that bind tightly and specifically to selected DNA sites.

A ribozyme is an enzyme that is made of RNA and primarily acts on RNA substrates. As used herein, ribozymes refer to RNA constructs that specifically cleave messenger RNA (*see, e.g.,* U.S. Patent Nos. 5,180,818, 5,116,742 and 5,093,246 to Cech et al.) and in particular refers to ribozymes that are designed to target RNA molecules for cleavage and that thereby in some manner inhibit or interfere with cell growth or with expression of a targeted mRNA or protein.

Extracellular protein binding oligonucleotides refer to oligonucleotides that specifically bind to proteins

Nucleic acids may be composed of the well-known deoxyribonucleotides and ribonucleotides composed of the bases: adenosine, cytosine, guanine, thymidine, and uridine. As well, various other nucleotide derivatives and non-phosphate backbones or phosphate-derivative backbones may be used. For example, because normal phosphodiester oligonucleotides (referred to as PO oligonucleotides) are sensitive to DNA- and RNA-specific nucleases (X = 0 (see structure, below); type I), several resistant types of oligonucleotides have been developed. These include types II-IV oligonucleotides: 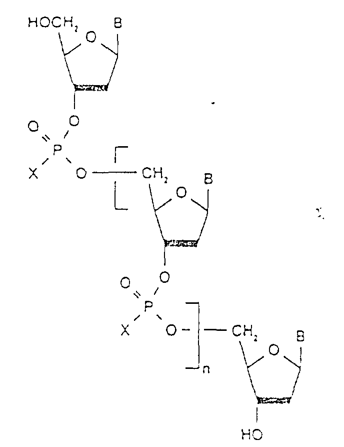 in which B is a nucleotide base; and X is OEt in phosphotriester (type II), X is Me in methylphosphonate (type III; referred to as MP oligos); and X is S in phosphorothioate (referred to as PS oligos; U.S. Patent No. 5,218,088 to Gorenstein et al. describes a method for preparation of PS oligos). Presently, MP and PS oligonucleotides have been the focus of most investigation. Nucleic acids may be single or double stranded and may be chimeric, that is composed of both DNA and RNA.

As used herein, a "therapeutic nucleic acid" refers to a nucleic acid that is used to effect genetic therapy by serving as a replacement for a defective gene, by encoding a therapeutic product, such as TNF, or by encoding a cytotoxic molecule, especially an enzyme, such as saporin. The therapeutic nucleic acid may encode all or a portion of a gene, and may function by recombining with DNA already present in a cell, thereby replacing a defective portion of a gene. It may also encode a portion of a protein and exert its effect by virtue of co-suppression of a gene product.

As used herein, "expression vector" includes vectors capable of expressing DNA fragments that are in operative linkage with regulatory sequences, such as promoter regions, that are capable of effecting expression of such DNA fragments. Thus, an expression vector refers to a recombinant DNA or RNA construct, such as a plasmid, a phage, recombinant virus or other vector that, upon introduction into an appropriate host cell, results in expression of the cloned DNA. Appropriate expression vectors are well known to those of skill in the art and include those that are replicable in eukaryotic cells and/or prokaryotic cells and those that remain episomal or may integrate into the host cell genome.

As used herein, a "promoter region" refers to the portion of DNA of a gene that controls transcription of DNA to which it is operatively linked. A portion of the promoter region includes specific sequences of DNA that are sufficient for RNA polymerase recognition, binding and transcription initiation. In addition, the promoter region includes cis- or trans-acting sequences that modulate this recognition, binding and transcription initiation activity of the RNA polymerase. Promoters, depending upon the nature of the regulation, may be constitutive or regulated. Preferred promoters for use herein are tightly regulated such that, absent induction, the DNA encoding the polypeptide is not expressed.

As used herein, a "transcription terminator region" has either (a) a subsegment that encodes a polyadenylation signal and polyadenylation site in the transcript, and/or (b) a subsegment that provides a transcription termination signal that terminates transcription by the polymerase that recognizes the selected promoter. Transcription terminators are optional components of the expression systems herein, but are employed in preferred embodiments.

As used herein, "transfection" refers to the taking up of DNA or RNA by a host cell. Transformation refers to this process performed in a manner such that the DNA is replicable, either as an extrachromosomal element or as part of the chromosomal DNA of the host. Methods and means for effecting transfection and transformation are well known to those of skill in this art (*see, e.g.,* Wigler et al., *Proc. Natl. Acad. Sci. USA 76*:1373-1376, 1979; Cohen et al., *Proc. Natl. Acad. Sci. USA 69*:2110, 1972).

As used herein, "FGF-mediated pathophysiological condition" refers to a deleterious condition characterized by or caused by proliferation of cells that are sensitive to bFGF mitogenic stimulation. Basic FGF-mediated pathophysiological conditions include, but are not limited to, certain tumors, rheumatoid arthritis, restenosis, Dupuytren's Contracture and certain complications of diabetes, such as proliferative retinopathy.

As used herein, "substantially pure" means sufficiently homogeneous to appear free of readily detectable impurities as determined by standard methods of analysis and which are sufficiently pure such that further purification would not detectably alter the physical and chemical properties, such as enzymatic and biological activities, of the substance. Methods for purification of the compounds to produce substantially chemically pure compounds are known to those of skill in the art. A substantially chemically pure compound may, however, be a mixture of stereoisomers. In such instances, further purification might increase the specific activity of the compound.

As used herein, to "hybridize" under conditions of a specified stringency is used to describe the stability of hybrids formed between two single-stranded nucleic acid fragments and refers to the conditions of ionic strength and temperature at which such hybrids are washed, following annealing under conditions of stringency less than or equal to that of the washing step. Typically high, medium and low stringency encompass the following conditions or equivalent conditions thereto:
1) high stringency: 0.1 x SSPE or SSC, 0.1% SDS, 65°C
2) medium stringency: 0.2 x SSPE or SSC, 0.1% SDS, 50°C
3) low stringency: 1.0 x SSPE or SSC, 0.1% SDS, 50°C.
Equivalent conditions refer to conditions that select for substantially the same percentage of mismatch in the resulting hybrids. Ingredients, such as formamide, Ficoll, and Denhardt's solution may be added and affected parameters such as the temperature under which the hybridization should be conducted and the rate of the reaction are adjusted according to well-known formulas. The recipes for SSPE, SSC and Denhardt's are described, for example, in Sambrook et al. (1989) *Molecular Cloning. A Laboratory Manual*, Cold Spring Harbor Laboratory Press, Chapter 8; *see,* Sambrook et al., vol. 3, p. B.13, *see, also*, numerous catalogs that describe commonly used laboratory solutions).

As used herein, "culture" means a propagation of cells in a medium conducive to their growth, and all sub-cultures thereof. The term "subculture" refers to a culture of cells grown from cells of another culture (source culture), or any subculture of the source culture, regardless of the number of subculturings that have been performed between the subculture of interest and the source culture.

As used herein, an "effective amount" of a compound for treating a particular disease is an amount that is sufficient to ameliorate, or in some manner reduce the symptoms associated with the disease. Such amount may be administered as a single dosage or may be administered according to a regimen, whereby it is effective. The amount may cure the disease but, typically, is administered in order to ameliorate the symptoms of the disease. Repeated administration may be required to achieve the desired amelioration of symptoms.

As used herein, "pharmaceutically acceptable" salts, esters or other derivatives of the conjugates include any salts, esters or derivatives that may be readily prepared by those of skill in this art using known methods for such derivatization and that produce compounds that may be administered to animals or humans without substantial toxic effects and that either are pharmaceutically active or are prodrugs.

As used herein, "treatment" means any manner in which the symptoms of a conditions, disorder or disease are ameliorated or otherwise beneficially altered. Treatment also encompasses any pharmaceutical use of the compositions herein. Amelioration of the symptoms of a particular disorder by administration of a particular pharmaceutical composition refers to any lessening, whether permanent or temporary, lasting or transient that can be attributed to or associated with administration of the composition.

As used herein, an "ophthalmically effective amount" is that amount which, in the composition administered and by the technique administered, provides an amount of therapeutic agent to the involved eye tissues sufficient to prevent or reduce corneal haze following excimer laser surgery, prevent closure of a trabeculectomy or prevent or substantially slow the recurrence of pterygii.

As used herein, "biological activity" refers to the *in vivo* activities of a compound or physiological responses that result upon *in vivo* administration of a compound, composition or other mixture. Biological activity, thus, encompasses therapeutic effects and pharmaceutical activity of such compounds, compositions and mixtures. The biological activity of a cytotoxic agent, such as saporin or an antisense nucleic acid, refers to the ability of such agent to interfere with the metabolism of the cell by inhibiting protein synthesis, DNA synthesis, or the activities of particular proteins and regulatory molecules. Thus, the biological activity of a RIP refers to its ability to inhibit protein synthesis by inactivation of ribosomes either *in vivo* or *in vitro* or to inhibit the growth of or kill cells upon internalization of the RIP by the cells. Such biological or cytotoxic activity may be assayed by any method known to those of skill in the art including, but not limited to, the *in vitro* assays that measure protein synthesis and *in vivo* assays that assess cytotoxicity by measuring the effect of a test compound on cell proliferation or on protein synthesis. Particularly preferred, however, are assays that assess cytotoxicity in targeted cells.

As used herein, "ED₅₀" refers to the concentration at which 50% of the cells are killed following a 72-hour incubation with a cytotoxic conjugate, such as FGF-SAP.

As used herein, "ID₅₀" refers to the concentration of a cytotoxic conjugate required to inhibit protein synthesis in treated cells by 50% compared to protein synthesis in the absence of the protein.

### PREPARATION OF CONJUGATES

The conjugates that are provided herein contain a heparin-binding growth factor protein linked via a linker to a targeted agent, such as a cytotoxic agent, DNA binding domain or nucleic acid. The linking is effected either chemically, by recombinant expression of a fusion protein in instances when the targeted agent is a protein, and by combinations of chemical and recombinant expression. Upon binding to an appropriate receptor, the conjugate is internalized by the cell and the conjugate is trafficked through the cell via the endosomal compartment, where at least a portion of it is cleaved.

Some of the linkers, such as the cathepsin D linkers, provided herein are designed to increase the portion of conjugate that is cleaved in the endosome of targeted cells, such as tumor cells, and to thereby increase the amount of targeted agent that is delivered to the cytoplasm and/or nucleus. Others of the linkers provided herein are designed to increase the serum stability and/or solubility of the conjugate and thereby increase the amount of targeted agent delivered to the targeted cell. In preferred embodiments, several linkers will be combined to take advantage of the desired properties of each.

### A. Preparation of heparin-binding growth factors and targeted agents

### 1. Heparin-binding growth factors

Numerous growth factors and families of growth factors that share structural and functional features have been identified, including families of growth factors that specifically bind to heparin. The ability of heparin-binding growth factors to interact with heparin appears in general to be a reflection of a physiologically more relevant interaction occurring *in vivo* between these factors and heparan sulfate proteoglycan molecules, which are found on the surface of cells and in extracellular matrix. The results to date on the heparin/heparan-binding growth factors indicate that the ultimate biological activity and bioavailability of some of these factors may depend not only on the spatial and temporal expression of the factors and their respective high affinity receptors, but also on the local expression of the heparan sulfate proteoglycans.

### a. Fibroblast growth factors

One family of growth factors that has a broad spectrum of activities is the fibroblast growth factor (FGF) family. These proteins share the ability to bind to heparin, induce intracellular receptor-mediated tyrosine phosphorylation and the expression of the c-fos mRNA transcript, and stimulate DNA synthesis and cell proliferation. This family of proteins includes FGFs designated FGF-1 through FGF-9 (or acidic FGF (aFGF), basic FGF (bFGF), int-2 (*see, e.g*., Moore et al., EMBO J. 5:919-924, 1986), hst-1/K-FGF (*see, e.g*., Sakamoto et al., Proc. Natl. Acad. Sci. U.S.A. 86:1836-1840, 1986; U.S. Patent No. 5,126,323), FGF-5 (*see, e.g*., U.S. Patent No. 5,155,217), FGF-6/hst-2 (*see, e*.*g*., published European Application EP 0 488 196 A2; Uda et al., Oncogene 7:303-309, 1992), keratinocyte growth factor (KGF; *see, e.g.,* Finch et al., Science 245:752-755, 1985; Rubin et al., Proc. Natl. Acad. Sci. U.S.A. 86:802-806, 1989; and International Application WO 90/08771), FGF-8 (*see, e.g.,* Tanaka et al., Proc Natl. Acad. Sci. U.S.A. 89:8528-8532. 1902). and FGF-9 (*see,* Miyamoto et al., Mol. Cell. Biol. 13:4251-4259, 1993), respectively

Acidic and basic FGF, which were the first members of the FGF family that were characterized, are about 55% identical at the amino acid level and are highly conserved among species. Basic FGF has a molecular weight of approximately 16 kD, is basic and temperature sensitive and has a high isoelectric point. Acidic FGF has an acidic isoelectric point. The other members of the FGF family have subsequently been identified on the basis of amino acid sequence homologies with aFGF and bFGF and common physical and biological properties. including the ability to bind to one or more FGF receptors. Basic FGF, int-2, hst-1/K-FGF, FGF-5, hst-2/FGF-6 and FGF-8 are oncogenes. bFGF is expressed in melanomas, int-2 is expressed in mammary tumor virus and hst-1/K-FGF is expressed in angiogenic tumors. Acidic FGF, bFGF, KGF and FGF-9 are expressed in normal cells and tissues.

FGFs exhibit a mitogenic effect on a wide variety of mesenchymal, endocrine and neural cells. They are also important in differentiation and development. Of particular interest is their stimulatory effect on collateral vascularization and angiogenesis. Such effects have stimulated considerable interest in FGFs as therapeutic agents, for example, as pharmaceuticals for wound healing, neovascularization, nerve regeneration and cartilage repair. In addition to potentially useful proliferative effects, FGF-induced mitogenic stimulation may, in some instances, be detrimental. For example, cell proliferation and angiogenesis are an integral aspect of tumor growth. Members of the FGF family, including bFGF, are thought to play a pathophysiological role, for example, in tumor development, rheumatoid arthritis, proliferative diabetic retinopathies and other complications of diabetes.

The effects of FGFs are mediated by high affinity receptor tyrosine kinases on the cell surface membranes or FGF-responsive cells (*see, e.g.,* Imamura et al., *Biochem. Biophys. Res. Comm. 155*:583-590, 1988; Huang et al., *J*. *Biol. Chem. 261*:9568-9571, 1986, which are incorporated herein by reference). Lower affinity receptors also play a role in mediating FGF activities. The high affinity receptor proteins, which are single chain polypeptides with molecular weights ranging from 110 to 150 kD, depending on cell type, constitute a family of structurally related FGF receptors. Four FGF receptor genes have been identified, and at least two of these genes generate multiple mRNA transcripts via alternative splicing of the primary transcript.

### b. Vascular endothelial growth factors

Vascular endothelial growth factors (VEGFs) were identified by their ability to directly stimulate endothelial cell growth, but do not appear to have mitogenic effects on other types of cells. VEGFs also cause a rapid and reversible increase in blood vessel permeability. The members of this family have been referred to variously as vascular endothelial growth factor (VEGF), vascular permeability factor (VPF) and vasculotropin (*see, e.g*., Plouet et al., *EMBO J. 8*:3801-3806, 1989) and are collectively referred to as VEGF.

VEGF was originally isolated from a guinea pig heptocarcinoma cell line, line 10, (*see, e.g.,* U.S. Patent No. 4,456,550 to Dvorak et al.) and has subsequently been identified in humans and in normal cells. It is a glycoprotein that binds to specific cell surface receptors and is expressed during normal development and in certain normal adult organs. Purified VEGF is a basic, heparin-binding, homodimeric glycoprotein, which is heat-stable, acid-stable and may be inactivated by reducing agents.

VEGF family members arise from a single gene organized as eight exons and spanning approximately 14 kb in the human genome. Four molecular species of VEGF result from alternative splicing of mRNA and contain 121, 165, 189 and 206 amino acids. The four species have similar biological activities, but differ markedly in their secretion patterns. The predominant isoform secreted by a variety of normal and transformed cells is VEGF₁₆₅. Transcripts encoding VEGF₁₂₁ and VEGF₁₈₉ are detectable in most cells and tissues that express the VEGF gene. In contrast, VEGF₂₀₆ is less abundant and has been identified only in a human fetal liver cDNA library. VEGF₁₂₁ is a weakly acidic polypeptide that lacks the heparin binding domain and, consequently, does not bind to heparin. VEGF₁₈₉ and VEGF₂₀₆ are more basic than VEGF₁₆₅ and bind to heparin with greater affinity. Although not every identified VEGF isoform binds heparin, all isoforms are considered to be heparin-binding growth factors within the context of this invention.

The secreted isoforms, VEGF₁₂₁ and VEGF₁₆₅ are preferred VEGF proteins. The longer isoforms, VEGF₁₈₉ and VEGF₂₀₆, are almost completely bound to the extracellular matrix and need to be released by an agent, such as suramin, heparin or heparinase, and plasmin. Other preferred VEGF proteins contain various combinations of VEGF exons, such that the protein still binds VEGF receptor and is internalized. It is not necessary that a VEGF protein used in the context of this invention either retain any of its *in vivo* biological activities, such as stimulating endothelial cell growth, or bind heparin. It is only necessary that the VEGF protein or fragment thereof bind the VEGF receptor and be internalized into the cell bearing the receptor. However, it may be desirable in certain contexts for VEGF to manifest certain of its biological activities. For example, if VEGF is used as a carrier for DNA encoding a molecule useful in wound healing, it would be desirable that VEGF exhibit vessel permeability activity and promotion of fibroblast migration and angiogenesis. It will be apparent from the teachings provided within which of the activities of VEGF are desirable to maintain.

VEGF promotes an array of responses in endothelium, including blood vessel hyperpermeability, endothelial cell growth, angiogenesis, and enhanced glucose transport. VEGF stimulates the growth of endothelial cells from a variety of sources (including brain capillaries, fetal and adult aortas, and umbilical veins) at low concentrations, but is reported to have no effect on the growth of vascular smooth muscle cells, adrenal cortex cells, keratinocytes, lens epithelial cells, or BHK-21 fibroblasts. VEGF also is a potent polypeptide regulator of blood vessel function; it causes a rapid but transient increase in microvascular permeability without causing endothelial cell damage or mast cell degranulation, and its action is not blocked by antihistamines. VEGF has also been reported to induce monocyte migration and activation.

VEGF has been implicated as a tumor angiogenesis factor in some human gliomas. Kaposi's sarcoma cells cultured from AIDS patients also express and secrete high levels of VEGF. A strong correlation exists between degree of vascularization of the malignancy and VEGF mRNA expression. Furthermore, monoclonal antibodies that inhibit VEGF-induced angiogenesis exert an inhibitory effect on the growth of human rhabdomyosarcoma, glioblastoma multiforme, or leiomyosarcoma cell lines in nude mice.

In addition, VEGF appears to have a role in wound healing. In wound healing, as in tumor stroma generation, VEGF probably exerts the dual functions of maintaining increased vessel permeability so that plasma proteins, such as fibrinogen extravasate, clot to form provisional matrix, and induce the migration of fibroblasts and new blood vessels; and stimulating endothelial cell division, thereby enhancing angiogenesis directly. VEGF is also expressed in healing wounds by some of the macrophages that populate the developing granulation tissue.

VEGF may have a role in certain types of chronic inflammation. Enhanced vascular permeability is one of the earliest events in the inflammatory response. VEGF has been detected in some non-cancerous human effusions and VEGF mRNA in activated macrophages suggests a role for VEGF in forms of inflammation characterized by macrophage infiltration, such as delayed hypersensitivity and chronic inflammation. Also, VEGF is a chemoattractant for monocytes and VEGF has been shown to enhance the activity of the inflammatory mediator tumor necrosis factor (TNF). VEGF may also play a role in the pathogenesis of the angiogenic disease rheumatoid arthritis as high levels of VEGF are found in the synovial fluid of rheumatoid arthritis patients.

Quiescent and proliferating endothelial cells display high-affinity binding to VEGF, and endothelial cell responses to VEGF appear to be mediated by high affinity cell surface receptors. Expression of either receptor on nonendothelial cells, however, does not confer upon such cells the ability to proliferate in response to VEGF. Two tyrosine kinases have been identified as VEGF receptors. The first, known as fms-like tyrosine kinase or FLT is a receptor tyrosine kinase that is specific for VEGF. In adult and embryonic tissues, expression of FLT mRNA is localized to the endothelium and to populations of cells that give rise to endothelium. The second receptor KDR (human kinase insert domain-containing receptor), and its mouse homologue FLK-1, are closely related to FLT. The KDR/FLK-1 receptor is expressed in endothelium during the fetal growth stage, during earlier embryonic development, and in adult tissues. FLT and KDR are membrane-spanning receptors that each contain seven immunoglobulin-like domains in the extracellular ligand-binding region, a single transmembrane-spanning sequence, and a cytoplasmic tyrosine kinase sequence that is interrupted by a "kinase insert" domain.

Messenger RNA encoding FLT and KDR have been identified in tumor blood vessels, and, as such, these receptors are likely to be relevant for VEGF-endothelial cell interactions in tumors. For example, FLT mRNA is expressed by endothelial cells of blood vessels supplying glioblastomas. Similarly, FLT and KDR mRNAs are upregulated in tumor blood vessels in invasive human colon adenocarcinoma, but not in the blood vessels of adjacent normal tissues.

Because of the role of VEGF in endothelial cell growth and its association with certain disease states, there is interest in it as a therapeutic agent and as a target for therapeutic intervention. For example, disorders characterized by inadequate tissue perfusion, such as obstructive atherosclerosis and diabetes, diabetic retinopathy and other angiogenesis in the posterior eye, and prevention of restenosis following percutaneous transluminal angioplasty are candidates for use of VEGF as a therapeutic agent. Inhibition of VEGF activity may serve as a means to inhibit pathological vessel formation and, thereby, is of considerable interest in a variety of clinical applications, particularly oncology. In this regard, subfragments of VEGF which bind receptor but do not stimulate vessel formation are ideal candidates for carrying DNA molecules encoding cytotoxins to tumor cells.

### c. Heparin-binding epidermal growth factors

Several new mitogens in the epidermal growth factor protein family have recently been identified that display the ability to bind the glycosaminoglycan, heparin. Among these is the mitogen known as heparin-binding EGF-like growth factor (HBEGF), which elutes from heparin-Sepharose columns at about 1.0 - 1.2 M NaCl and which was first identified as a secreted product of cultured human monocytes, macrophages, and the macrophage-like U-937 cell line (Higashiyama et al., *Science 251*:936-939, 1991; Besner et al., *Cell Regul. 1*:811-19, 1990). HBEGF has been shown to interact with the same high affinity receptors as EGF on bovine aortic smooth muscle cells and human A431 epidermoid carcinoma cells (Higashiyama, *Science 251*:936-939, 1991).

HBEGFs exhibit a mitogenic effect on a wide variety of cells including BALB/c 3T3 fibroblast cells and smooth muscle cells, but unlike VEGFs, are not mitogenic for endothelial cells (Higashiyama et al., *Science 251*:936-939, 1991). HBEGF appears to be a more potent mitogen for smooth muscle cells than either EGF or TGF-α, although all three factors bind to EGF receptors. Of particular interest, HBEGF has a stimulatory effect on collateral vascularization and angiogenesis. In some instances, however, HBEGF-induced mitogenic stimulation may be detrimental. For example, cell proliferation and angiogenesis are an integral aspect of tumor growth. Members of the HBEGF family are thought to play a pathophysiological role, for example, in a variety of tumors, such as bladder carcinomas, breast tumors and non-small cell lung tumors.

HBEGF isolated from U-937 cells is heterogeneous in structure and contains at least 86 amino acids and two sites of *O*-linked glycosyl groups (Higashiyama et al., *J*. *Biol. Chem. 267*:6203-6212, 1992). The carboxyl-terminal half of the secreted HBEGF shares approximately 35% sequence identity with human EGF, including six cysteines spaced in the pattern characteristic of members of the EGF protein family. In contrast, the amino-terminal portion of the mature factor is characterized by stretches of hydrophilic residues and has no structural equivalent in EGF. Site-directed mutagenesis of HBEGF and studies with peptide fragments have indicated that the heparin-binding sequences of HBEGF reside primarily in a twenty one-amino acid stretch upstream of and slightly overlapping the EGF-like domain.

The effects of HBEGFs are mediated by EGF receptor tyrosine kinases expressed on cell surfaces of HBEGF-responsive cells (*see, e.g*., U.S. Patent Nos. 5,183,884 and 5,218,090; and Ullrich et al., *Nature 309*:4113-425, 1984), which are incorporated herein by reference). The EGF receptor proteins, which are single chain polypeptides with molecular weights 170 kD, constitute a family of structurally related EGF receptors. Cells known to express the EGF receptors include, for example, smooth muscle cells, fibroblasts, keratinocytes, and numerous human cancer cell lines, such as the: A431 (epidermoid); KB3-1 (epidermoid); COLO 205 (colon); CRL 1739 (gastric); HEP G2 (hepatoma); LNCAP (prostate); MCF-7 (breast); MDA-MB-468 (breast); NCI 417D (lung); MG63 (osteosarcoma); U-251 (glioblastoma); D-54MB (glioma); and SW-13 (adrenal).

For the purposes of this invention, HBEGF need only bind a specific HBEGF receptor and be internalized. Any member of the HBEGF family, whether or not it binds heparin, is useful within the context of this invention as long as it meets the requirements set forth above. Members of the FIBEGF. family are those that have sufficient nucleotide identity to hybridize under normal stringency conditions (typically greater than 75% nucleotide identity). Subfragments or subportions of a fall-length HBEGF may also be desirable. One skilled in the art may find from the teachings provided within that certain biological activities are more or less desirable, depending upon the application. Thus, HBEGF may be customized for the particular application. Means for modifying proteins is provided in detail below. Briefly, additions, substitutions and deletions of amino acids may be produced by any commonly employed recombinant DNA method.

### d. Selection of targeting polypeptides for use herein

Any polypeptide or peptidomimetic that is reactive with an FGF receptor, a VEGF receptor, or an HBEGF receptor may be used in the methods herein. These include members of the families and fragments thereof, as well as constrained analogs of such peptides that bind to one of the receptor and internalize a linked targeted agent. Members of the FGF peptide family, including FGF-1 - FGF-9, are particularly preferred. Modified peptides, including FGF polypeptides that have the nuclear translocating sequence (NTS) removed (*see,* 2b(3) and Table 2, below) and chimeric peptides, which retain the specific binding and internalizing activities are also contemplated for use herein. FGF polypeptides that have been modified by removal of the NTS are particularly suited for use herein. Such polypeptides will not be transported to the nucleus and, as such, administration of conjugates containing the modified FGF should not exhibit mitogenic activity.

Modification of the polypeptide may be effected by any means known to those of skill in this art. The preferred methods herein rely on modification of DNA encoding the polypeptide and expression of the modified DNA.

As an example, DNA encoding the FGF polypeptide may be isolated, synthesized or obtained from commercial sources (the amino acid sequences of FGF-1 - FGF-9 are set forth in SEQ ID NOS. 24-32; DNA sequences may be based on these amino acid sequences or may be those that are known to those of skill in this art (*see, e.g*., Genbank, release 86); *see, also*, U.S. Patent No. 4,956,455, U.S. Patent No. 5,126,323, U.S. Patent No. 5,155,217, U.S. Patent No. 4,868,113, published International Application WO 90/08771 (and the corresponding U.S. patent, upon its issuance), which is based on U.S. Application Serial No. 07/304,281, filed January 31, 1989; EP Application 0 488 196 A2;, and Miyamoto et al., *Mol. Cell. Biol. 13*:4251-4259, 1993). Expression of a recombinant bFGF protein in yeast and *E. coli* is described in Barr et al., *J. Biol. Chem. 263*:16471-16478, 1988, in copending International PCT Application Serial No. PCT/US93/05702 and co-pending United States Application Serial No. 07/901,718. Expression of recombinant FGF proteins may be performed as described herein or using methods known to those of skill in the art; and DNA encoding FGF proteins may be used as the starting materials for the methods herein.

Similarly, DNA encoding VEGF or HBEGF may also be isolated, synthesized or obtained from commercial sources. DNA sequences are available in public databases, such as Genbank and in SEQ ID NOS 78-95. Based on these sequences, oligonucleotide primers may be designed and used to amplify the gene from cDNA or mRNA by polymerase chain reaction technique.

Mutation may be effected by any method known to those of skill in the art, including site-specific or site-directed mutagenesis of DNA encoding the protein and the use of DNA amplification methods using primers to introduce and amplify alterations in the DNA template, such as PCR splicing by overlap extension (SOE). Site-specific mutagenesis is typically effected using a phage vector that has single- and double-stranded forms, such as M13 phage vectors, which are well-known and commercially available. Other suitable vectors that contain a single-stranded phage origin of replication may be used (*see, e.g*., Veira et al., *Meth. Enzymol*. *15*:3, 1987). In general, site-directed mutagenesis is performed by preparing a single-stranded vector that encodes the protein of interest (*i.e*., a member of the FGF family or a cytotoxic molecule, such as a saporin). An oligonucleotide primer that contains the desired mutation within a region of homology to the DNA in the single-stranded vector is annealed to the vector followed by addition of a DNA polymerase, such as *E. coli* DNA polymerase I (Klenow fragment), which uses the double stranded region as a primer to produce a heteroduplex in which one strand encodes the altered sequence and the other the original sequence. The heteroduplex is introduced into appropriate bacterial cells and clones that include the desired mutation are selected. The resulting altered DNA molecules may be expressed recombinantly in appropriate host cells to produce the modified protein.

Suitable conservative substitutions of amino acids are known to those of skill in this art may be made generally without altering the biological activity of the resulting molecule. For example, such substitutions may be made in accordance with those set forth in TABLE 1 as follows:

**TABLE 1**

| Original residue | Conservative substitution |
|---|---|
| Ala (A) | Gly; Ser |
| Arg (R) | Lys |
| Asn (N) | Gln; His |
| Cys (C) | Ser |
| Gln (Q) | Asn |
| Glu (E) | Asp |
| Gly (G) | Ala; Pro |
| His (H) | Asn; Gln |
| Ile (I) | Leu: Val |
| Leu (L) | Ile; Val |
| Lys (K) | Arg: Gln; Glu |
| Met (M) | Leu; Tyr; Ile |
| Phe (F) | Met; Leu; Tyr |
| Ser (S) | Thr |
| Thr (T) | Ser |
| Trp (W) | Tyr |
| Tyr (Y) | Trp; Phe |
| Val (V) | Ile; Leu |

Other similarly conservative substitutions may be made. If necessary such substitutions may be determined empirically merely by testing the resulting modified protein for the ability to bind to and internalize upon binding to FGF receptors. Those that retain this ability are suitable for use in the conjugates and methods herein. In addition, muteins of the FGFs are known to those of skill in the art (*see, e.g*., U.S. Patent No. 5,175,147; International Application No. WO 89/00198, which is based on U.S. Applications Ser. No. 07/070,797; and International Application No. WO 91/15229, which is based on U.S. Applications Ser. No. 07/505,124).

### 2. The targeted agent

The targeted agents include any agent intended for intracellular delivery, such as cytotoxins, therapeutic drugs, drugs for imaging (*see, e.g*., U.S. Patent No. 5,256,399) and nucleic acids. For the ophthalmic applications herein, the targeted agent is a cytotoxic agent or, if DNA, encodes a therapeutic compound that inhibits or prevents cell proliferation.

### a. Cytotoxic agents

Any agent that, upon internalization by a cell, inhibits cell growth or proliferation or results in cell death, is suitable for use herein. Cytotoxic agents include ribosome inactivating proteins, small metabolic inhibitors, antisense nucleic acids, toxic drugs, such as anticancer agents, and small molecules, such as porphyrins.

### (1) Ribosome inactivating proteins

Ribosome-inactivating-proteins (RIPs), which include ricin, abrin and saporin, are plant proteins that catalytically inactivate eukaryotic ribosomes. Some RIPs, such as the toxins abrin and ricin, contain two constituent chains: a cell-binding chain that mediates binding to cell surface receptors and internalizing the molecule; and a chain responsible for toxicity. Single chain RIPs (type I RIPS), such as the saporins, do not have a cell-binding chain. As a result, unless internalized, they are substantially less toxic to whole cells than the RIPs that have two chains.

RIPS inactivate ribosomes by interfering with the protein elongation step of protein synthesis. For example, the RIP saporin (hereinafter also referred to as SAP) has been shown to inactivate 60S ribosomes by cleavage of the n-glycosidic bond of the adenine at position 4324 in the rat 28S ribosomal RNA (rRNA). The particular region in which A₄₃₂₄ is located in the rRNA is highly conserved among prokaryotes and eukaryotes. A₄₃₂₄ in 28S rRNA corresponds to A₂₆₆₀ in *Escherichia coli* (*E. coli*) 23S rRNA. Several RIPs also appear to interfere with protein synthesis in prokaryotes, such as *E. coli*.

Saporin and other ribosome inactivating proteins (RIPs) are the preferred cytotoxic agent for use herein. Any cytotoxic agent that, when internalized inhibits or destroys cell growth, cell proliferation or other essential cell functions may be used herein. Such cytotoxic agents are considered to be functionally equivalent to the RIPs described herein, and include, but are not limited to, saporin, the ricins, abrin and other RIPs, *Pseudomonas exotoxin*, inhibitors of DNA, RNA or protein synthesis or other metabolic inhibitors that are known to those of skill in this art. Saporin is preferred, but other suitable RIPs include, but are not limited to, ricin, ricin A chain, maize RIP, gelonin, diphtheria toxin, diphtheria toxin A chain, trichosanthin, tritin, pokeweed antiviral protein (PAP), mirabilis antiviral protein (MAP), Dianthins 32 and 30, abrin, monordin, bryodin, shiga and others known to those of skill in this art (*see, e.g*., Barbieri et al., *Cancer Surveys 1*:489-520, 1982, and European published patent application No. 0466 222, incorporated herein by reference, which provide lists of numerous RIPs and their sources; *see, also*, U.S. Patent No. 5,245,608 to Walsh et al., which provides a RIP from maize).

Several structurally related RIPs have been isolated from seeds and leaves of the plant *Saponaria officinalis* (soapwort). Among these, SAP-6 is the most active and abundant, representing 7% of total seed proteins. Saporin is very stable, has a high isoelectric point, does not contain carbohydrates, and is resistant to denaturing agents, such as sodium dodecyl sulfate (SDS), and a variety of proteases. The amino acid sequences of several saporin-6 isoforms from seeds are known and there appear to be families of saporin RIPs differing in few amino acid residues. Because saporin is a type I RIP, it does not possess a cell-binding chain. Consequently, its toxicity to whole cells is much lower than other toxins, such as ricin and abrin. When internalized by eukaryotic cells, however, its cytotoxicity is 100- to 1000-fold more potent than ricin A chain.

If necessary, the selected cytotoxic agent is derivatized to produce a group reactive with a cysteine on the selected FGF. If derivatization results in a mixture of reactive species, a mono-derivatized form of the cytotoxic agent is isolated and is then conjugated to the mutated FGF.

### (a) Isolation of saporin and DNA encoding saporin

The saporin polypeptides include any of the isoforms of saporin that may be isolated from *Saponaria officinalis* or related species or modified form that retain cytotoxic activity. In particular, such modified saporin may be produced by modifying the DNA encoding the protein (*see, e.g.,* International PCT Application Serial No. PCT/US93/05702, filed on June 14, 1993, which is a continuation-in-part of United States Application Serial No. 07/901,718; *see, also,* copending U.S. Patent Application No. 07/885,242 filed May 20, 1992, and Italian Patent No. 1231914), by altering one or more amino acids or deleting or inserting one or more amino acids, such as a cysteine that may render it easier to conjugate to FGF or other cell surface binding protein. Any such protein, or portion thereof, that, when conjugated to FGF as described herein, that exhibits cytotoxicity in standard *in vitro* or *in vivo* assays within at least about an order of magnitude of the saporin conjugates described herein is contemplated for use herein.

Thus, the SAP used herein includes any protein that is isolated from natural sources or that is produced by recombinant expression (*see, e.g*., copending International PCT Application Serial No. PCT/US93/05702, filed on June 14, 1993, which is a continuation-in-part of United States Application Serial No. 07/901,718, filed June 16, 1992; *see, also,* Example 1, below).

DNA encoding SAP or any cytotoxic agent may be used in the recombinant methods provided herein. In instances in which the cytotoxic agent does not contain a cysteine residue, such as instances in which DNA encoding SAP is selected, the DNA may be modified to include cysteine codon. The codon may be inserted into any locus that does not reduce or reduces by less than about one order of magnitude the cytotoxicity of the resulting protein may be selected. Such locus may be determined empirically by modifying the protein and testing it for cytotoxicity in an assay, such as a cell-free protein synthesis assay. The preferred loci in SAP for insertion of the cysteine residue is at or near the N-terminus (within about 10 residues of the N-terminus).

### (b) Host cells for expression of cytotoxic agents and conjugates that contain cytotoxic agents

Host organisms include those organisms in which recombinant production of heterologous proteins have been carried out and in which the cytotoxic agent, such as saporin is not toxic or of sufficiently low toxicity to permit expression before cell death. Presently preferred host organisms are strains of bacteria. Most preferred host organisms are strains of *E. coli*, particularly. BL21(DE3) cells (NOVAGEN, Madison, WI).

### (c) Methods for recombinant production of cytotoxic agents

The DNA encoding the cytotoxic agent, such as saporin protein, is introduced into a plasmid in operative linkage to an appropriate promoter for expression of polypeptides in a selected host organism. The presently preferred saporin proteins are saporin proteins that have been modified by addition of a Cys residue or replacement of a non-essential residue at or near the amino- or carboxyl terminus of the saporin with Cys. Saporin, such as that of SEQ ID NO. 7 has been modified by insertion of Met-Cys residue at the N-terminus and has also been modified by replacement of the Asn or Ile residue at positions 4 and 10, respectively (*see* Example 4). The DNA fragment encoding the saporin may also include a protein secretion signal that functions in the selected host to direct the mature polypeptide into the periplasm or culture medium. The resulting saporin protein can be purified by methods routinely used in the art, including, methods described hereinafter in the Examples.

Methods of transforming suitable host cells, preferably bacterial cells, and more preferably *E. coli* cells, as well as methods applicable for culturing said cells containing a gene encoding a heterologous protein, are generally known in the art. See, for example, Sambrook et al., *Molecular Cloning: A Laboratory Manual*, Cold Spring Harbor Laboratory Press, Cold Spring Harbor. NY, 1989.

The DNA construct encoding the saporin protein is introduced into the host cell by any suitable means, including, but not limited to transformation employing plasmids, viral, or bacterial phage vectors, transfection, electroporation, lipofection, and the like. The heterologous DNA can optionally include sequences, such as origins of replication that allow for the extrachromosomal maintenance of the saporin-containing plasmid, or can be designed to integrate into the genome of the host (as an alternative means to ensure stable maintenance in the host).

Positive transformants can be characterized by Southern blot analysis (Sambrook et al., *Molecular Cloning: A Laboratory Manual*, Cold Spring Harbor Laboratory Press, Cold Spring Harbor, NY, 1989) for the site of DNA integration; Northern blots for inducible-promoter-responsive saporin gene expression; and product analysis for the presence of saporin-containing proteins in either the cytoplasm, periplasm, or the growth media.

Once the saporin-encoding DNA fragment has been introduced into the host cell, the desired saporin-containing protein is produced by subjecting the host cell to conditions under which the promoter is induced, whereby the operatively linked DNA is transcribed. In a preferred embodiment, such conditions are those that induce expression from the *E. coli* lac operon. The plasmid containing the DNA encoding the saporin-containing protein also includes the lac operator (O) region within the promoter and may also include the lac I gene encoding the lac repressor protein (*see, e.g.,* Muller-Hill et al., *Proc. Natl. Acad. Sci. USA 59*:1259-12649, 1968). The lac repressor represses the expression from the lac promoter until induced by the addition of IPTG in an amount sufficient to induce transcription of the DNA encoding the saporin-containing protein.

The expression of saporin in *E. coli* is, thus accomplished in a two-stage process. In the first stage, a culture of transformed *E. coli* cells is grown under conditions in which the expression of the saporin-containing protein within the transforming plasmid, preferably a encoding a saporin, such as described in Example 4, is repressed by virtue of the lac repressor. In this stage cell density increases. When an optimum density is reached, the second stage commences by addition of IPTG, which prevents binding of repressor to the operator thereby inducing the lac promoter and transcription of the saporin-encoding DNA.

In a preferred embodiment, the promoter is the T7 RNA polymerase promoter, which may be linked to the lac operator and the *E. coli* host strain includes DNA encoding T7 RNA polymerase operably linked to the lac operator and a promoter, preferably the lacUV5 promoter. The presently preferred plasmid is pET 11a (NOVAGEN, Madison, WI), which contains the T71ac promoter, T7 terminator, the inducible *E. coli* lac operator, and the lac repressor gene. The plasmid pET 15b (NOVAGEN, Madison, WI), which contains a His-Tag™ leader sequence (Seq. ID No. 36) for use in purification with a His column and a thrombin cleavage site that permits cleavage following purification over the column, the T7-lac promoter region and the T7 terminator, has been used herein for expression of saporin. Addition of IPTG induces expression of the T7 RNA polymerase and the T7 promoter, which is recognized by the T7 RNA polymerase.

Transformed strains, which are of the desired phenotype and genotype, are grown in fermentors by suitable methods well known in the art. In the first, or growth stage, expression hosts are cultured in defined minimal medium lacking the inducing condition, preferably IPTG. When grown in such conditions, heterologous gene expression is completely repressed, which allows the generation of cell mass in the absence of heterologous protein expression. Subsequent to the period of growth under repression of heterologous gene expression, the inducer, preferably IPTG, is added to the fermentation broth, thereby inducing expression of any DNA operatively linked to an IPTG-responsive promoter (a promoter region that contains lac operator). This last stage is the induction stage.

The resulting saporin-containing protein can be suitably isolated from the other fermentation products by methods routinely used in the art, *e.g*., using a suitable affinity column as described in Example 1.E-F and 2.D; precipitation with ammonium sulfate; gel filtration; chromatography, preparative flat-bed iso-electric focusing; gel electrophoresis, high performance liquid chromatography (HPLC); and the like. A method for isolating saporin is provided in EXAMPLE 1 (*see, also*, Lappi et al., *Biochem. Biophys. Res*. *Commun. 129*:934-942, 1985). The expressed saporin protein is isolated from either the cytoplasm, periplasm, or the cell culture medium (see, discussion below B.1.b below and *see, e.g*., EXAMPLE 4 for preferred methods and saporin proteins).

### (2) Porphyrins

Porphyrins are well-known light activatable toxins that can be readily cross-linked to proteins (*see, e.g*., U.S. Patent No. 5,257,970; U.S. Patent No. 5,252,720; U.S. Patent No. 5,238,940; U.S. Patent No. 5,192,788; U.S. Patent No. 5,171,749; U.S. Patent No. 5,149,708; U.S. Patent No. 5,202,317; U.S. Patent No. 5,217,966; U.S. Patent No. 5,053,423; U.S. Patent No. 5,109,016; U.S. Patent No. 5,087,636; U.S. Patent No. 5,028,594; U.S. Patent No. 5,093,349; U.S. Patent No. 4,958,715; U.S. Patent No. 4,920,143 and International Application WO 93/02192).

### b. Nucleic acids for targeted delivery

The conjugates provided herein are also designed to deliver nucleic acids to targeted cells. The nucleic acids include those intended to deliver a cytotoxic signal to a cell or to modify expression of genes in a cell and thereby effect genetic therapy. Examples of nucleic acids include antisense RNA, DNA, ribozymes and other oligonucleotides that bind proteins. The nucleic acids can also include RNA trafficking signals, such as viral packaging sequences (*see, e.g*., Sullenger et al., *Science 262*:1566-1569, 1994). The nucleic acids also include DNA molecules that encode intact genes or that encode proteins useful for gene therapy or for cell cytotoxicity. Especially of interest are DNA molecules that encode an enzyme that results in cell death or renders a cell susceptible to cell death upon the addition of another product. For example, saporin is an enzyme that cleaves rRNA and inhibits protein synthesis. Other enzymes that inhibit protein synthesis are especially well suited for the present invention. Other enzymes may be used where the enzyme activates a compound with little or no cytotoxicity into a toxic product.

DNA (or RNA) that may be delivered to a cell to effect genetic therapy also includes DNA that encodes tumor-specific cytotoxic molecules, such as tumor necrosis factor, viral antigens and other proteins to render a cell susceptible to anti-cancer agents, and DNA encoding genes, such as the such as the defective gene (CFTR) associated with cystic fibrosis (*see, e.g*., International Application WO 93/03709, which is based on U.S. Application Serial No. 07/745,900; and Riordan et al., *Science 245*:1066-1073, 1989), to replace defective genes.

Nucleic acids and oligonucleotides for use as described herein can be synthesized by any method known to those of skill in this art (*see, e.g*., Wo 93/01286, which is based on U.S. Application Serial No. 07/723,454; U.S.. Patent No. 5,218,088; U.S. Patent No. 5,175,269; U.S. Patent No. 5,109,124). Identification of oligonucleotides and ribozymes for use as antisense agents is well within the skill in this art. Selection of DNA encoding genes for targeted delivery for genetic therapy is also well within the level of skill of those in this art. For example, the desirable properties, lengths and other characteristics of such oligonucleotides are well known. Antisense oligonuclotides are designed to resist degradation by endogenous nucleolytic enzymes and include, but are not limited to: phosphorothioate, methylphosphonate, sulfone, sulfate, ketyl, phosphorodithioate, phosphoramidate, phosphate esters, and other such linkages (*see, e.g*., Agrwal et al., *Tetrehedron Lett. 28*:3539-3542, 1987; Miller et al., *J. Am. Chem. Soc. 93*:6657-6665, 1971; Stec et al., *Tetrehedron Lett. 26*:2191-2194, 1985; Moody et al., *Nucl. Acids Res. 12*:4769-4782, 1989; Uznanski et al., *Nucl. Acids Res*., 1989; Letsinger et al., *Tetrahedron 40*:137-143, 1984; Eckstein, *Annu. Rev. Biochem. 54*:367-402, 1985; Eckstein, *Trends Biol. Sci. 14*:97-100, 1989; Stein, In: *Oligodeoxynucleotides. Antisense Inhibitors of Gene Expression*, Cohen (Ed.), Macmillan Press, London, pp. 97-117, 1989; Jager et al., *Biochemistry 27*:7237-7246, 1988).

### (1) Antisense oligonucleotides; triplex molecules; dumbbell oligonucleotides; DNA; extracellular protein binding oligonucleotides; and small nucleotide molecules

Antisense nucleotides are oligonucleotides that specifically bind to mRNA that has complementary sequences, thereby preventing translation of the mRNA (*see, e.g*., U.S. Patent No. 5,168,053 to Altman et al., U.S. Patent No. 5,190.931 to Inouye, U.S. Patent No. 5,135,917 to Burch; U.S. Patent No. 5,087,617 to Smith and Clusel et al., *Nucl. Acids Res. 21*:3405-3411, 1993, which describes dumbbell antisense oligonucleotides). Triplex molecules refer to single DNA strands that target duplex DNA and thereby prevent transcription (*see, e.g*., U.S. Patent No. 5,176,996 to Hogan et al., which describes methods for making synthetic oligonucleotides that bind to target sites on duplex DNA).

Particularly useful antisense nucleotides and triplex molecules are molecules that are complementary or bind to the sense strand of DNA or mRNA that encodes an oncogene, such as bFGF, int-2, hst-1/K-FGF, FGF-5, hst-2/FGF-6, FGF-8 or protein that promotes unwanted cell proliferation or differentiation. Linkage of such antisense or triplex molecules to the corresponding FGF actor should specifically target tumor cells that express the oncogene. Since many tumors express FGF receptors, other oncogenes, such as p53, c-myc and erb-2, may also be targeted using an FGF, particularly bFGF linked to an antisense oligonucleotide or triplex molecule.

Other useful antisense oligonucleotides include those that are specific for IL-8 (*see, e.g*., U.S. Patent No. 5,241,049; and International applications WO 89/004836; WO 90/06321; WO 89/10962; WO 90/00563; and WO 91/08483, and the corresponding U.S. applications for descriptions of DNA encoding IL-8 and amino acid sequences of IL-S), which can be linked to bFGF for the treatment of psoriasis, antisense oligonucleotides that are specific for nonmuscle myosin heavy chain and/or c-myb (*see, e.g*., Simons et al., *Circ. Res. 70*:835-843, 1992; WO 93/01286, which is based on U.S. application Serial No. 07/723,454: LeClerc et al., *J. Am. Coll. Cardiol. 17 (2 Suppl*. *A)*:105A, 1991: Ebbecke et al., *Basic Res. Cardiol. 87*:585-591, 1992), which can be targeted by an FGF to inhibit smooth muscle cell proliferation, such as that following angioplasty and thereby prevent restenosis or inhibit viral gene expression in transformed or infected cells.

### (2) Ribozymes

Ribozymes are RNA constructs that specifically cleave messenger RNA. There are at least five classes of ribozymes that are known that are involved in the cleavage and/or ligation of RNA chains. Ribozymes can be targeted to any RNA transcript and can catalytically cleave such transcript (*see, e.g*., U.S. Patent No. 5,272,262; U.S. Patent No. 5,144,019; and U.S. Patent Nos. 5,168,053, 5,180,818, 5,116,742 and 5,093,246 to Cech et al., which described ribozymes and methods for production thereof). Any such ribosome may be linked to the growth factor for delivery to FGF-receptor bearing cells.

The ribozymes may be delivered to the targeted cells, such tumor cells that express an FGF receptor, as DNA encoding the ribozyme linked to a eukaryotic promoter, such as a eukaryotic viral promoter, generally a later promoter, such that upon introduction into the nucleus, the ribozyme will be directly transcribed. In such instances, the construct will also include a nuclear translocation sequence (NTS; see Table 2, below), generally as part of the growth factor or as part of a linker between the growth factor and linked DNA.

### (3) Delivery of nucleic acid molecules

In order to deliver the nucleic acid to the nucleus, the conjugate should include an NTS. If the conjugate is designed such that the heparin-binding growth factor and linked DNA is cleaved or dissociated in the cytoplasm, then the NTS should be included in a portion of the linker that remains bound to the DNA, so that, upon internalization, the conjugate will be trafficked to the nucleus. The nuclear translocation sequence (NTS) may be a heterologous sequence or a may be derived from the selected growth factor. All presently identified members of the FGF family of peptides contain an NTS (*see, e.g*., International Application WO 91/15229 and Table 2). If a portion of an FGF that binds to an FGF receptor and internalizes a linked ligand is used to deliver DNA to the nucleus, the conjugate must include a NTS located such that the linked DNA is translocated to the nucleus. A typical consensus NTS sequence contains an amino-terminal proline or glycine followed by at least three basic residues in a array of seven to nine amino acids (*see*, *e.g.* Dang et al., *J. Biol. Chem. 264*:18019-18023, 1989; Dang et al. *Mol. Cell. Biol. 8*:4049-4058, 1988, and Table 2, which sets forth examples of NTSs and regions of proteins that share homology with known NTSs).

**TABLE 2**

| Source | Sequence* | SEQ ID NO. |
|---|---|---|
| SV40 large T | Pro¹²⁶LysLysArgLysValGlu | 58 |
| Polyoma large T | Pro²⁷⁹ ProLysLysAlaArgGluVal | 59 |
| Human c-Myc | Pro¹²⁰AlaAlaLysArgValLysLeuAsp | 60 |
| Adenovirus E1A | Lys²⁸¹ArgProArgPro | 61 |
| Yeast mat α₂ | Lys³IleProIleLys | 62 |
| c-Erb-A | A. Gly²² LysArgLysArgLysSer | 63 |
| | B. Ser¹²⁷LysArgValAlaLysArgLysleu | 64 |
| | C. Ser¹⁸¹HisTrpLysGlnLysArgLysPhe | 65 |
| c-Myb | Pro⁵²¹LeuLeuLysLysIleLysGln | 66 |
| p53 | Pro³¹⁶GlnProLysLysLysPro | 67 |
| Nucleolin | Pro²⁷⁷GlyLysArgLysLysGluMetThrLysGlnLysGluValPro | 68 |
| HIV Tat | Gly⁴⁸ArgLysLysArgArgGlnArgArgArgAlaPro | 69 |
| FGF-1 | AsnTyrLysLysProLysLeu | 70 |
| FGF-2 | HisPheLysAspProLysArg | 71 |
| FGF-3 | AlaProArgArgArgLysLeu | 72 |
| FGF-4 | IleLysArgLeuArgArg | 73 |
| FGF-5 | GlyArgArg | - |
| FGF-6 | IleLysArgGlnArgArg | 74 |
| FGF-7 | IleArgValArgArg | 75 |

| | | |
|---|---|---|
| *Superscript indicates position in protein | | |

### 3. Plasmids and host cells for expression of heparin-binding growth factors

Host organisms include those organisms in which recombinant production of heterologous proteins have been carried out, such as, but not limited to, bacteria (for example, *E. coli*), yeast (for example, *Saccharomyces cerevisiae* and *Pichia pastoris*), mammalian cells, insect cells. Presently preferred host organisms are strains of bacteria.

The DNA construct is introduced into a plasmid for expression in a desired host. In preferred embodiments, the host is a bacterial host. The sequences of nucleotides in the plasmids that are regulatory regions, such as promoters and operators, are operationally associated with one another for transcription. The sequence of nucleotides encoding the growth factor or growth factor-chimera may also include DNA encoding a secretion signal, whereby the resulting peptide is a precursor protein. The resulting processed protein may be recovered from the periplasmic space or the fermentation medium.

In preferred embodiments the DNA plasmids also include a transcription terminator sequence. The promoter regions and transcription terminators are each independently selected from the same or different genes.

The plasmids used herein preferably include a promoter in operable association with the DNA encoding the protein or polypeptide of interest and are designed for expression of proteins in a bacterial host. It has been found that tightly regulatable promoters are preferred for expression of saporin. Suitable promoters for expression of proteins and polypeptides herein are widely available and are well known in the art. Inducible promoters or constitutive promoters that are linked to regulatory regions are preferred. Such promoters include, but are not limited to, the T7 phage promoter and other T7-like phage promoters, such as the T3, T5 and SP6 promoters, the trp, lpp, and lac promoters, such as the lacUV5, from *E. coli;* the P10 or polyhedron gene promoter of baculovirus/insect cell expression systems (*see, e.g*., U.S. Patent Nos. 5,243,041, 5,242,687, 5,266,317, 4,745,051, and 5,169,784) and inducible promoters from other eukaryotic expression systems. For expression of the proteins such promoters are inserted in a plasmid in operative linkage with a control region such as the lac operon.

Preferred promoter regions are those that are inducible and functional in *E. coli.* Examples of suitable inducible promoters and promoter regions include, but are not limited to: the *E. coli* lac operator responsive to isopropyl β-D-thiogalactopyranoside (IPTG; *see*, et al. Nakamura et al., *Cell 18*:1109-1117, 1979); the metallothionein promoter metal-regulatory-elements responsive to heavy-metal (e.g., zinc) induction (*see, e.g*., U.S. Patent No. 4,870,009 to Evans et al.); the phage T71ac promoter responsive to IPTG (*see, e.g*., U.S. Patent No. 4,952,496; and Studier et al., *Meth. Enzymol. 185*:60-89, 1990) and the TAC promoter.

The plasmids also preferably include a selectable marker gene or genes that are functional in the host. A selectable marker gene includes any gene that confers a phenotype on bacteria that allows transformed bacterial cells to be identified and selectively grown from among a vast majority of untransformed cells. Suitable selectable marker genes for bacterial hosts, for example, include the ampicillin resistance gene (Amp^{r}), tetracycline resistance gene (Tc^{r}) and the kanamycin resistance gene (Kan^{r}). The kanamycin resistance gene is presently preferred.

The preferred plasmids also include DNA encoding a signal for secretion of the operably linked protein. Secretion signals suitable for use are widely available and are well known in the art. Prokaryotic and eukaryotic secretion signals functional in *E. coli* may be employed. The presently preferred secretion signals include, but are not limited to, those encoded by the following *E. coli* genes: ompA, ompT, ompF, ompC, beta-lactamase, and alkaline phosphatase, and the like (von Heijne, *J. Mol. Biol. 184*:99-105, 1985). In addition, the bacterial pelB gene secretion signal (Lei et al., *J. Bacteriol. 169*:4379, 1987), the phoA secretion signal, and the cek2 functional in insect cell may be employed. The most preferred secretion signal is the *E. coli* ompA secretion signal. Other prokaryotic and eukaryotic secretion signals known to those of skill in the art may also be employed (*see, e.g*., von Heijne, *J. Mol. Biol. 184*:99-105, 1985). Using the methods described herein, one of skill in the art can substitute secretion signals that are functional in either yeast, insect or mammalian cells to secrete proteins from those cells.

Particularly preferred plasmids for transformation of *E. coli* cells include the pET expression vectors (*see,* U.S patent 4,952,496; available from NOVAGEN, Madison, WI; *see, also*, literature published by Novagen describing the system). Such plasmids include pET 11a, which contains the T71ac promoter, T7 terminator, the inducible *E. coli* lac operator, and the lac repressor gene; pET 12a-c, which contains the T7 promoter, T7 terminator, and the *E*. *coli* ompT secretion signal; and pET 15b (NOVAGEN, Madison, WI), which contains a His-Tag™ leader sequence (Seq. ID No. 36) for use in purification with a His column and a thrombin cleavage site that permits cleavage following purification over the column; the T7-lac promoter region and the T7 terminator.

Other preferred plasmids include the pKK plasmids, particularly pKK 223-3, which contains the TAC promoter, (available from Pharmacia; *see, also*, Brosius et al., *Proc.. Natl. Acad. Sci. 81*:6929, 1984; Ausubel et al., *Current Protocols in Molecular* *Biology*; U.S. Patent Nos. 5.122,463, 5,173,403, 5,187,153, 5,204,254, 5,212,058, 5,212,286, 5,215,907, 5,220,013, 5,223,483, and 5,229,279), which contain the TAC promoter. Plasmid pKK has been modified by replacement of the ampicillin resistance marker gene, by digestion with *Eco*RI, with a kanamycin resistance cassette with EcoRI sticky ends (purchased from Pharmacia; obtained from pUC4K, *see, e.g.,* Vieira et al. (*Gene 19*:259-268, 1982; and U.S. Patent No. 4,719,179). Baculovirus vectors, such as a pBlueBac (also called pJVETL and derivatives thereof) vector, particularly pBlueBac III, (*see, e.g.,* U.S. Patent Nos. 5,278,050, 5,244,805, 5,243,041, 5,242,687, 5,266,317, 4,745,051, and 5,169,784; available from INVITROGEN, San Diego) may also be used for expression of the polypeptides in insect cells. The pBlueBacIII vector is a dual promoter vector and provides for the selection of recombinants by blue/white screening as this plasmid contains the β-galactosidase gene (lacZ) under the control of the insect recognizable ETL promoter and is inducible with IPTG. A DNA construct iso a baculovirus vector pBluebac III (INVITROGEN, San Diego, CA) and then co-transfected with wild type virus into insect cells *Spodoptera frugiperda* (sf9 cells; *see, e.g.,* Luckow et al., *Bio/technology 6*:47-55, 1988, and U.S. Patent No. 4,745,051).

Other plasmids include the pIN-IIIompA plasmids (*see,* U.S. Patent No. 4,575,013 to Inouye; *see, also*. Duffaud et al., *Meth. Enz. 153*:492-507, 1987), such as pIN-IIIompA2. The pIN-IIIompA plasmids include an insertion site for heterologous DNA linked in transcriptional reading frame with four functional fragments derived from the lipoprotein gene of *E. coli*. The plasmids also include a DNA fragment coding for the signal peptide of the ompA protein of *E. coli*, positioned such that the desired polypeptide is expressed with the ompA signal peptide at its amino terminus, thereby allowing efficient secretion across the cytoplasmic membrane. The plasmids further include DNA encoding a specific segment of the *E. coli* lac promoter-operator, which is positioned in the proper orientation for transcriptional expression of the desired polypeptide, as well as a separate functional *E. coli* lacI gene encoding the associated repressor molecule that, in the absence of lac operon inducer, interacts with the lac promoter-operator to prevent transcription therefrom. Expression of the desired polypeptide is under the control of the lipoprotein (lpp) promoter and the lac promoter-operator, although transcription from either promoter is normally blocked by the repressor molecule. The repressor is selectively inactivated by means of an inducer molecule thereby inducing transcriptional expression of the desired polypeptide from both promoters.

In a preferred embodiment, the DNA fragment is replicated in bacterial cells, preferably in *E. coli*. The preferred DNA fragment also includes a bacterial origin of replication, to ensure the maintenance of the DNA fragment from generation to generation of the bacteria. In this way, large quantities of the DNA fragment can be produced by replication in bacteria. Preferred bacterial origins of replication include, but are not limited to, the f1-ori and col E1 origins of replication. Preferred hosts contain chromosomal copies of DNA encoding T7 RNA polymerase operably linked to an inducible promoter, such as the lacUV promoter (*see,* U.S. Patent No. 4,952,496). Such hosts include, but are not limited to, lysogens *E. coli* strains HMS174(DE3)pLysS, BL21(DE3)pLysS, HMS174(DE3) and BL21(DE3). Strain BL21(DE3) is preferred. The pLys strains provide low levels of T7 lysozyme, a natural inhibitor of T7 RNA polymerase.

The DNA fragments provided may also contain a gene coding for a repressor-protein. The repressor-protein is capable of repressing the transcription of a promoter that contains sequences of nucleotides to which the repressor-protein binds. The promoter can be derepressed by altering the physiological conditions of the cell. The alteration can be accomplished by the addition to the growth medium of a molecule that inhibits, for example, the ability to interact with the operator or with regulatory proteins or other regions of the DNA or by altering the temperature of the growth media. Preferred repressor-proteins include, but are not limited to the *E. coli*. lacI repressor responsive to IPTG induction, the temperature sensitive cI857 repressor, and the like. The *E. coli* lacI repressor is preferred.

DNA encoding full-length bFGF or the bFGF muteins has been linked to DNA encoding the mature saporin protein and introduced into the pET vectors, including pET-11a and pET-12a expression vectors (NOVAGEN, Madison, WI), for intracellular and periplasmic expression, respectively, of FGF-SAP fusion proteins. The resulting fusion proteins exhibit cytotoxic activity and appear to be at least as potent as the chemically conjugated FGF-SAP preparations.

The resulting bFGF-fusion proteins are highly cytotoxic when internalized by targeted cells.

### B. Linkers

In order to increase the serum stability, solubility and/or intracellular concentration of the targeted agent, one or more linkers (are) inserted between the FGF protein and the targeted moiety or the heparin-binding growth factor and the DNA binding domain. These linkers include peptide linkers, such as intracellular protease substrates, and chemical linkers, such as acid labile linkers, ribozyme substrate linkers and others. Peptides linkers may be inserted using heterobifunctional reagents, described below, or, preferably, are linked to FGF and other heparin-binding growth factors by linking DNA encoding the substrate to the DNA encoding the protein and expressing the resulting chimera. In instances in which the targeted agent is a protein, such as a RIP or nucleic acid binding domain, the DNA encoding the linker can be inserted between the DNA encoding the heparin-binding growth factor protein and the DNA encoding the targeted protein agent.

Chemical linkers may be inserted by covalently coupling the linker to the FGF or other growth factor protein and the targeted agent. The heterobifunctional agents, described below, may be used to effect such covalent coupling.

### 1. Protease substrates

Peptides encoding protease-specific substrates are introduced between the heparin-binding growth factor protein and the targeted moiety. The peptides may be inserted using heterobiofunctional reagents, described below, or, preferably, are linked to heparin-binding growth factor by linking DNA encoding the substrate to the DNA encoding the FGF protein and expressing the resulting chimera. In instances in which the targeted agent is a protein, such as a RIP, the DNA encoding the linker can be inserted between the DNA encoding the heparin-binding growth factor protein and the DNA encoding the targeted protein agent. For example, DNA encoding substrates specific for intracellular proteases has been inserted between the DNA encoding the FGF protein and a targeted agent, such as saporin.

Any protease specific substrate (*see*, *e.g.,* O'Hare et al., *FEBS 273*:200-204, 1990; Forsberg et al., *J. Protein Chem. 10*:517-526, 1991; Westby et al., *Bioconjuugate Chem. 3*:375-381, 1992) may be introduced as a linker between the FGF protein and linked targeting agent as long as the substrate is cleaved in an intracellular compartment. Preferred substrates include those that are specific for proteases that are expressed at higher levels in tumor cells or that are preferentially expressed in the endosome. The following substrates are among those contemplated for use in accord with the methods herein: cathepsin B substrate, cathepsin D substrate, trypsin substrate, thrombin substrate, and recombinant subtilisin substrate (PheAlaHisTyr, SEQ ID NO. 56).

### 2. Flexible linkers and linkers that increase the solubility of the conjugates

Flexible linkers and linkers that increase solubility of the conjugates are contemplated for use, either alone or with other linkers, such as the protease specific substrate linkers. Such linkers include, but are not limited to, (Gly₄Ser)ₙ, (Ser₄Gly)ₙ and (AlaAlaProAla)ₙ in which n is 1 to 6, preferably 1-4, such as:
**a.** Gly₄Ser SEQ ID NO:40 CCATGGGCGG CGGCGGCTCT GCCATGG
**b.** (Gly₄Ser)₂ SEQ ID NO:41 CCATGGGCGG CGGCGGCTCT GGCGGCGGCG GCTCTGCCAT GG
**c.** (Ser₄Gly)₄ SEQ ID NO:42 
CCATGGCCTC GTCGTCGTCG GGCTCGTCGT CGTCGGGCTC GTCGTCGTCG
GGCTCGTCGT CGTCGGGCGC CATGG
**d.** (Ser₄Gly)₂ SEQ ID NO:43 CCATGGCCTC GTCGTCGTCG GGCTCGTCGT CGTCGGGCGC CATGG
**e.** (AlaAlaProAla)ₙ, where n is 1 to 4, preferably 2 (*see*, SEQ ID NO:55)

### 3. Heterobifunctional cross-linking reagents

Numerous heterobifunctional cross-linking reagents that are used to form covalent bonds between amino groups and thiol groups and to introduce thiol groups into proteins, are known to those of skill in this art (*see*, *e.g*., the PIERCE CATALOG, ImmunoTechnology Catalog & Handbook, 1992-1993, which describes the preparation of and use of such reagents and provides a commercial source for such reagents; *see, also, e.g.,* Cumber et al., *Bioconjugate Chem. 3*:397-401, 1992; Thorpe et al., *Cancer Res. 47*:5924-5931, 1987; Gordon et al., *Proc. Natl. Acad Sci. 84*:308-312, 1987; Walden et al., *J*. *Mol. Cell Immunol*. *2*:191-197, 1986; Carlsson et al., *Biochem. J. 173*:723-737, 1978; Mahan et al., *Anal. Biochem. 162*:163-170, 1987; Wawryznaczak et al., *Br. J. Cancer 66*:361-366, 1992; Fattom et al., *Infection & Immun. 60*:584-589, 1992). These reagents may be used to form covalent bonds between the FGF proteins or FGF proteins with protease substrate peptide linkers and targeted protein agent. These reagents include, but are not limited to: N-succinimidyl-3-(2-pyridyldithio)propionate (SPDP; disulfide linker); sulfosuccinimidyl 6-[3-(2-pyridyldithio)propionamido]hexanoate (sulfo-LC-SPDP); succinimidyloxycarbonyl-α-methyl benzyl thiosulfate (SMBT, hindered disulfate linker); succinimidyl 6-[3-(2-pyridyldithio) propionamido]hexanoate (LC-SPDP); sulfosuccinimidyl 4-(N-maleimidomethyl)cyclohexane-1-carboxylate (sulfo-SMCC); succinimidyl 3-(2-pyridyldithio)butyrate (SPDB; hindered disulfide bond linkder); sulfosuccinimidyl 2-(7-azido-4-methylcoumarin-3-acetamide) ethyl-1,3'-dithiopropionate (SAED); sulfosuccinimidyl 7-azido-4-methylcoumarin-3-acetate (SAMCA); sulfosuccinimidyl 6-[alpha-methyl-alpha-(2-pyridyldithio)toluamido]hexanoate (sulfo-LC-SMPT); 1,4-di-[3'-(2'-pyridyldithio)propionamido]butane (DPDPB); 4-succinimidyloxycarbonyl-α-methyl-α-(2-pyridylthio)toluene (SMPT, hindered disulfate linker);sulfosuccinimidyl6[ α-methyl-α-(2-pyridyldithio)toluamido]hexanoate (sulfo-LC-SMPT); *m*-maleimidobenzoyl-N-hydroxysuccinimide ester (MBS); m-maleimidobenzoyl-N-hydroxysulfosuccinimide ester (sulfo-MBS); N-succinimidyl(4-iodoacetyl)aminobenzoate (SIAB; thioether linker); sulfosuccinimidyl(4-iodoacetyl)amino benzoate (sulfo-SIAB); succinimidyl4(*p*-maleimidophenyl)butyrate (SMPB); sulfosuccinimidyl4-(*p*-maleimidophenyl)butyrate (sulfo-SMPB); azidobenzoyl hydrazide (ABH).

These linkers should be particularly useful when used in combination with peptide linkers, such as those that increase flexibility.

### 4. Acid cleavable linkers, photocleavable and heat sensitive linkers

Acid cleavable linkers include, but are not limited to, bismaleimideothoxy propane; and adipic acid dihydrazide linkers (*see, e.g*., Fattom et al., *Infection & Immun*. *60*:584-589, 1992) and acid labile transferrin conjugates that contain a sufficient portion of transferrin to permit entry into the intracellular transferrin cycling pathway (*see*, *e.g*., Welhöner et al., *J. Biol. Chem. 266*:4309-4314, 1991). Conjugates linked via acid cleavable linkers should be preferentially cleaved in acidic intracellular compartments, such as the endosome.

Photocleavable linkers are linkers that are cleaved upon exposure to light (*see, e.g*., Goldmacher et al., *Bioconj. Chem. 3*:104-107, 1992, which linkers are herein incorporated by reference), thereby releasing the targeted agent upon exposure to light. Photocleavable linkers are linkers that are cleaved upon exposure to light are known (*see, e.g*., Hazum et al., *Pept., Proc. Eur. Pept. Symp., 16th*, Brunfeldt, K (Ed), pp. 105-110, 1981, which describes the use of a nitrobenzyl group as a photocleavable protective group for cysteine; Yen et al., *Makromol. Chem 190*:69-82, 1989, which describes water soluble photocleavable copolymers, including hydroxypropylmethacrylamide copolymer, glycine copolymer, fluorescein copolymer and methylrhodamine copolymer; Goldmacher et al., *Bioconj. Chem. 3*:104-107, 1992, which describes a cross-linker and reagent therefor that undergoes photolytic degradation upon exposure to near UV light (350 nm); and Senter et al., *Photochem. Photobiol 42*:231-237, 195, which describes nitrobenzyloxycarbonyl chloride cross linking reagents that produce photocleavable linkages), thereby releasing the targeted agent upon exposure to light. Such linkers would have particular use in treating dermatological or ophthalmic conditions and other tissues, such as blood vessels during angioplasty in the prevention or treatment of restenosis, that can be exposed to light using fiber optics. After administration of the conjugate, the eye or skin or other body part can be exposed to light, resulting in release of the targeted moiety from the conjugate. This should permit administration of higher dosages of such conjugates compared to conjugates that release a cytotoxic agent upon internalization. Heat sensitive linkers would also have similar applicability.

### C. Methods of preparation of conjugates with linked targeted agent

Cytotoxic conjugates with linked targeted agents can be prepared either by chemical conjugation, recombinant DNA technology, or combinations of recombinant expression and chemical conjugation. The methods herein are described with particular reference to bFGF and saporin. It is understood, however, that the same methods may be used prepare and use conjugates of any member of the FGF family with SAP, modified SAP, a nucleic acid binding domain, a nucleic acid or any other targeted agent via linkers as described herein.

### 1. Chemical conjugation

To effect chemical conjugation herein, the FGF protein is linked via one ore more selected linkers or directly to the targeted agent. Chemical conjugation must be used if the targeted agent is other than a peptide or protein, such a nucleic acid or a non-peptide drug.

### a. Preparation of the FGF protein

The FGF protein is prepared by any suitable method, including recombinant DNA technology, isolation from a suitable source, purchase from a commercial source, or chemical synthesis. The selected linker or linkers is (are) linked to the FGF protein by chemical combination, generally relying on an available thiol or amine group on the FGF. Heterobifunctional linkers are particularly suited for chemical conjugation. Alternatively, if the linker is a peptide linker, then the FGF and linker can be expressed recombinantly as a fusion protein. If the targeted agent is a protein or peptide and the linker is a peptide, then the entire conjugate can be expressed as a fusion protein.

Any protein that is reactive with an FGF receptor may be used herein. In particular, any member of the FGF family of peptides or portion thereof that binds to an FGF receptor and internalizes a linked agent may be used herein. For the chemical conjugation methods the protein may be produced recombinantly, produced synthetically or obtained from commercial or other sources. For the preparation of fusion proteins, the DNA encoding the FGF may be obtained from any known source or synthesized according to its DNA or amino acid sequences (*see, e.g*., SEQ ID NOS. 12, 13 and 24-32; see discussion in A1 above).

In addition, any of the proteins reactive with an FGF may be modified as described herein in order to reduce the heterogeneity the resulting preparations of cytotoxic conjugates.

### (1) Selection of desired modifications of the FGF protein

If it is necessary or desired, the heterogeneity of preparations of FGF protein-containing chemical conjugates and fusion proteins can be reduced by modifying the FGF protein by deleting or replacing a site(s) on the FGF that cause the heterogeneity and/or by modifying the targeted agent. Such sites in FGF are typically cysteine residues that, upon folding of the protein, remain available for interaction with other cysteines or for interaction with more than one cytotoxic molecule per molecule of FGF peptide. Thus, such cysteine residues do not include any cysteine residue that are required for proper folding of the FGF peptide or for retention of the ability to bind to an FGF receptor and internalize. For chemical conjugation, one cysteine residue that, in physiological conditions, is available for interaction, is not replaced because it is used as the site for linking the cytotoxic moiety. The resulting modified FGF is conjugated with a single species of targeted agent.

### (2) Preparation of modified FGF polypeptides

The polypeptide reactive with an FGF receptor is modified by removing one or more reactive cysteines that are not required for receptor binding, but that are available for reaction with appropriately derivatized cytotoxic agent, so that the resulting FGF protein has only one cysteine residue available for conjugation with the cytotoxic agent. If necessary, the contribution of each cysteine to the ability to bind to FGF receptors may be determined empirically. Each cysteine residue may be systematically replaced with a conservative amino acid change (see Table 1, above) or deleted. The resulting mutein is tested for the requisite biological activity, the ability to bind to FGF receptors and internalize linked cytotoxic moieties. If the mutein retains this activity, then the cysteine residue is not required. Additional cysteines are systematically deleted and replaced and the resulting muteins are tested for activity. In this manner the minimum number and identity of the cysteines needed to retain the ability to bind to an FGF receptor and internalize may be determined. The resulting mutant FGF is then tested for retention of the ability to target a cytotoxic agent to a cell that expresses an FGF receptor and to internalize the cytotoxic agent into such cells. Retention of proliferative activity is indicative, though not definitive, of the retention of such activities. Proliferative activity may be measured by any suitable proliferation assay, such as the assay, exemplified below, that measures the increase in cell number of adrenal capillary endothelial cells.

It is noted, however, that modified or mutant FGFs may exhibit reduced or no proliferative activity, but may be suitable for use herein, if they retain the ability to target a linked targeted agent to cells bearing receptors to which the unmodified FGF binds and result in internalization of the targeted moiety.

Any of FGF-1 - FGF-9 may be any of FGF-1 - FGF-9 may be so modified. The complete amino acid sequence of each of FGF-1 - FGF-9 is known (*see, e.g*., SEQ ID NO. 24 (FGF-1) and SEQ ID NOS. 26-32 (FGF-3 - FGF-9, respectively). The sequence is examined and cysteine residues are identified. Comparison among the amino acid sequences of FGF-1 -FGF-9 reveals that one Cys is conserved among FGF family of peptides (see Table 3). These cysteine residues may be required for secondary structure and should not be altered. Each of the remaining cysteine residues may be systematically deleted and/or replaced by a serine residue or other residue that would not be expected to alter the structure of the protein. The resulting peptide is tested for biological activity. If the cysteine residue is necessary for retention of biological activity it is not deleted; if it not necessary, then it is preferably replaced with a serine or other residue that should not alter the secondary structure of the resulting protein.

The cysteine residues from each of FGF-1-FGF-9 that appear to be essential for retention of biological activity and that should not deleted or replaced are as follows:

**TABLE 3**

| | |
|---|---|
| FGF-1 | cys⁹⁸ |
| FGF-2 | cys¹⁰¹ |
| FGF-3 | cys¹¹⁵ |
| FGF-4 | cys¹⁵⁵ |
| FGF-5 | cys¹⁶⁰ |
| FGF-6 | cys¹⁴⁷ |
| FGF-7 | cys¹³⁷ |
| FGF-8 | cys¹²⁷ |
| FGF-9 | cys¹³⁴ |

For example, FGF-1 has cysteines at positions 31, 98 and 132; FGF-2 has cysteines at positions 34, 78, 96 and 101; FGF-3 has cysteines at positions 50 and 115; FGF-4 has cysteines at positions 88 and 155; FGF-5 has cysteines at positions 19, 93, 160 and 202; FGF-6 has cysteines at positions 80 and 147; FGF-7 has cysteines at positions 18, 23, 32, 46, 71, 133 and 137; FGF-8 has cysteines at positions 10, 19, 109 and 127; and FGF-9 has cysteines at positions 68 and 134.

Since FGF-3, FGF-4 and FGF-6 have only two cysteines, for purposes of chemical conjugation, preferably neither cysteine is deleted or replaced, unless another residue, preferably one near either terminus, is replaced with a cysteine. With respect to the other FGF family members, at least one cysteine must remain available for conjugation with the cytotoxic conjugate and probably two cysteines, but at least the cysteine residues set forth in Table 3. A second cysteine may be required to form a disulfide bond. Thus, any FGF peptide that has more than three cysteines is be modified for chemical conjugation by deleting or replacing the other cysteine residues. FGF peptides that have three cysteine residues are modified by elimination of one cysteine, conjugated to a cytotoxic moiety and tested for the ability to bind to FGF receptors and internalize the cytotoxic moiety.

In accord with the methods herein, several muteins of basic FGF for chemical conjugation have been produced (preparation of muteins for recombinant expression of the conjugate is described below). DNA, obtained from pFC80 (see, copending International PCT Application Serial No. PCT/US93/05702, which is a continuation-in-part of United States Application Serial No. 07/901,718; *see, also*, SEQ ID NO. 12) encoding basic FGF has been mutagenized. Mutagenesis of cysteine 78 of basic FGF to serine ([C78S]FGF) or cysteine 96 to serine ([C96S]FGF) produced two mutants that retain virtually complete proliferative activity of native basic FGF as judged by the ability to stimulate endothelial cell proliferation in culture. The activities of the two mutants and the native protein do not significantly differ as assessed by efficacy or maximal response. Sequence analysis of the modified DNA verified that each of the mutants has one codon for cysteine converted to that for serine.

The resulting mutein FGF or unmodified FGF is reacted with a single species of cytotoxic agent to produced. The bFGF muteins have been reacted with a single species of derivatized saporin (mono-derivatized saporin) thereby resulting in monogenous preparations of FGF-SAP conjugates and homogeneous compositions of FGF-SAP chemical conjugates. The resulting chemical conjugate does not aggregate and retains the requisite biological activities.

### b. Preparation of saporin for chemical conjugation

Saporin for chemical conjugation may be produced by isolating the protein from the leaves or seeds of *Saponaria officinalis* (*see*, *e.g*., Example 1) or using recombinant methods and the DNA provided herein or known to those of skill in the art or obtained by screening appropriate libraries (see Examples 1 and 4 below and the discussion in A.2.a(1)-(3) above).

### (1) Isolation of mono-derivatized SAP

For chemical conjugation, the SAP may be derivatized or modified such that it includes a cysteine residue for conjugation to the FGF protein. Typically, SAP is derivatized by reaction with SPDP. This results in a heterogeneous population. For example, SAP that is derivatized by SPDP to a level of 0.9 moles pyridine-disulfide per mole of SAP includes a population of non-derivatized, mono-derivatized and di-derivatized SAP. Ribosome-inactivating proteins, which are overly derivatized with SPDP, may lose activity because of reaction with sensitive lysines (Lambert et al., *Cancer Treat. Res. 37*:175-209, 1988). The quantity of non-derivatized SAP in the preparation of the non-purified material can be difficult to judge and this may lead to errors in being able to estimate the correct proportion of derivatized SAP to add to the reaction mixture.

Because of the removal of a negative charge by the reaction of SPDP with lysine, the three species, however, have a charge difference. The methods herein rely on this charge difference for purification of mon-derivatized SAP by Mono-S cation exchange chromatography. The use of purified mono-derivatized SAP has distinct advantages over the non-purified material. The amount of basic FGF that can react with SAP is limited to one molecule with the mono-derivatized material, and it is seen in the results presented herein that a more homogeneous conjugate is produced. There are still sources of heterogeneity with the mono-derivatized SAP used here. Native SAP as purified from the seed itself is a mixture of four isoforms, as judged by protein sequencing (*see, e.g.,* International PCT Application Serial No. PCT/US93/05702 and copending United States Application Serial No. 07/901,718; *see, also*, Montecucchi et al., *Int. J. Pept. Prot. Res. 33*:263-267, 1989; Maras et al., *Biochem. Internat*. *21*:631-638, 1990; and Barra et al., *Biotechnol. Appl. Biochem. 13*:48-53, 1991). This creates a some heterogeneity in the conjugates, since the reaction with SPDP probably occurs equally each isoform. This source of heterogeneity can be addressed by use of SAP expressed in *E. coli*.

### (2) Recombinant expression of saporin

DNA provided herein includes a sequence of nucleotides encoding a saporin polypeptide or a modified saporin polypeptide and may include an N-terminal extension sequence linked to the amino terminus of the saporin that encodes a linker so that, if desired, the SAP and linker can be expressed as a fusion protein.

Saporin for chemical conjugation may be produced by isolating the protein from the leaves or seeds of *Saponaria officinalis* (*see*, *e.g*., Example 1) or using recombinant methods and the DNA provided herein or known to those of skill in the art or obtained by screening appropriate libraries (*see* Examples 1 and 4 below and the discussion in A.2.a(1)-(3) above). DNA provided herein includes a sequence of nucleotides encoding a saporin polypeptide and may include an N-terminal extension sequence linked to the amino terminus of the saporin that encodes a linker so that, if desired, the SAP and linker can be expressed as a fusion protein.

The DNA molecules provided herein encode saporin that has substantially the same amino acid sequence and ribosome-inactivating activity as that of saporin-6 (SO-6), including any of four isoforms, which have heterogeneity at amino acid positions 48 and 91 (*see*, *e.g*., Maras et al., *Biochem. Internat. 21*:631-638, 1990, and Barra et al., *Biotechnol. Appl. Biochem. 13*:45-53, 1991 and SEQ ID NOS. 3-7). Other suitable saporin polypeptides include other members of the multi-gene family coding for isoforms of saporin-type RIP's including SO-1 and SO-3 (Fordham-Skelton et al., *Mol*. *Gen. Genet. 221*:134-138, 1990), SO-2 (*see, e.g.,* U.S. Application Serial No. 07/885,242, which corresponds to GB 2,216,891; *see, also*, Fordham-Skelton et al., *Mol. Gen. Genet. 229*:460-466, 1991), SO-4 (*see*, *e.g*., GB 2,194,241 B; *see*, *also*, Lappi et al., *Biochem. Biophys. Res. Commun. 129*:934-942, 1985) and SO-5 (*see*, *e*.*g*., GB 2,194,241 B; *see, also*, Montecucchi et al., *Int. J. Peptide Protein Res. 33*:263-267, 1989). SO-4, which includes the N-terminal 40 amino acids set forth in SEQ ID NO. 33, is isolated from the leaves of *Saponaria officinalis* by extraction with 0.1 M phosphate buffer at pH 7, followed by dialysis of the supernatant against sodium borate buffer, pH 9, and selective elution from a negatively charged ion exchange resin, such as Mono S (Pharmacia Fine Chemicals, Sweden) using gradient of 1 to 0.3 M. NaCI and first eluting chromatographic fraction that has SAP activity. The second eluting fraction is SO-5.

Tne saporin polypeptides exemplified herein include those having substantially the same amino acid sequence as those listed in SEQ ID NOS. 3-7. The isolation and expression of the DNA encoding these proteins is described in Example 1.

### (3) Modification of saporin

Because more than one amino group on SAP may react with the succinimidyl moiety, it is possible that more than one amino group on the surface of the protein is reactive. This creates the potential for heterogeneity in the mono-derivatized SAP. This source of heterogeneity has been solved by the conjugating modified SAP expressed in *E. coli* that has an additional cysteine inserted, as described above, in the coding sequence.

As discussed above, muteins of saporin that contain a Cys at or near the amino or carboxyl terminus can be prepared. Thus, instead of derivatizing saporin to introduce a sulfhydryl, the saporin can be modified by the introduction of a cysteine residue into the SAP such that the resulting modified saporin protein reacts with the FGF protein to produce a monogenous cytotoxic conjugate and the conjugate binds to FGF receptors on eukaryotic cells is cytotoxic upon internalization by such cells. Preferred loci for introduction of a cysteine residue include the N-terminus region, preferably within about one to twenty residues from the N-terminus of the cytotoxic agent, such as SAP. For expression of SAP in the bacterial host systems herein, it is also desirable to add DNA encoding a methionine linked to the DNA encoding the N-terminus of the saporin protein. DNA encoding SAP has been modified by inserting a DNA encoding Met-Cys (ATG TGT or ATG TGC) at the N-terminus immediately adjacent to the codon for first residue of the mature protein.

Muteins in which a cysteine residue has been added at the N-terminus and muteins in which the amino acid at position 4 or 10 has been replaced with cysteine have been prepared by modifying the DNA encoding saporin (*see*, EXAMPLE 4). The modified DNA may be expressed and the resulting saporin protein purified, as described herein for expression and purification of the resulting SAP. The modified saporin can then be reacted with the modified FGF to form disulfide linkages between the single exposed cysteine residue on the FGF and the cysteine residue on the modified SAP.

Using either methodology (reacting mono-derivatized SAP the FGF peptide or introducing a cys residue into SAP), the resulting preparations of FGF-SAP chemical conjugates are monogenous; compositions containing the conjugates also appear to be free of aggregates.

The above-described sources for heterogeneity also can be avoided by producing the cytotoxic conjugate as a fusion protein by expression of DNA encoding the modified FGF protein linked to DNA encoding the cytotoxic agent, as described below.

### 2. Recombinant production of the conjugates

Expression of DNA encoding a fusion of an FGF protein linked to the targeted agent results in a monogenous preparation of cytotoxic conjugates and is suitable for use, when the selected targeting agent and linker are polpypeptides. Preparations containing the fusion proteins may be rendered more homogeneous by modifying the FGF and/or the targeted agent to prevent interactions between each conjugate, such as via unreacted cysteines.

Aggregate formation has been eliminated or substantially reduced by preparing mutein constructs in which the cysteine residues on the FGF are deleted or replaced (*see,* discussion in 1A(1), above). Conjugates containing bFGF in which the cysteines at positions 78 and 96 residues have been replaced by serines have been prepared. The resulting preparations of cytotoxic conjugates retain cytotoxic activity, are monogenous and are free of aggregates.

### a. Preparation of muteins for recombinant production of the conjugates

For recombinant expression using to the methods herein, all of the cysteines the FGF peptide that are not required for biological activity are deleted or replaced; and for use in the chemical conjugation methods herein, all except for one of these cysteines, which will be used for chemical conjugation to the cytotoxic agent ,are deleted or replaced. In practice, it appears that only two cysteines (including each of the cysteine residues set forth in Table 3), and perhaps only the cysteines set forth in Table 3, are required for retention of the requisite biological activity of the FGF peptide. Thus, FGF peptides that have more than two cysteines are modified by replacing the remaining cysteines with serines. The resulting muteins may be tested for the requisite biological activity.

FGF peptides, such as FGF-3, FGF-4 and FGF-6, that have two cysteines can be modified by replacing the second cysteine, which is not listed in Table 3, and the resulting mutein used as part of a construct containing DNA encoding the cytotoxic agent linked to the FGF-encoding DNA. The construct is expressed in a suitable host cell and the resulting protein tested for the ability to bind to FGF receptors and internalize the cytotoxic agent.

As exemplified below, conjugates containing bFGF muteins in which Cys⁷⁸ and Cys⁹⁶ have been replaced with serine residues have been prepared. The resulting conjugates are at least as active as recombinant conjugates that have wild type FGF components and at least as active as chemical conjugates of FGF. In addition, it appears that the recombinantly produced conjugates are less toxic, and thus, can, if necessary, be administered in higher dosages.

### b. DNA constructs and expression of the DNA constructs

To produce monogenous preparations of cytotoxic conjugates using recombinant means, the DNA encoding the FGF protein is modified so that, upon expression, the resulting FGF portion of the fusion protein does not include any cysteines available for reaction. In preferred embodiments, DNA encoding an FGF polypeptide is linked to DNA encoding a saporin polypeptide. The DNA encoding the FGF polypeptide is modified in order to remove the translation stop codon and other transcriptional or translational stop signals that may be present and to remove or replace DNA encoding the available cysteines. The DNA is then ligated to the DNA encoding the saporin polypeptide directly or via a linker region of one or more codons between the first codon of the saporin and the last codon of the FGF. The size of the linker region is any length as long as the resulting conjugate exhibits cytotoxic activity upon internalization by a target cell. Presently, spacer regions of from about one to about seventy-five to ninety codons are preferred.

DNA encoding FGF peptides and/or the amino acid sequences FGFs are known to those of skill in this art (*see, e.g*., SEQ ID NOS. 24-32). DNA may be prepared synthetically based on the amino acid sequence or known DNA sequence of an FGF or may be isolated using methods known to those of skill in the art or obtained from commercial or other sources known to those of skill in this art. For example, DNA encoding virtually all of the FGF family of peptides is known. For example human aFGF (Jaye et al., *Science 233*:541-545, 1986), bovine bFGF (Abraham et al., *Science 233*:545-548, 1986), human bFGF (Abraham et al., *EMBO J. 5*:2523-2528, 1986; and Abraham et al., *Quant. Biol. 51*:657-668, 1986) and rat bFGF (*see* Shimasaki et al., *Biochem. Biophys. Res. Comm*., 1988, and Kurokawa et al., *Nucleic Acids Res*. *16*:5201, 1988), FGF-3, FGF-7 and FGF-9 are known (*see, also*, U.S. Patent No. 5,155,214; U.S. Patent No. 4,956,455; U.S. Patent No. 5,026,839; and U.S. Patent No. 4,994,559, the DNASTAR database, and references discussed above and below). The amino acid sequence of an exemplary mammalian bFGF isolated from bovine pituitary tissue is also known (*see, e.g*., in Esch et al., *Proc. Natl. Acad. Sci. USA 82*:6507-6511, 1985; and U.S. Patent No. 4,956,455).

Such DNA may then be mutagenized using standard methodologies to delete or delete and replace any cysteine residues, as describe herein, that are responsible for aggregate formation. If necessary, the identity of cysteine residues that contribute to aggregate formation may be determined empirically, by deleting and/or deleting and replacing a cysteine residue and ascertaining whether the resulting FGF with the deleted cysteine form aggregates in solutions containing physiologically acceptable buffers and salts.

As discussed above, any FGF protein, in addition to basic FGF (bFGF) and acidic FGF (aFGF), including *HST, INT*/2, FGF-5. FGF-6, KGF(FGF-7), FGF-8. and FGF-9 (*see, e.g*., Baird et al., *Brit. Med*. *Bull 45*:438-452, 1989; Tanaka et al., *Proc.* *Natl. Acad. Sci. USA 89*:8928-8932, 1992; Miyamoto et al., *Mol. Cell. Biol. 13*:4251-4259, 1993; *see, also,* the data base, DNA (July, 1993 release from DNASTAR, Inc. Madison, WI) for DNA and amino acid sequences of the FGF family; see SEQ ID NOS. 24-32 for amino acid sequences of FGF-1 - FGF-9, respectively), may be modified and expressed in accord with the methods herein. All of the FGF proteins induce mitogenic activity in a wide variety of normal diploid mesoderm-derived and neural crest-derived cells and this activity is mediated by binding to an FGF cell surface receptor followed by internalization. Binding to an FGF receptor followed by internalization are the activities required for an FGF protein to be suitable for use herein. A test of such "FGF mitogenic activity", which reflects the ability to bind to FGF receptors and to be internalized, is the ability to stimulate proliferation of cultured bovine aortic endothelial cells (*see, e.g*., Gospodarowicz et al., *J. Biol. Chem. 257*:12266-12278, 1982; Gospodarowicz et al., *Proc. Natl. Acad*. *Sci. USA 73*:4120-4124, 1976).

The DNA encoding the resulting modified FGF-SAP can be inserted into a plasmid and expressed in a selected host, as described above, to produce monogenous preparations of FGF-SAP and homogeneous compositions containing monogenous FGF-SAP.

Multiple copies of the modified FGF-SAP chimera or modified FGF-cytotoxic agent chimera can be inserted into a single plasmid in operative linkage with one promoter. When expressed, the resulting protein will be an FGF-SAP multimer. Typically two to six copies of the chimera are inserted, preferably in a head to tail fashion, into one plasmid.

DNA encoding human bFGF-SAP having SEQ ID NO. 12 has been mutagenized as described in the Examples using splicing by overlap extension (SOE). Another preferred coding region is set forth in SEQ ID NO. 13, nucleotides 1 - 465. In both instances, in preferred embodiments, the DNA is modified by replacing the cysteines at positions 78 and 96 with serine. The codons encoding cysteine residues at positions 78 and 96 of FGF in the FGF-SAP encoding DNA (SEQ ID NO. 12) were converted to serine codons by SOE. Each application of the SOE method uses two amplified oligonucleotide products, which have complementary ends as primers and which include an altered codon at the locus at which the mutation is desired, to produce a hybrid product. A second amplification reaction that uses two primers that anneal at the non-overlapping ends amplify the hybrid to produce DNA that has the desired alteration.

### D. Methods for preparation of heparin-binding growth factor and nucleic acid complexes

Many methods have been developed to deliver nucleic acid into cells including retroviral vectors, electroporation, CaPO4 precipitation and microinjection, but each of these methods has distinct disadvantages. Microinjecting nucleic acid into cells is very time consuming because each cell must be manipulated individually. Retroviral vectors can only hold a limited length of nucleic acid and can activate oncogenes depending upon the insertion site in the target chromosome. Conditions for electroporation and CaPO₄-mediated transfection are harsh and cause much cell death.

By comparison, receptor mediated gene delivery as described herein is a more desirable method of selectively targeting toxic genes into cells that have "more active" receptors or that overexpress the specific receptor on the cell surface. A receptor may be more active because it has a higher rate of internalization or higher cycling rate through the endosome to the cell surface. Advantages of this method over other gene delivery methods include increased specificity of delivery, the absence of nucleic acid length limitations, reduced toxicity, and reduced immunogemeity of the conjugate. These characteristics allow for repeated administration of the material with minimal harm to cells and may allow increased level of expression of the toxic protein. In addition, primary cultures can also be treated using this method.

Receptor mediated gene delivery is also useful for delivering other types of nucleic acids. Antisense and ribozymes will interfere with specific gene expression in a cell. With these nucleic acids, an inhibitory signal is delivered which may result in cytotoxicity or decreased gene expression without concomitant cytotoxicity. Conversely, gene delivery can be used to increase gene expression of specific genes. Thus, genetic defects may be corrected, or novel proteins expressed to effect a "foreign" biological function on a cell, such as drug sensitivity, capability to bind to a substrate, enzymatic activity, and the like.

Specificity of delivery is achieved by coupling a nucleic acid binding domain to a growth factor, either by chemical conjugation or by generating a fusion protein. The growth factor part of the conjugate or fusion confers specificity thus generating a cell-specific nucleic acid delivery conjugate. The choice of the growth factor to use will depend upon the receptor expressed by the target cells. The receptor type of the target cell population may be determined by conventional techniques like antibody staining, PCR of cDNA using receptor-specific primers, and biochemical or functional receptor binding assays. It is preferable that the receptor be cell type specific or have increased expression or activity within the target cell population.

The nucleic acid binding domain can be non-specific in its ability to bind nucleic acid or it can be highly specific so that the amino acid residues bind only the desired nucleic acid sequence. Nonspecific binding proteins, polypeptides, or compounds are generally polycations or highly basic. Lys and Arg are basic residues and proteins enriched for these residues are candidate nucleic acid binding domains. Examples of basic proteins include histones, protamines, and repeating units of lysine and arginine. Poly-L-lysine is a well-used nucleic acid binding domain. Polycations, such as spermine and spermidine, are also widely used to bind nucleic acids. Examples of sequence-specific proteins include Sp-1, AP-1, and the rev protein from HIV. Specific nucleic acid binding domains such as AP-1 and Sp-1 can be cloned in tandem, individually, in tandem, or in multiple repeats into a desired region of the growth factor of interest. Alternatively, the domains can be chemically conjugated to the growth factor.

The corresponding response elements that bind the domain are incorporated into the nucleic acid molecule to be delivered. Condensation of the nucleic acid and protein will result in specific binding of response element to the nucleic acid binding domain. Even greater specificity of binding may be achieved by identifying and using the minimal amino acid sequence that binds to the nucleic acid of interest. For example, phage display methods can be used to identify amino acids residues of varying length that will bind to specific nucleic acid sequences with high affinity. (See U.S. Patent No. 5,223,409.) The peptide sequence can then be cloned into the ligand as a single copy or multiple copies. Alternatively, the peptide may be chemically conjugated to the ligand. Incubation of the nucleic acid with the conjugated protein will result in a specific binding between the two.

These complexes may be used to deliver nucleic acids that encode saporin or other toxic proteins into cells that have FGF, VEGF, HBEGF, or other heparin-binding growth factor receptors that are more active in intemalization upon binding or over expressed on the cell surfaces. When a therapeutic gene is to be delivered, the cDNA encoding the gene is cloned downstream of a mammalian promoter such as SV40. CMV, TK or Adenovirus promoter. As outlined below, promoters of interest may be active in any cell type, active only in a tissue-specific manner, such as α-crystalline or tyrosinase, or inducible, such as the MMTV LTR.

The nucleic acid construct containing the therapeutic gene, antisense, ribozyme, or the like, is bound onto a nucleic acid binding domain such as Sp-2, AP-1, poly-L-lysine or the like, as described below, which has been chemically conjugated or fused by recombinant means to a heparin-binding growth factor acting as a cell-specific ligand.

### 1. Nucleic acid binding domain

As noted above, nucleic acid binding domains (NABD) interact with the target nucleic acid either in a sequence-specific manner or a nonspecific manner. When the interaction is non-specific, the NABD binds nucleic acid regardless of the sequence. For example, poly-L-lysine is a basic polypeptide that binds to oppositely charged DNA. Other highly basic proteins or polycationic compounds, such as histones, protamines, and spermidine, also bind to nucleic acids in a nonspecific manner.

Many proteins have been identified that bind specific sequences of DNA. These proteins are responsible for genome replication, transcription and repair of damaged DNA. The transcription factors regulate gene expression and are a diverse group of proteins. These factors are especially well suited for purposes of the subject invention because of their sequence-specific recognition. Transcription factors are grouped into one of seven well-established classes based upon the structural motif used for recognition. Other classes or subclasses may eventually be delineated as more factors are discovered and defined. Proteins from those classes or proteins that do not fit within one of these classes, such as SV40 T antigen and p53 may also be used. The major families include helix-turn-helix (HTH) proteins, homeodomains, zinc finger proteins, steroid receptors, leucine zipper proteins, the helix-loop-helix proteins, and β-sheets.

Examples of members of these families are generally available. Many of these factors are cloned and the precise DNA-binding region delineated for some of them. When the sequence of the DNA-binding domain is known, a gene encoding it may be synthesized if the region is short. Alternatively, the genes may be cloned from the genome or from cDNA libraries using oligonucleotides as probes or primers for polymerase chain reaction methods. Such methods may be found in (Sambrook et al., supra).

Helix-turn-helix proteins include the well studied λ Cro protein, λcI, and E. coli CAP proteins (*see,* Steitz et al., *Proc. Natl. Acad. Sci. USA 79*:3097-3100, 1982; Ohiendorf et al., *J. Mol. Biol. 169*:757-769, 1983). In addition, the Lac repressor (Kaptein et al., *J. Mol. Biol. 182*:179-182, 1985) and Trp repressor (Scheritz et al., *Nature 317*:782-786, 1985) belong to this family. Members of the homeodomain family include the Drosophila protein Antennapaedia (Qian et al., *Cell. 59*:573-580, 1989) and yeast MATα2 (Wolberger et al., *Cell. 67*:517-528, 1991). Zinc finger proteins include TFIIIA (Miller et al., *EMBOJ 4*:1609-1614, 1985), Sp-1, zif 268 and many others (*see,* generally Krizek et al., *J. Am. Chem. Soc. 113*:4518-4523, 1991): Steroid receptor proteins include receptros for steroid hormones, retinoids, vitamin D, thyroid hormones, as well as other compounds. Specific examples include retenoic acid, knirps, progesterone, androgen, glucocosteroid and estrogen receptor proteins. The leucine zipper family was defined by a heptad repeat of leucines over a region of 30 to 40 residues. Specific members of this family include C/EBP, C-fos, c-jun, GCN4, sis-A, and CREB (*see,* generally O'Shea et al., *Science 254*:539-544, 1991). The helix-look-helix (HLH) family of proteins appears to have some similarities to the leucine zipper family. Well-known members of this family myoD (Weintraub et al., *Science 251*:761-766, 1991); c-myg; and AP-2 (Williams and Tijan, *Science 251*:1067-1071, 1991). The β-sheet family uses an antiparallel β-sheet for DNA binding, rather than the more common α-helix. The family contains the MetJ (Phillips, *Curr. Opin. Struc. Biol. 1*:89-98, 1991), Arc (Breg et al., *Nature 346*:586-589, 1990) and Mnt repressors. In addition, other motifs are used for DNA binding, such as the cysteine-rich motif in yeast GAL4 repressor, and the GATA factor. In addition, viruses contain genes that bind specific sequences. One of the most-studied viral genes in the *rev* gene from HIV. The *rev* gene product binds a sequence called RRE (*rev* responsive element) found in the *env* gene. Other proteins or peptides that bind DNA may be discovered on the basis of sequence similarity to the known classes or functionally by selection.

A technique that may be useful for selection of nucleic acid binding domains is phage display. (*See,* for example, U.S. Patent No. 5,223,409.) In this method, DNA sequences are inserted into the gene III or gene VIII gene of filamentous phage, such as M13. The DNA sequences may be randomly generated or variants of a known DNA-binding domain. Generally, the inserts encode from 6 to 20 amino acids. Several vectors with multicloning sites have been developed for insertion (McLafferty et al., *Gene 128*:29-36, 1993; Scott and Smith, *Science 249*:386-390, 1990; Smith and Scott, *Methods Enzymol. 217*:228-257, 1993). The peptide encoded by the inserted sequence is displayed on the surface of the bacteriophage. Bacteriophage expressing a desired DNA-binding domain are selected for by binding to the DNA molecule that is to used in gene therapy. The DNA molecule used for selection may be single stranded or double stranded. When DNA molecules for delivery are single-stranded, such as ribozymes and antisense, the appropriate target is single-stranded. When DNA molecules for delivery encode a therapeutic gene, the target molecule is preferably double-stranded, but single-stranded molecules may also be used. Preferably, the entire coding region of the DNA molecule is used as the target. In addition, elements necessary for transcription that are included for *in vivo* or *in vitro* delivery may be present in the target DNA molecule. Recovered bacteriophage are propagated and subsequent rounds of selection may be performed. The final selected phage are propagated and the DNA sequence of the insert is determined. Once the predicted amino acid sequence of the peptide is known, sufficient peptide for use herein may be made either by recombinant means or synthetically, depending on the method of coupling. In addition, the peptide may be generated as a tandem array of 2 or more peptides, in order to maximize affinity or binding of multiple DNA molecules to a single polypeptide.

As an example of the phage display selection technique, a DNA-binding domain/peptide that recognizes DNA encoding saporin is isolated. DNA fragments encoding saporin may be isolated from a plasmid containing these sequences. The plasmid FPFS1 contains the entire coding region of saporin. Digestion of the piasmid with *Nco*I and *Eco*RI restriction enzymes liberates the saporin specific sequence as a single fragment of approximately 780 bp. This fragment may be purified by any one of a number of methods, such as agarose gel electrophoresis and subsequent elution from the gel. The saporin fragment is fixed to a solid support, such as in the wells of a 96-well plate. If the double-stranded fragment does not bind well to the plate, a coating, a positively charged molecule, may be used to promote DNA adherence. The phage library is added to the wells and an incubation period allows for binding of the phage to the DNA. Unbound phage are removed by a wash, typically containing 10mM Tris, 1mM EDTA, and not containing any salt or low salt concentration. Bound phage are eluted starting at a 0.1M NaCI containing buffer. The NaCl concentration is increased in a step-wise fashion until all the phage are eluted. Typically, phage binding with higher affinity will only be released by higher salt concentrations.

Eluted phage are propagated in the bacteria host. Further rounds of selection may be performed to select for a few phage binding with high affinity. The DNA sequence of the insert in the binding phage is then determined. In addition, peptides having a higher affinity may be isolated by making variants of the insert sequence and subjecting these variants to further rounds of selection.

### 2. Chemical conjugation of heparin-binding growth factor and DNA binding domain

Either FGF, VEGF, or HBEGF may be conjugated to the nucleic acid binding domain. FGF may be conjugated essentially as described in Section C.1 above with the substitution of the nucleic acid binding domain for saporin. Unkers, as described in B above, may be incorporated into the chemical conjugates or fusion proteins.

### a. Preparation of proteins for chemical conjugation

### (1) Preparation of HBEGF polypeptides for chemical conjugation

FGF, VEGF or HBEGF may be isolated from a suitable source or may be produced using recombinant DNA methodology, discussed below. To effect chemical conjugation herein, the heparin-binding growth factor protein is conjugated generally via a reactive amine group or thiol group to the targeted agent or to a linker, which has been or is subsequently linked to the targeted agent. The heparin-binding growth factor protein is conjugated either via its N-terminus, C-terminus, or elsewhere in the polypeptide. In preferred embodiments, the heparin-binding growth factor protein is conjugated via a reactive cysteine residue to the linker or to the targeted agent. The heparin-binding growth factor can also be modified by addition of a cysteine residue, either by replacing a residue or by inserting the cysteine, at or near the amino or carboxyl terminus, within about 20, preferably 10 residues from either end, and preferably at or near the amino terminus.

In preferred embodiments, to reduce the heterogeneity of preparations, the heparin-binding growth factor protein is modified by mutagenesis to replace reactive cysteines, leaving, preferably, only one available cysteine for reaction. The heparin-binding growth factor protein is modified by deleting or replacing a site(s) on the heparin-binding growth factor that causes the heterogeneity. Such sites are typically cysteine residues that, upon folding of the protein, remain available for interaction with other cysteines or for interaction with more than one cytotoxic molecule per molecule of heparin-binding growth factor peptide. Thus, such cysteine residues do not include any cysteine residue that are required for proper folding of the growth factor or for retention of the ability to bind to a heparin-binding growth factor receptor and internalize. For chemical conjugation, one cysteine residue that, in physiological conditions, is available for interaction, is not replaced because it is used as the site for linking the cytotoxic moiety. The resulting modified heparin-binding growth factor is conjugated with a single species of cytotoxic conjugate.

Alternatively, the contribution of each cysteine to the ability to bind to heparin-binding growth factor receptors may be determined empirically. Each cysteine residue may be systematically replaced with a conservative amino acid change (see Table 1, above) or deleted. The resulting mutein is tested for the requisite biological activity: the ability to bind to heparin-binding growth factor receptors and internalize linked cytotoxic moieties. If the mutein retains this activity, then the cysteine residue is not required. Additional cysteines are systematically deleted and replaced and the resulting muteins are tested for activity. Each of the remaining cysteine residues may be systematically deleted and/or replaced by a serine residue or other residue that would not be expected to alter the structure of the protein. The resulting peptide is tested for biological activity. If the cysteine residue is necessary for retention of biological activity it is not deleted; if it not necessary, then it is preferably replaced with a serine or other residue that should not alter the secondary structure of the resulting protein. In this manner the minimum number and identity of the cysteines needed to retain the ability to bind to a heparin-binding growth factor receptor and internalize may be determined. It is noted, however, that modified or mutant heparin-binding growth factors may exhibit reduced or no proliferative activity, but may be suitable for use herein, if they retain the ability to target a linked cytotoxic agent to cells bearing receptors to which the unmodified heparin-binding growth factor binds and result in internalization of the cytotoxic moiety. In the case of VEGF, VEGF₁₂₁ contains 9 cysteines and each of VEGF₁₆₅, VEGF₁₈₉ and VEGF₂₀₆ contain 7 additional residues in the region not present in VEGF₁₂₁. Any of the 7 are likely to be non-essential for targeting and internalization of linked cytotoxic agents. Recently, the role of Cys-25, Cys-56, Cys-67, Cys-101, and Cys-145 in dimerization and biological activity was assessed (Claffery et al., *Biochem. Biophys. Acta 1246*:1-9, 1995). Dimerization requires Cys-25, Cys-56, and Cys-67. Substitution of anyone of these cysteine residues resulted in secretion of a monomeric VEGF, which was inactive in both vascular permeability and endothelial cell mitotic assays. In contrast, substitution of Cys 145 had no effect on dimerization, although biological activities were somewhat reduced. Substitution of Cys-101 did not result in the production of a secreted or cytoplasmic protein. Thus, substitution of Cys-145 is preferred.

The VEGF monomers are preferably linked via non-essential cvsteine residues to the linkers or to the targeted agent. VEGF that has been modified by introduction of a cys residue at or near one terminus, preferably the N-terminus is preferred for use in chemical conjugation (see Examples for preparation of such modified VEGF). For use herein, preferably the VEGF is dimerized prior to linkage to the linker and/or targeted agent. Methods for coupling proteins to the linkers, such as the heterobifunctional agents, or to nucleic acids, or to proteins are known to those of skill in the art and are also described herein.

For recombinant expression using the methods described herein, up to all cysteines in the HBEGF polypeptide that are not required for biological activity can be deleted or replaced. Alternatively, for use in the chemical conjugation methods herein, all except one of these cysteines, which will be used for chemical conjugation to the cytotoxic agent, can be deleted or replaced. Each of the HBEGF polypeptides described herein have six cysteine residues. Each of the six cysteines may independently be replaced and the resulting mutein tested for the ability to bind to HBEGF receptors and to be internalized. Alternatively, the resulting mutein-encoding DNA is used as part of a construct containing DNA encoding the NABD linked to the HBEGF-encoding DNA. The construct is expressed in a suitable host cell and the resulting protein tested for the ability to bind to HBEGF receptors and internalize. As long as this ability is retained the mutein is suitable for use herein.

Methods for chemical conjugation of proteins are known to those of skill in the art. The preferred methods for chemical conjugation depend on the selected components, but preferably rely on disulfide bond formation. For example, if the targeted agent is SPDP-derivatized saporin, then it is advantageous to dimerize the VEGF moiety prior coupling or conjugating to the derivatized saporin. If VEGF is modified to include a cysteine residue at or near the N-, preferably, or C- terminus, then dimerization should follow coupling to the targeted agent.

To effect chemical conjugation herein, the HBEGF polypeptide is linked via one or more selected linkers or directly to the targeted agent. Chemical conjugation must be used if the targeted agent is other than a peptide or protein, such a nucleic acid or a non-peptide drug.

### b. Preparation of NABD for chemical conjugation

A nucleic acid binding domain is prepared for chemical conjugation essentially as described in C(1)(b) above. Briefly, a protein binding domain may be derivatized with SPDP of other chemical. If the binding domain does not have a Cys residue available for reaction, one can be either inserted or substituted for another amino acid. Mono-derivatized species may be isolated, essentially as described above.

### 3. Fusion protein of heparin-binding growth factor and DNA binding domain

Expression of DNA encoding a fusion of a heparin-binding growth factor polypeptide linked to the targeted agent results in a more homogeneous preparation of cytotoxic conjugates and is suitable for use, when the selected targeting agent and linker are polypeptides. Aggregate formation can be reduced in preparations containing the fusion proteins by modifying the growth factor, such as by removal of nonessential cysteines in the heparin-binding domain and/or the nucleic acid domain to prevent interactions between each conjugate, such as via unreacted cysteines.

DNA encoding the polypeptides may be isolated, synthesized or obtained from commercial sources or prepared as described herein. Expression of recombinant heparin-binding growth factor polypeptides may be performed as described herein; and DNA encoding these polypeptides may be used as the starting materials for the methods herein.

DNA encoding FGF, VEGF, and HBEGF polypeptides and/or the amino acid sequences of these factors are known to those of skill in this art (*see, e.g*., SEQ ID NOS. 24-32, 78-95). DNA may be prepared synthetically based on the amino acid sequence or known DNA sequence or may be isolated using methods known to those of skill in the art or obtained from commercial or other sources known to those of skill in this art. For example, suitable methods are described in Examples 3 and 5 for amplifying FGF encoding cDNA from plasmids containing FGF encoding cDNA.

As described herein, such DNA may then be mutagenized using standard methodologies to delete or replace any eysteine residues that are responsible for aggregate formation. If necessary, the identity of cysteine residues that contribute to aggregate formation may be determined empirically, by deleting and/or deleting and replacing a cysteine residue and ascertaining whether the resulting growth factor with the deleted cysteine forms aggregates in solutions containing physiologically acceptable buffers and salts. Loci for insertion of cysteine residues may also be determined empirically. Generally, regions at or near (within 20, preferably 10 amino acids) the C-or, preferably, the N-terminus are preferred.

The DNA construct encoding the conjugate can be inserted into a plasmid and expressed in a selected host, as described above, to produce a recombinant heparin-binding growth factor-NABD conjugate. Multiple copies of the chimera can be inserted into a single plasmid in operative linkage with one promoter. When expressed, the resulting protein will then be a multimer. Typically, two to six copies of the chimera are inserted, preferably in a head to tail fashion, into one plasmid.

### 4. Nucleic acid construct

The nucleic acids suitable for delivery include those described above. Briefly, these include antisense, ribozymes, single-strands that will form triplexes, protein-binding oligonucleotides, and therapeutic genes. Generally, antisense, ribozymes and the like, will be delivered without requiring further propagation or transcription/translation, while therapeutic genes will need to be transcribed and translated for effectiveness. However, any of these molecules may be contained on self-replicating vectors for amplification of the nucleic acid. In the case of cytotoxic agents such as the ribosome-inactivating proteins, very few molecules need be present for cell killing. Indeed, only a single molecule of diphtheria toxoid is necessary to kill a cell. In other cases, it may be that propagation or stable maintenance of the construct is necessary to attain sufficient numbers or concentrations of the gene product for effective gene therapy. Examples of replicating and stable eukaryotic plasmids are found in the scientific literature.

In general, nucleic acid constructs containing therapeutic genes will also contain elements necessary for transcription and translation. The choice of the promoter will depend upon the cell type to be transformed and the control desired. Promoters can be constitutive or active in any cell type, tissue specific, cell specific or inducible. Examples of contitutive or nonspecific promoters include the SV40 early promoter, the SV40 late promoter, CMV early gene promoter, HIV LTR, and adenovirus promoter. In addition to viral promoters, cellular promoters are also amenable within the context of this invention. In particular, cellular promoters for the so-called housekeeping genes are useful. Viral promoters are preferred, because generally they are stronger promoters than cellular promoters.

Tissue specific promoters are particularly useful when a particular tissue type is to be targeted for transformation. By using one of this class of promoters, an extra margin of specificity can be attained. For example, when the indication to be treated is ophthalmological, either the alpha-crystalline promoter or gamma-crystalline promoter is preferred. When a tumor is the subject of gene delivery, cellular promoters for specific tumor markers or promoters more active in tumor cells should be chosen. Thus, to transform prostate tumor cells the prostate-specific antigen promoter is especially useful. Similarly, the tyrosinase promoter or tyrosinase-related protein promoter is a preferred promoter for melanoma treatment. For B lymphocytes, the immunoglobulin variable region gene promoter, for T lymphocytes, the TCR receptor variable region promoter, for helper T lymphocytes, the CD4 promoter, for liver, the albumin promoter, are but a few examples of tissue specific promoters. Many other examples of tissue specific promoters are readily available to one skilled in the art.

Inducible promoters may also be used. These promoters include the MMTV LTR, which is inducible by dexamethasone, metallothionein, which is inducible by heavy metals, and promoters with cAMP response elements, which are inducible by cAMP. By using an inducible promoter, the nucleic acid may be delivered to a cell and will remain quiescent until the addition of the inducer. This allows further control on the timing of production of the therapeutic gene.

Therapeutic gene products may be noncytotoxic but activate a compound, which is endogenously produced or exogenously applied, from a nontoxic form to a toxic product. Gene products that activate compounds to be cytotoxic include HSVTK, which selectively monophosphorylates certain purine arabinosides and substituted pyrimidine compounds. More specifically, exposure of the drugs ganiclovir, acyclovir, or any of their analogues (*e.g*., FIAU, FIAC, DHPG) to HSVTK, phosphorylates the drug into its corresponding active nucleotide triphosphate form.

Other gene products may also be utilizes within the context of the present invention. These include *E. coli.* guanine phosphoribosyl transferase which converts thioxanthine into toxic thioxanthine monophosphate (Besnard et al., *Mol. Cell. Biol.* 7:4139-4141, 1987); alkaline phosphatase, which converts inactive phosphorylated compounds such as mitomycin phosphate and doxorubicin-phosphate to toxic dephosphorylated compounds; fungal (*e.g*., *Fusarium oxysporum*) or bacterial cytosine deaminase which converts 5-fluorocytosine to the toxic compound 5-fluorouracil (Mullen, *PNAS 89*:33, 1992); carboxypeptidase G2 which cleaves glutamic acid from para-N-bis (2-chloroethyl) aminobenzoyl glutamic acid, creating a toxic benzoic acid mustard; and Penicillin-V amidase, which converts phenoxyacetabide derivatives of doxorubicin and melphalan to toxic compounds (*see*, generally, Vrudhula et al., *J*. *of Med. Chem. 36*(7):919-923, 1993; Kern et al., *Canc. Immum. Immunother. 31*(4):202-206, 1990):

Additionally, promoters that are coordinately regulated with a particular cellular gene may be used. For example, promoters of genes that are coordinately expressed when a particular FGF receptor gene is expressed may be used. Then, the nucleic acid will be transcribed when the FGF receptor, such as FGFR1, is expressed, and not when FGFR2 is expressed. This type of promoter is especially useful when one knows the pattern of FGF receptor expression in a particular tissue, so that specific cells within that tissue may be killed upon transcription of a cytotoxic agent gene without affecting the surrounding tissues.

### 5. Condensation of the heparin-binding growth factor and nucleic acids

The growth factor/NABD is incubated with the nucleic acid to be delivered under conditions that allow binding of the NABD to nucleic acid. Conditions will vary somewhat depending on the nature of the NABD, but will typically occur in 0.1M NaCl and 20 mM HEPES or other similar buffer.

One desired application of nucleic acid delivery is the delivery of cytotoxic agents, such as saporin, in a non-toxic form. By delivering a nucleic acid molecule capable of expressing saporin, the timing of cytotoxicity may be exquisitely controlled. For example, if saporin is expressed under the control of a tissue-specific promoter, then uptake of the complex by cells having the tissue-specific factors necessary for promoter activation will result in the killing of those cells. On the other hand, if cells taking up the complex do not have those tissue-specific factors, the cells will be spared.

As an example of the constructs that may be used to effect this strategy, test constructs have been made and assayed. The first construct is a chemical conjugate of FGF and poly-L-lysine. The FGF molecule is a variant in which the cys residue at position 96 has been changed to a serine; only the cys at position 78 is available for conjugation. The poly-L-lysine was derivatized with SPDP and coupled to FGF2-3. This conjugate was used to condense a plasmid able to express the β-galactosidase gene.

The ability of a construct to bind nucleic acid molecules may be assessed by agarose gel electrophoresis. A convenient test is to digest a plasmid, such as pSVβ, with restriction enzymes to yield a variety of fragment sizes. For ease of detection, the fragments may be labeled with ³²P either by filling in of the ends with DNA polymerase I or phosphorylation of the 5'-end with polynucleotide kinase following a dephosphorylation using alkaline phosphatase. The plasmid fragments are then incubated with the heparin-binding growth factor/nucleic acid binding domain in a buffered saline solution, such as 20mM HEPES, pH 7.3, 0.1M NaCl. The reaction mixture is electrophoresed on an agarose gel along side similarly digested, but nonreacted fragments. If a radioactive label was incorporated, the gel may be dried and autoradiographed. If no radioactive label is present, the gel may be stained with ethidium bromide and the DNA visualized by excitation with UV. Binding has occurred if the mobility of the fragments is retarded compared to the control. In the example case, the mobility of the fragments was retarded after binding with the FGF2-3/poly-L-lysine conjugate.

Further examination of the conjugate is performed to show that it binds to the cell surface receptor and is internalized into the cell. It is not necessary that the heparin-binding growth factor as part of the conjugate retain complete biological activity. For example, FGF is mitogenic on certain cell types. As discussed above, this activity may be desirable or not, depending upon the intended purpose of the nucleic acid delivery. If this activity is desirable or necessary, a proliferation assay is performed. Likewise, for each desirable activity, an appropriate assay is performed. However, for application of the subject invention, the only criteria that need be met are receptor binding and internalization.

Receptor binding and internalization may be measured by the following three assays. A competitive inhibition assay of the complex to cells expressing the appropriate receptor is used to demonstrate receptor binding. Receptor binding and internalization may be assayed by measuring β-gal expression (*e*.*g*., enzymatic activity) in cells that have been transformed with a complex of a β-gal containing plasmid condensed with a heparin-binding growth factor/NABD. This assay is particularly useful for optimizing conditions to give maximal transformation. Thus, the optimum ratio of growth factor/NABD to nucleic acid and the amount of DNA per cell may readily be determined by assaying and comparing the enzymatic activity of β-gal. As such, these first two assays are useful for preliminary analysis and failure to show receptor binding or β-gal activity does not eliminate a candidate heparin-binding growth factor/NABD conjugate or fusion protein from further analysis. A third, preferred, assay is a cytotoxicity assay performed on cells transformed with a nucleic acid encoding a cytotoxic agent condensed with heparin-binding growth factor/NABD. While, in general, any cytotoxic agent may be used, ribosome-inactivating proteins are preferred and saporin, or another type I RIP, is particularly preferred. A statistically significant reduction in cell number demonstrates the ability of the growth factor/NABD conjugate or fusion to deliver nucleic acids into a cell.

In a similar fashion, functional assays may be used to assess the ability of any conjugate described herein to bind to a receptor and be intemalized. Thus, when the conjugate delivers a cytotoxic signal, cytotoxicity on test cells is measured.

In the exemplary conjugate provided herein, FGF-poly-L-lysine was used to condense pSVβ and introduced into COS cells and ABAE, an endothelial cell line. Both of these cell lines express FGF receptors. Maximal β-galactosidase activity was achieved when 30 µg of pSVβ per 100 µg of FGF2-3-poly-L-lysine was used. Approximately 30% of the cells showed demonstrable staining with X-gal. Moreover, the transformation was dependent upon the presence of FGF receptors; β-gal activity was not significantly above background when cells were incubated with pSVβ alone, poly-L-lysine plus pSVβ, or unconjugated FGF-3, poly-L-lysine, plus pSVβ.

### 6. Covalent coupling of nucleic acids to proteins

To effect chemical conjugation herein, the heparin-binding growth factor protein is linked to the nucleic acid either directly or via one or more linkers. Methods for conjugating nucleic acids, at the 5' ends, 3' ends and elsewhere, to the amino and carboxyl termini and other sites in proteins are known to those of skill in the art (for a review *see, e.g*., Goodchild, (1993) In: Perspectives in Bioconjugate Chemistry, Mears, Ed., American Chemical Society, Washington, D.C. pp. 77-99). For example, proteins have been linked to nucleic acids using ultraviolet irradiation (Sperling et al., *Nucleic Acids Res. 5*:2755-2773, 1976; Fiser et al., *FEBS Lett. 52*:281-283, 1975), bifunctional chemicals (Bäumert et al., *Eur. J. Biochem. 89*:353-359, 1978; and Oste et al., *Mol. Gen. Genet. 168*:81-16, 1979) photochemical cross-linking (Vanin et al., *FEBS Lett. 124*:89-92, 1981; Rinke et al., *J. Mol. Biol. 137*:301-314, 1980; Millon et al., *Eur. J. Biochem. 110*:485-454, 1980).

In particular, the reagents (N-acetyl-N'-(p-glyoxylylbenzolyl)cystamine and 2-iminothiolane have been used to couple DNA to proteins, such as α2macroglobulin (α 2M) via mixed disulfide formation (*see,* Cheng et al., *Nucleic Acids Res. 11*:659-669, 1983). N-acetyl-N'-(p-glyoxylylbenzolyl)cystamine reacts specifically with nonpaired guaninine residues and, upon reduction, generates a free sulfhydryl group. 2-Iminothiolane reacts with proteins to generate sulfhydryl groups that are then conjugated to the derivatized DNA by an intermolecular disulfide interchange reaction. Any linkage may be used provided that, upon internalization of the conjugate the targeted nucleic acid is active. Thus, it is expected that cleavage of the linkage may be necessary, although it is contemplated that for some reagents, such as DNA encoding ribozymes linked to promoters or DNA encoding therapeutic agents for delivery to the nucleus, such cleavage may not be necessary.

Thiol linkages can be readily formed using heterbiofunctional reagents. Amines have also been attached to the terminal 5' phosphate of unprotected oligonucleotides or nucleic acids in aqueous solutions by reacting the nucleic acid with a water-soluble carbodiimide, such as 1-ethyl-3'[3-dimethylaminopropyl]carbodiimide (EDC) or N-ethyl-N'(3-dimethylaminopropylcarbodiimidehydrochloride (EDCI), in imidazole buffer at pH 6 to produce the 5'phosphorimidazolide. Contacting the 5'phosphorimidazolide with amine-containing molecules, such as an FGF, and ethylenediamine, results in stable phosphoramidates (*see, e.g*., Chu et al., *Nucleic Acids Res. 11*:6513-6529, 1983; and WO 88/05077 in which the U.S. is designated). In particular, a solution of DNA is saturated with EDC, at pH 6 and incubated with agitation at 4o C overnight. The resulting solution is then buffered to pH 8.5 by adding, for example about 3 volutes of 100 mM citrate buffer, and adding about 5 µg - about 20 µg of an FGF, and agitating the resulting mixture at 40°C for about 48 hours. The unreacted protein may be removed from the mixture by column chromatography using, for example, SEPHADEX G75 (Pharmacia) using 0.1 M ammonium carbonate solution, pH 7.0 as an eluting buffer. The isolated conjugate may be lyophilized and stored until used.

U.S. Patent No. 5,237,016 provides methods for preparing nucleotides that are bromacetylated at their 5' termini and reacting the resulting oligonucleotides with thiol groups. Oligonucleotides derivatized at their 5'-termini bromoacetyl groups can be prepared by reacting 5'-aminohexyl-phosphoramidate oligonucleotides with bromoacetic acid-N-hydroxysuccinimide ester as described in U.S. Patent No. 5,237,016. U.S. Patent No. 5,237,016 also describes methods for preparing thiol-derivatized nucleotides, which can then be reacted with thiol groups on the selected growth factor. Briefly, thiol-derivatized nucleotides are prepared using a 5'-phosphorylated nucleotide in two steps: (1) reaction of the phosphate group with imidazole in the presence of a diimide and displacement of the imidazole leaving group with cystamine in one reaction step; and reduction of the disulfide bond of the cystamine linker with dithiothreitol (*see, also,* Orgel et al., *Nucl. Acids Res. 14*:651, 1986, which describes a similar procedure). The 5'-phosphorylated starting oligonucleotides can be prepared by methods known to those of skill in the art (*see, e.g*., Maniatis et al., *Molecular Cloning: A Laboratory Manual*, Cold Spring Harbor Laboratory, New York, p. 122, 1982).

The antisense oligomer or nucleic acid, such as a methylphosphonate oligonucleotide (MP-oligomer), may be derivatized by reaction with SPDP or SMPB. The resulting MP-oligomer may be purified by HPLC and then coupled to an FGF, such as an FGF or FGF mutein, modified by replacement of one or more cysteine residues, as described above. The MP-oligomer (about 0.1 µM) is dissolved in about 40-50 µl of 1:1 acetonitrile/water to which phosphate buffer (pH 7.5, final concentration 0.1 M) and a 1 mg MP-oligomer in about 1 ml phosphate buffered saline is added. The reaction is allowed to proceed for about 5-10 hours at room temperature and is then quenched with about 15 µL 0.1 iodoacetamide. The FGF-oligonucleotide conjugates can be purified on heparin sepharose Hi Trap columns (1 ml, Pharmacia) and eluted with a linear or step gradient. The conjugate should elute in 0.6 M NaCl.

### E. Properties and use of the resulting chemical conjugates and fusion proteins

Using the methods and materials described above and in the Examples numerous chemical conjugates and fusion proteins have been synthesized. These include the following constructs:

**TABLE 4**

| FGF CONJUGATES | | |
|---|---|---|
| DESCRIPTION | Protein name | Plasmid Name |
| wild type chemical conjugate | CCFS1 | |
| mutein C78S chemical conjugate | CCFS2 | |
| mutein C96S chemical conjugate | CCFS3 | |
| mutein C96S Cys-Sap chemical conjugate | CCFS4 | |

| wild type fusion protein (FGF-Ala-Met-SAP) | FPFS1 | pZ1A, pZ1B, pZ1C, pZ1D, pZ1E, pZ1G, pZ1H, pZ1J |
|---|---|---|
| mutein C78S protein | FGF2-2 | |
| mutein C96S protein | FGF2-3 | |
| mutein C78 & C96S fusion protein | FPFS4 | pZ2B |
| mutein C78 & C96S fusion protein with cathepsin D substrate linker | FPFS5 | pZ3B |
| wild type fusion protein with D.T. Trypsin substrate linker | FPFS6 | pZ4B |
| wild type fusion protein with Gly₄Ser linker | FPFS7 | pZ6B |
| wild type fusion protein with (Gly₄Ser)₂ linker | FPFS8 | pZ7B |
| wild type fusion protein with cathepsin B substrate linker | FPFS9 | pZ5B |
| wild type fusion protein with Ser₄Gly linker | FPFS10 | pZ8B |
| wild type fusion protein with (Ser₄ Gly)₂ linker | FPFS11 | pZ9B |
| wild type fusion protein with (Ser₄Gly)₄ linker | FPFS12 | pZ10B |
| wild type fusion protein with (Gly₄Ser)₄ linker | FPFS13 | pZ11B |
| mutein C78 & C96S fusion protein with trypsin substrate linker | FPFS14 | pZ12B |
| FGF-Ala-Met-SAP-Ala-Met-Sap | FPFS16 | pZ13 B |
| wild type fusion protein (SAP-Ala-Met-FGF) | FPSF1 | pZ15B |
| SAP-(Gly₄Ser)₂-FGF | FPSF2 | pZ16B |
| SAP-AMEFELGTRGSSRVD-FGF-AM-SAP | FPSFS1 | pZ17B |
| FGF-poly-L-lysine | | |

Particular details of the syntheses of the constructs are set forth in EXAMPLES 7 and 11. The above constructs have been synthesized and have been or can be inserted into plasmids including pET 11 (with and without the T7 transcription terminator), pET 12 and pET 15 (INVITROGEN, San Diego), λpPL and pKK223-3 (PHARMACIA, P.L.) and derivatives of pKK223-3. The resulting plasmids have been and can be transformed into bacterial hosts including BL21, BL231(DE3)+pLYS S, HMS175(DE3), HMS175(DE3)+pLYS S (NOVAGEN, Madison, WI) and N4830(c1857) (*see,* Gottesman et al., *J. Mol. Biol. 140*:57-75, 1980, commercially available from PL Biochemicals, Inc., *also, see, e.g*., U.S. Patent Nos. 5,266,465, 5,260,223, 5,256,769, 5,256,769, 5,252,725, 5,250,296, 5,244,797, 5,236,828, 5,234,829, 5,229,273, 4,798,886, 4,849,350, 4,820,631 and 4,780,313). N4830 harbors a heavily deleted phage lambda prophage carrying the mutant c1857 temperature sensitive repressor and an active N gene.

The chemical conjugates exhibit comparable biological activities to the fusion proteins. For example, the FGF conjugates appear to exhibit potent anti-tumor activity. Weekly intravenous injections of bFGF-SAP conjugates (total dose 125 µg/kg) over four weeks in mice, with established SK-Mel-5 xenografts, resulted in a mean tumor volume that was 49% of the control volume. Modification of the weekly regiment to include cis-platin (5 mg/kg intraperitoneally once per week on the day following FGF-SAP treatment) resulted in a mean tumor volume at sixty days that was that was 23% of the controls. The combined treatment resulted in complete tumor remission in 10% of the treated mice. Similarly, administration of bFGF-SAP to mice with established PA-1 tumors (human ovarian teratocarcinoma), HT-1197 (human bladder carcinoma), and DU-145 (human prostate carcinoma) resulted in prolonged survival and statistically significant dose-related suppression in tumor growth.

Conjugates produced herein have been injected into rats and appear to have low toxicity. The fusions proteins FPFS1, FPFS4, and FPFS5 were injected at a dosage of 75µg/kg into groups of 4 rats on 3 successive days (r animals survived at day 4 as compared to 3 and 2 animals). In a second experiment, rats were injected with 75µg/kg on 3 successive days with either CCFS-SMPB (containing a non-cleavable linker), CCFS-LC (a long chain SPOP linker), FPFS1, FPFS6 (containing a trypsin sensitive linker or CCFS1. On day 7, only 1/4 animals in the group receiving FPFS1 had survived compared to 4/4 surviving in all other groups.

Moreover, the chemical conjugate and fusion protein bFGF-SAP demonstrated an anti-proliferative effect on smooth muscle cells in rabbit balloon injury models of restenosis (*see*, *also*, U.S. Patent No. 5,308,622) and on cultured fibroblasts for pterygii. Incubation of subconfluent cultures of pterygial fibroblasts with FGF-SAP yielded a dose and time dependent inhibition of cell growth as assayed by cell number, with a ID₅₀ of 50 and 5nM for 0.5 hr and 6 days exposure, respectively. bFGF-SAP was more cytotoxic than 5-fluorouricol and mitomycin C as compared by the ID₅₀ values. Therefore, these results suggest that intraoperative application of bFGF-SAP may be an effective adjunct for preventing and minimizing reoccurrence of pterygium after excision.

FGF-SAP was also applied to the episcleral surface in eyes of rabbits having posterior lip sclerectomy. One randomly selected eye in each of 7 surgically-treated rabbits had FGF-SAP applied for 10 minutes. Mean filtering bleb survival time was statistically significantly greater (p=0.03) for the treated eyes (15.3 ± 5.7 days) than for control eyes (10.2 ± 1.5 days). The conjunctival blebs in the treated eyes appeared significantly more avascular, although marked surrounding conjunctival hyperemia and chemoses were noted in all treated eyes. These results appear to demonstrate an inhibitory effect of FGF-SAP on wound healing following glaucoma filtering surgery.

In *in vitro* cytotoxicity assays, the conjugates and fusion proteins containing linkers demonstrate activity at least comparable to FPFS1 and CCFS1. The fusion protein FPFS4, containing a trypsin sensitive linker and FPFS1 or CCFS1 were tested for cytotoxicity on BHK-21 cells and SK-Mel-28 cells. In all experiments FPFS4 demonstrated similar cytotoxic activity. In a much larger test FPFS4, FPFS5 (cathepsin B linker), FPFS6 (gly₄ser linker), FPFS7 ((gly₄ser)₂ linker), FPFS8 (ser₄gly linker) and FPFS12 (mutein C965 with trypsin linker), CCFS3 and CCFS4 were assayed against FPFS1 and CCFS1 as standards on SK-MEL 28 and BHK-21 cells. FPFS4, 6, and 12 had ID₅₀ values equivalent to FPFS1. FPFS5, 7, and 8 had somewhat higher ID₅₀ values, generally 2-3 fold higher. The chemical conjugate CCFS4 performed as well or better than CCFS1, while CCFS3 performed equivalently. Taken together, these data suggest that conjugates and fusions containing linkers are at least as efficacious as conjugates without linkers.

### F. Formulation and administration of pharmaceutical compositions

The conjugates herein may be formulated into pharmaceutical compositions suitable for topical, local, intravenous and systemic application. For the ophthalmic uses herein, local administration, either by topical administration or by injection is preferred. Time release formulations are also desirable. Effective concentrations of one or more of the conjugates are mixed with a suitable pharmaceutical carrier or vehicle. The concentrations or amounts of the conjugates that are effective requires delivery of an amount, upon administration, that ameliorates the symptoms or treats the disease. Typically, the compositions are formulated for single dosage administration. Therapeutically effective concentrations and amounts may be determined empirically by testing the conjugates in known *in vitro* and *in vivo* systems, such as those described here; dosages for humans or other animals may then be extrapolated therefrom.

The conjugates herein are formulated into ophthamologically acceptable compositions and are applied to the affected area of the eye during or immediately after surgery. In particular, following excimer laser surgery, the composition is applied to the cornea; following trabeculectomy the composition is applied to the fistula; and following removal of pterygii the composition is applied to the cornea. The compositions may also be used to treat pterygii. The conjugates are applied during and immediately following surgery and may, if possible be applied post-operatively, until healing is complete. The compositions are applied as drops for topical and subconjunctival application or are injected into the eye for intraocular application. The compositions may also be absorbed to a biocompatible support, such as a cellulosic sponge or other polymer delivery device, and contacted with the affected area.

Upon mixing or addition of the conjugate(s) with the vehicle, the resulting mixture may be a solution, suspension, emulsion or the like. The form of the resulting mixture depends upon a number of factors, including the intended mode of administration and the solubility of the conjugate in the selected carrier or vehicle. The effective concentration is sufficient for ameliorating the symptoms of the disease, disorder or condition treated and may be empirically determined based upon *in vitro* and/or *in vivo* data, such as the data from the mouse xenograft model for tumors or rabbit ophthalmic model. If necessary, pharmaceutically acceptable salts or other derivatives of the conjugates may be prepared.

Pharmaceutical carriers or vehicles suitable for administration of the conjugates provided herein include any such carriers known to those skilled in the art to be suitable for the particular mode of administration. In addition, the conjugates may be formulated as the sole pharmaceutically active ingredient in the composition or may be combined with other active ingredients.

The conjugates can be administered by any appropriate route, for example, orally, parenterally, intravenously, intradermally, subcutaneously, or topically, in liquid, semi-liquid or solid form and are formulated in a manner suitable for each route of administration. Preferred modes of administration depend upon the indication treated. Dermatological and ophthalmologic indications will typically be treated locally; whereas, tumors and restenosis, will typically be treated by systemic, intradermal or intramuscular, modes of administration.

Therapeutically, *i.e*., ophthamologically, effective concentrations and amounts may be determined for each application herein empirically by testing the conjugates in known *in vitro* and *in vivo* systems (rabbit and baboon models), such as those described herein; dosages for humans or other animals may then be extrapolated therefrom. Demonstration that the conjugates prevent or inhibit proliferation of serum stimulated corneal keratocytes or fibroblasts explanted from eyes, as shown herein, and demonstration of any inhibition of proliferation of such tissues in rabbits should establish human efficacy. The rabbit eye model is a recognized model for studying the effects of topically and locally applied drugs (*see, e.g*., U.S. Patent Nos. 5,288,735, 5,263,992, 5,262,178, 5,256,408, 5,252,319, 5,238,925, 5,165,952; *see, also*, Mirate et al., *Curr. Eye Res. 1*:491-493, 1981).

The conjugate is included in the pharmaceutically acceptable carrier in an amount sufficient to exert a therapeutically useful effect in the absence of undesirable side effects on the patient treated. It is understood that number and degree of side effects depends upon the condition for which the conjugates are administered. For example, certain toxic and undesirable side effects are tolerated when treating life-threatening illnesses, such as tumors, that would not be tolerated when treating disorders of lesser consequence. The concentration of conjugate in the composition will depend on absorption, inactivation and excretion rates thereof, the dosage schedule, and amount administered as well as other factors known to those of skill in the art.

Typically a therapeutically effective dosage should produce a serum concentration of active ingredient of from about 0.1 ng/ml to about 50-100 µg/ml. The pharmaceutical compositions typically should provide a dosage of from about 0.01 mg to about 100 - 2000 mg of conjugate, depending upon the conjugate selected, per kilogram of body weight per day. For example, for treatment of restenosis a daily dosage of about between 0.05 and 0.5 mg/kg (based on FGF-SAP chemical conjugate or an amount of conjugate provided herein equivalent on a molar basis thereto) should be sufficient. Local application for ophthalmic disorders and dermatological disorders should provide about 1 ng up to 100 µg, preferably about 1 ng to about 10 µg, per single dosage administration. It is understood that the amount to administer will be a function of the conjugate selected, the indication treated, and possibly the side effects that will be tolerated.

The active ingredient may be administered at once, or may be divided into a number of smaller doses to be administered at intervals of time. It is understood that the precise dosage and duration of treatment is a function of the disease being treated and may be determined empirically using known testing protocols or by extrapolation from *in vivo* or *in vitro* test data. It is to be noted that concentrations and dosage values may also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed compositions.

Solutions or suspensions used for parenteral, intradermal, subcutaneous, or topical application can include any of the following components: a sterile diluent, such as water for injection, saline solution, fixed oil, polyethylene glycol, glycerine, propylene glycol or other synthetic solvent; antimicrobial agents, such as benzyl alcohol and methyl parabens; antioxidants, such as ascorbic acid and sodium bisulfite; chelating agents, such as ethylenediaminetetraacetic acid (EDTA); buffers, such as acetates, citrates and phosphates; and agents for the adjustment of toxicity such as sodium chloride or dextrose. Parental preparations can be enclosed in ampules, disposable syringes or multiple dose vials made of glass, plastic or other suitable material.

If administered intravenously, suitable carriers include physiological saline or phosphate buffered saline (PBS), and solutions containing thickening and solubilizing agents, such as glucose, polyethylene glycol, and polypropylene glycol and mixtures thereof. Liposomal suspensions may also be suitable as pharmaceutically acceptable carriers. These may be prepared according to methods known to those skilled in the art.

The conjugates may be formulated for local or topical application, such as for topical application to the skin and mucous membranes, such as in the eye, in the form of gels, creams, and lotions and for application to the eye or for intracisternal or intraspinal application. Such solutions, particularly those intended for ophthalmic use, may be formulated as 0.01% -10% isotonic solutions, pH about 5-7, with appropriate salts. The ophthalmic compositions may also include additional components. such as hyaluronic acid. The conjugates may be formulated as aerosols for topical application (*see*, *e*.*g*., U.S. Patent Nos. 4,044,126, 4,414,209, and 4,364,923).

The conjugates may be prepared with carriers that protect them against rapid elimination from the body, such as time release formulations or coatings. Such carriers include controlled release formulations, such as, but not limited to, implants and microencapsulated delivery systems, and biodegradable, biocompatible polymers, such as ethylene vinyl acetate, polyanhydrides, polyglycolic acid, polyorthoesters, polylactic acid and others. For example, the composition may be applied during surgery using a sponge, such as a commercially available surgical sponges (*see*, *e.g.,* U.S. Patent Nos. 3,956,044 and 4,045,238; available from Weck, Alcon, and Mentor), that has been soaked in the composition and that releases the composition upon contact with the eye. These are particularly useful for application to the eye for ophthalmic indications following or during surgery in which only a single administration is possible. The compositions may also be applied in pellets (such as Elvax pellets(ethylene-vinyl acetate copolymer resin); about 1- 5 µg of conjugate per 1 mg resin) that can be implanted in the eye during surgery.

If oral administration is desired, the conjugate should be provided in a composition that protects it from the acidic environment of the stomach. For example, the composition can be formulated in an enteric coating that maintains its integrity in the stomach and releases the active compound in the intestine. The composition may also be formulated in combination with an antacid or other such ingredient.

Oral compositions will generally include an inert diluent or an edible carrier and may be compressed into tablets or enclosed in gelatin capsules. For the purpose of oral therapeutic administration, the active compound or compounds can be incorporated with excipients and used in the form of tablets, capsules or troches. Pharmaceutically compatible binding agents and adjuvant materials can be included as part of the composition.

The tablets, pills, capsules, troches and the like can contain any of the following ingredients, or compounds of a similar nature: a binder, such as microcrystalline cellulose, gum tragacanth and gelatin; an excipient such as starch and lactose, a disintegrating agent such as, but not limited to, alginic acid and corn starch; a lubricant such as, but not limited to, magnesium stearate: a glidant, such as, but not limited to, colloidal silicon dioxide; a sweetening agent such as sucrose or saccharin; and a flavoring agent such as peppermint, methyl salicylate, and fruit flavoring.

When the dosage unit form is a capsule, it can contain, in addition to material of the above type, a liquid carrier such as a fatty oil. In addition, dosage unit forms can contain various other materials which modify the physical form of the dosage unit, for example, coatings of sugar and other enteric agents. The conjugates can also be admimstered as a component of an elixir, suspension, syrup, wafer, chewing gum or the like. A syrup may contain, in addition to the active compounds, sucrose as a sweetening agent and certain preservatives, dyes and colorings and flavors.

The conjugates may be formulated for local or topical application, such as for topical application to the skin and mucous membranes, such as in the eye, in the form of gels, creams, and lotions and for application to the eye. Such solutions, particularly those intended for ophthalmic use, may be formulated as 0.01%-10% isotonic solutions, pH about 5-7, with appropriate salts. Suitable ophthalmic solutions are known (*see, e.g.*, U.S. Patent No. 5,116,868, which describes typical compositions of ophthalmic irrigation solutions and solutions for topical application). Such solutions, which have a pH adjusted to about 7.4, contain, for example, 90-100 mM sodium chloride, 4-6 mM dibasic potassium phosphate, 4-6 mM dibasic sodium phosphate, 8-12 mM sodium citrate, 0.5-1.5 mM magnesium chloride, 1.5-2.5 mM calcium chloride, 15-25 mM sodium acetate, 10-20 mM D.L.-sodium β-hydroxybutyrate and 5-5.5 mM glucose.

The active materials can also be mixed with other active materials, that do not impair the desired action, or with materials that supplement the desired action, including viscoelastic materials, such as hyaluronic acid, which is sold under the trademark HEALON (solution of a high molecular weight (MW of about 3 millions) fraction of sodium hyaluronate; manufactured by Pharmacia, Inc. *see, e.g*., U.S. Patent Nos. 5,292,362, 5,282,851, 5,273,056, 5,229,127, 4,517,295 and 4,328,803), VISCOAT (fluorine-containing (meth)acrylates, such as, 1H,1H,2H,2H-hepta-decafluorodecylmethacrylate; *see, e.g.,* U.S. Patent Nos. 5,278,126, 5,273,751 and 5,214,080; commercially available from Alcon Surgical, Inc.), ORCOLON (*see, e.g.,* U.S. Patent Nos. 5,273,056; commercially available from Optical Radiation Corporation), methylcellulose, methyl hyaluronate, polyacrylamide and polymethacrylamide (*see, e.g.,* U.S. Patent No. 5,273,751). The viscoelastic materials are present generally in amounts ranging from about 0.5 to 5.0%, preferably 1 to 3% by weight of the conjugate material and serve to coat and protect the treated tissues. The compositions may also include a dye, such as methylene blue or other inert dye, so that the composition can be seen when injected into the eye or contacted with the surgical site during surgery.

Ophthamologically effective concentrations or amounts of one or more of the conjugates are mixed with a suitable pharmaceutical carrier or vehicle. The concentrations or amounts of the conjugates that are effective requires delivery of an amount, upon administiation, that prevents or substantially reduces corneal clouding, trabeculectomy closure, or pterygii recurrence.

The ophthalmologic indications herein are typically be treated locally either by the application of drops to the affected tissue(s), contacting with a biocompatible sponge that has absorbed a solution of the conjugates or by injection of a composition. For the indications herein, the composition will be applied during or immediately after surgery in order to prevent closure of the trabeculectomy, prevent a proliferation of keratocytes following excimer laser surgery, or to prevent a recurrence of pterygii. The composition may also be injected into the affected tissue following surgery and applied in drops following surgery until healing is completed. For example, to administer the formulations to the eye, it can be slowly injected into the bulbar conjunctiva of the eye.

Conjugates with photocleavable linkers are among those preferred for use in the methods herein. Upon administration of such composition to the affected area of the eye, the eye is exposed to light of a wavelength, typically visible or UV that cleaves the linker, thereby releasing the cytotoxic agent.

The active materials can also be mixed with other active materials that do not impair the desired action, or with materials that supplement the desired action, such as cis-platin for treatment of tumors.

Finally, the compounds may be packaged as articles of manufacture containing packaging material, one or more conjugates or compositions as provided herein within the packaging material, and a label that indicates the indication for which the conjugate is provided.

The following examples are included for illustrative purposes only and are not intended to limit the scope of the invention.

### EXAMPLE 1

### RECOMBINANT PRODUCTION OF SAPORIN

### A. Materials and methods

### 1. Bacterial Strains

*E. coli* strain JA221 (lpp⁻ hdsM+ trpE5 leuB6 lacY recA1 F'[lacI^{q} lac⁺ pro⁺]) is publicly available from the American Type Culture Collection (ATCC), Rockville, MD 20852, under the accession number ATCC 33875. (JA221 is also available from the Northern Regional Research Center (NRRL), Agricultural Research Service, U.S. Department of Agriculture, Peoria, IL 61604, under the accession number NRRL B-15211; *see, also*, U.S. Patent No. 4,757,013 to Inouye; and Nakamura et al., *Cell 18*:1109-1117, 1979). Strain INV1α is commercially available from Invitrogen, San Diego, CA.

### 2. DNA Manipulations

The restriction and modification enzymes employed herein are commercially available in the U.S. Native saporin and rabbit polyclonal antiserum to saporin were obtained as previously described in Lappi et al., *Biochem. Biophys. Res. Comm. 129*:934-942. Ricin A chain is commercially available from SIGMA, Milwaukee, WI. Antiserum was linked to Affi-gel 10 (BIO-RAD, Emeryville, CA) according to the manufacturer's instructions. Sequencing was performed using the Sequenase kit of United States Biochemical Corporation (version 2.0) according to the manufacturer's instructions. Minipreparation and maxipreparation of plasmids, preparation of competent cells, transformation, M13 manipulation, bacterial media. Western blotting, and ELISA assays were according to Sambrook et al., (*Molecular Cloning: A Laboratory Manual*, Cold Spring Harbor Laboratory Press, Cold Spring Harbor, NY, 1989). The purification of DNA fragments was done using the Geneclean II kit (Bio 101) according to the manufacturer's instructions. SDS gel electrophoresis was performed on a Phastsystem (Pharmacia).

Western blotting was accomplished by transfer of the electrophoresed protein to nitrocellulose using the PhastTransfer system, as described by the manufacturer. The antiserum to SAP was used at a dilution of 1:1000. Horseradish peroxidase labeled anti-IgG was used as the second antibody (*see* Davis et al., *Basic Methods In Molecular Biology,* New York, Elsevier Science Publishing Co., pp 1-338, 1986).

### B. Isolation of DNA encoding saporin

### 1. Isolation of genomic DNA and preparation of polymerase chain reaction (PCR) primers

*Saponaria officinalis* leaf genomic DNA was prepared as described in Bianchi et al., *Plant Mol. Biol. 11*:20-214, 1988. Primers for genomic DNA amplifications were synthesized in a 380B automatic DNA synthesizer. The primer corresponding to the "sense" strand of saporin (SEQ ID NO. 1) includes an *Eco*R I restriction site adapter immediately upstream of the DNA codon for amino acid -15 of the native saporin N-terminal leader sequence (SEQ ID NO. 1): 5'-CTGCAGAATTCGCATGGATCCTGCTTCAAT-3'. The primer 5'-CTGCAGAATTCGCCTCGTTTGACTACTTTG-3' (SEQ ID NO. 2) corresponds to the "antisense" strand of saporin and complements the coding sequence of saporin starting from the last 5 nucleotides of the DNA encoding the carboxyl end of the mature peptide. Use of this primer introduced a translation stop codon and an *Eco*RI restriction site after the sequence encoding mature saporin.

### 2. Amplification of DNA encoding saporin

Unfractionated *Saponaria officinalis* leaf genomic DNA (1 µl) was mixed in a final volume of 100 µl containing 10 mM Tris-HCl (pH 8.3), 50 mM KCl, 0.01% gelatin, 2 mM MgCl₂, 0.2 mM dNTPs, 0.8 µg of each primer. Next, 2.5 U TaqI DNA polymerase (Perkin Elmer Cetus) was added and the mixture was overlaid with 30 µl of mineral oil (Sigma). Incubations were done in a DNA Thermal Cycler (Ericomp). One cycle included a denaturation step (94OC for 1 min.), an annealing step (60OC for 2 min.), and an elongation step (72OC for 3 min.). After 30 cycles, a 10 µl aliquot of each reaction was run on a 1.5% agarose gel to verify the correct structure of the amplified product.

The amplified DNA was digested with *Eco*RI and subcloned into *Eco*R I-restricted M13mp18 (NEW ENGLAND BIOLABS, Beverly, MA; *see, also,* Yanisch-Perron et al. (1985), "Improved M13 phage cloning vectors and host strains: Nucleotide sequences of the M13mp18 and pUC19 vectors", *Gene 33*:103). Single-stranded DNA from recombinant phages was sequenced using oligonucleotides based on internal points in the coding sequence of saporin (*see*, Bennati et al., *Eur*. *J.* *Biochem. 183*:465-470, 1989). Nine of the M13mp18 derivatives were sequenced and compared. Of the nine sequenced clones, five had unique sequences, set forth as SEQ ID NOS. 3-7, respectively. The clones were designated M13mp18-G4, -G1, -G2, -G7, and -G9. Each of these clones contains all of the saporin coding sequence and 45 nucleotides of DNA encoding the native saporin N-terminal leader peptide.

### C. pOMPAG4 Plasmid Construction

M13 mp18-G4, containing the SEQ ID NO. 3 clone from Example 1.B.2., was digested with *Eco*R I, and the resulting fragment was ligated into the *Eco*R I site of the vector pIN-IIIompA2 (*see*, *e*.*g*., *see,* U.S. Patent No. 4,575,013 to Inouye; and Duffaud et al., *Meth. Enz. 153*:492-507, 1987) using the methods described in Example 1.A.2. The ligation was accomplished such that the DNA encoding saporin, including the N-terminal extension, was fused to the leader peptide segment of the bacterial ompA gene. The resulting plasmid pOMPAG4 contains the lpp promoter (Nakamura et al., *Cell 18*:1109-1117, 1987), the *E*. *coli* lac promoter operator sequence (lac O) and the *E. coli* ompA gene secretion signal in operative association with each other and with the saporin and native N-terminal leader-encoding DNA listed in SEQ ID NO. 3. The plasmid also includes the *E*. *coli* lac repressor gene (lac I).

The M13 mp18-G1, -G2, -G7, and -G9 clones obtained from Example 1.B.2, containing SEQ ID NOS. 4-7 respectively, are digested with *Eco*R I and ligated into *Eco*R I digested pIN-IIIompA2 as described for M13 mp18-G4 above in this example. The resulting plasmids, labeled pOMPAG1, pOMPAG2, pOMPAG7, pOMPA9, are screened, expressed, purified, and characterized as described for the plasmid pOMPAG4.

INV1α competent cells were transformed with pOMPAG4 and cultures containing the desired plasmid structure were grown further in order to obtain a large preparation of isolated pOMPAG4 plasmid using methods described in Example 1.A.2.

### D. Saporin expression in E. coli

The pOMPAG4 transformed *E. coli* cells were grown under conditions in which the expression of the saporin-containing protein is repressed by the lac repressor to an O.D. in or at the end of the log phase of growth after which IPTG was added to induce expression of the saporin-encoding DNA.

To generate a large-batch culture of pOMPAG4 transformed *E. coli* cells, an overnight culture (lasting approximately 16 hours) of JA221 *E. coli* cells transformed with the plasmid pOMPAG4 in LB broth (*see e.g.,* Sambrook et al., *Molecular Cloning: A Laboratory Manual*, Cold Spring Harbor Laboratory Press, Cold Spring Harbor, NY, 1989) containing 125 mg/ml ampicillin was diluted 1:100 into a flask containing 750 ml LB broth with 125 mg/ml ampicillin. Cells were grown at logarithmic phase shaking at 37OC until the optical density at 550 nm reached 0.9 measured in a spectrophotometer.

In the second step, saporin expression was induced by the addition of IPTG (Sigma) to a final concentration of 0.2 mM. Induced cultures were grown for 2 additional hours and then harvested by centrifugation (25 min., 6500 x g). The cell pellet was resuspended in ice cold 1.0 M TRIS, pH 9.0, 2 mM EDTA (10 ml were added to each gram of pellet). The resuspended material was kept on ice for 20-60 minutes and then centrifuged (20 min., 6500 x g) to separate the periplasmic fraction of *E. coli*, which corresponds to the supernatant, from the intracellular fraction corresponding to the pellet.

### E. Purification of secreted recombinant Saporin

### 1. Anti-SAP immuno-affinity purification

The periplasmic fraction from Example 1.D. was dialyzed against borate-buffered saline (BBS: 5 mM boric acid, 1.25 mM borax, 145 mM sodium chloride, pH 8.5). The dialysate was loaded onto an immunoaffinity column (0.5 x 2 cm) of anti-saporin antibodies, obtained as described in Lappi et al., *Biochem. Biophys. Res. Comm., 129*:934-942, 1985, bound to Affi-gel 10 and equilibrated in BBS at a flow rate of about 0.5 ml/min. The column was washed with BBS until the absorbance at 280 nm of the flow-through was reduced to baseline. Next the column containing the antibody bound saporin was eluted with 1.0 M acetic acid and 0.5 ml fractions were collected in tubes containing 0.3 ml of 2 M ammonium hydroxide, pH 10. The fractions were analyzed by ELISA (*see, e.g*., Sambrook et al., *Molecular Cloning: A Laboratory Manual,* Cold Spring Harbor Laboratory Press, Cold Spring Harbor, NY, 1989). The peak fraction of the ELISA was analyzed by Western blotting as described in Example 1.A.2 and showed a single band with a slightly higher molecular weight than native saporin. The fractions that contained saporin protein, as determined by the ELISA, were then pooled for further purification.

### 2. Reverse Phase High Performance Liquid Chromatography purification

To further purify the saporin secreted into the periplasm, the pooled fractions from Example 1.E.1. were diluted 1:1 with 0.1% trifluoroacetic acid (TFA) in water and chromatographed in reverse phase high pressure liquid chromatography (HPLC) on a Vydac C4 column (Western Analytical) equilibrated in 20% acetonitrile, 0.1% TFA in water. The protein was eluted with a 20 minute gradient to 60% acetonitrile. The HPLC produced a single peak that was the only area of immunoreactivity with anti-SAP antiserum when analyzed by a western blot as described in Example 1.E.1. Samples were assayed by an ELISA.

Sequence analysis was performed by Edman degradation in a gas-phase sequenator (Applied Biosystems) (*see, e.g*., Lappi et al., *Biochem. Biophys. Res. Comm. 129*:934-942, 1985). The results indicated that five polypeptides were obtained that differ in the length, between 7 and 12 amino acids, of the N-terminal saporin leader before the initial amino acid valine of the mature native saporin (SEQ ID NO.3: residue -12 through -7). All of the N-terminal extended variants retained cytotoxic activity. The size of the native leader is 18 residues, indicating that the native signal peptide is not properly processed by bacterial processing enzymes. The ompA signal was, however, properly processed.

To obtain homogeneous saporin, the recombinantly produced saporin can be separated by size and one of the five polypeptides used to produce the conjugates.

### F. Purification of intracellular soluble saporin

To purify the cytosolic soluble saporin protein, the pellet from the intracellular fraction of Example 1.E. above was resuspended in lysis buffer (30 mM TRIS, 2 mM EDTA, 0.1% Triton X-100, pH 8.0, with 1 mM PMSF, 10 µg/ml pepstatin A, 10 µg aprotinin, µg/ml leupeptin and 100 µg/ml lysozyme, 3.5 ml per gram of original pellet). To lyse the cells, the suspension was left at room temperature for one hour, then frozen in liquid nitrogen and thawed in a 37OC bath three times, and then sonicated for two minutes. The lysate was centrifuged at 11,500 x g for 30 min. The supernatant was removed and stored. The pellet was resuspended in an equal volume of lysis buffer, centrifuged as before, and this second supernatant was combined with the first. The pooled supernatants were dialyzed versus BBS and chromatographed over the immunoaffinity column as described in Example 1.E.1. This material also retained cytotoxic activity.

### G. Assay for cytotoxic activity

The RIP activity of recombinant saporin was compared to the RIP activity of native SAP in an *in vitro* assay measuring cell-free protein synthesis in a nuclease-treated rabbit reticulocyte lysate (Promega). Samples of immunoaffinity-purified saporin, obtained in Example 1.E.1., were diluted in PBS and 5 µl of sample was added on ice to 35 µl of rabbit reticulocyte lysate and 10 µl of a reaction mixture containing 0.5 µl of Brome Mosaic Virus RNA, 1 mM amino acid mixture minus leucine, 5 µCi of tritiated leucine and 3 µl of water. Assay tubes were incubated 1 hour in a 30OC water bath. The reaction was stopped by transferring the tubes to ice and adding 5 µl of the assay mixture, in triplicate, to 75 µl of 1 N sodium hydroxide, 2.5% hydrogen peroxide in the wells of a Millititer HA 96-well filtration plate (Millipore). When the red color had bleached from the samples, 300 µl of ice cold 25% trichloroacetic acid (TCA) were added to each well and the plate left on ice for another 30 min. Vacuum filtration was performed with a Millipore vacuum holder. The wells were washed three times with 300 µl of ice cold 8% TCA. After drying, the filter paper circles were punched out of the 96-well plate and counted by liquid scintillation techniques.

The IC₅₀ for the recombinant and native saporin were approximately 20 pM. Therefore, recombinant saporin-containing protein has full protein synthesis inhibition activity when compared to native saporin.

### EXAMPLE 2

### PREPARATION OF MONO-DERIVATIZED SAPORIN

### A. Materials and Methods

### 1. Reagents

Restriction and modification enzymes were purchased from BRL (Gaithersburg, MD), Stratagene (La Jolla, CA) and New England Biolabs (Beverly, MA). Native SAP was obtained from *Saponaria officinalis* (*see*, *e*.*g*., Stirpe et al., *Biochem*. *J. 216*:617-625, 1983). Briefly, the seeds were extracted by grinding in 5 mM sodium phosphate buffer, pH 7.2 contining 0.14 M NaCl, straining the extracts through cheesecloth, followed by centrifugation at 28,00 g for 30 min to produce a crude extract, which was dialyzed against 5 mM sodium phosphate buffer, pH 6.5, centrifuged and applied to CM-cellulose (CM 52, Whatman, Maidstone, Kent, U.K.). The CM column was washed and SO-6 was eluted with a 0-0.3 M NaCl gradient in the phosphate buffer.

Plasmid isolation, production of competent cells, transformation and M13 manipulations were carried out according to published procedures (Sambrook et al. (1989) Molecular Cloning, a Laboratory Manual Cold Spring Harbor Laboratory Press, Cold Spring Harbor, NY). Purification of DNA fragments was achieved using the Geneclean II kit, purchased from Bio 101 (La Jolla, CA). Sequencing of the different constructions was performed using the Sequenase kit (version 2.0) of USB (Cleveland, OH).

### 2. Bacterial strains

### Novablue and BL21(DE3) (NOVAGEN, Madison WI)

### 3. Sodium dodecyl sulfate (SDS) gel electrophoresis and Western blotting.

SDS gel electrophoresis was performed on a PhastSystem utilizing 20% gels (Pharmacia). Western blotting was accomplished by transfer of electrophoresed protein to nitrocellulose using the PhastTransfer system (Pharmacia), as described by the manufacturer. The antisera to SAP and basic FGF were used at a dilution of 1:1000. Horseradish peroxidase labeled anti-IgG was used as the second antibody as described (Davis, L., Dibner et al., *Basic Methods in Molecular Biology*, p. 1, Elsevier Science Publishing Co., New York, 1986).

### 4. Cytotoxicity assays of conjugates.

Cytotoxicity experiments were performed with the Promega (Madison, WI) CellTiter 96 Cell Proliferation/Cytotoxicity Assay Cell types used were SK-Mel-28, human melanoma Swiss 3T3 mouse fibroblasis (from Dr. Pamela Maher, La Jolla. CA), B16F10, mouse melanoma, PA-1, human ovarian carcinoma (from Dr. Julie Beitz, Roger Williams Hospital, Providence RI), and baby hamster kidney (BHK) (obtained from the American Type Culture Collection (ATCC)). 2500 cells were plated per well.

### B. Derivatization and purification of mono-derivatized SAP

Saporin (49 mg) at a concentration of 4.1 mg/ml was dialyzed against 0.1 M sodium phosphate, 0.1 M sodium chloride, pH 7.5. A 1.1 molar excess (563 µg in 156 µl of anhydrous ethanol) of SPDP (Pharmacia, Uppsala, Sweden) was added and the reaction mixture immediately agitated and put on a rocker platform for 30 minutes. The solution was then dialyzed against the same buffer. An aliquot of the dialyzed solution was examined for extent of derivatization according to the Pharmacia instruction sheet. The extent of derivatization was 0.86 moles of SPDP per mole of SAP. During these experiments, another batch of SAP was derivatized using an equimolar quantity of SPDP in the reaction mixture with a resulting 0.79 molar ratio of SPDP to SAP.

Derivatized SAP (32.3 mg) was dialyzed in 0.1 M sodium borate, pH 9.0 and applied to a Mono S 16/10 column equilibrated with 25 mM sodium chloride in dialysis buffer. A gradient of 25 mM to 125 mM sodium chloride in dialysis buffer was run to elute SAP and derivatized SAP. The flow rate was 4.0 ml/min. and 4 ml fractions were collected. Aliquots of fractions were assayed for protein concentration (BCA Protein Assay, Pierce Chemical, Chicago, IL) and for pyridylthione released by reducing agent. Individual fractions (25 to 37) were analyzed for protein concentration and pyridyl-disulfide concentration. The data indicated a separation according to the level of derivatization by SPDP. The initial eluting peak was composed of SAP that is approximately di-derivatized; the second peak is mono-derivatized and the third peak shows no derivatization. The di-derivatized material accounts for 20% of the three peaks; the second accounts for 48% and the third peak contains 32%. Material from the second peak was pooled and gave an average ratio of pyridyl-disulfide to SAP of 0.95. Fraction 33, which showed a divergent ratio of pyridine-2-thione to protein, was excluded from the pool. Fractions that showed a ratio of SPDP to SAP greater than 0.85 but less than 1.05 were pooled, dialyzed against 0.1 M sodium chloride, 0.1 M sodium phosphate, pH 7.5 and used for derivatization with basic FGF. A pool of these materials had a molar ratio SPDP:SAP of 0.9 with a final yield of 4.6 mg.

### EXAMPLE 3

### RECOMBINANT PRODUCTION OF FGF-SAP FUSION PROTEIN

### A. General Descriptions

### 1. Bacterial Strains and Plasmids:

*E. coli* strains BL21(DE3), BL21(DE3)pLysS, HMS174(DE3) and HMS174(DE3)pLysS were purchased from NOVAGEN, Madison, WI. Plasmid pFC80, described below, has been described in the WIPO International Patent Application No. WO 90/02800, except that the bFGF coding sequence in the plasmid designated pFC80 herein has the sequence set forth as SEQ ID NO. 12, nucleotides 1-465. The plasmids described herein may be prepared using pFC80 as a starting material or, alternatively, by starting with a fragment containing the CII ribosome binding site (SEQ ID NO. 15) linked to the FGF-encoding DNA (SEQ ID NO. 12).

*E. coli* strain JA221 (lpp- hdsM+ trpE5 leuB6 lacY recA1 F'[lacI^{q} lac⁺ pro⁺]) is publicly available from the American Type Culture Collection (ATCC), Rockville, MD 20852, under the accession number ATCC 33875. (JA221 is also available from the Northern Regional Research Center (NRRL), Agricultural Research Service, U.S. Department of Agriculture, Peoria, IL 61604, under the accession number NRRL B-15211; *see, also*, U.S. Patent No. 4,757,013 to Inouye; and Nakamura et al., *Cell 18*:1109-1117, 1979). Strain INV1α is commercially available from Invitrogen, San Diego, CA.

### 2. DNA Manipulations

The restriction and modification enzymes employed here are commercially available in the U.S. Native SAP, chemically conjugated bFGF-SAP and rabbit polyclonal antiserum to SAP and FGF were obtained as described in Lappi et al., *Biochem. Biophys. Res*. *Comm. 129*:934-942, 1985, and Lappi et al., *Biochem. Biophys., Res. Comm. 160*:917-923, 1989. The pET System Induction Control was purchased from NOVAGEN, Madison, WI. The sequencing of the different constructions was done using the Sequenase kit of United States Biochemical Corporation (version 2.0). Minipreparation and maxipreparations of plasmids, preparation of competent cells, transformation, M13 manipulation, bacterial media and Western blotting were performed using routine methods (*see, e.g.*, Sambrook et al., *Molecular Cloning: A Laboratory Manual*, Cold Spring Harbor Laboratory Press, Cold Spring Harbor, NY, 1989). The purification of DNA fragments was done using the Geneclean II kit, purchased from Bio 101. SDS gel electrophoresis was performed on a Phastsystem (Pharmacia).

Rabbit polyclonal antiserum to SAP and FGF were obtained as described in Lappi et al., *Biochem. Biophys. Res. Comm. 129*:934-942, 1985. and Lappi et al., *Biochem. Biophys., Res. Comm. 160*:917-923, 1989. The pET System Induction Control was purchased from NOVAGEN, Madison, WI. Minipreparation and maxipreparations of plasmids, preparation of competent cells, transformation, M13 manipulation, bacterial media and Western blotting were performed using routine methods (*see, e.g*., Sambrook et al., *Molecular Cloning: A Laboratory Manual*, Cold Spring Harbor Laboratory Press, Cold Spring Harbor, NY, 1989). The purification of DNA fragments was done using the Geneclean II kit, purchased from Bio 101. SDS gel electrophoresis was performed on a Phastsystem (Pharmacia).

Western blotting was accomplished by transfer of the electrophoresed protein to nitrocellulose using the PhastTransfer system, as described by the manufacturer. Horseradish peroxidase labeled anti-IgG was used as the second antibody (*see* Davis et al., *Basic Methods In Molecular Biology*. New York, Elsevier Science Publishing Co., pp. 1-338, 1986).

### B. Construction of plasmids encoding FGF-SAP fusion proteins

### 1. Construction of FGFM13 that contains DNA encoding the CI ribosome binding site linked to FGF

A Nco I restriction site was introduced into the SAP-encoding DNA the M13mp18-G4 clone, prepared as described in Example 1.B.2. by site-directed mutagenesis method using the Amersham *In vitro*-mutagenesis system 2.1. The oligonucleotide employed to create the *Nco* I restriction site was synthesized using a 380B automatic DNA synthesizer (Applied Biosystems) and is listed as: SEQ ID NO. 8 - CAACAACTGCCATGGTCACATC. This oligonucleotide containing the *Nco* I site replaced the original SAP-containing coding sequence at SEQ ID NO.3, nts 32-53. The resulting M13mp18-G4 derivative is termed mpNG4.

In order to produce a bFGF coding sequence in which the stop codon was removed, the FGF-encoding DNA was subcloned into a M13 phage and subjected to site-directed mutagenesis. Plasmid pFC80 is a derivative of pDS20 (*see, e.g*., Duester et al., *Cell 30*:855-864, 1982; *see, also*, U.S. Patent Nos. 4,914,027, 5,037,744, 5,100,784, and 5,187,261; *see, also*, PCT International Application No. WO 90/02800; and European Patent Application No. EP 267703 A1), which is almost the same as plasmid pKG1800 (*see*, Bernardi et al., *DNA Sequence 1*:147-150, 1990; *see, also*, McKenney et al. (1981) pp. 383-415 in *Gene Amplification and Analysis 2: Analysis of Nucleic Acids by Enzymatic Methods*, Chirikjian et al. (eds.), North Holland Publishing Company, Amsterdam) except that it contains an extra 440 bp at the distal end of *galK* between nucleotides 2440 and 2880 in pDS20. Plasmid pKG1800 includes the 2880 bp *Eco*R I-*Pvu* II of pBR322 that contains the contains the ampicillin resistance gene and an origin of replication.

Plasmid pFC80 was prepared from pDS20 by replacing the entire *galK* gene with the FGF-encoding DNA of SEQ ID NO. 12, inserting the trp promoter (SEQ ID NO. 14) and the bacteriophage lambda CII ribosome binding site (SEQ. ID No. 15; *see, e.g.,* Schwarz et al., *Nature 272*:410, 1978) upstream of and operatively linked to the FGF-encoding DNA. The Trp promoter can be obtained from plasmid pDR720 (Pharmacia PL Biochemicals) or synthesized according to SEQ ID NO. 14. Plasmid pFC80, contains the 2880 bp *Eco*R I-*Bam*H I fragment of plasmid pSD20, a synthetic *Sal* I*-Nde* I fragment that encodes the Trp promoter region (SEQ ID NO. 14):

EcoR I
AATTCCCCTGTTGACAATTAATCATCGAACTAGTTAACTAGTACGCAGCTTGGCTGCAG
and the CII ribosome binding site (SEQ ID NO. 15)):

Sal I Nde I
GTCGACCAAGCTTGGGCATACATTCAATCAATTGTTATCTAAGGAAATACTTACATATG

The FGF-encoding DNA was removed from pFC80 by treating it as follows. The pFC80 plasmid was digested by *Hga* I and *Sal* I, which produces a fragment containing the CII ribosome binding site linked to the FGF-encoding DNA. The resulting fragment was blunt ended with Klenow's reagent and inserted into M13mp18 that had been opened by Sma I and treated with alkaline phosphatase for blunt-end ligation. In order to remove the stop codon, an insert in the ORI minus direction was mutagenized using the Amersham kit, as described above, using the following oligonucleotide (SEQ ID NO. 9): GCTAAGAGCGCCATGGAGA. SEQ ID NO. 9 contains one nucleotide between the FGF carboxy terminal serine codon and a *Nco* I restriction site; it replaced the following wild type FGF encoding DNA having SEQ ID NO. 10:

GCT AAG AGC TGA  CCA TGG AGA[?]
Ala Lys Ser STOP Pro Trp Arg

The resulting mutant derivative of M13mp18, lacking a native stop codon after the carboxy terminal serine codon of bFGF, was designated FGFM13. The mutagenized region of FGFM13 contained the correct sequence (SEQ ID NO. 11).

### 2. Preparation of plasmids pFS92 (PZ1A), PZ1B and PZ1C that encode the FGF-SAP fusion protein (FPFS1)

### a. Plasmid pFS92 (also designated PZ1A)

Plasmid FGFM13 was cut with *Nco* I and *Sac* I to yield a fragment containing the CII ribosome binding site linked to the bFGF coding sequence with the stop codon replaced.

The M13mp18 derivative mpNG4 containing the saporin coding sequence was also cut with restriction endonucleases *Nco* I and *Sac* I, and the bFGF coding fragment from FGFM13 was inserted by ligation to DNA encoding the fusion protein bFGF-SAP into the M13mp18 derivative to produce mpFGF-SAP, which contains the CII ribosome binding site linked to the FGF-SAP fusion gene. The sequence of the fusion gene is set forth in SEQ ID NO. 12 and indicates that the FGF protein carboxy terminus and the saporin protein amino terminus are separated by 6 nucleotides (SEQ ID NOS. 12 and 13, nts 466-471) that encode two amino acids Ala Met.

Plasmid mpFGF-SAP was digested with *Xba* I and *Eco*R I and the resulting fragment containing the bFGF-SAP coding sequence was isolated and ligated into plasmid pET-11a (available from NOVAGEN, Madison, WI; for a description of the plasmids see U.S. Patent No. 4,952,496; *see, also*, Studier et al., *Meth. Enz. 185*:60-89, 1990; Studier et al., *J. Mol. Biol. 189*:113-130, 1986; Rosenberg et al., *Gene 56*:125-135, 1987) that had also been treated with *Eco*R I and *Xba* I. The resulting plasmid was designated pFS92. It was renamed PZ1A.

Plasmid pFS92 (or PZ1A) contains DNA the entire basic FGF protein (SEQ ID NO. 12), a 2-amino acid long connecting peptide, and amino acids 1 to 253 of the mature SAP protein. Plasmid pFS92 also includes the CII ribosome binding site linked to the FGF-SAP fusion protein and the T7 promoter region from pET-11a.

*E. coli* strain BL21(DE3)pLysS (NOVAGEN, Madison WI) was transformed with pFS92 according to manufacturer's instructions and the methods described in Example 2.A.2.

### b. Plasmid PZ1B

Plasmid pFS92 was digested with *Eco*R I, the ends repaired by adding nucleoside triphosphates and Klenow DNA polymerase, and then digested with *Nde* I to release the FGF-encoding DNA without the CII ribosome binding site. This fragment was ligated into pET 11a, which had been *Bam*H I digested, treated to repair the ends, and digested with *Nde* I. The resulting plasmid was designated PZ1B. PZ1B includes the T7 transcription terminator and the pET-11a ribosome binding site.

*E. coli* strain BL21(DE3) (NOVAGEN, Madison WI) was transformed with PZ1B according to manufacturer's instructions and the methods described in Example 2.A.2.

### c. Plasmid PZ1C

Plasmid PZ1C was prepared from PZ1B by replacing the ampicillin resistance gene with a kanarnycin resistance gene.

### d. Plasmid PZ1D

Plasmid pFS92 was digested with *Eco*R I and *Nde* I to release the FGF-encoding DNA without the CII ribosome binding site and the and the ends were repaired. This fragment was ligated into pET 12a, which had been *Bam*H I digested and treated to repair the ends. The resulting plasmid was designated PZ1D. PZ1D includes DNA encoding the OMP T secretion signal operatively linked to DNA encoding the fusion protein.

*E. coli* strains BL21(DE3), BL21(DE3)pLysS, HMS174(DE3) and HMS174(DE3)pLysS (NOVAGEN, Madison WI) were transformed with PZ1D according to manufacturer's instructions and the methods described in Example 2.A.2.

### C. Expression of the recombinant bFGF-SAP fusion proteins (FPFS1)

The two-stage method described above was used to produce recombinant bFGF-SAP protein (hereinafter bFGF-SAP fusion protein).

### 1. Expression of rbFGF-SAP from pFS92 (PZ1A)

Three liters of LB broth containing ampicillin (50 µg/ml) and chloramphenicol (25 µg/ml) were inoculated with pFS92 plasmid-containing bacterial cells (strain BL21(DE3)pLysS) from an overnight culture (1:100 dilution) that were obtained according to Example 2.B. Cells were grown at 37°C in an incubator shaker to an OD₆₀₀ of 0.7. IPTG (Sigma Chemical, St. Louis, MO) was added to a final concentration of 0.2 mM and growth was continued for 1.5 hours at which time cells were centrifuged.

Subsequent experiments have shown that growing the BL21(DE3)pLysS cells at 30°C instead of 37°C improves yields. When the cells are grown at 30°C they are grown to an OD₆₀₀ of 1.5 prior to induction. Following induction, growth is continued for about 2 to 2.5 hours at which time the cells are harvested by centrifugation.

The pellet was resuspended in lysis solution (45-60 ml per 16 g of pellet; 20 mM TRIS, pH 7.4, 5 mM EDTA, 10% sucrose, 150 mM NaCl, lysozyme, 100 µg/ml, aprotinin, 10 µg/ml, leupeptin, 10 µg/ml, pepstatin A, 10 µg/ml and 1 mM PMSF) and incubated with stirring for 1 hour at room temperature. The solution was frozen and thawed three times and sonicated for 2.5 minutes. The suspension was centrifuged at 12,000 X g for 1 hour; the resulting first-supematant was saved and the pellet was resuspended in another volume of lysis solution without lysozyme. The resuspended material was centrifuged again to produce a second-supernatant, and the two supernatants were pooled and dialyzed against borate buffered saline, pH 8.3.

### 2. Expression of bFGF-SAP fusion protein from PZ1B and PZ1C

Two hundred and fifty mls. of LB medium containing ampicillin (100 µg/ml) were inoculated with a fresh glycerol stock of PZ1D. Cells were grown at 30°C in an incubator shaker to an OD₆₀₀ of 0.7 and stored overnight at 4°C. The following day the cells were pelleted and resuspended in fresh LB medium (no ampicillin). The cells were divided into 5 1-liter batches and grown at 30°C in an incubator shaker to an OD₆₀₀ of 1.5. IPTG (SIGMA CHEMICAL, St. Louis, MO) was added to a final concentration of 0.1 mM and growth was continued for about 2 to 2.5 hours at which time cells were harvested by centrifugation.

In order to grow PZ1C, prior to induction, the cells are grown in medium containing kanamycin (50µg/ml) in place of ampicillin.

### 3. Expression of bFGF-SAP fusion protein from PZ1D

Two hundred and fifty mls of LB medium containing ampicillin (100 µg/ml; LB AMP₁₀₀ medium) were inoculated with a fresh glycerol stock of PZ1B. Cells were grown at 30°C in an incubator shaker to an OD₆₀₀ of 0.7 and stored overnight at 4°C. The following day the cells were pelleted and resuspended in fresh LB medium (no ampicillin). The cells were used to inoculate a 1 liter batch of LB medium and grown at 30°C in an incubator shaker to an OD₆₀₀ of 1.5. IPTG (SIGMA CHEMICAL. St. Louis, MO) was added to a final concentration of 0.1 mM and growth was continued for about 2 to 2.5 hours at which time cells were harvested by centrifugation. The cell pellet was resuspended in ice cold 1.0 M Tris pH 9.0. 2 mM EDTA. The resuspended material is kept on ice for another 20-60 minutes and then centrifuged to separate the periplasmic fraction (supernatant) from the intracellular fraction (pellet).

### D. Affinity purification of bFGF-SAP fusion protein

Thirty ml of the dialyzed solution containing the bFGF-SAP fusion protein from Example 2.C. was applied to HiTrap heparin-Sepharose column (Pharmacia, Uppsala, Sweden) equilibrated with 0.15 M NaCl in 10 mM TRIS, pH 7.4 (buffer A). The column was washed: first with equilibration buffer; second with 0.6 M NaCl in buffer A; third with 1.0 M NaCl in buffer A; and finally eluted with 2 M NaCl in buffer A into 1.0 ml fractions. Samples were assayed by the ELISA method.

The results indicate that the bFGF-SAP fusion protein elutes from the heparin-Sepharose column at the same concentration (2 M NaCl) as native and recombinantly-produced bFGF. This indicates that the heparin affinity is retained in the bFGF-SAP fusion protein.

### E. Characterization of the bFGF-SAP fusion protein

### 1. Western blot of affinity-purified bFGF-SAP fusion protein

SDS gel electrophoresis was performed on a Phastsystem utilizing 20% gels (Pharmacia). Western blotting was accomplished by transfer of the electrophoresed protein to nitrocellulose using the PhastTransfer system (Pharmacia), as described by the manufacturer. The antisera to SAP and bFGF were used at a dilution of 1:1000 dilution. Horseradish peroxidase labeled anti-IgG was used as the second antibody (Davis et al., *Basic Methods in Molecular Biology*, New York, Elsevier Science Publishing Co., pp 1-338, 1986).

The anti-SAP and anti-FGF antisera bound to a protein with an approximate molecular weight of 48,000 kd, which corresponds to the sum of the independent molecular weights of SAP (30,000) and bFGF (18,000).

### 2. Assays to assess the cytotoxicity of the FGF-SAP fusion protein

### a. Effect of bFGF-SAP fusion protein on cell-free protein synthesis

The RIP activity of bFGF-SAP fusion protein compared to the FGF-SAP chemical conjugate was assayed as described in Example 1.G. The results indicated that the IC₅₀ of the bFGF-SAP fusion protein is about 0.2 nM and the IC₅₀ of chemically conjugated FGF-SAP is about 0.125 nm.

### b. Cytotoxicity of bFGF-SAP fusion protein

Cytotoxicity experiments were performed with the Promega (Madison, WI) Cell Titer 96 Cell Proliferation/Cytotoxicity Assay. About 1,500 SK-Mel-28 cells (available from ATCC), a human melanoma cell line, were plated per well in a 96 well plate in 90 µl HDMEM plus 10% FCS and incubated overnight at 37°C, 5% CO₂. The following morning 10 µl of media alone or 10 µl of media containing various concentrations of the rbFGF-SAP fusion protein, basic FGF or saporin were added to the wells. The plate was incubated for 72 hours at 37°C. Following the incubation period, the number of living cells was determined by measuring the incorporation and conversion of the commonly available dye MTT supplied as a part of the Promega kit. Fifteen µl of the MTT solution was added to each well, and incubation was continued for 4 hours. Next, 100 µl of the standard solubilization solution supplied as a part of the Promega kit was added to each well. The plate was allowed to stand overnight at room temperature and the absorbance at 560 nm was read on an ELISA plate reader (Titertek Multiskan PLUS, ICN, Flow, Costa Mesa, CA).

The results indicated that the chemical FGF-SAP conjugate has an ID₅₀ of 0.3 nM, the bFGF-SAP fusion protein has a similar ID₅₀ of 0.6 nM, and unconjugated SAP, which is unable to bind to the cell surface, has an ID₅₀ of 200 nM. Therefore, when internalized, the bFGF-SAP fusion protein appears to have approximately the same cytotoxic activity as the chemically conjugated FGF-SAP.

### EXAMPLE 4

### PREPARATION OF MODIFIED SAPORIN

Instead of derivatizing SAP, SAP is modified by addition of a cysteine residue by at the N-terminus-encoding portion of the DNA or the addition of a cysteine at position 4 or 10. The resulting saporin can then reacted with an available cysteine on an FGF or reacted with a linker or a linker attached to an FGF to produce conjugates that are linked via the added Cys or Met-Cys on saporin.

Modified SAP has prepared by modifying DNA encoding the saporin by inserting DNA encoding Met-Cys or Cys at position -1 or by replacing the Ile or the Asp codon within 10 or fewer residues of the N-terminus. The resulting DNA has been inserting into pET11a and pET15b and expressed in BL21 cells. The resulting saporin proteins are designated FPS1 (saporin with Cys at -1), FPS2 (saporin with Cys at position 4) and FPS3 (saporin with Cys at position 10). A plasmid that encodes FPS1 and that has been for expression of FPS1 has been designated PZ50B. Plasmids that encode FPS2 and that have been used for expression of FPS2 have been designated PZ51B (pET11a-based plasmid) and PZ51E (pet15b-based plasmid). Plasmids that encode FPS3 and that have been used for expression of FPS3 have been designated PZ52B (pET11a-based plasmid) and PZ52E (pet15b-based plasmid).

### A. Materials and Methods

### 1. Bacterial strains

Novablue (NOVAGEN, Madison, WI) and BL21(DE3) (NOVAGEN, Madison WI).

### 2. DNA manipulations

DNA manipulations were performed as described in Examples 1 and 2. Plasmid PZ1B (designated PZ1B1) described in Example 2 was used as the DNA template.

### B. Preparation of saporin with an added cysteine residue at the N-terminus

### 1. Primers

(a) Primer #1 corresponding to the sense strand of saporin, nucleotides 472-492 of SEQ ID NO. 12, incorporates a *Nde*I site and adds a cys codon 5' to the start site for the mature protein TATGTGTGTCACATCAATCACATTAGAT (SEQ ID NO. 34)
(b) Primer #2 - Antisense primer complements the coding sequence of saporin spanning nucleotides 547-567 of SEQ ID NO. 12 and contains a *BamH*I site

CAGGTTTGGATCCTTTACGTT (SEQ ID NO. 35)

### 2. Isolation of saporin-encoding DNA

PZIB1 DNA was amplified by PCR as follows using the above primers. PZ1B DNA (1 µl) was mixed in a final volume of 100 µl containing 10 mM Tris-HCl (pH 8.3), 50 mM KCl, 0.01% gelatin, 2 mM MgCl₂, 0.2 mM dNTPs, 0.8 µg of each primer. Next, 2.5 U TaqI DNA polymerase (Boehringer Mannheim) was added and the mixture was overlaid with 30 µl of mineral oil (Sigma). Incubations were done in a DNA Thermal Cycler (Ericomp). One cycle included a denaturation step (94°C for 1 min.), an annealing step (60°C for 2 min.), and an elongation step (72°C for 3 min.). After 35 cycles, a 10 µl aliquot of each reaction was run on a 1.5% agarose gel to verify the correct structure of the amplified product.

The amplified DNA was gel purified and digested with *Nde*I and *Bam*HI and subcloned into *Nde*I and *Bam*HI-digested pZ1B1. This digestion and subcloning step removed the FGF-encoding DNA and 5' portion of SAP up to the *Bam*HI site at nucleotides 555-560 (SEQ ID NO. 12) and replaced this portion with DNA encoding a saporin molecule that contains a cysteine residue at position -1 relative to the start site of the native mature SAP protein. The resulting plasmid is designated pZ50B1.

### C. Preparation of saporin with a cysteine residue at position 4 or 10 of the native protein

These constructs were designed to introduce a cysteine residue at position 4 or 10 of the native protein by replacing the isoleucine residue at position 4 or the asparagine residue at position 10 with cysteine.

### 1. Materials

### (a) Bacterial strains

The bacterial strains were Novablue and BL21(DE3) (NOVAGEN, Madison, WI).

### (b) DNA manipulations

DNA manipulations as described above.

### 2. Preparation of modified SAP-encoding DNA

SAP was amplified by polymerase chain reaction (PCR) from the parental plasmid pZ1B1 encoding the FGF-SAP fusion protein.

### (a) Primers

(1) The primer corresponding to the sense strand of saporin, spanning nucleotides 466-501 of SEQ ID NO. 12, incorporates a *Nde*I site and replaces the Ile codon with a Cys codon at position 4 of the mature protein (SEQ ID NO. 76): CATATGGTCACATCATGTACATTAGATCTAGTAAAT.
(2) The primer corresponding to the sense strand of saporin, nucleotides 466-515 of SEQ ID NO. 12, incorporates a *Nde*I site and replaces the Asp codon with a cys codon at position 10 of the mature protein (SEQ ID NO. 77) CATATGGTCACATCAATCACATTAGATCTAGTATGTCCGACCGCGGTCA.
(3) Primer #2 - Antisense primer complements the coding sequence of saporin spanning nucleotides 547-567 of SEQ ID NO. 12 and contains a *BamH*I site (SEQ ID NO. 35):

CAGGTTTGGATCCTTTACGTT.

### (b) Amplification

The PCR conditions was performed as described above, using the following cycles: denaturation step 94°C for 1 minute, annealing for 2 minutes at 60°C, and extension for 2 minutes at 72°C for 35 cycles. The amplified DNA was gel purified, digested with *Nde*I and *BamH*I, and subcloned into NdeI and BamHI digested pZ1B1. This digestion removed the FGF and 5' portion of SAP (up to the newly added *BamH*I) from the parental FGF-SAP vector (pZ1B1) and replaced this portion with a SAP molecule containing a CYS at position 4 or 10 relative to the start site of the native mature SAP protein. The resulting plasmids are designated pZ51B1 and pZ52B1, respectively.

### D. Cloning of DNA encoding SAP mutants in vector pET15b

The initial step in this construction was the mutagenesis of the internal *Bam*HI site at nucleotides 555-560 (SEQ ID NO. 12) in pZ1B1 by PCR using a sense primer corresponding to nucleotides 143-570 (SEQ ID NO. 12) but changing the G at nucleotide 555 (the third position in the Lys codon) to an A. The complement of the sense primer was used as the antisense primer. The PCR reactions were conducted as in B above. One µl of the resulting PCR product was used in a second PCR reaction using the same sense oligonucleotide as in B., above, in order to introduce a *Nde*I site and a Cys codon onto the 5' end of the saporin-encoding DNA. The antisense primer was complementary to the 3' end of the saporin protein and encoded a *Bam*HI site for cloning and a stop codon (SEQ ID NO. 37):

GGATCCGCCTCGTTTGACTACTT.

The resulting plasmid was digested with *Nde*I/*BamH*I and inserted into pET15b (NOVAGEN, Madison, WI), which has a His-Tag™ leader sequence (SEQ ID NO. 36), that had also been digested *Nde*I/*BamH*I.

The SAP-Cys-4 and Sap-Cys-10 mutants were similarly inserted into pET15b using SEQ ID NOS. 76 and 77, respectively, as the sense primers and SEQ ID NO. 37 as the antisense primer.

DNA encoding unmodified SAP (Example 1) can be similarly inserted into a pet15b or pet11A and expressed as described below for the modified SAP-encoding DNA.

### E. Expression of the modified saporin-encoding DNA

BL21(DE3) cells were transformed with the resulting plasmids and cultured as described in Example 2, except that all incubations were conducted at 30°C instead of 37°C. Briefly, a single colony was grown in LB AMP₁₀₀ to and OD₆₀₀ of 1.0-1.5 and then induced with IPTG (final concentration 0.1 mM) for 2 h. The bacteria were spun down.

### F. Purification of modified saporin

Lysis buffer (20 mM NaPO₄, pH 7.0, 5 mM EDTA, 5 mM EGTA, 1 mM DTT, 0.5 µg/ml leupeptin, 1 µg/ml aprotinin, 0.7 µg/ml pepstatin) was added to the rSAP cell paste (produced from pZ50B1 in BL21 cells, as described above) in a ratio of 1.5 ml buffer/g cells. This mixture was evenly suspended via a Polytron homogenizer and passed through a microfluidizer twice.

The resulting lysate was centrifuged 50,000 rpm for 45 min. The supernatant was diluted with SP Buffer A (20 mM NaPO₄, 1 mM EDTA, pH 7.0) so that the conductivity was below 2.5 mS/cm. The diluted lysate supernatant was then loaded onto a SP-Sepharose column, and a linear gradient of 0 to 30% SP Buffer B (1 M NaCl, 20 mM NaPO₄, 1 mM EDTA, pH 7.0) in SP Buffer A with a total of 6 column volumes was applied. Fractions containing rSAP were combined and the resulting rSAP had a purity of greater than 90%.

A buffer exchange step was used here to get the SP eluate into a buffer containing 50 mM NaBO₃, 1 mM EDTA, pH 8.5 (S Buffer A). This sample was then applied to a Resource S column (Pharmacia, Sweden) pre-equilibrated with S Buffer A. Pure rSAP was eluted off the column by 10 column volumes of a linear gradient of 0 to 300 mM NaCI in SP Buffer A. The final rSAP was approximately 98% pure and the overall yield of rSAP was about 50% (the amount of rSAP in crude lysate was determined by ELISA).

In this preparation. ultracentrifugation was used clarify the lysate; other methods, such as filtration and using floculents also can be used. In addition, Streamline S (PHARMACIA, Sweden) may also be used for large scale preparations.

### EXAMPLE 5

### PREPARATION OF FGF MUTEINS

### A. Materials and Methods

### 1. Reagents

Restriction and modification enzymes were purchased from BRL (Gaithersburg, MD), Stratagene (La Jolla, CA) and New England Biolabs (Beverly, MA). Native SAP, chemically conjugated basic FGF-SAP and rabbit polyclonal antiserum to SAP and basic FGF were obtained from *Saponaria officinalis* (*see, e.g*., Stirpe et al., *Biochem. J. 216*:617-625, 1983). Briefly, the seeds were extracted by grinding in 5 mM sodium phosphate buffer, pH 7.2 containing 0.14 M NaCI, straining the extracts through cheesecloth, followed by centrifugation at 28,00 g for 30 min to produce a crude extract, which was dialyzed against 5 mM sodium phosphate buffer, pH 6.5, centrifuged and applied to CM-cellulose (CM 52, Whatman, Maidstone, Kent, U.K.). The CM column was washed and SO-6 was eluted with a 0-0.3 M NaCl gradient in the phosphate buffer.

Plasmid pFC80, containing the basic FGF coding sequence, was a gift of Drs. Paolo Sarmientos and Antonella Isacchi of Farmitalia Carlo Erba (Milan, Italy). Plasmid pFC80, has been described in the WIPO International Patent Application No. WO 90/02800 and co-pending International PCT Application Serial No. PCT/US93/05702, which are herein incorporated in their entirety by reference. The sequence of DNA encoding bFGF in pFC80 is that set forth in copending International PCT Application Serial No. PCT/US93/05702 and in SEQ ID NO. 12. The construction of pFC80 is set forth above in Example 2.

Plasmid isolation, production of competent cells, transformation and M13 manipulations were carried out according to published procedures (Sambrook et al. (1989) Molecular Cloning, a Laboratory Manual Cold Spring Harbor Laboratory Press, Cold Spring Harbor, NY). Purification of DNA fragments was achieved using the Geneclean II kit, purchased from Bio 101 (La Jolla, CA). Sequencing of the different constructions was performed using the Sequenase kit (version 2.0) of USB (Cleveland, OH).

### 2. Sodium dodecyl sulphate (SDS) gel electrophoresis and Western blotting.

SDS gel electrophoresis was performed on a PhastSystem utilizing 20% gels (Pharmacia). Western blotting was accomplished by transfer of electrophoresed protein to nitrocellulose using the PhastTransfer system (Pharmacia), as described by the manufacturer. The antisera to SAP and basic FGF were used at a dilution of 1:1000. Horseradish peroxidase labeled anti-IgG was used as the second antibody as described (Davis, L., Dibner et al. (1986) Basic Methods in Molecular Biology, p. 1, Elsevier Science Publishing Co., New York).

### B. Preparation of the mutagenized FGF by site-directed mutagenesis

Cysteine to serine substitutions were made by oligonucleotide-directed mutagenesis using the Amersham (Arlington Heights, IL) *in vitro*-mutagenesis system 2.1. Oligonucleotides encoding the new amino acid were synthesized using a 380B automatic DNA synthesizer (Applied Biosystems, Foster City, CA).

### 1. Mutagenesis

The oligonucleotide used for *in vitro* mutagenesis of cysteine 78 was AGGAGTGTCTGCTAACC (SEQ ID NO. 16), which spans nucleotides 225-241 of SEQ ID NO. 12). The oligonucleotide for mutagenesis of cysteine 96 was TTCTAAATCGGTTACCGATGACTG (SEQ ID NO. 17), which spans nucleotides 279-302 of SEQ ID NO. 12). The mutated replicative form DNA was transformed into *E. coli* strain JM109 and single plaques were picked and sequenced for verification of the mutation. The FGF mutated gene was then cut out of M13, ligated into the expression vector pFC80, which had the non-mutated form of the gene removed, and transformed into *E. coli* strain JM109. Single colonies were picked and the plasmids sequenced to verify the mutation was present. Plasmids with correct mutation were then transformed into the *E. coli* strain FICE 2 and single colonies from these transformations were used to obtain the mutant basic FGFs. An excellent level of expression, approximately 20 mg per liter of fermentation broth, was achieved.

### 2. Purification of mutagenized FGF

Cells were grown overnight in 20 ml of LB broth containing 100 µg/ml ampicillin. The next morning the cells were pelleted and transferred to 500 ml of M9 medium with 100 µg/ml ampicillin and grown for 7 hours. The cells were pelleted and resuspended in lysis solution (10 mM TRIS, pH 7.4, 150 mM NaCl, lysozyme, 10 µ g/mL, aprotinin, 10 µg/mL, leupeptin, 10 µg/mL, pepstatin A, 10 µg/mL and 1 mM PMSF; 45-60 ml per 16 g of pellet) and incubated while stirring for 1 hour at room temperature. The solution was frozen and thawed three times and sonicated for 2.5 minutes. The suspension was centrifuged; the supernatant saved and the pellet resuspended in another volume of lysis solution without lysozyme, centrifuged again and the supernatants pooled. Extract volumes (40 ml) were diluted to 50 ml with 10 mM TRIS, pH 7.4 (buffer A). Pools were loaded onto a 5 ml Hi-Trap heparin-Sepharose column (Pharmacia, Uppsala, Sweden) equilibrated in 150 mM sodium chloride in buffer A. The column was washed with 0.6 M sodium chloride and 1 M sodium chloride in buffer A and then eluted with 2 M sodium chloride in buffer A. Peak fractions of the 2 M elution, as determined by optical density at 280 nm, were pooled and purity determined by gel electrophoresis. Yields were 10.5 mg of purified protein for the Cys₇₈ mutant and 10.9 mg for the Cys₉₆ mutant.

The biological activity of [C78S]FGF and [C96S]FGF was measured on adrenal capillary endothelial cells in culture. Cells were plated 3,000 per well of a 24 well plate in 1 ml of 10% calf serum-HDMEM. When cells were attached, samples were added in taplicate at the indicated concentration and incubated for 48 h at 37°C. An equal quantity of samples was added and further incubated for 48 h. Medium was aspirated; cells were treated with trypsin (1 ml volume) to remove cells to 9 ml of Hematall diluent and counted in a Coulter Counter. The results show that the two mutants that retain virtually complete proliferative activity of native basic FGF as judged by the ability to stimulate endothelial cell proliferation in culture.

### EXAMPLE 6

### PREPARATION OF CONJUGATES CONTAINING FGF MUTEINS

### A. Cytotoxicity assays of conjugates

Cytotoxicity experiments were performed with the Promega (Madison, WI) CellTiter 96 Cell Proliferation/Cytotoxicity Assay. Cell types used were SK-Mel-28, human melanoma Swiss 3T3 mouse fibroblasts (from Dr. Pamela Maher, La Jolla, CA), B16F10, mouse melanoma, PA-1, human ovarian carcinoma (from Dr. Julie Beitz, Roger Williams Hospital, Providence RI), and baby hamster kidney (BHK) [obtained from the American Type Culture Collection (ATCC)]. 2500 cells were plated per well.

### B. Coupling of FGF muteins to SAP

### 1. Chemical Synthesis of [C78S]FGF-SAP (CCFS2) and [C96S]FGF-SAP (CCFS3)

[C78S]FGF or [C96S]FGF (1 mg; 56 nmol) that had been dialyzed against phosphate-buffered saline was added to 2.5 mg mono-derivatized SAP (a 1.5 molar excess over the basic FGF mutants) and left on a rocker platform overnight. The next morning the ultraviolet-visible wavelength spectrum was taken to determine the extent of reaction by the release of pyridylthione, which adsorbs at 343 nm with a known extinction coefficient. The ratio of pyridylthione to basic FGF mutant for [C785]FGF was 1.05 and for [C96S]FGF was 0.92. The reaction mixtures were treated identically for purification in the following manner: reaction mixture was passed over a HiTrap heparin-Sepharose column (1 ml) equilibrated with 0.15 M sodium chloride in buffer A at a flow rate of 0.5 ml/min. The column was washed with 0.6 M NaCl and 1.0 M NaCl in buffer A and the product eluted with 2.0 M NaCl in buffer A. Fractions (0.5 ml) were analyzed by gel electrophoresis and absorbance at 280 nm. Peak tubes were pooled and dialyzed versus 10 mM sodium phosphate, pH 7.5 and applied to a Mono-S 5/5 column equilibrated with the same buffer. A 10 ml gradient between 0 and 1.0 M sodium chloride in equilibration buffer was used to elute the product. Purity was determined by gel electrophoresis and peak fractions were pooled. The yield for [C78S]FGF-SAP was 1.6 mg (60% with respect to starting amount of [C78S]FGF) and for was 0.96 mg [C96S]FGF-SAP (35%).

Virtually 100% of the mutant FGFs reacted with mono-derivatized SAP ([C78SJFGF: 105%, [C96S]FGF: 92%). Because the free surface cysteine of each mutant acts as a free sulfhydryl, it was unnecessary to reduce cysteines after purification from the bacteria. The resulting product was purified by heparin-Sepharose (data not shown), thus establishing that heparin binding activity of the conjugate is retained.

Coomassie staining and Western blotting of the purified proteins showed a prominent band at a molecular weight of about 48,000, corresponding to the combined molecular weights of SAP and bFGF. A much lighter band at a slightly lower molecular weight was detected and attributed to the described mobility of an artifact produced by the high isoelectric point (10.5) (Gelfi et al., *J. Biochem. Biophys. Meth. 15*:41-48, 1987) of SAP that causes a smearing in SDS gel electrophoresis (*see, e.g.,* Lappi et al., *Biochem. Biophys. Res. Commun. 129*:934-942, 1985). No higher molecular weight bands, corresponding to conjugates containing more than one molecule of SAP per molecule of basic FGF or more than one molecule of basic FGF per molecule of SAP were detected on Coomassie-stained gels of [C78S]FGF-SAP) and of ([C96S]FGF-SAP). Such bands were present in lanes on the gel in which an equal quantity (by weight) of heterogeneous FGF-SAP, synthesized from wild-type bFGF and non-purified derivatized SAP, had been loaded.

Western blotting using antibodies to SAP or basic FGF revealed that, while 480 ng of either [C78S]FGF-SAP or [C96S]FGF-SAP results in a well-visualized band (with the additional slight lower molecular weight band) the same quantity of conjugate produced by the previous procedure is almost undetectable. As in the Coomassie staining, the Western blotting of the mutant FGF-SAPs reveals much greater homogeneity than with heterogeneous FGF-SAP synthesized with non-mutagenized basic FGF and non-purified derivatized SAP.

### 2. Preparation of [C96S]FGF-rSAP (CCFS4)

Recombinant saporin that has the cys added at the N-terminus (SAP-CYS-(-1)) that was cloned and expressed in BL21 cells and isolated as described in EXAMPLE 4 was coupled to [C96S]FGF using (5,5'-dithiobis-(2-nitrobenzoic acid)) DTNB also called Ellman's reagent. The rSAP and [C96S]FGF were each treated with 10 mM dithiothreitol (DTT), incubated for 1 h at room temperature, and the DTT was removed by gel filtration in conjugation buffer (0.1 M NaPO₄, 100 NaCl and 1 mM EDTA, pH 7.5). A 100-fold molar excess of DTNB was added to the rSAP, incubated for 1 h at room temperature. Unreacted DTNB was removed by gel filtration. The [C96S]FGF was added to DTNB-treated SAP (3:1 molar ratio of [C96S]FGF:SAP) and incubated at room temperature for about 1 hr or for 16 hrs at 4°C. The mixture was loaded on heparin sepharose in 10 mM NaPO₄, 1 mM EDTA, pH 6 and the conjugate and free [C96S]FGF were eluted with 2 M NaCl in 10 mM NaPO₄, 1 mM EDTA, pH 6. The free [C96S]FGF was removed by gel filtration on Sephacryl S100 (Pharmacia). The resulting conjugate was designated CCFS4.

### C. Cytotoxicity of [C78S]FGF-SAP (CCFS2), [C96S]FGF-SAP (CCFS3) and [C96S]FGF-rSAP (CCFS4)

Cytotoxicity of the two mutant FGF-SAPs to several cell types has been tested. Heterogeneous FGF-SAP (CCFS1) is very cytotoxic to SK-MEL-28 cells, human melanoma cells, with an ED₅₀ of approximately 8 ng/ml. The mutant FGF-SAPs are also potently cytotoxic to these cells. [C78S]FGF-SAP and [C96S]FGF-SAP each have an ED₅₀ comparable to the heterogeneous chemically conjugates, indicting that mutant FGFs are able to internalize SAP to virtually the same extent as the heterogeneous FGF-SAP.

Similar results were obtained with an ovarian carcinoma cell type, PA-1, Swiss 3T3 cells, B16F10, a mouse melanoma and BHK cells.

CCFS4 was tested in the *in vitro* cytotoxicity assay and its activity is at least as good to the wild-type chemical conjugate (CCFS1).

### D. Preparation of homogeneous mixtures of FGF-SAP muteins by splicing by overlap extension (SOE)

### 1. Conversion of Cys 78 to Ser 78

### (a) Materials

### (1) Plasmids

Plasmid PZ1B (designated PZ1B1) described in Example 2 was used as the DNA template. The primers were prepared as follows:

### (2) Primers

(a) Primer #1 spanning the *Nde*I site at the 5' end of the FGF-encoding DNA from plasmid pZIB AAATACTTACATATGGCAGCAGGATC (SEQ ID NO. 18).
(b) Primer #2 - Antisense primer to nucleotides spanning the Cys 78 (nucleotides 220-249 of SEQ ID NO. 12 with base change to generate Ser 78) CAGGTAACGGTTAGCAGACACTCCTTTGAT (SEQ ID NO. 19).
(c) Primer #3 - Sense primer to nucleotides spanning the Cys 78 (nucleotides 220 - 249 of SEQ ID NO. 12 with base change to generate Ser 78) ATCAAAGGAGTGTCTGCTAACCGTTACCTG (SEQ ID NO. 20).
(d) Primer #4 - Antisense primer to spanning the *Nco*I site of FGF in pZ1B (corresponding to nucleotides 456-485 of SEQ ID NO. 12)

GTGATTGATGTGACCATGGCGCTCTTAGCA (SEQ ID NO. 21).

### (b) Reactions

### (1) Reaction A

PZ1B1 DNA (100 ng) was mixed (final volume of 100 µl upon addition of the Taq polymerase) with primer #1 (50 µM); primer #2 (50 µM), 10 mM Tri-HCl (pH 8.3), 50 mM KCl, 0.01% gelatin, 2 mM MgCl₂, 0.2 mM dNTPs.

### (2) Reaction B

Same as above except that primer #3 (50 µM) and primer #4 (50 µM) were used in place of primers #1 and #2.

Each reaction mixture was heated to 95°C for 5 min, 0.5 U TaqI DNA polymerase (1 µl; Boehringer Mannheim) was added and the mixture was overlaid with 100 µl of mineral oil (Perkin Elmer Cetus). Incubations were done in a DNA Thermal Cycler (Ericomp). Each cycle included a denaturation step (95°C for 1 min.), an annealing step (60°C for 1.5 min.), and an elongation step (75°C for 3 min.). After 20 cycles, the reaction mixture was incubated at 75°C for 10 minutes for a final elongation. The products were resolved on a 2% agarose gel and DNA of the correct size (247 bp and 250 bp) was purified. The ends were repaired by adding nucleoside triphosphates and Klenow DNA polymerase.

### (3) Reaction C

One µl of each product of reactions A and B were mixed (final volume of 100 µ L upon addition of Taq polymerase) with primers #1 and #4 (final concentration of each was 50 µM); 10 mM Tri-HCl (pH 8.3), 50 mM KCl, 0.01% gelatin, 2 mM MgCl₂, 0.2 mM dNTPs.

The resulting reaction mixture was heated to 95°C for 5 min, 0.5 U TaqI DNA polymerase (1 µl; Boehringer Mannheim) was added and the mixture was overlaid with 100 µl of mineral oil (Perkin Elmer Cetus). Incubations were done in a DNA Thermal Cycler (Erricomp). Each cycle included a denaturation step (95°C for 1 min.), an annealing step (60°C for 1.5 min.), and an elongation step (75°C for 3 min.), followed, after 20 cycles, by a final elongation step at 75°C for 10 minutes.

The amplified product was resolved on a 1.5% agarose gel and the correct size fragment (460 bp), designated FGFC78S-SAP was purified.

### 2. Generation of DNA encoding FGFC78/C96S-SAP

### (a) Materials

### (1) Template

DNA encoding FGFC78S-SAP

### (2) Primers

(a) Primer #5-Sense primer spanning the Cys 96 (nucleotides 275-300 of SEQ ID NO. 12 with base change to generate Ser 96) TGGCTTCTAAATCTGTTACGGATGAG (SEQ ID NO. 22).
(b) Primer #6-Antisense primer spanning the Cys 96 (nucleotides 275-300 of SEQ ID NO. 12 with base change to generate Ser 96)

CTCATCCGTAACAGATTTAGAAGCCA (SEQ ID NO. 23).

### (b) Reactions

### (1) Reaction D

FGFC78S-SAP-encoding DNA (100 ng) was mixed (final volume of 100 µl upon addition of the Taq polymerase) with primer #1 (50 µM); primer #5 (50 µM), 10 mM Tri-HCl (pH 8.3), 50 mM KCl, 0.01% gelatin, 2 mM MgCl₂ and 0.2 mM dNTPs.

### (2) Reaction E

Same as above, except that primers #4 and #6 (50 µM final concentration of each) were used instead of primers #1 and #5.

Each reaction mixture was heated to 95°C for 5 min, 0.5 U TaqI DNA polymerase (1 µl; Boehringer Mannheim) was added and the mixture was overlaid with 100 µl of mineral oil (Perkin Elmer Cetus). Incubations were done in a DNA Thermal Cycler (Ericomp). Each cycle included a denaturation step (95°C for 1 min), an annealing step (60°C for 1.5 min.), and an elongation step (75°C for 3 min.) for 20 cycles, followed by a final elongation step at 75°C for 10 minutes. The products were resolved on a 2% agarose gel and DNA of the correct size (297 bp and 190 bp) was purified. The ends were repaired by adding nucleoside triphosphates and Klenow DNA polymerase.

### (3) Reaction F

The product of reactions D and E (100 ng of each) were mixed (final volume of 100 µL upon addition of Taq polymerase) with primers #1 and #4 and amplified as described above. The amplified product resolved on a 1.5% agarose gel and the correct size fragment (465 bp) was purified. The resulting product, DNA that encodes FGFC78/96S-SAP, had *Nde*I and *Nco*I ends. It was digested with *Nde*I and *Nco*I and ligated into *Nde*I/*Nco*I-digested PZ1B1 and into *Nde*I/*Nco*I-digested PZ1C1 (PZIC described in Example 2 above). The resulting constructs were designated PZ2B1 and PZ2C1, respectively.

### E. Expression of the recombinant FGFC78/96S-SAP fusion proteins (FPFS4) from PZ2B1 and PZ2C1

The two-stage method described above for production of FPFS1 was used to produce recombinant FGFC78/96S-SAP protein (hereinafter FPFS4). Two hundred and fifty mls. of LB medium containing ampicillin (100 µg/ml) were inoculated with a fresh glycerol stock of PZ1B. Cells were grown at 30°C in an incubator shaker to an OD₆₀₀ of 0.7 and stored overnight at 4°C. The following day the cells were pelleted and resuspended in fresh LB medium (no ampicillin). The cells were divided into 5 1-liter batches and grown at 30°C in an incubator shaker to an OD₆₀₀ of 1.5. IPTG (SIGMA CHEMICAL, St. Louis, MO) was added to a final concentration of 0.1 mM and growth was continued for about 2 to 2.5 hours at which time cells were harvested by centrifugation.

In order to grow PZ2C1, prior to induction, the cells were grown in medium containing kanamycin (50µg/ml) in place of ampicillin.

### F. Biological Activity

The cytotoxicity of the mutein FGF-SAP produced from PZ2B1 (FPFS4) was assessed on SK MEL 28 cells and was at least equivalent to the activity of the wild type FGF-SAP chemical conjugate, and recombinant FGF-SAP produced from PZ1B1.

The *in vivo* activity of the mutein FGF-SAP produced from PZ2B1 has been tested in animals, and it appears to be less toxic than FGF-SAP from PZ1B1 (FPFS1).

### EXAMPLE 7

### PREPARATION OF FGF-SAP CONJUGATES THAT CONTAIN LINKERS ENCODING PROTEASE SUBSTRATES

**A. Synthesis of oligos encoding protease substrates** Complementary single-stranded oligos in which the sense strand encodes a protease substrate, have been synthesized either using a cyclone machine (Millipore, MA) according the instructions provided by the manufacturer, or were made by Midland Certified Reagent Co. (MIDLAND, TX)or by National Biosciences, INC. (MN). The following oligos have been synthesized and introduced into constructs encoding bFGF-SAP.
**1.** Cathepsin B substrate linker: 5'- CCATGGCCCTGGCCCTGGCCCTGGCCCTGGCCATGG SEQ ID NO:38
**2.** Cathepsin D substrate linker 
5'- CCATGGGCCGATCGGGCTTCCTGGGCTTCGGCTTCCTGG
GCTTCGCCAT GG -3' SEQ ID NO:39
**3x.** Trypsin substrate linker 
5'- CCATGGGCCGATCGGGCGGTGGGTGCGCTGGTAATAGAGT
CAGAAGATCAGTCGGAAGCAGCCTGTCTTGCGGTGGTCTC
GACCTGCAGG CCATGG-3' SEQ ID NO:44
**4.** Gly₄Ser 5'- CCATGGGCGG CGGCGGCTCT GCCATGG -3' SEQ ID NO:40
**5.** (Gly₄Ser)₂ 5'- CCATGGGCGGCGGCGGCTCTGGCGGCGGCGGCTC TGCCATGG -3' SEQ ID NO:41
**6.** (Ser₄Gly)₄ 
5'- CCATGGCCTCGTCGTCGTCGGGCTCGTCGTCGTC
GGGCTCGTCGTCGTCGGGCTCGTCGTCGTCGGGC
GCCATGG -3' SEQ ID NO:42
**7.** (Ser₄Gly)₂ 
5- CCATGGCCTCGTCGTCGTCGGGCTCGTCGTCGTC
GGGCGCCATGG -3' SEQ ID NO:43
**8.** Thrombin substrate linker CTG GTG CCG CGC GGC AGC SEQ ID NO. 52 Leu Val Pro Arg Gly Ser
**9.** Enterokinase substrate linker GAC GAC GAC GAC CCA  SEQ ID NO. 53 Asp Asp Asp Asp Lys
**10.** Factor Xa substrate ATC GAA GGT CGT SEQ ID NO. 54 Ile Glu gly Arg

### B. Preparation of DNA constructs encoding FGF-Linker-SAP

The complementary oligos were annealed by heating at 95°C for 15 min., cooled to room temperature, and then incubated at 4°C for a minute to about an hour. Following the incubation, the oligos were digested with *Nco*I and ligated overnight, at a 3:1 (insert:vector) ratio, at 15°C to *Nco*I-digested PZ1B, PZ1C or PZ2B (see Examples 2B and 6), which had been treated with alkaline phosphatase (Boehringer Mannheim).

Bacteria (Novablue (NOVAGEN, Madison, WI)) were transformed with ligation mixture (1 µl) and plated on LB-amp or LB-Kan, depending upon the plasmid). Colonies were selected, clones isolated and sequenced to determine orientation of the insert. Clones with correct orientation were used to transform strain expression strain BL21(DE3) (NOVAGEN, Madison WI). Glycerol stocks were generated from single transformed colonies. The transformed strains were cultured as described in Example 2 and fusion proteins with linkers were expressed.

The DNA and amino acid sequences of exemplary fusion proteins, containing cathepsin B substrate (FPFS9), cathepsin D substrate (FPFS5), Gly₄Ser (FPFS7), (Gly₄Ser)₂ (FPFS8), trypsin substrate (FPFS6), (Ser₄Gly)₄ (FPFS12) and (Ser₄Gly)₂ (FPFS11) linkers, respectively, are set forth in SEQ ID NOS. 45-51 (*see, also*, Table 4).

### C. Expression of conjugates with linkers

DNA encoding the conjugates set forth above and summarized in Table 4 have been expressed in BL21 cells as described above for PZ1B1 using plasmids prepared as described above and summarized in TABLE 5.

### EXAMPLE 8

### ANTIPROLIFERATIVE ACTIVITY OF BASIC FIBROBLAST GROWTH FACTOR-SAPORIN MITOTOXIN IN CULTURED KERATOCYTES

One of the most serious complications of excimer laser photorefractive keratectomy (PRK) is corneal haze and it may be caused by keratocyte proliferation and new collagen formation after surgery. The use of basic fibroblast growth factor-saporing (bFGF-SAP), a conjugate of bFGF and the ribosome-inactivation protein, saporin, to inhibit keratocytes proliferation following excimer laser PRK has been evaluated.

### A. Experiment #1

### (1) Materials and Methods

Rabbit keratocytes cell lines were established by primary explant culture from New Zealand white rabbits. Subconfluent culture of keratocytes were incubated with 6 different concentrations (10⁻⁴, 10⁻³, 10⁻², 10⁻¹, 1, and 10 ug/ml) of bFGF-SAP (CCFS1) for 3 hours and analyzed for their effects on keratocyte proliferation 1, 2, 3, and 7 days following drug exposure. Keratocyte proliferation was quantified by hemocytometer.

### (2) Results

Keratocyte proliferation was inhibited by 3 hours exposure to bFGF-SAP in a dose-dependent manner. The indicated that short-term application of bFGF-SAP may be useful in limiting keratocyte proliferation following excimer laser surgery.

### B. Experiment #2

Rabbit keratocytes cell lines were established by primary explant culture from New Zealand white rabbits. Keratocytes were incubated with 11 different concentrations (5 x 10⁻¹² M- 1 x 10⁻⁶ M) of bFGF-SAP (FPFS1), bFGF-SAP (CCFS1), FGF, SAP and FGF SAP for 48 hours and analyzed for their effects on keratocyte proliferation. CCFS1 inhibited cell proliferation in a dose dependent manner with an ID₅₀ of about 0.1 x 10⁻⁹ M; FPFS1 inhibited cell proliferation in a dose dependent manner with an ID₅₀ of about 0.2 x 10⁻⁹ M; SAP and FGF + SAP inhibited cell proliferation with an ID₅₀ of about 5 x 10⁻⁸ M, and FGF had little effect.

This experiment was repeated with a 3 hour exposure time to the conjugates. The results were qualitatively similar. Higher concentrations of conjugate were required to achieve inhibition of proliferation.

### C. Experiment #3

Rabbit keratocytes cell lines were established by primary explant culture from New Zealand white rabbits. Keratocytes were serum starved and then serum stimulated in order to ascertain the effects of the conjugates on proliferating keratocytes. The serum stimulated kertocytes were incubated with varying (1 x 10⁻⁸ M - 1 x 10⁻⁴ M) of bFGF-SAP (FPFS1), bFGF-SAP (CCFS1), FGF, and SAP for 3 hours. CCFS1 inhibited cell proliferation in a dose dependent manner with an ID₅₀ of between about 1 and 10 nM; and FPFS1 inhibited cell proliferation in a dose dependent manner with an ID₅₀ of between about 1 and 10 nM: FGF appeared to stimulate cell proliferation, and SAP slightly inhibited cell proliferation.

### D. In vivo Rabbit Model

After systemic anesthesia with ketamine and xylazine (4:I), 15 rabbits undergo a mechanical removal of the epithelium and excimer laser photorefractive keratectomy. After surgery the rabbits are treated with the following drugs four times daily until epithelial healing: a) PBS, 0.1%BSA; b) bFGF, c) bFGF-SAP. The animals are be examined every two weeks with documentary photographs and sacrificed after two months. Corneas are prepared for hyaluronic Acid and BrdU immunohistochemistry study.

A sample size of 15 rabbits, male and female, divided into 3 groups according to the surgical technique used, is sufficient to detect a statistically significant difference among the groups at p<0.05, if there is a treatment benefit.

### EXAMPLE 9

### THERAPEUTIC ACTIVITY OF THE WILD-TYPE CHEMICAL CONJUGATE AND FUSION PROTEIN bFGF-SAP IN THE MOUSE TUMOR XENOGRAFT MODEL

### A. Materials and methods

The methods set forth below were performed substantially as described in Beitz et al., *Cancer Research 52*:227-230, 1992).

### (1) Study Design

Sixty-three athymic mice bearing subcutaneous tumors received four weekly bolus IV injections of the test materials. Tumor volumes were measured twice weekly for 61 days.

### (2) Test Materials

Wild-type chemical conjugate bFGF-SAP was supplied in Dulbecco's phosphate buffered saline (PBS) at a concentration of 1.0 mg/ml. Fusion protein bFGF-SAP in *E. coli* was supplied in Dulbecco's PBS at a concentration of 9.0 mg/ml. Basic FGF was supplied in Dulbecco's PBS at a concentration of 1.0 mg/ml. Saporin was supplied in Dulbecco's PBS (0.01 M Phosphate, 0.14 M NaCl, pH 7.4) at a concentration of 1.0 mg/ml. All dilutions were made in Dulbecco's PBS with 0.1% bovine serum albumin (NB 1005-18).

### (3) Species

Female Balb/c nu/nu athymic mice (Roger Williams Hospital Animal Facility, Providence, RI), 8-12 weeks old, were maintained in an aseptic environment. Sixty-three animals were selected for the study, and body weights ranged from 25-30 grams the day prior to dosing.

### (4) Husbandry

Animals were maintained in a quarantined room and handled under aseptic conditions. Food and water were supplied *ad libitum* throughout the experiment.

### (5) Tumor Cells

PA-1 human ovarian teratocarcinoma cells were obtained from the American Type Culture Collection (Rockville, MD; ATCC accession no. CRL1572) were grown in modified Eagle's medium supplemented with 10% fetal calf serum.

### (6) Tumor Implantation

Five days prior to injection of the test material, mice received a subcutaneous injection of tumor cells (approximately 2 x 10⁶ PA-1 human ovarian teratocarcinoma cells/mouse) in the right rear flank.

### (7) Tumor Size Measurements

Calipers were used to measure the dimensions of each tumor. Measurements (mm) of maximum and minimum width were performed prior to injection of the test material and at bi-weekly intervals for 61 days. Tumor volumes (mm³) were computed using the formula Volume=[(minimum measurement)²(maximum measurement)]/2.

### (8) Dose Preparation

Dosing material was prepared by mixing the test material with appropriate volumes of PBS/0.1% BSA to achieve the final doses.

### (9) Dosing Procedures

Individual syringes were prepared for each animal. Mice received four weekly IV injection (250-300 ul) into the tail vein on days 5, 12, 19 and 26 with day 1 designated as the day that the tumor cells were injected into the mice. Doses were individualized for differences in body weight.

### B. Results - Inhibition of tumor growth

In all animals, tumors were measured prior to injection of the test material and at bi-weekly intervals for 61 days. Tumors from animals in all groups were approximately 55-60 mm³ on day 5 when treatment began. The vehicle-treated group (PBS with 0.1% BSA) showed a 50-fold increase in tumor volume over the 61 days of the study. The other control groups demonstrated similar levels of tumor growth: the SAP control group showed a 30-fold increase, the bFGF control group showed a 50-fold increase, and the bFGF plus SAP group showed a 50-fold increase in tumor volume. In all the control groups, the rate of growth of the tumor was fairly consistent over the 61-day period. In the treated groups, with wild-type chemical conjugate bFGF-SAP and fusion protein bFGF-SAP, there appeared to be a statistically significant dose-related suppression in tumor growth compared to controls over the first 30 days; however, tumor volumes increased again after this period such that there was no longer a statistical difference between the treated and control groups.

The 50 µg/kg/week fusion protein bFGF-SAP-treated groups exhibited tumor volumes that were 29% of controls, but a statistical comparison to controls was not done because only two animals in the treated group survived to 30 days. The fusion protein bFGF-SAP 5.0 µg/kg/week dose achieved significant suppression of tumor growth, with tumor volumes at 48% of control values. The 0.5 µg/kg/week fusion protein bFGF-SAP group showed significant suppression of tumor growth to day 26 when tumors were at 71% of controls. There was no statistical difference between tumor volumes in the 0.5 µg/kg/week wild-type chemical conjugate bFGF-SAP and fusion protein bFGF-SAP groups at 30 days. A statistical comparison of the two 50 µg/kg/week treatment groups was not done because there were only two surviving animals in the fusion protein bFGF-SAP group.

All seven animals survived the 61-day study in all groups with the exception of the 50 µg/kg/week chemical conjugate bFGF-SAP group (3 of 7 survived to 61 days) and the 50 µg/kg/week fusion protein bFGF-SAP group (1 of 7 survived to 61 days).

### EXAMPLE 10

### TESTS OF THE EFFECTS OF AN FGF-SAPORIN CONJUGATE IN A RAT BALLOON INJURY MODEL OF RESTENOSIS

### A. Summary

Wild-type chemical conjugate bFGF-SAP and fusion protein bFGF-SAP were evaluated for anti-proliferative activity against smooth muscle cells in a rat balloon injury model of restenosis. Thirty-six male Sprague-Dawley rats (300 to 350 g) were randomized into six treatment groups (n=6/treatment); animals underwent balloon denudation of the left carotid artery and then received three doses of wild-type chemical conjugate bFGF-SAP (75 ug/kg/day), fusion protein bFGF-SAP (1.25, 5.0, 25, 75 ug/kg/day), or vehicle (PBS with 0.1% BSA) via tail vein injections at 5 min, 24 hr, and 48 hr. Animals were sacrificed six days postdenudation surgery, and carotid arteries were sectioned and analyzed for intimal smooth muscle cell number.

The six animals in the high dose fusion protein bFGF-SAP group (75 ug/kg/day) either died or were sacrificed before the end of the study. Deaths also occurred in the 25 ug/kg/day fusion protein bFGF-SAP group; two of the six animals survived. Three of the animals in the wild-type chemical conjugate bFGF-SAP group (75 ug/kg/day) showed thrombosis at the balloon injury site upon necropsy and were not included in the calculated average.

Anti-proliferative activity was seen with wild-type chemical conjugate bFGF-SAP and fusion protein bFGF-SAP; intimal smooth muscle cell number was decreased relative to controls with 75 ug/kg/day wild-type chemical conjugate bFGF-SAP and with 1.25 And 25 ug/kg/day fusion protein bFGF-SAP. There was notable variability between control animals treated on different days and within treatment groups treated on the same day. This variability in the model may be due to the early time point chosen for sacrifice of the animals (six days) and may be responsible for the apparent lack of activity of the 5.0 Ug/kg/day fusion protein bFGF-SAP group relative to controls.

### B. Materials And Methods

### 1. Test Materials

Wild-type chemical conjugate bFGF-SAP was supplied in Dulbecco's phosphate buffered saline (PBS) at a concentration of 1.0 mg/ml. Fusion protein bFGF-SAP (NB 1008-118) produced in *E. coli* was supplied in Dulbecco's PBS at a concentration of 0.5 mg/ml. All dilutions were made in Dulbecco's PBS with 0.1% bovine serum albumin (NB 1005-62).

### 2. Species

Thirty-six male Sprague-Dawley rats (B&K Laboratories, Seattle), 3-4 months old, were selected for the study. Body weights ranged from 300-350 grams the day prior to dosing.

### 3. Treatment

The left common carotid artery was denuded of endothelium by intraluminal passage of a 2F Fogarty balloon. On day 6 (96 hr after the last dose), the animals were sacrificed with an overdose of sodium pentobarbital. Fifteen minutes before the rats were sacrificed, Evans blue (0.5 ml, 5% in saline) was injected intravenously, and the animals were perfusion-fixed by placing a catheter in the abdominal aorta and infusing 4% paraformadehyde in phosphate buffer (0.1 M, pH 7.3) at physiological pressure and flow for 4 min. Segments from the denuded carotid arteries were embedded in paraffin and counter-stained with hematoxylin, and the total number of intimal smooth muscle cells was determined by light microscopy.

### 4. Determination of intimal smooth muscle cell number

Four non-serial cross sections were taken from each of two areas of the left carotid artery. Intimal smooth muscle cell number was counted under light microscopy. Means ± standard deviations were calculated, and the results were reported as the average number of intimal smooth muscle cells per cross section.

### 5. Dose Preparation

Dosing material was prepared by mixing the test material with appropriate volumes of PBS/0.1% BSA to achieve the final doses.

### 6. Dosing Procedures

Individual syringes were prepared for each animal. Rats were injected via the tail vein at 5 min after ballooning and again at 24 and 48 hours after ballooning (200 ul/injection). Doses were individualized for differences in body weight.

### EXAMPLE 11

### FGF-POLY-L-LYSINE CONDENSATION OF A PLASMID ENCODING β-GALACTOSIDASE

### A. Derivitization of poly-L-lysine

Poly- L- lysine was dissolved in 0.1 M NaPO4, 0.1 M NaCl, 1 mM EDTA, pH 7.5 (buffer A) at 3 mg/ml. Approximately 30 mg of poly-L-lysine solution was mixed with 0.187 ml of 3 mg/ml SPDP in anhydrous ethanol resulting in a molar ratio of SPDP/poly-L-lysine of 1.5. The derivitization reaction was carried out at room temperature for 30 minutes. The reaction mixture was then dialyzed against 4 liters of Buffer A for 4 hours at room temperature.

### B. Conjugation of Derivitized polylysine to FGF2-3

A solution containing 28.5 mg of poly-L-lysine-SPDP was added to 12.9 mg of FGF2-3 in buffer A incubated overnight at 4°C. The molar ratio of poly-L-lysine-SPDP/FGF2-3 was approximately 1.5. Following incubation, the conjugation reaction mixture was applied to a 6 ml Resource S column. A gradient of 0.15 M to 2.55 M NaCl in 20 mM NaPO4, 1mM EDTA, pH 8.0 (Buffer B) over 30 column volumes was used for elution. The FGF2-3/poly-L-lysine conjugate, CCFL, was eluted off the column at 1.05-1.74 M NaCl concentration. Unreacted FGF2-3 was eluted off by 0.5-0.6 M NaCl.

The fractions containing CCFL were concentrated and loaded onto a gel-filtration column (Sephacryl S100) for buffer exchange into 20 mM HEPES, 0.1 M NaCl, pH 7.3. The molecular weight of CCFL as determined by size exclusion HPLC is approximately 42 kD. To determine if the conjugation procedure interfered with the ability of FGF2-3 to bind heparin, the chemical conjugate CCFL was loaded onto a heparin column and was eluted off the column at 1.8-2.0 M NaCl. In comparison, unconjugated FGF2-3 is eluted off heparin at 1.4 - 1.6 M NaCl. This suggests that poly-L-lysine contributes to FGF2-3 ability to bind heparin. The ability of poly-L-lysine to bind heparin was not determined.

A sample of CCFL was electorphoresed on SDS/PAGE under reducing conditions. The protein migrated at the same molecular weight as FGF. Under nonreducing conditions the conjugate did not enter the gel because of its high charge density.

A proliferation assay was performed to determine if the conjugation procedure reduced the ability of FGF2-3 ability to stimulate mitogenesis. The results revealed that CCFL is equivalent to FGF2-3 in stimulating proliferation.

### C. FGF2-3-poly-L-lysine-nucleic acid Complex Formation

Optimal conditions for complex formation were established. Varying quantities (0.2 to 200 µg) of β-galactosidase encoding plasmid nucleic acid pSVβ were mixed with 100 µg of CCFL in 20mM HEPES pH7.3, 0.1 M NaCl. The reaction was incubated for 1 hour at room temperature. nucleic acid binding to the FGF-lysine conjugate was confirmed by gel mobility shift assay using ³²P-labeled SV40-β-gal nucleic acid cut with *Pvu*I restriction endonuclease. In brief, SV40β-gal nucleic acid was digested with *Pvu*I restriction endonucleases; ends were labeled by filling in ³²PdNTPs with DNA polymerase I (Klenow fragment). To each sample of 30ng of 32P labeled nucleic acid increasing amounts of FGF-polylysine conjugate was added to the mixture. The protein/nucleic acid mixture was electrophoresed in a 1% agarose gel with 1 X TAE buffer.

A proliferation assay was performed to determine if the condensed nucleic acid had an effect the ability of CCFL to stimulate mitogenesis. The proliferation assay showed that only the highest dose of nucleic acid (200ug) had an inhibitory effect on proliferation as compared to FGF2-3 and CCFL.

The CCFL/nucleic acid mixture was introduced into COS cells and an endothelial cell line, ABAE, both of which express FGF receptors. The cells were subsequently assayed for b-galactosidse enzyme activity. COS and ABEA cells were grown on coverslips and incubated with the different ratios of CCFL:DNA for 48 hours. The cells were then fixed and stained with X-gal. Maximal β-galactosidase enzyme activity was seen when 30 ug of pSVβ per 100 ug of FGF2-3-polylysine conjugate was used.

Sensitivity of the receptor mediated gene delivery system was determined using the optimized CCFL/DNA ratio for complex formation. Increasing amounts of the CCFL/DNA complex was added to cells. 100 µg of CCFL was mixed with 30 ug of pSVB for 1 hour at room temperature. The COS and endothelial cells were incubated with increasing amounts of condensed material (0 ng, 1 ng, 10 ng, 100 ng, 1000 ng and 10,000 ng). The cells were incubated for 48 hours and then were assayed for β-galactosidase activity. In addition, cells grown on cover slips were treated with 1000 ng of CCFL-DNA for 48 hours, then fixed and stained using X-gal. The β-gal enzyme assay revealed that with increasing amounts of material there is an increase in enzyme activity. Cells incubated with X-gal showed blue staining throughout the cytoplasm in approximately 30% of the cells on the coverslip.

To determine if the CCFL complex was being taken up through the FGF receptor by a specific receptor mediated endocytosis pathway, three controls were performed. β-galactosidase activity was determined for cells treated with nucleic acid alone, with nucleic acid poly-L-lysine and with FGF2-3 + poly-L-lysine + nucleic acid. β-galactosidase gene expression was not significantly above background, indicating that a covalent linkage between FGF2-3 and poly-L-lysine is necessary for β-galactosidase expression to occur in the cells.

In addition, to show that CCFL/nucleic acid is being taken up through the FGF receptors, an excess of free FGF2-3 is added to a constant amount of CCFL/nucleic acid to compete for binding to its cognate receptor. If CCFL-β-gal is being delivered to the cytoplasm by the FGF receptor, then increasing doses of free FGF should decrease or eliminate β-gal expression.

From the foregoing it will be appreciated that, although specific embodiments of the invention have been described herein for purposes of illustration, various modifications may be made without deviating from the spirit and scope of the invention. Accordingly, the invention is not limited except as by the appended claims.

## Claims

1. A composition having the formula:
polypeptide that binds to a fibroblast growth factor (FGF) receptor-nucleic acid binding domain-nucleic acid molecule, wherein:
the nucleic acid binding domain being chemically conjugated or fused to the polypeptide that binds to an FGF receptor,
the nucleic acid molecule being bound to the nucleic acid binding domain; and wherein the composition binds to an FGF receptor and is internalized specifically in cells bearing the FGF receptor.

2. The composition of claim 1 wherein the nucleic acid molecule is a cytocide-encoding agent.

3. The composition of claim 2 wherein the cytocide encoding agent encodes a ribosome inactivating protein.

4. The composition of claim 3 wherein the ribosome inactivating protein is saporin.

5. The composition of claim 3 wherein the ribosome inactivating protein is gelonin.

6. The composition of claim 2 wherein the cytocide encoding agent encodes a protein that inhibits elongation factor 2.

7. The composition of claim 6 wherein the protein is diphtheria toxin.

8. The composition of claim 1 wherein the nucleic acid molecule is a therapeutic protein encoding agent.

9. The composition of either of claims 1, 2, or 8 wherein the polypeptide that binds to the FGF receptor is FGF-2 and the nucleic acid binding domain is poly-L-lysine.

10. The composition of either of claims 1, 2, or 8 wherein the nucleic acid binding domain is a transcription factor.

11. The composition of either of claims 1, 2, or 8 wherein the nucleic acid binding domain is a protein selected from the group consisting of AP-1, Sp-1, rev, GCN4, ëcro, ëcI, TFIIA, myoD, retinoic acid receptor, glucocosteroid receptor, SV40 large T antigen, and GAL4 polypeptides.

12. The composition of either of claims 1, 2, or 8 wherein the nucleic acid binding domain is a polycation.

13. The composition of claim 12 wherein the polycation is selected from the group consisting of poly-L-lysine, protamine, histone and spermine.

14. The composition of claim 2 wherein the cytocide-encoding agent further comprises a tissue-specific promoter operably linked thereto.

15. The composition of claim 8 wherein the therapeutic protein-encoding agent further comprises a tissue-specific promoter operably linked thereto.

16. The composition of either of claims 14 or 15 wherein the tissue-specific promoter is selected from the group consisting of alpha-crystalline promoter, tyrosinase promoter, prostate specific antigen promoter, and gamma-crystalline promoter.

17. The composition of claim 1, further comprising a tissue-specific promoter operably linked thereto.

18. The composition of either of claims 1, 2, or 8, further comprising between one to six linkers that are selected from the group consisting of a cleavable linker, a linker that provides a sorting signal, a linker that reduces steric hindrance and a linker that contributes to a condensing ability of the nucleic acid binding domain, the addition of said one to six linkers resulting in one of the following formulas:
polypeptide that binds to an FGF receptor-L-nucleic acid binding domain-nucleic acid molecule,
polypeptide that binds to an FGF receptor-L-nucleic acid binding domain-cytocide encoding agent, or the formula:
polypeptide that binds to an FGF receptor-L-nucleic acid binding domain-therapeutic protein encoding agent wherein:
L is one to six linkers; and
wherein the conjugate retains the ability to bind to an FGF receptor and internalize the nucleic acid molecule, cytocide-encoding agent or therapeutic protein encoding agent.

19. The composition of claim 18 wherein the linker is selected from the group consisting of (GlyₘSerₚ)ₙ, (SerₘGlyₚ)ₙ, and (AlaAlaProAla)ₙ in which n is 1 to 6, m is 1 to 6, and p is 1 to 4.

20. The composition of claim 19 wherein m is 4, p is 1, and n is 2 to 4.

21. The composition of claim 18 wherein the linker is a disulfide bond.

22. A method of delivering a nucleic acid molecule to a cell, comprising contacting a cell with the composition according to any one of claims 1 and 5-11, wherein the nucleic acid molecule is internalized in the cell.

23. A composition having the formula:
polypeptide that binds to an fibroblast growth factor (FGF) receptor-nucleic acid molecule-nucleic acid binding domain, wherein:
the nucleic acid molecule being conjugated to the polypeptide that binds to an FGF receptor; and wherein the nucleic acid molecule is bound to the nucleic acid binding domain; and wherein the composition binds to an FGF receptor and is internalized specifically in cells bearing the FGF receptor.

24. The composition of either of claims 1, 2, 8, or 23 wherein the polypeptide that binds to an FGF receptor is selected from the group consisting of an FGF-1 polypeptide, an FGF-2 polypeptide, an FGF-3 polypeptide, an FGF-4 polypeptide, an FGF-5 polypeptide, an FGF-6 polypeptide, an FGF-7 polypeptide, an FGF-8 polypeptide and an FGF-9 polypeptide.

25. The composition of either of claims 1, 2, 8, or 23 wherein the polypeptide that binds to an FGF receptor is an FGF-2 polypeptide.

26. The composition of claim 25 wherein FGF-2 has a serine residue at position 96.

27. The composition of either of claims 1, 2, 8, or 23 wherein the polypeptide that binds to an FGF receptor is an FGF-7 polypeptide.

28. The composition of claim 18 wherein the polypeptide that binds to an FGF receptor is selected from the group consisting of an FGF-1 polypeptide, an FGF-2 polypeptide, an FGF-3 polypeptide, an FGF-4 polypeptide, an FGF-5 polypeptide, an FGF-6 polypeptide, an FGF-7 polypeptide, an FGF-8 polypeptide and an FGF-9 polypeptide.

29. The composition of claim 18 wherein the polypeptide that binds to an FGF receptor is an FGF-2 polypeptide.

30. The composition of claim 29 wherein FGF-2 has a serine residue at position 96.

31. The composition of claim 18 wherein the polypeptide that binds to an FGF receptor is an FGF-7 polypeptide.

32. The composition of claim 18 wherein the cleavable linker is cleavable by a protease.

33. The composition of claim 32 wherein the protease is selected from the group consisting of cathepsin B, cathepsin D and trypsin.

34. The composition of claim 8 wherein the therapeutic protein-encoding agent encodes HSV-thymidine kinase or cytosine deaminase.

35. A composition having the formula:
receptor-binding internalized ligand―nucleic acid binding domain―polypeptide-encoding agent, wherein:
receptor-binding internalized ligand is a polypeptide reactive with a cell surface receptor;
nucleic acid binding domain binds to a polypeptide-encoding agent, the domain being conjugated or fused to the receptor-binding internalized ligand;
polypeptide-encoding agent is a nucleic acid molecule encoding a polypeptide, the agent being bound to the nucleic acid binding domain; and wherein
the receptor-binding internalized ligand―nucleic acid binding domain―polypeptide-encoding agent binds to the cell surface receptor and internalizes the polypeptide-encoding agent in cells bearing the receptor.

36. The composition of claim 35 wherein the receptor-binding internalized ligand is selected from the group consisting of a polypeptide reactive with a VEGF receptor and a polypeptide reactive with an HBEGF receptor.

37. The composition of claim 35 wherein the receptor-binding internalized ligand is a cytokine.

38. The composition of claim 35 wherein the polypeptide-encoding agent encodes a therapeutic protein.

39. The composition of claim 38 wherein the protein is a ribosome inactivating protein.

40. The composition of claim 39 wherein the ribosome inactivating protein is saporin.

41. The composition of claim 39 wherein the ribosome inactivating protein is gelonin.

42. The composition of claim 38 wherein the protein inhibits elongation factor 2.

43. The composition of claim 42 wherein the protein is diphtheria toxin.

44. The composition of claim 35 wherein the polypeptide-encoding agent encodes HSV-thymidine kinase.

45. The composition of claim 35 wherein the growth factor is a polypeptide reactive with the FGF receptor and the nucleic acid binding domain is poly-L-lysine.

46. The composition of claim 35 wherein the nucleic acid binding domain is selected from the group consisting of helix-turn-helix motif proteins, homeodomain proteins, zinc finger motif proteins, steroid receptor proteins, leucine zipper motif proteins, helix-loop-helix motif proteins, and â-sheet motif proteins.

47. The composition of claim 35 wherein the nucleic acid binding domain is selected from the group consisting of AP-1, Sp-1, *rev*, GCN4, ëcro, ëcI, TFIIA, myoD, retinoic acid receptor, glucocosteroid receptor, SV40 large T antigen, and GAL4.

48. The composition of claim 35 wherein the nucleic acid binding domain is selected from the group consisting of poly-L-lysine, protamine, histone, and spermine.

49. The composition of claim 35 wherein the nucleic acid binding domain binds a DNA molecule that encodes a ribosome inactivating protein.

50. The composition of claim 35 wherein the nucleic acid binding domain binds the coding region of saporin DNA.

51. The composition of claim 35 wherein the polypeptide-encoding agent further comprises a tissue-specific promoter.

52. The composition of claim 52 wherein the tissue-specific promoter is selected from the group consisting of alpha-crystalline, prostate-specific antigen, and gamma-crystalline promoter.

53. The composition of any one of claims 35-52, further comprising at least one linker that increases the serum stability or intracellular availability of the nucleic acid binding domain, the addition of said linker(s) resulting in the formula:
receptor-binding internalized ligand―(L)_{q}―nucleic acid binding domain-polypeptide encoding agent or the formula:
receptor-binding internalized ligand―(L)_{q}―nucleic acid binding domain-therapeutic protein encoding agent wherein:
L is at least one linker; and
q is 1 or more, such that the conjugate retains the ability to bind to a cell surface receptor and internalize the polypeptide/therapeutic protein-encoding agent, and wherein the polypeptide/therapeutic protein-encoding agent is bound to the nucleic acid binding domain.

54. The composition of claim 53 wherein the linker increases the flexibility of the conjugate.

55. The composition of claim 53 wherein the linker is a disulfide bond.

56. A composition according to any one of the claims 1, 35 or 53, for use in the manufacture of a medicament for effecting a genetic therapy, comprising contacting animal cells with said composition.

57. A composition according to any one of claims 1, 35 or 53, for use in the manufacture of a medicament for treating cancer, comprising contacting the cancer cells with an amount of the composition sufficient for inhibiting proliferation of the cancer cells.

58. A composition according to any one of claims 1, 35 or 53, for use in the manufacture of a medicament for treating smooth muscle cells hyperplasia, comprising contacting the smooth muscle cells with an amount of the composition sufficient for inhibiting hyperplasia of smooth muscle cells.

59. A composition having the formula:
receptor-binding internalized ligand-polypeptide encoding agent--nucleic acid binding domain, wherein:
receptor-binding internalized ligand is a polypeptide reactive with a cell surface receptor;
polypeptide-encoding agent is a nucleic acid molecule encoding a therapeutic protein, the agent being conjugated to the receptor-binding internalized ligand; and wherein the polypeptide-encoding agent is bound to the nucleic acid binding domain; and wherein the receptor-binding internalized ligand-polypeptide-encoding agent nucleic acid binding domain binds to the cell surface receptor and is internalized in cells bearing the receptor.

60. The composition of claim 59 wherein the nucleic acid binding domain is poly-L-lysine.

61. The composition of claim 59 wherein the receptor binding internalized ligand is a polypeptide reactive with an FGF receptor.


SEQUENCE LISTING

(1) GENERAL INFORMATION:

(i) APPLICANT: Prizm Pharmaceuticals, Inc.

(ii) TITLE OF INVENTION: COMPOSITIONS CONTAINING HEPARIN-BINDING
GROWTH FACTORS FOR GENE THERAPY AND TREATMENT OF ANTERIOR
EYE DISORDERS

(iii) NUMBER OF SEQUENCES: 95

(iv) CORRESPONDENCE ADDRESS:
(A) ADDRESSEE: SEED and BERRY
(B) STREET: 6300 Columbia Center, 701 Fifth Avenue
(C) CITY: Seattle
(D) STATE: Washington
(E) COUNTRY: USA
(F) ZIP: 98104-7092

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30

(vi) CURRENT APPLICATION DATA:
(A) APPLICATION NUMBER:
(B) FILING DATE: 15-MAR-1995
(C) CLASSIFICATION:

(viii) ATTORNEY/AGENT INFORMATION:
(A) NAME: Nottenburg, Carol
(B) REGISTRATION NUMBER: P-39,317
(C) REFERENCE/DOCKET NUMBER: 760100.406PC

(ix) TELECOMMUNICATION INFORMATION:
(A) TELEPHONE: (206) 622-4900
(B) TELEFAX: (206) 682-6031
(C) TELEX: 3723836 SEEDANDBERRY

(2) INFORMATION FOR SEQ ID NO:1:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: misc_recomb
(B) LOCATION: 6..11
(D) OTHER INFORMATION: /standard_name= "EcoRI Restriction Site"

(ix) FEATURE:
(A) NAME/KEY: sig_peptide
(B) LOCATION: 12..30
(D) OTHER INFORMATION: /function= "N-terminal extension"
/product= "Native saporin signal peptide"

(xi) SEQUENCE DESCRIPTION: SEQ ID NO:1:

CTGCAGAATT CGCATGGATC CTGCTTCAAT 30

(2) INFORMATION FOR SEQ ID NO:2:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(ix) FEATURE:
(A) NAME/KEY: misc_recomb
(B) LOCATION: 6..11
(D) OTHER INFORMATION: /standard_name= "EcoRI Restriction Site"

(ix) FEATURE:
(A) NAME/KEY: terminator

(B) LOCATION: 23..25
(D) OTHER INFORMATION: /note= "Anti-sense stop codon"

(ix) FEATURE:
(A) NAME/KEY: mat_peptide
(B) LOCATION: 26..30
(D) OTHER INFORMATION: /note= "Anti-sense to carboxyl
terminus of mature peptide"

(xi) SEQUENCE DESCRIPTION: SEQ ID NO:2:

CTGCAGAATT CGCCTCGTTT GACTACTTTG

(2) INFORMATION FOR SEQ ID NO:3:

(i) SEQUENCE CHARACTERISTICS:
(A) LENGTH: 804 base pairs
(B) TYPE: nucleic acid
(C) STRANDEDNESS: double
(D) TOPOLOGY: unknown

(ii) MOLECULE TYPE: cDNA

(ix) FEATURE:
(A) NAME/KEY: CDS
(B) LOCATION: 1..804

(ix) FEATURE:
(A) NAME/KEY: misc_feature
(B) LOCATION: 1..804
(D) OTHER INFORMATION: /note= "Nucleotide sequence
corresponding to the clone M13 mp18-G4 in Example I.B.2."

(ix) FEATURE:
(A) NAME/KEY: mat_peptide
(B) LOCATION: 46..804
(D) OTHER INFORMATION: /product= ""Saporin""

(xi) SEQUENCE DESCRIPTION: SEQ ID NO:3:

GCA TGG ATC CTG CTT CAA TTT TCA GCT TGG ACA ACA ACT GAT GCG GTC
Ala Trp Ile Leu Leu Gln Phe Ser Ala Trp Thr Thr Thr Asp Ala Val
-15 -10 -5 1

ACA TCA ATC ACA TTA GAT CTA GTA AAT CCG ACC GCG GGT CAA TAC TCA
Thr Ser Ile Thr Leu Asp Leu Val Asn Pro Thr Ala Gly Gln Tyr Ser
                5                   10                  15

TCT TTT GTG GAT AAA ATC CGA AAC AAT GTA AAG GAT CCA AAC CTG AAA
Ser Phe Val Asp Lys Ile Arg Asn Asn Val Lys Asp Pro Asn Leu Lys
            20                  25                  30

TAC GGT GGT ACC GAC ATA GCC GTG ATA GGC CCA CCT TCT AAA GAA AAA
Tyr Gly Gly Thr Asp Ile Ala Val Ile Gly Pro Pro Ser Lys Glu Lys

TTC CTT AGA ATT AAT TTC CAA AGT TCC CGA GGA ACG GTC TCA CTT GGC
Phe Leu Arg Ile Asn Phe Gln Ser Ser Arg Gly Thr Val Ser Leu Gly
 50                 55                  60                  65

CTA AAA CGC GAT AAC TTG TAT GTG GTC GCG TAT CTT GCA ATG GAT AAC
Leu Lys Arg Asp Asn Leu Tyr Val Val Ala Tyr Leu Ala Met Asp Asn
                70                  75                  80

ACG AAT GTT AAT CGG GCA TAT TAC TTC AAA TCA GAA ATT ACT TCC GCC
Thr Asn Val Asn Arg Ala Tyr Tyr Phe Lys Ser Glu Ile Thr Ser Ala
            85                  90                  95

GAG TTA ACC GCC CTT TTC CCA GAG GCC ACA ACT GCA AAT CAG AAA GCT
Glu Leu Thr Ala Leu Phe Pro Glu Ala Thr Thr Ala Asn Gln Lys Ala
        100                 105                 110

TTA GAA TAC ACA GAA GAT TAT CAG TCG ATC GAA AAG AAT GCC CAG ATA
Leu Glu Tyr Thr Glu Asp Tyr Gln Ser Ile Glu Lys Asn Ala Gln Ile
    115                 120                 125

ACA CAG GGA GAT AAA AGT AGA AAA GAA CTC GGG TTG GGG ATC GAC TTA
Thr Gln Gly Asp Lys Ser Arg Lys Glu Leu Gly Leu Gly Ile Asp Leu
130                 135                 140                 145

CTT TTG ACG TTC ATG GAA GCA GTG AAC AAG AAG GCA CGT GTG GTT AAA
Leu Leu Thr Phe Met Glu Ala Val Asn Lys Lys Ala Arg Val Val Lys
                150                 155                 160

AAC GAA GCT AGG TTT CTG CTT ATC GCT ATT CAA ATG ACA GCT GAG GTA
Asn Glu Ala Arg Phe Leu Leu Ile Ala Ile Gln Met Thr Ala Glu Val
            165                 170                 175

GCA CGA TTT AGG TAC ATT CAA AAC TTG GTA ACT AAG AAC TTC CCC AAC
Ala Arg Phe Arg Tyr Ile Gln Asn Leu Val Thr Lys Asn Phe Pro Asn
        180                 185                 190

AAG TTC GAC TCG GAT AAC AAG GTG ATT CAA TTT GAA GTC AGC TGG CGT
Lys Phe Asp Ser Asp Asn Lys Val Ile Gln Phe Glu Val Ser Trp Arg
    195                 200                 205

AAG ATT TCT ACG GCA ATA TAC GGG GAT GCC AAA AAC GGC GTG TTT AAT
Lys Ile Ser Thr Ala Ile Tyr Gly Asp Ala Lys Asn Gly Val Phe Asn
210                 215                 220                 225

AAA GAT TAT GAT TTC GGG TTT GGA AAA GTG AGG CAG GTG AAG GAC TTG
Lys Asp Tyr Asp Phe Gly Phe Gly Lys Val Arg Gln Val Lys Asp Leu
                230                 235                 240

CAA ATG GGA CTC CTT ATG TAT TTG GGC AAA CCA AAG
Gln Met Gly Leu Leu Met Tyr Leu Gly Lys Pro Lys
            245                 250

(2) INFORMATION FOR SEQ ID NO:4:

(i) SEQUENCE CHARACTERISTICS:
(A) LENGTH: 804 base pairs
(B) TYPE: nucleic acid
(C) STRANDEDNESS: double
(D) TOPOLOGY: unknown

(ii) MOLECULE TYPE: cDNA

(ix) FEATURE:
(A) NAME/KEY: CDS
(B) LOCATION: 1..804

(ix) FEATURE:
(A) NAME/KEY: misc_feature
(B) LOCATION: 1..804
(D) OTHER INFORMATION: /note= "Nucleotide sequence
corresponding to the clone M13 mp18-G1 in Example I.B.2."

(ix) FEATURE:
(A) NAME/KEY: mat_peptide
(B) LOCATION: 46..804
(D) OTHER INFORMATION: /product= "Saporin"

(xi) SEQUENCE DESCRIPTION: SEQ ID NO:4:

GCA TGG ATC CTG CTT CAA TTT TCA GCT TGG ACA ACA ACT GAT GCG GTC
Ala Trp Ile Leu Leu Gln Phe Ser Ala Trp Thr Thr Thr Asp Ala Val
-15 -10 -5 1

ACA TCA ATC ACA TTA GAT CTA GTA AAT CCG ACC GCG GGT CAA TAC TCA
Thr Ser Ile Thr Leu Asp Leu Val Asn Pro Thr Ala Gly Gln Tyr Ser
                5                   10                  15

TCT TTT GTG GAT AAA ATC CGA AAC AAC GTA AAG GAT CCA AAC CTG AAA
Ser Phe Val Asp Lys Ile Arg Asn Asn Val Lys Asp Pro Asn Leu Lys
            20                  25                  30

TAC GGT GGT ACC GAC ATA GCC GTG ATA GGC CCA CCT TCT AAA GAA AAA
Tyr Gly Gly Thr Asp Ile Ala Val Ile Gly Pro Pro Ser Lys Glu Lys
        35                  40                  45

TTC CTT AGA ATT AAT TTC CAA AGT TCC CGA GGA ACG GTC TCA CTT GGC
Phe Leu Arg Ile Asn Phe Gln Ser Ser Arg Gly Thr Val Ser Leu Gly
50 55 60 65

CTA AAA CGC GAT AAC TTG TAT GTG GTC GCG TAT CTT GCA ATG GAT AAC
Leu Lys Arg Asp Asn Leu Tyr Val Val Ala Tyr Leu Ala Met Asp Asn
70 75 80

ACG AAT GTT AAT CGG GCA TAT TAC TTC AGA TCA GAA ATT ACT TCC GCC
Thr Asn Val Asn Arg Ala Tyr Tyr Phe Arg Ser Glu Ile Thr Ser Ala
                85                  90                  95

GAG TTA ACC GCC CTT TTC CCA GAG GCC ACA ACT GCA AAT CAG AAA GCT

Glu Leu Thr Ala Leu Phe Pro Glu Ala Thr Thr Ala Asn Gln Lys Ala
        100                 105                 110

TTA GAA TAC ACA GAA GAT TAT CAG TCG ATC GAA AAG AAT GCC CAG ATA
Leu Glu Tyr Thr Glu Asp Tyr Gln Ser Ile Glu Lys Asn Ala Gln Ile
    115                 120                 125

ACA CAG GGA GAT AAA TCA AGA AAA GAA CTC GGG TTG GGG ATC GAC TTA
Thr Gln Gly Asp Lys Ser Arg Lys Glu Leu Gly Leu Gly Ile Asp Leu
130                 135                 140                 145

CTT TTG ACG TCC ATG GAA GCA GTG AAC AAG AAG GCA CGT GTG GTT AAA
Leu Leu Thr Ser Met Glu Ala Val Asn Lys Lys Ala Arg Val Val Lys
                150                 155                 160

AAC GAA GCT AGG TTT CTG CTT ATC GCT ATT CAA ATG ACA GCT GAG GTA
Asn Glu Ala Arg Phe Leu Leu Ile Ala Ile Gln Met Thr Ala Glu Val
            165                 170                 175

GCA CGA TTT CGG TAC ATT CAA AAC TTG GTA ACT AAG AAC TTC CCC AAC
Ala Arg Phe Arg Tyr Ile Gln Asn Leu Val Thr Lys Asn Phe Pro Asn
        180                 185                 190

AAG TTC GAC TCG GAT AAC AAG GTG ATT CAA TTT GAA GTC AGC TGG CGT
Lys Phe Asp Ser Asp Asn Lys Val Ile Gln Phe Glu Val Ser Trp Arg
    195                 200                 205

AAG ATT TCT ACG GCA ATA TAC GGA GAT GCC AAA AAC GGC GTG TTT AAT
Lys Ile Ser Thr Ala Ile Tyr Gly Asp Ala Lys Asn Gly Val Phe Asn
210                 215                 220                 225

AAA GAT TAT GAT TTC GGG TTT GGA AAA GTG AGG CAG GTG AAG GAC TTG
Lys Asp Tyr Asp Phe Gly Phe Gly Lys Val Arg Gln Val Lys Asp Leu
                230                 235                 240

CAA ATG GGA CTC CTT ATG TAT TTG GGC AAA CCA AAG
Gln Met Gly Leu Leu Met Tyr Leu Gly Lys Pro Lys
            245                 250

(2) INFORMATION FOR SEQ ID NO:5:

     (i) SEQUENCE CHARACTERISTICS:
          (A) LENGTH: 804 base pairs
          (B) TYPE: nucleic acid
          (C) STRANDEDNESS: double
          (D) TOPOLOGY: unknown

    (ii) MOLECULE TYPE: cDNA

    (ix) FEATURE:
          (A) NAME/KEY: CDS
          (B) LOCATION: 1..804

    (ix) FEATURE:
          (A) NAME/KEY: misc_feature

(B) LOCATION: 1..804
(D) OTHER INFORMATION: /note= "Nucleotide sequence
corresponding to the clone M13 mp18-G2 in Example I.B.2."

(ix) FEATURE:
(A) NAME/KEY: mat_peptide
(B) LOCATION: 46..804
(D) OTHER INFORMATION: /product= "Saporin"

(xi) SEQUENCE DESCRIPTION: SEQ ID NO:5:

GCA TGG ATC CTG CTT CAA TTT TCA GCT TGG ACA ACA ACT GAT GCG GTC
Ala Trp Ile Leu Leu Gln Phe Ser Ala Trp Thr Thr Thr Asp Ala Val
-15 -10 -5 1

ACA TCA ATC ACA TTA GAT CTA GTA AAT CCG ACT GCG GGT CAA TAC TCA
Thr Ser Ile Thr Leu Asp Leu Val Asn Pro Thr Ala Gly Gln Tyr Ser
                5                   10                  15

TCT TTT GTG GAT AAA ATC CGA AAC AAC GTA AAG GAT CCA AAC CTG AAA
Ser Phe Val Asp Lys Ile Arg Asn Asn Val Lys Asp Pro Asn Leu Lys
            20                  25                  30

TAC GGT GGT ACC GAC ATA GCC GTG ATA GGC CCA CCT TCT AAA GAT AAA
Tyr Gly Gly Thr Asp Ile Ala Val Ile Gly Pro Pro Ser Lys Asp Lys
        35                  40                  45

TTC CTT AGA ATT AAT TTC CAA AGT TCC CGA GGA ACG GTC TCA CTT GGC
Phe Leu Arg Ile Asn Phe Gln Ser Ser Arg Gly Thr Val Ser Leu Gly
50 55 60 65

CTA AAA CGC GAT AAC TTG TAT GTG GTC GCG TAT CTT GCA ATG GAT AAC
Leu Lys Arg Asp Asn Leu Tyr Val Val Ala Tyr Leu Ala Met Asp Asn
70 75 80

ACG AAT GTT AAT CGG GCA TAT TAC TTC AAA TCA GAA ATT ACT TCC GCC
Thr Asn Val Asn Arg Ala Tyr Tyr Phe Lys Ser Glu Ile Thr Ser Ala
                85                  90                  95

GAG TTA ACC GCC CTT TTC CCA GAG GCC ACA ACT GCA AAT CAG AAA GCT
Glu Leu Thr Ala Leu Phe Pro Glu Ala Thr Thr Ala Asn Gln Lys Ala
            100                 105                 110

TTA GAA TAC ACA GAA GAT TAT CAG TCG ATC GAA AAG AAT GCC CAG ATA
Leu Glu Tyr Thr Glu Asp Tyr Gln Ser Ile Glu Lys Asn Ala Gln Ile
        115                 120                 125

ACA CAG GGA GAT AAA AGT AGA AAA GAA CTC GGG TTG GGG ATC GAC TTA
Thr Gln Gly Asp Lys Ser Arg Lys Glu Leu Gly Leu Gly Ile Asp Leu
130 135 140 145

CTT TTG ACG TTC ATG GAA GCA GTG AAC AAG AAG GCA CGT GTG GTT AAA
Leu Leu Thr Phe Met Glu Ala Val Asn Lys Lys Ala Arg Val Val Lys
150 155 160

AAC GAA GCT AGG TTT CTG CTT ATC GCT ATT CAA ATG ACA GCT GAG GTA
Asn Glu Ala Arg Phe Leu Leu Ile Ala Ile Gln Met Thr Ala Glu Val
            165                 170                     175

GCA CGA TTT AGG TAC ATT CAA AAC TTG GTA ACT AAG AAC TTC CCC AAC
Ala Arg Phe Arg Tyr Ile Gln Asn Leu Val Thr Lys Asn Phe Pro Asn
        180                 185                 190

AAG TTC GAC TCG GAT AAC AAG GTG ATT CAA TTT GAA GTC AGC TGG CGT
Lys Phe Asp Ser Asp Asn Lys Val Ile Gln Phe Glu Val Ser Trp Arg
    195                 200                 205

AAG ATT TCT ACG GCA ATA TAC GGG GAT GCC AAA AAC GGC GTG TTT AAT
Lys Ile Ser Thr Ala Ile Tyr Gly Asp Ala Lys Asn Gly Val Phe Asn
210                 215                 220                     225

AAA GAT TAT GAT TTC GGG TTT GGA AAA GTG AGG CAG GTG AAG GAC TTG
Lys Asp Tyr Asp Phe Gly Phe Gly Lys Val Arg Gln Val Lys Asp Leu
                230                 235                 240

CAA ATG GGA CTC CTT ATG TAT TTG GGC AAA CCA AAG
Gln Met Gly Leu Leu Met Tyr Leu Gly Lys Pro Lys
            245                 250

(2) INFORMATION FOR SEQ ID NO:6:

     (i) SEQUENCE CHARACTERISTICS:
          (A) LENGTH: 804 base pairs
          (B) TYPE: nucleic acid
          (C) STRANDEDNESS: double
          (D) TOPOLOGY: unknown

    (ii) MOLECULE TYPE: cDNA

    (ix) FEATURE:
          (A) NAME/KEY: CDS
          (B) LOCATION: 1..804

    (ix) FEATURE:
          (A) NAME/KEY: misc_feature
          (B) LOCATION: 1..804
          (D) OTHER INFORMATION: /note= "Nucleotide sequence
               corresponding to the clone M13 mp18-G7 in Example I.B.2."

    (ix) FEATURE:
          (A) NAME/KEY: mat_peptide
          (B) LOCATION: 46..804
          (D) OTHER INFORMATION: /product= "Saporin"

    (xi) SEQUENCE DESCRIPTION: SEQ ID NO:6:

GCA TGG ATC CTG CTT CAA TTT TCA GCT TGG ACA ACA ACT GAT GCG GTC
Ala Trp Ile Leu Leu Gln Phe Ser Ala Trp Thr Thr Thr Asp Ala Val
-15                 -10                 -5                      1

ACA TCA ATC ACA TTA GAT CTA GTA AAT CCG ACC GCG GGT CAA TAC TCA
Thr Ser Ile Thr Leu Asp Leu Val Asn Pro Thr Ala Gly Gln Tyr Ser
                5                   10                  15

TCT TTT GTG GAT AAA ATC CGA AAC AAC GTA AAG GAT CCA AAC CTG AAA
Ser Phe Val Asp Lys Ile Arg Asn Asn Val Lys Asp Pro Asn Leu Lys
            20                  25                  30

TAC GGT GGT ACC GAC ATA GCC GTG ATA GGC CCA CCT TCT AAA GAA AAA
Tyr Gly Gly Thr Asp Ile Ala Val Ile Gly Pro Pro Ser Lys Glu Lys
        35                  40                  45

TTC CTT AGA ATT AAT TTC CAA AGT TCC CGA GGA ACG GTC TCA CTT GGC
Phe Leu Arg Ile Asn Phe Gln Ser Ser Arg Gly Thr Val Ser Leu Gly
    50                  55                  60                  65

CTA AAA CGC GAT AAC TTG TAT GTG GTC GCG TAT CTT GCA ATG GAT AAC
Leu Lys Arg Asp Asn Leu Tyr Val Val Ala Tyr Leu Ala Met Asp Asn
                    70                  75                  80

ACG AAT GTT AAT CGG GCA TAT TAC TTC AGA TCA GAA ATT ACT TCC GCC
Thr Asn Val Asn Arg Ala Tyr Tyr Phe Arg Ser Glu Ile Thr Ser Ala
                85                  90                  95

GAG TTA ACC GCC CTT TTC CCA GAG GCC ACA ACT GCA AAT CAG AAA GCT
Glu Leu Thr Ala Leu Phe Pro Glu Ala Thr Thr Ala Asn Gln Lys Ala
            100                 105                 110

TTA GAA TAC ACA GAA GAT TAT CAG TCG ATC GAA AAG AAT GCC CAG ATA
Leu Glu Tyr Thr Glu Asp Tyr Gln Ser Ile Glu Lys Asn Ala Gln Ile
        115                 120                 125

ACA CAG GGA GAT AAA TCA AGA AAA GAA CTC GGG TTG GGG ATC GAC TTA
Thr Gln Gly Asp Lys Ser Arg Lys Glu Leu Gly Leu Gly Ile Asp Leu
130                 135                 140                 145

CTT TTG ACG TCC ATG GAA GCA GTG AAC AAG AAG GCA CGT GTG GTT AAA
Leu Leu Thr Ser Met Glu Ala Val Asn Lys Lys Ala Arg Val Val Lys
                150                 155                 160

AAC GAA GCT AGA TTC CTT CTT ATC GCT ATT CAG ATG ACG GCT GAG GCA
Asn Glu Ala Arg Phe Leu Leu Ile Ala Ile Gln Met Thr Ala Glu Ala
            165                 170                 175

GCA CGA TTT AGG TAC ATA CAA AAC TTG GTA ATC AAG AAC TTT CCC AAC
Ala Arg Phe Arg Tyr Ile Gln Asn Leu Val Ile Lys Asn Phe Pro Asn
        180                 185                 190

AAG TTC AAC TCG GAA AAC AAA GTG ATT CAG TTT GAG GTT AAC TGG AAA
Lys Phe Asn Ser Glu Asn Lys Val Ile Gln Phe Glu Val Asn Trp Lys
    195                 200                 205

AAA ATT TCT ACG GCA ATA TAC GGG GAT GCC AAA AAC GGC GTG TTT AAT
Lys Ile Ser Thr Ala Ile Tyr Gly Asp Ala Lys Asn Gly Val Phe Asn
210                 215                 220                 225

AAA GAT TAT GAT TTC GGG TTT GGA AAA GTG AGG CAG GTG AAG GAC TTG 768
Lys Asp Tyr Asp Phe Gly Phe Gly Lys Val Arg Gln Val Lys Asp Leu
230 235 240

CAA ATG GGA CTC CTT ATG TAT TTG GGC AAA CCA AAG 804
Gln Met Gly Leu Leu Met Tyr Leu Gly Lys Pro Lys
                245                 250

(2) INFORMATION FOR SEQ ID NO:7:

(i) SEQUENCE CHARACTERISTICS:
(A) LENGTH: 804 base pairs
(B) TYPE: nucleic acid
(C) STRANDEDNESS: double
(D) TOPOLOGY: unknown

(ii) MOLECULE TYPE: cDNA

(ix) FEATURE:
(A) NAME/KEY: CDS
(B) LOCATION: 1..804

(ix) FEATURE:
(A) NAME/KEY: misc_feature
(B) LOCATION: 1..804
(D) OTHER INFORMATION: /note= "Nucleotide sequence
corresponding to the clone M13 mp18-G9 in Example I.B.2."

(ix) FEATURE:
(A) NAME/KEY: mat_peptide
(B) LOCATION: 46..804
(D) OTHER INFORMATION: /product= "Saporin"

(xi) SEQUENCE DESCRIPTION: SEQ ID NO:7:

GCA TGG ATC CTG CTT CAA TTT TCA GCT TGG ACA ACA ACT GAT GCG GTC 48
Ala Trp Ile Leu Leu Gln Phe Ser Ala Trp Thr Thr Thr Asp Ala Val
-15 -10 -5 1

ACA TCA ATC ACA TTA GAT CTA GTA AAT CCG ACC GCG GGT CAA TAC TCA 96
Thr Ser Ile Thr Leu Asp Leu Val Asn Pro Thr Ala Gly Gln Tyr Ser
                5                   10                  15

TCT TTT GTG GAT AAA ATC CGA AAC AAC GTA AAG GAT CCA AAC CTG AAA 144
Ser Phe Val Asp Lys Ile Arg Asn Asn Val Lys Asp Pro Asn Leu Lys
            20                  25                  30

TAC GGT GGT ACC GAC ATA GCC GTG ATA GGC CCA CCT TCT AAA GAA AAA 192
Tyr Gly Gly Thr Asp Ile Ala Val Ile Gly Pro Pro Ser Lys Glu Lys
        35                  40                  45

TTC CTT AGA ATT AAT TTC CAA AGT TCC CGA GGA ACG GTC TCA CTT GGC 240
Phe Leu Arg Ile Asn Phe Gln Ser Ser Arg Gly Thr Val Ser Leu Gly
50 55 60 65

CTA AAA CGC GAT AAC TTG TAT GTG GTC GCG TAT CTT GCA ATG GAT AAC 288
Leu Lys Arg Asp Asn Leu Tyr Val Val Ala Tyr Leu Ala Met Asp Asn
70 75 80

ACG AAT GTT AAT CGG GCA TAT TAC TTC AGA TCA GAA ATT ACT TCC GCC 336
Thr Asn Val Asn Arg Ala Tyr Tyr Phe Arg Ser Glu Ile Thr Ser Ala
                85                  90                  95

GAG TTA ACC GCC CTT TTC CCA GAG GCC ACA ACT GCA AAT CAG AAA GCT 384
Glu Leu Thr Ala Leu Phe Pro Glu Ala Thr Thr Ala Asn Gln Lys Ala
            100                 105                 110

TTA GAA TAC ACA GAA GAT TAT CAG TCG ATT GAA AAG AAT GCC CAG ATA 432
Leu Glu Tyr Thr Glu Asp Tyr Gln Ser Ile Glu Lys Asn Ala Gln Ile
        115                 120                 125

ACA CAA GGA GAT CAA AGT AGA AAA GAA CTC GGG TTG GGG ATT GAC TTA 480
Thr Gln Gly Asp Gln Ser Arg Lys Glu Leu Gly Leu Gly Ile Asp Leu
130 135 140 145

CTT TCA ACG TCC ATG GAA GCA GTG AAC AAG AAG GCA CGT GTG GTT AAA 528
Leu Ser Thr Ser Met Glu Ala Val Asn Lys Lys Ala Arg Val Val Lys
150 155 160

GAC GAA GCT AGA TTC CTT CTT ATC GCT ATT CAG ATG ACG GCT GAG GCA 576
Asp Glu Ala Arg Phe Leu Leu Ile Ala Ile Gln Met Thr Ala Glu Ala
                165                 170                 175

GCG CGA TTT AGG TAC ATA CAA AAC TTG GTA ATC AAG AAC TTT CCC AAC 624
Ala Arg Phe Arg Tyr Ile Gln Asn Leu Val Ile Lys Asn Phe Pro Asn
            180                 185                 190

AAG TTC AAC TCG GAA AAC AAA GTG ATT CAG TTT GAG GTT AAC TGG AAA 672
Lys Phe Asn Ser Glu Asn Lys Val Ile Gln Phe Glu Val Asn Trp Lys
        195                 200                 205

AAA ATT TCT ACG GCA ATA TAC GGG GAT GCC AAA AAC GGC GTG TTT AAT 720
Lys Ile Ser Thr Ala Ile Tyr Gly Asp Ala Lys Asn Gly Val Phe Asn
210 215 220 225

AAA GAT TAT GAT TTC GGG TTT GGA AAA GTG AGG CAG GTG AAG GAC TTG 768
Lys Asp Tyr Asp Phe Gly Phe Gly Lys Val Arg Gln Val Lys Asp Leu
230 235 240

CAA ATG GGA CTC CTT ATG TAT TTG GGC AAA CCA AAG 804
Gln Met Gly Leu Leu Met Tyr Leu Gly Lys Pro Lys
                245                 250

(2) INFORMATION FOR SEQ ID NO:8:

(i) SEQUENCE CHARACTERISTICS:
(A) LENGTH: 22 base pairs
(B) TYPE: nucleic acid
(C) STRANDEDNESS: single

(D) TOPOLOGY: linear

(ii) MOLECULE TYPE: DNA (genomic)

(ix) FEATURE:
(A) NAME/KEY: misc_recomb
(B) LOCATION: 10..15
(D) OTHER INFORMATION: /standard_name= "Nco I restriction enzyme
recognition site"

(ix) FEATURE:
(A) NAME/KEY: mat_peptide
(B) LOCATION: 15..22
(D) OTHER INFORMATION: /product= "N-terminus of Saporin
protein"

(xi) SEQUENCE DESCRIPTION: SEQ ID NO:8:

CAACAACTGC CATGGTCACA TC 22

(2) INFORMATION FOR SEQ ID NO:9:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: misc_recomb
(B) LOCATION: 11..16
(D) OTHER INFORMATION: /standard_name= "Nco I restriction enzyme
recognition site."

(ix) FEATURE:
(A) NAME/KEY: mat_peptide
(B) LOCATION: 1..10
(D) OTHER INFORMATION: /product= "Carboxy terminus of
mature FGF protein"

(xi) SEQUENCE DESCRIPTION: SEQ ID NO:9:

GCTAAGAGCG CCATGGAGA 19

(2) INFORMATION FOR SEQ ID NO:10:

(i) SEQUENCE CHARACTERISTICS:
(A) LENGTH: 21 base pairs
(B) TYPE: nucleic acid
(C) STRANDEDNESS: double
(D) TOPOLOGY: unknown

(ii) MOLECULE TYPE: cDNA

(ix) FEATURE:
(A) NAME/KEY: CDS
(B) LOCATION: 1..12
(D) OTHER INFORMATION: /product= "Carboxy terminus of
wild type FGF"

(ix) FEATURE:
(A) NAME/KEY: misc_recomb
(B) LOCATION: 13..18
(D) OTHER INFORMATION: /standard_name= "Nco I restriction enzyme
recognition site"

(xi) SEQUENCE DESCRIPTION: SEQ ID NO:10:

GCT AAG AGC TGACCATGGA GA 21
Ala Lys Ser
1

(2) INFORMATION FOR SEQ ID NO:11:

(i) SEQUENCE CHARACTERISTICS:
(A) LENGTH: 102 base pairs
(B) TYPE: nucleic acid
(C) STRANDEDNESS: double
(D) TOPOLOGY: linear

(ii) MOLECULE TYPE: cDNA

(ix) FEATURE:
(A) NAME/KEY: CDS
(B) LOCATION: 1..96
(D) OTHER INFORMATION: /product= "pFGFNcoI"
/note= "Equals the plasmid pFC80 wih native FGF
stop codon removed."

(ix) FEATURE:
(A) NAME/KEY: misc_recomb
(B) LOCATION: 29..34
(D) OTHER INFORMATION: /standard_name= "Nco I restriction enzyme
recognition site"

(xi) SEQUENCE DESCRIPTION: SEQ ID NO:11:

CTT TTT CTT CCA ATG TCT GCT AAG AGC GCC ATG GAG ATC CGG CTG AAT 48
Leu Phe Leu Pro Met Ser Ala Lys Ser Ala Met Glu Ile Arg Leu Asn
1               5                   10                  15

GGT GCA GTT CTG TAC CGG TTT TCC TGT GCC GTC TTT CAG GAC TCC TGAAATCTT 102
Gly Ala Val Leu Tyr Arg Phe Ser Cys Ala Val Phe Gln Asp Ser
            20                  25                  30

(2) INFORMATION FOR SEQ ID NO:12:

(i) SEQUENCE CHARACTERISTICS:

(A) LENGTH: 1230 base pairs
(B) TYPE: nucleic acid
(C) STRANDEDNESS: double
(D) TOPOLOGY: unknown

(ii) MOLECULE TYPE: cDNA

(ix) FEATURE:
(A) NAME/KEY: CDS
(B) LOCATION: 1..1230

(ix) FEATURE:
(A) NAME/KEY: mat_peptide
(B) LOCATION: 1..465
(D) OTHER INFORMATION: /product= "bFGF"

(ix) FEATURE:
(A) NAME/KEY: mat_peptide
(B) LOCATION: 472..1230
(D) OTHER INFORMATION: /product= "Saporin"

(xi) SEQUENCE DESCRIPTION: SEQ ID NO:12:

ATG GCA GCA GGA TCA ATA ACA ACA TTA CCC GCC TTG CCC GAG GAT GGC 48
Met Ala Ala Gly Ser Ile Thr Thr Leu Pro Ala Leu Pro Glu Asp Gly
1               5                   10                  15

GGC AGC GGC GCC TTC CCG CCC GGC CAC TTC AAG GAC CCC AAG CGG CTG 96
Gly Ser Gly Ala Phe Pro Pro Gly His Phe Lys Asp Pro Lys Arg Leu
            20                  25                  30

TAC TGC AAA AAC GGG GGC TTC TTC CTG CGC ATC CAC CCC GAC GGC CGA 144
Tyr Cys Lys Asn Gly Gly Phe Phe Leu Arg Ile His Pro Asp Gly Arg
        35                  40                  45

GTT GAC GGG GTC CGG GAG AAG AGC GAC CCT CAC ATC AAG CTT CAA CTT 192
Val Asp Gly Val Arg Glu Lys Ser Asp Pro His Ile Lys Leu Gln Leu
    50                  55                  60

CAA GCA GAA GAG AGA GGA GTT GTG TCT ATC AAA GGA GTG TGT GCT AAC 240
Gln Ala Glu Glu Arg Gly Val Val Ser Ile Lys Gly Val Cys Ala Asn
65                  70                  75                  80

CGT TAC CTG GCT ATG AAG GAA GAT GGA AGA TTA CTG GCT TCT AAA TGT 288
Arg Tyr Leu Ala Met Lys Glu Asp Gly Arg Leu Leu Ala Ser Lys Cys
                85                  90                  95

GTT ACG GAT GAG TGT TTC TTT TTT GAA CGA TTG GAA TCT AAT AAC TAC 336
Val Thr Asp Glu Cys Phe Phe Phe Glu Arg Leu Glu Ser Asn Asn Tyr
            100                 105                 110

AAT ACT TAC CGG TCA AGG AAA TAC ACC AGT TGG TAT GTG GCA TTG AAA 384
Asn Thr Tyr Arg Ser Arg Lys Tyr Thr Ser Trp Tyr Val Ala Leu Lys
        115                 120                 125

CGA ACT GGG CAG TAT AAA CTT GGA TCC AAA ACA GGA CCT GGG CAG AAA 432
Arg Thr Gly Gln Tyr Lys Leu Gly Ser Lys Thr Gly Pro Gly Gln Lys
    130                 135                 140

GCT ATA CTT TTT CTT CCA ATG TCT GCT AAG AGC GCC ATG GTC ACA TCA 480
Ala Ile Leu Phe Leu Pro Met Ser Ala Lys Ser Ala Met Val Thr Ser
145                 150                 155                 160

ATC ACA TTA GAT CTA GTA AAT CCG ACC GCG GGT CAA TAC TCA TCT TTT 528
Ile Thr Leu Asp Leu Val Asn Pro Thr Ala Gly Gln Tyr Ser Ser Phe
                165                 170                 175

GTG GAT AAA ATC CGA AAC AAC GTA AAG GAT CCA AAC CTG AAA TAC GGT 576
Val Asp Lys Ile Arg Asn Asn Val Lys Asp Pro Asn Leu Lys Tyr Gly
            180                 185                 190

GGT ACC GAC ATA GCC GTG ATA GGC CCA CCT TCT AAA GAA AAA TTC CTT 624
Gly Thr Asp Ile Ala Val Ile Gly Pro Pro Ser Lys Glu Lys Phe Leu
        195                 200                 205

AGA ATT AAT TTC CAA AGT TCC CGA GGA ACG GTC TCA CTT GGC CTA AAA 672
Arg Ile Asn Phe Gln Ser Ser Arg Gly Thr Val Ser Leu Gly Leu Lys
    210                 215                 220

CGC GAT AAC TTG TAT GTG GTC GCG TAT CTT GCA ATG GAT AAC ACG AAT 720
Arg Asp Asn Leu Tyr Val Val Ala Tyr Leu Ala Met Asp Asn Thr Asn
225                 230                 235                 240

GTT AAT CGG GCA TAT TAC TTC AAA TCA GAA ATT ACT TCC GCC GAG TTA 768
Val Asn Arg Ala Tyr Tyr Phe Lys Ser Glu Ile Thr Ser Ala Glu Leu
                245                 250                 255

ACC GCC CTT TTC CCA GAG GCC ACA ACT GCA AAT CAG AAA GCT TTA GAA 816
Thr Ala Leu Phe Pro Glu Ala Thr Thr Ala Asn Gln Lys Ala Leu Glu
            260                 265                 270

TAC ACA GAA GAT TAT CAG TCG ATC GAA AAG AAT GCC CAG ATA ACA CAG 864
Tyr Thr Glu Asp Tyr Gln Ser Ile Glu Lys Asn Ala Gln Ile Thr Gln
        275                 280                 285

GGA GAT AAA AGT AGA AAA GAA CTC GGG TTG GGG ATC GAC TTA CTT TTG 912
Gly Asp Lys Ser Arg Lys Glu Leu Gly Leu Gly Ile Asp Leu Leu Leu
    290                 295                 300

ACG TTC ATG GAA GCA GTG AAC AAG AAG GCA CGT GTG GTT AAA AAC GAA 960
Thr Phe Met Glu Ala Val Asn Lys Lys Ala Arg Val Val Lys Asn Glu
305                 310                 315                 320

GCT AGG TTT CTG CTT ATC GCT ATT CAA ATG ACA GCT GAG GTA GCA CGA 1008
Ala Arg Phe Leu Leu Ile Ala Ile Gln Met Thr Ala Glu Val Ala Arg
                325                 330                 335

TTT AGG TAC ATT CAA AAC TTG GTA ACT AAG AAC TTC CCC AAC AAG TTC 1056
Phe Arg Tyr Ile Gln Asn Leu Val Thr Lys Asn Phe Pro Asn Lys Phe
            340                 345                 350

GAC TCG GAT AAC AAG GTG ATT CAA TTT GAA GTC AGC TGG CGT AAG ATT 1104
Asp Ser Asp Asn Lys Val Ile Gln Phe Glu Val Ser Trp Arg Lys Ile
        355                 360                 365

TCT ACG GCA ATA TAC GGG GAT GCC AAA AAC GGC GTG TTT AAT AAA GAT 1152
Ser Thr Ala Ile Tyr Gly Asp Ala Lys Asn Gly Val Phe Asn Lys Asp
    370                 375                 380

TAT GAT TTC GGG TTT GGA AAA GTG AGG CAG GTG AAG GAC TTG CAA ATG 1200
Tyr Asp Phe Gly Phe Gly Lys Val Arg Gln Val Lys Asp Leu Gln Met
385                 390                 395                 400

GGA CTC CTT ATG TAT TTG GGC AAA CCA AAG 1230
Gly Leu Leu Met Tyr Leu Gly Lys Pro Lys
                405                 410

(2) INFORMATION FOR SEQ ID NO:13:

(i) SEQUENCE CHARACTERISTICS:
(A) LENGTH: 1230 base pairs
(B) TYPE: nucleic acid
(C) STRANDEDNESS: double
(D) TOPOLOGY: unknown

(ii) MOLECULE TYPE: cDNA

(ix) FEATURE:
(A) NAME/KEY: CDS
(B) LOCATION: 1..1230

(ix) FEATURE:
(A) NAME/KEY: mat_peptide
(B) LOCATION: 1..465
(D) OTHER INFORMATION: /product= "bFGF"

(ix) FEATURE:
(A) NAME/KEY: mat_peptide
(B) LOCATION: 472..1230
(D) OTHER INFORMATION: /product= "Saporin"

(xi) SEQUENCE DESCRIPTION: SEQ ID NO:13:

ATG GCT GCT GGT TCT ATC ACT ACT CTG CCG GCT CTG CCG GAA GAC GGT 48
Met Ala Ala Gly Ser Ile Thr Thr Leu Pro Ala Leu Pro Glu Asp Gly
1               5                   10                  15

GGT TCT GGT GCT TTC CCG CCC GGC CAC TTC AAG GAC CCC AAG CGG CTG 96
Gly Ser Gly Ala Phe Pro Pro Gly His Phe Lys Asp Pro Lys Arg Leu
            20                  25                  30

TAC TGC AAA AAC GGG GGC TTC TTC CTG CGC ATC CAC CCC GAC GGC CGA 144
Tyr Cys Lys Asn Gly Gly Phe Phe Leu Arg Ile His Pro Asp Gly Arg
        35                  40                  45

GTT GAC GGG GTC CGG GAG AAG AGC GAC CCT CAC ATC AAG CTT CAA CTT 192
Val Asp Gly Val Arg Glu Lys Ser Asp Pro His Ile Lys Leu Gln Leu
    50                  55                  60

CAA GCA GAA GAG AGA GGA GTT GTG TCT ATC AAA GGA GTG TGT GCT AAC 240
Gln Ala Glu Glu Arg Gly Val Val Ser Ile Lys Gly Val Cys Ala Asn
65                  70                  75                  80

CGT TAC CTG GCT ATG AAG GAA GAT GGA AGA TTA CTG GCT TCT AAA TGT 288
Arg Tyr Leu Ala Met Lys Glu Asp Gly Arg Leu Leu Ala Ser Lys Cys
                85                  90                  95

GTT ACG GAT GAG TGT TTC TTT TTT GAA CGA TTG GAA TCT AAT AAC TAC 336
Val Thr Asp Glu Cys Phe Phe Phe Glu Arg Leu Glu Ser Asn Asn Tyr
            100                 105                 110

AAT ACT TAC CGG TCA AGG AAA TAC ACC AGT TGG TAT GTG GCA TTG AAA 384
Asn Thr Tyr Arg Ser Arg Lys Tyr Thr Ser Trp Tyr Val Ala Leu Lys
        115                 120                 125

CGA ACT GGG CAG TAT AAA CTT GGA TCC AAA ACA GGA CCT GGG CAG AAA 432
Arg Thr Gly Gln Tyr Lys Leu Gly Ser Lys Thr Gly Pro Gly Gln Lys
    130                 135                 140

GCT ATA CTT TTT CTT CCA ATG TCT GCT AAG AGC GCC ATG GTC ACA TCA 480
Ala Ile Leu Phe Leu Pro Met Ser Ala Lys Ser Ala Met Val Thr Ser
145                 150                 155                 160

ATC ACA TTA GAT CTA GTA AAT CCG ACC GCG GGT CAA TAC TCA TCT TTT 528
Ile Thr Leu Asp Leu Val Asn Pro Thr Ala Gly Gln Tyr Ser Ser Phe
                165                 170                 175

GTG GAT AAA ATC CGA AAC AAC GTA AAG GAT CCA AAC CTG AAA TAC GGT 576
Val Asp Lys Ile Arg Asn Asn Val Lys Asp Pro Asn Leu Lys Tyr Gly
            180                 185                 190

GGT ACC GAC ATA GCC GTG ATA GGC CCA CCT TCT AAA GAA AAA TTC CTT 624
Gly Thr Asp Ile Ala Val Ile Gly Pro Pro Ser Lys Glu Lys Phe Leu
        195                 200                 205

AGA ATT AAT TTC CAA AGT TCC CGA GGA ACG GTC TCA CTT GGC CTA AAA 672
Arg Ile Asn Phe Gln Ser Ser Arg Gly Thr Val Ser Leu Gly Leu Lys
    210                 215                 220

CGC GAT AAC TTG TAT GTG GTC GCG TAT CTT GCA ATG GAT AAC ACG AAT 720
Arg Asp Asn Leu Tyr Val Val Ala Tyr Leu Ala Met Asp Asn Thr Asn
225                 230                 235                 240

GTT AAT CGG GCA TAT TAC TTC AAA TCA GAA ATT ACT TCC GCC GAG TTA 768
Val Asn Arg Ala Tyr Tyr Phe Lys Ser Glu Ile Thr Ser Ala Glu Leu
                245                 250                 255

ACC GCC CTT TTC CCA GAG GCC ACA ACT GCA AAT CAG AAA GCT TTA GAA 816
Thr Ala Leu Phe Pro Glu Ala Thr Thr Ala Asn Gln Lys Ala Leu Glu

            260                 265                 270

TAC ACA GAA GAT TAT CAG TCG ATC GAA AAG AAT GCC CAG ATA ACA CAG 864
Tyr Thr Glu Asp Tyr Gln Ser Ile Glu Lys Asn Ala Gln Ile Thr Gln
        275                 280                 285

GGA GAT AAA AGT AGA AAA GAA CTC GGG TTG GGG ATC GAC TTA CTT TTG 912
Gly Asp Lys Ser Arg Lys Glu Leu Gly Leu Gly Ile Asp Leu Leu Leu
    290                 295                 300

ACG TTC ATG GAA GCA GTG AAC AAG AAG GCA CGT GTG GTT AAA AAC GAA 960
Thr Phe Met Glu Ala Val Asn Lys Lys Ala Arg Val Val Lys Asn Glu
305                 310                 315                 320

GCT AGG TTT CTG CTT ATC GCT ATT CAA ATG ACA GCT GAG GTA GCA CGA 1008
Ala Arg Phe Leu Leu Ile Ala Ile Gln Met Thr Ala Glu Val Ala Arg
                325                 330                 335

TTT AGG TAC ATT CAA AAC TTG GTA ACT AAG AAC TTC CCC AAC AAG TTC 1056
Phe Arg Tyr Ile Gln Asn Leu Val Thr Lys Asn Phe Pro Asn Lys Phe
            340                 345                 350

GAC TCG GAT AAC AAG GTG ATT CAA TTT GAA GTC AGC TGG CGT AAG ATT 1104
Asp Ser Asp Asn Lys Val Ile Gln Phe Glu Val Ser Trp Arg Lys Ile
        355                 360                 365

TCT ACG GCA ATA TAC GGG GAT GCC AAA AAC GGC GTG TTT AAT AAA GAT 1152
Ser Thr Ala Ile Tyr Gly Asp Ala Lys Asn Gly Val Phe Asn Lys Asp
    370                 375                 380

TAT GAT TTC GGG TTT GGA AAA GTG AGG CAG GTG AAG GAC TTG CAA ATG 1200
Tyr Asp Phe Gly Phe Gly Lys Val Arg Gln Val Lys Asp Leu Gln Met
385                 390                 395                 400

GGA CTC CTT ATG TAT TTG GGC AAA CCA AAG 1230
Gly Leu Leu Met Tyr Leu Gly Lys Pro Lys
                405                 410

(2) INFORMATION FOR SEQ ID NO:14:

(i) SEQUENCE CHARACTERISTICS:
(A) LENGTH: 59 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:14:

AATTCCCCTG TTGACAATTA ATCATCGAAC TAGTTAACTA GTACGCAGCT TGGCTGCAG 59

(2) INFORMATION FOR SEQ ID NO:15:

(i) SEQUENCE CHARACTERISTICS:

(A) LENGTH: 59 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:15:

GTCGACCAAG CTTGGGCATA CATTCAATCA ATTGTTATCT AAGGAAATAC TTACATATG 59

(2) INFORMATION FOR SEQ ID NO:16:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:16:

AGGAGTGTCT GCTAACC 17

(2) INFORMATION FOR SEQ ID NO:17:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:17:

TTCTAAATCG GTTACCGATG ACTG 24

(2) INFORMATION FOR SEQ ID NO:18:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:18:

AAATACTTAC ATATGGCAGC AGGATC 26

(2) INFORMATION FOR SEQ ID NO:19:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:19:

CAGGTAACGG TTAGCAGACA CTCCTTTGAT 30

(2) INFORMATION FOR SEQ ID NO:20:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:20:

ATCAAAGGAG TGTCTGCTAA CCGTTACCTG 30

(2) INFORMATION FOR SEQ ID NO:21:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:21:

GTGATTGATG TGACCATGGC GCTCTTAGCA 30

(2) INFORMATION FOR SEQ ID NO:22:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:22:

TGGCTTCTAA ATCTGTTACG GATGAG 26

(2) INFORMATION FOR SEQ ID NO:23:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:23:

CTCATCCGTA ACAGATTTAG AAGCCA 26

(2) INFORMATION FOR SEQ ID NO:24:

(i) SEQUENCE CHARACTERISTICS:
(A) LENGTH: 155 amino acids
(B) TYPE: amino acid
(C) STRANDEDNESS: single
(D) TOPOLOGY: unknown

(ii) MOLECULE TYPE: peptide

(xi) SEQUENCE DESCRIPTION: SEQ ID NO:24:

Met Ala Glu Gly Glu Ile Thr Thr Phe Thr Ala Leu Thr Glu Lys Phe
1               5                   10                  15

Asn Leu Pro Pro Gly Asn Tyr Lys Lys Pro Lys Leu Leu Tyr Cys Ser
            20                  25                  30

Asn Gly Gly His Phe Leu Arg Ile Leu Pro Asp Gly Thr Val Asp Gly
        35                  40                  45

Thr Arg Asp Arg Ser Asp Gln His Ile Gln Leu Gln Leu Ser Ala Glu
    50                  55                  60

Ser Val Gly Glu Val Tyr Ile Lys Ser Thr Glu Thr Gly Gln Tyr Leu
65                  70                  75                  80

Ala Met Asp Thr Asp Gly Leu Leu Tyr Gly Ser Gln Thr Pro Asn Glu
                85                  90                  95

Glu Cys Leu Phe Leu Glu Arg Leu Glu Glu Asn His Tyr Asn Thr Tyr
            100                 105                 110

Ile Ser Lys Lys His Ala Glu Lys Asn Trp Phe Val Gly Leu Lys Lys
        115                 120                 125

Asn Gly Ser Cys Lys Arg Gly Pro Arg Thr His Tyr Gly Gln Lys Ala
    130                 135                 140

Ile Leu Phe Leu Pro Leu Pro Val Ser Ser Asp
145                 150                 155

(2) INFORMATION FOR SEQ ID NO:25:

    (i) SEQUENCE CHARACTERISTICS:
        (A) LENGTH: 155 amino acids
        (B) TYPE: amino acid
        (C) STRANDEDNESS: single
        (D) TOPOLOGY: unknown

    (ii) MOLECULE TYPE: peptide

    (xi) SEQUENCE DESCRIPTION: SEQ ID NO:25:

(2) INFORMATION FOR SEQ ID NO:26:

    (i) SEQUENCE CHARACTERISTICS:
        (A) LENGTH: 239 amino acids
        (B) TYPE: amino acid
        (C) STRANDEDNESS: single
        (D) TOPOLOGY: unknown

(ii) MOLECULE TYPE: peptide

(xi) SEQUENCE DESCRIPTION: SEQ ID NO:26:

(2) INFORMATION FOR SEQ ID NO:27:

(i) SEQUENCE CHARACTERISTICS:
(A) LENGTH: 206 amino acids
(B) TYPE: amino acid

(C) STRANDEDNESS: single
(D) TOPOLOGY: unknown

(ii) MOLECULE TYPE: peptide

(xi) SEQUENCE DESCRIPTION: SEQ ID NO:27:

Met Ser Gly Pro Gly Thr Ala Ala Val Ala Leu Leu Pro Ala Val Leu
1               5                   10                  15

Leu Ala Leu Leu Ala Pro Trp Ala Gly Arg Gly Gly Ala Ala Ala Pro
            20                  25                  30

Thr Ala Pro Asn Gly Thr Leu Glu Ala Glu Leu Glu Arg Arg Trp Glu
        35                  40                  45

Ser Leu Val Ala Leu Ser Leu Ala Arg Leu Pro Val Ala Ala Gln Pro
    50                  55                  60

Lys Glu Ala Ala Val Gln Ser Gly Ala Gly Asp Tyr Leu Leu Gly Ile
65                  70                  75                  80

Lys Arg Leu Arg Arg Leu Tyr Cys Asn Val Gly Ile Gly Phe His Leu
                85                  90                  95

Gln Ala Leu Pro Asp Gly Arg Ile Gly Gly Ala His Ala Asp Thr Arg
            100                 105                 110

Asp Ser Leu Leu Glu Leu Ser Pro Val Glu Arg Gly Val Val Ser Ile
        115                 120                 125

Phe Gly Val Ala Ser Arg Phe Phe Val Ala Met Ser Ser Lys Gly Lys
    130                 135                 140

Leu Tyr Gly Ser Pro Phe Phe Thr Asp Glu Cys Thr Phe Lys Glu Ile
145                 150                 155                 160

Leu Leu Pro Asn Asn Tyr Asn Ala Tyr Glu Ser Tyr Lys Tyr Pro Gly
                165                 170                 175

Met Phe Ile Ala Leu Ser Lys Asn Gly Lys Thr Lys Lys Gly Asn Arg
            180                 185                 190

Val Ser Pro Thr Met Lys Val Thr His Phe Leu Pro Arg Leu
        195                 200                 205

(2) INFORMATION FOR SEQ ID NO:28:

(i) SEQUENCE CHARACTERISTICS:
(A) LENGTH: 268 amino acids
(B) TYPE: amino acid
(C) STRANDEDNESS: single
(D) TOPOLOGY: unknown

(ii) MOLECULE TYPE: peptide

(xi) SEQUENCE DESCRIPTION: SEQ ID NO:28:

Met Ser Leu Ser Phe Leu Leu Leu Leu Phe Phe Ser His Leu Ile Leu
1               5                   10                  15

Ser Ala Trp Ala His Gly Glu Lys Arg Leu Ala Pro Lys Gly Gln Pro
            20                  25                  30

Gly Pro Ala Ala Thr Asp Arg Asn Pro Ile Gly Ser Ser Ser Arg Gln
        35                  40                  45

Ser Ser Ser Ser Ala Met Ser Ser Ser Ser Ala Ser Ser Ser Pro Ala
    50                  55                  60

Ala Ser Leu Gly Ser Gln Gly Ser Gly Leu Glu Gln Ser Ser Phe Gln
65                  70                  75                  80

Trp Ser Pro Ser Gly Arg Arg Thr Gly Ser Leu Tyr Cys Arg Val Gly
                85                  90                  95

Ile Gly Phe His Leu Gln Ile Tyr Pro Asp Gly Lys Val Asn Gly Ser
            100                 105                 110

His Glu Ala Asn Met Leu Ser Val Leu Glu Ile Phe Ala Val Ser Gln
        115                 120                 125

Gly Ile Val Gly Ile Arg Gly Val Phe Ser Asn Lys Phe Leu Ala Met
    130                 135                 140

Ser Lys Lys Gly Lys Leu His Ala Ser Ala Lys Phe Thr Asp Asp Cys
145                 150                 155                 160

Lys Phe Arg Glu Arg Phe Gln Glu Asn Ser Tyr Asn Thr Tyr Ala Ser
                165                 170                 175

Ala Ile His Arg Thr Glu Lys Thr Gly Arg Glu Trp Tyr Val Ala Leu
            180                 185                 190

Asn Lys Arg Gly Lys Ala Lys Arg Gly Cys Ser Pro Arg Val Lys Pro
        195                 200                 205

Gln His Ile Ser Thr His Phe Leu Pro Arg Phe Lys Gln Ser Glu Gln
    210                 215                 220

Pro Glu Leu Ser Phe Thr Val Thr Val Pro Glu Lys Lys Asn Pro Pro
225                 230                 235                 240

Ser Pro Ile Lys Ser Lys Ile Pro Leu Ser Ala Pro Arg Lys Asn Thr
                245                 250                 255

Asn Ser Val Lys Tyr Arg Leu Lys Phe Arg Phe Gly
            260                 265

(2) INFORMATION FOR SEQ ID NO:29:

(i) SEQUENCE CHARACTERISTICS:
(A) LENGTH: 198 amino acids
(B) TYPE: amino acid
(C) STRANDEDNESS: single
(D) TOPOLOGY: unknown

(ii) MOLECULE TYPE: peptide

(xi) SEQUENCE DESCRIPTION: SEQ ID NO:29:

Met Ser Arg Gly Ala Gly Arg Leu Gln Gly Thr Leu Trp Ala Leu Val
1               5                   10                  15

Phe Leu Gly Ile Leu Val Gly Met Val Val Pro Ser Pro Ala Gly Thr
            20                  25                  30

Arg Ala Asn Asn Thr Leu Leu Asp Ser Arg Gly Trp Gly Thr Leu Leu
        35                  40                  45

Ser Arg Ser Arg Ala Gly Leu Ala Gly Glu Ile Ala Gly Val Asn Trp
    50                  55                  60

Glu Ser Gly Tyr Leu Val Gly Ile Lys Arg Gln Arg Arg Leu Tyr Cys
65                  70                  75                  80

Asn Val Gly Ile Gly Phe His Leu Gln Val Leu Pro Asp Gly Arg Ile
                85                  90                  95

Ser Gly Thr His Glu Glu Asn Pro Tyr Ser Leu Leu Glu Ile Ser Thr
            100                 105                 110

Val Glu Arg Gly Val Val Ser Leu Phe Gly Val Arg Ser Ala Leu Phe
        115                 120                 125

Val Ala Met Asn Ser Lys Gly Arg Leu Tyr Ala Thr Pro Ser Phe Gln
    130                 135                 140

Glu Glu Cys Lys Phe Arg Glu Thr Leu Leu Pro Asn Asn Tyr Asn Ala
145                 150                 155                 160

Tyr Glu Ser Asp Leu Tyr Gln Gly Thr Tyr Ile Ala Leu Ser Lys Tyr
                165                 170                 175

Gly Arg Val Lys Arg Gly Ser Lys Val Ser Pro Ile Met Thr Val Thr
            180                 185                 190

His Phe Leu Pro Arg Ile
        195

(2) INFORMATION FOR SEQ ID NO:30:

(i) SEQUENCE CHARACTERISTICS:
(A) LENGTH: 194 amino acids
(B) TYPE: amino acid

(C) STRANDEDNESS: single
(D) TOPOLOGY: unknown

(ii) MOLECULE TYPE: peptide

(xi) SEQUENCE DESCRIPTION: SEQ ID NO:30:

Met His Lys Trp Ile Leu Thr Trp Ile Leu Pro Thr Leu Leu Tyr Arg
1               5                   10                  15

Ser Cys Phe His Ile Ile Cys Leu Val Gly Thr Ile Ser Leu Ala Cys
            20                  25                  30

Asn Asp Met Thr Pro Glu Gln Met Ala Thr Asn Val Asn Cys Ser Ser
        35                  40                  45

Pro Glu Arg His Thr Arg Ser Tyr Asp Tyr Met Glu Gly Gly Asp Ile
    50                  55                  60

Arg Val Arg Arg Leu Phe Cys Arg Thr Gln Trp Tyr Leu Arg Ile Asp
65                  70                  75                  80

Lys Arg Gly Lys Val Lys Gly Thr Gln Glu Met Lys Asn Asn Tyr Asn
                85                  90                  95

Ile Met Glu Ile Arg Thr Val Ala Val Gly Ile Val Ala Ile Lys Gly
            100                 105                 110

Val Glu Ser Glu Phe Tyr Leu Ala Met Asn Lys Glu Gly Lys Leu Tyr
        115                 120                 125

Ala Lys Lys Glu Cys Asn Glu Asp Cys Asn Phe Lys Glu Leu Ile Leu
    130                 135                 140

Glu Asn His Tyr Asn Thr Tyr Ala Ser Ala Lys Trp Thr His Asn Gly
145                 150                 155                 160

Gly Glu Met Phe Val Ala Leu Asn Gln Lys Gly Ile Pro Val Arg Gly
                165                 170                 175

Lys Lys Thr Lys Lys Glu Gln Lys Thr Ala His Phe Leu Pro Met Ala
            180                 185                 190

Ile Thr

(2) INFORMATION FOR SEQ ID NO:31:

(i) SEQUENCE CHARACTERISTICS:
(A) LENGTH: 215 amino acids
(B) TYPE: amino acid
(C) STRANDEDNESS: single
(D) TOPOLOGY: unknown

(ii) MOLECULE TYPE: peptide

(xi) SEQUENCE DESCRIPTION: SEQ ID NO:31:

Met Gly Ser Pro Arg Ser Ala Leu Ser Cys Leu Leu Leu His Leu Leu
1               5                   10                  15

Val Leu Cys Leu Gln Ala Gln Val Thr Val Gln Ser Ser Pro Asn Phe
            20                  25                  30

Thr Gln His Val Arg Glu Gln Ser Leu Val Thr Asp Gln Leu Ser Arg
        35                  40                  45

Arg Leu Ile Arg Thr Tyr Gln Leu Tyr Ser Arg Thr Ser Gly Lys His
    50                  55                  60

Val Gln Val Leu Ala Asn Lys Arg Ile Asn Ala Met Ala Glu Asp Gly
65                  70                  75                  80

Asp Pro Phe Ala Lys Leu Ile Val Glu Thr Asp Thr Phe Gly Ser Arg
                85                  90                  95

Val Arg Val Arg Gly Ala Glu Thr Gly Leu Tyr Ile Cys Met Asn Lys
            100                 105                 110

Lys Gly Lys Leu Ile Ala Lys Ser Asn Gly Lys Gly Lys Asp Cys Val
        115                 120                 125

Phe Thr Glu Ile Val Leu Glu Asn Asn Tyr Thr Ala Leu Gln Asn Ala
    130                 135                 140

Lys Tyr Glu Gly Trp Tyr Met Ala Phe Thr Arg Lys Gly Arg Pro Arg
145                 150                 155                 160

Lys Gly Ser Lys Thr Arg Gln His Gln Arg Glu Val His Phe Met Lys
                165                 170                 175

Arg Leu Pro Arg Gly His His Thr Thr Glu Gln Ser Leu Arg Phe Glu
            180                 185                 190

Phe Leu Asn Tyr Pro Pro Phe Thr Arg Ser Leu Arg Gly Ser Gln Arg
        195                 200                 205

Thr Trp Ala Pro Glu Pro Arg
    210                 215

(2) INFORMATION FOR SEQ ID NO:32:

(i) SEQUENCE CHARACTERISTICS:
(A) LENGTH: 208 amino acids
(B) TYPE: amino acid
(C) STRANDEDNESS: single
(D) TOPOLOGY: unknown

(ii) MOLECULE TYPE: peptide

(xi) SEQUENCE DESCRIPTION: SEQ ID NO:32:

(2) INFORMATION FOR SEQ ID NO:33:

    (i) SEQUENCE CHARACTERISTICS:
        (A) LENGTH: 40 amino acids
        (B) TYPE: amino acid
        (C) STRANDEDNESS: single
        (D) TOPOLOGY: unknown

    (ii) MOLECULE TYPE: peptide

    (xi) SEQUENCE DESCRIPTION: SEQ ID NO:33:

Val Ile Ile Tyr Glu Leu Asn Leu Gln Gly Thr Thr Lys Ala Gln Tyr
                5                   10                  15

Ser Thr Ile Leu Lys Gln Leu Arg Asp Asp Ile Lys Asp Pro Asn Leu

            20                  25                  30

Xaa Tyr Gly Xaa Xaa Asp Tyr Ser
        35                  40

(2) INFORMATION FOR SEQ ID NO:34:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:34

CATATGTGTG TCACATCAAT CACATTAGAT 30

(2) INFORMATION FOR SEQ ID NO:35:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:35

CAGGTTTGGA TCCTTTACGT T 21

(2) INFORMATION FOR SEQ ID NO:36:

(i) SEQUENCE CHARACTERISTICS:
(A) LENGTH: 82 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:36

AAGGAGATATACC ATG GGC AGC AGC CAT CAT CAT CAT CAT CAC AGC AGC 43
Met Gly Ser Ser His His His His His His Ser Ser
1               5                   10

GGC CTG GTG CCG CGC GGC AGC CAT ATG CTC GAG GAT CCG 82
Gly Leu Val Pro Arg Gly Ser His Met Leu Glu Asp Pro
        15                  20                  25

(2) INFORMATION FOR SEQ ID NO:37:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:37

GGATCCGCCT CGTTTGACTA CTT 23

(2) INFORMATION FOR SEQ ID NO:38:

(i) SEQUENCE CHARACTERISTICS:
(A) LENGTH: 36 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: CDS
(B) LOCATION: 3..35
(A) NAME/KEY: Cathepsin B[?]linker

(xi) SEQUENCE DESCRIPTION: SEQ ID NO:38

CCATGGCCCT GGCCCTGGCC CTGGCCCTGG CCATGG 36

(2) INFORMATION FOR SEQ ID NO:39:

(i) SEQUENCE CHARACTERISTICS:
(A) LENGTH: 51 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: CDS
(B) LOCATION: 3..50
(A) NAME/KEY: Cathepsin D linker

(xi) SEQUENCE DESCRIPTION: SEQ ID NO:39

CCATGGGCCGATCGGGCTTCCTGGGCTTCGGCTTCCTGGGCTTCGCCATGG 51

(2) INFORMATION FOR SEQ ID NO:40:

(i) SEQUENCE CHARACTERISTICS:
(A) LENGTH: 27 base pairs
(B) TYPE: nucleic acid

(C) STRANDEDNESS: single
(D) TOPOLOGY: linear

(ii) MOLECULE TYPE: DNA (genomic)

(ix) FEATURE:
(A) NAME/KEY: CDS
(B) LOCATION: 3..26
(A) NAME/KEY: Gly4Ser

(xi) SEQUENCE DESCRIPTION: SEQ ID NO:40

CCATGGGCGG CGGCGGCTCT GCCATGG 27

(2) INFORMATION FOR SEQ ID NO:41:

(i) SEQUENCE CHARACTERISTICS:
(A) LENGTH: 42 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: CDS
(B) LOCATION: 3..41
(A) NAME/KEY: (Gly4Ser)2

(xi) SEQUENCE DESCRIPTION: SEQ ID NO:41

CCATGGGCGG CGGCGGCTCT GGCGGCGGCG GCTCTGCCAT GG 42

(2) INFORMATION FOR SEQ ID NO:42:

(i) SEQUENCE CHARACTERISTICS:
(A) LENGTH: 75 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: CDS
(B) LOCATION: 3..74
(A) NAME/KEY: (Ser4Gly)4

(xi) SEQUENCE DESCRIPTION: SEQ ID NO:42

CCATGGCCTC GTCGTCGTCG GGCTCGTCGT CGTCGGGCTC GTCGTCGTCG GGCTCGTCGT 60

CGTCGGGCGC CATGG 75

(2) INFORMATION FOR SEQ ID NO:43:

(i) SEQUENCE CHARACTERISTICS:
(A) LENGTH:45 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: CDS
(B) LOCATION: 3..45
(A) NAME/KEY: (Ser4Gly)2

(xi) SEQUENCE DESCRIPTION: SEQ ID NO:43

CCATGGCCTC GTCGTCGTCG GGCTCGTCGT CGTCGGGCGC CATGG 45

(2) INFORMATION FOR SEQ ID NO:44:

(i) SEQUENCE CHARACTERISTICS:
(A) LENGTH: 96 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: CDS
(B) LOCATION: 3..95
(A) NAME/KEY: "Trypsin linker"

(xi) SEQUENCE DESCRIPTION: SEQ ID NO:44

CCATGGGCCG ATCGGGCGGT GGGTGCGCTG GTAATAGAGT CAGAAGATCA GTCGGAAGCA 60

GCCTGTCTTG CGGTGGTCTC GACCTGCAGG CCATGG 96

(2) INFORMATION FOR SEQ ID NO:45:

(i) SEQUENCE CHARACTERISTICS:
(A) LENGTH: 1260 base pairs
(B) TYPE: nucleic acid
(C) STRANDEDNESS: double
(D) TOPOLOGY: unknown

(ii) MOLECULE TYPE: cDNA

(ix) FEATURE:
(A) NAME/KEY: CDS
(B) LOCATION: 1..1260

(ix) FEATURE:
(A) NAME/KEY: mat_peptide
(B) LOCATION: 1..465
(D) OTHER INFORMATION: /product= "bFGF"

(ix) FEATURE:
(A) NAME/KEY: mat_peptide
(B) LOCATION: 466...501
(D) OTHER INFORMATION: /product= "Cathepsin B linker"

(ix) FEATURE:
(A) NAME/KEY: mat_peptide
(B) LOCATION: 502..1260
(D) OTHER INFORMATION: /product= "Saporin"

(xi) SEQUENCE DESCRIPTION: SEQ ID NO:45:

ATG GCA GCA GGA TCA ATA ACA ACA TTA CCC GCC TTG CCC GAG GAT GGC 48
Met Ala Ala Gly Ser Ile Thr Thr Leu Pro Ala Leu Pro Glu Asp Gly
1               5                   10                  15

GGC AGC GGC GCC TTC CCG CCC GGC CAC TTC AAG GAC CCC AAG CGG CTG 96
Gly Ser Gly Ala Phe Pro Pro Gly His Phe Lys Asp Pro Lys Arg Leu
            20                  25                  30

TAC TGC AAA AAC GGG GGC TTC TTC CTG CGC ATC CAC CCC GAC GGC CGA 144
Tyr Cys Lys Asn Gly Gly Phe Phe Leu Arg Ile His Pro Asp Gly Arg
        35                  40                  45

GTT GAC GGG GTC CGG GAG AAG AGC GAC CCT CAC ATC AAG CTT CAA CTT 192
Val Asp Gly Val Arg Glu Lys Ser Asp Pro His Ile Lys Leu Gln Leu
    50                  55                  60

CAA GCA GAA GAG AGA GGA GTT GTG TCT ATC AAA GGA GTG TGT GCT AAC 240
Gln Ala Glu Glu Arg Gly Val Val Ser Ile Lys Gly Val Cys Ala Asn
65                  70                  75                  80

CGT TAC CTG GCT ATG AAG GAA GAT GGA AGA TTA CTG GCT TCT AAA TGT 288
Arg Tyr Leu Ala Met Lys Glu Asp Gly Arg Leu Leu Ala Ser Lys Cys
                85                  90                  95

GTT ACG GAT GAG TGT TTC TTT TTT GAA CGA TTG GAA TCT AAT AAC TAC 336
Val Thr Asp Glu Cys Phe Phe Phe Glu Arg Leu Glu Ser Asn Asn Tyr
            100                 105                 110

AAT ACT TAC CGG TCA AGG AAA TAC ACC AGT TGG TAT GTG GCA TTG AAA 384
Asn Thr Tyr Arg Ser Arg Lys Tyr Thr Ser Trp Tyr Val Ala Leu Lys
        115                 120                 125

CGA ACT GGG CAG TAT AAA CTT GGA TCC AAA ACA GGA CCT GGG CAG AAA 432
Arg Thr Gly Gln Tyr Lys Leu Gly Ser Lys Thr Gly Pro Gly Gln Lys
    130                 135                 140

GCT ATA CTT TTT CTT CCA ATG TCT GCT AAG AGC GCC ATG GCC CTG GCC 480

Ala Ile Leu Phe Leu Pro Met Ser Ala Lys Ser Ala Met Ala Leu Ala
145                 150                 155                 160

CTG GCC CTG GCC CTG GCC ATG GTC ACA TCA ATC ACA TTA GAT CTA GTA     528
Leu Ala Leu Ala Leu Ala Met Val Thr Ser Ile Thr Leu Asp Leu Val
                165                 170                 175

AAT CCG ACC GCG GGT CAA TAC TCA TCT TTT GTG GAT AAA ATC CGA AAC     576
Asn Pro Thr Ala Gly Gln Tyr Ser Ser Phe Val Asp Lys Ile Arg Asn
            180                 185                 190

AAC GTA AAG GAT CCA AAC CTG AAA TAC GGT GGT ACC GAC ATA GCC GTG     624
Asn Val Lys Asp Pro Asn Leu Lys Tyr Gly Gly Thr Asp Ile Ala Val
        195                 200                 205

ATA GGC CCA CCT TCT AAA GAA AAA TTC CTT AGA ATT AAT TTC CAA AGT     672
Ile Gly Pro Pro Ser Lys Glu Lys Phe Leu Arg Ile Asn Phe Gln Ser
    210                 215                 220

TCC CGA GGA ACG GTC TCA CTT GGC CTA AAA CGC GAT AAC TTG TAT GTG     720
Ser Arg Gly Thr Val Ser Leu Gly Leu Lys Arg Asp Asn Leu Tyr Val
225                 230                 235                 240

GTC GCG TAT CTT GCA ATG GAT AAC ACG AAT GTT AAT CGG GCA TAT TAC     768
Val Ala Tyr Leu Ala Met Asp Asn Thr Asn Val Asn Arg Ala Tyr Tyr
                245                 250                 255

TTC AAA TCA GAA ATT ACT TCC GCC GAG TTA ACC GCC CTT TTC CCA GAG     816
Phe Lys Ser Glu Ile Thr Ser Ala Glu Leu Thr Ala Leu Phe Pro Glu
            260                 265                 270

GCC ACA ACT GCA AAT CAG AAA GCT TTA GAA TAC ACA GAA GAT TAT CAG     864
Ala Thr Thr Ala Asn Gln Lys Ala Leu Glu Tyr Thr Glu Asp Tyr Gln
        275                 280                 285

TCG ATC GAA AAG AAT GCC CAG ATA ACA CAG GGA GAT AAA AGT AGA AAA     912
Ser Ile Glu Lys Asn Ala Gln Ile Thr Gln Gly Asp Lys Ser Arg Lys
    290                 295                 300

GAA CTC GGG TTG GGG ATC GAC TTA CTT TTG ACG TTC ATG GAA GCA GTG     960
Glu Leu Gly Leu Gly Ile Asp Leu Leu Leu Thr Phe Met Glu Ala Val
305                 310                 315                 320

AAC AAG AAG GCA CGT GTG GTT AAA AAC GAA GCT AGG TTT CTG CTT ATC    1008
Asn Lys Lys Ala Arg Val Val Lys Asn Glu Ala Arg Phe Leu Leu Ile
                325                 330                 335

GCT ATT CAA ATG ACA GCT GAG GTA GCA CGA TTT AGG TAC ATT CAA AAC    1056
Ala Ile Gln Met Thr Ala Glu Val Ala Arg Phe Arg Tyr Ile Gln Asn
            340                 345                 350

TTG GTA ACT AAG AAC TTC CCC AAC AAG TTC GAC TCG GAT AAC AAG GTG    1104
Leu Val Thr Lys Asn Phe Pro Asn Lys Phe Asp Ser Asp Asn Lys Val
        355                 360                 365

ATT CAA TTT GAA GTC AGC TGG CGT AAG ATT TCT ACG GCA ATA TAC GGG    1152

Ile Gln Phe Glu Val Ser Trp Arg Lys Ile Ser Thr Ala Ile Tyr Gly
    370                 375                 380

GAT GCC AAA AAC GGC GTG TTT AAT AAA GAT TAT GAT TTC GGG TTT GGA      1200
Asp Ala Lys Asn Gly Val Phe Asn Lys Asp Tyr Asp Phe Gly Phe Gly
385                 390                 395                 400

AAA GTG AGG CAG GTG AAG GAC TTG CAA ATG GGA CTC CTT ATG TAT TTG      1248
Lys Val Arg Gln Val Lys Asp Leu Gln Met Gly Leu Leu Met Tyr Leu
                405                 410                 415

GGC AAA CCA AAG                                                      1260
Gly Lys Pro Lys
            420

(2) INFORMATION FOR SEQ ID NO:46:

     (i) SEQUENCE CHARACTERISTICS:
          (A) LENGTH: 1275 base pairs
          (B) TYPE: nucleic acid
          (C) STRANDEDNESS: double
          (D) TOPOLOGY: unknown

    (ii) MOLECULE TYPE: cDNA

    (ix) FEATURE:
          (A) NAME/KEY: CDS
          (B) LOCATION: 1..1275

    (ix) FEATURE:
          (A) NAME/KEY: mat_peptide
          (B) LOCATION: 1..465
          (D) OTHER INFORMATION: /product= "bFGF"

    (ix) FEATURE:
          (A) NAME/KEY: mat_peptide
          (B) LOCATION: 466...516
          (D) OTHER INFORMATION: /product= "Cathepsin D linker"

   (ix) FEATURE:
          (A) NAME/KEY: mat_peptide
          (B) LOCATION: 517..1275
          (D) OTHER INFORMATION: /product= "Saporin"

    (xi) SEQUENCE DESCRIPTION: SEQ ID NO:46:

ATG GCA GCA GGA TCA ATA ACA ACA TTA CCC GCC TTG CCC GAG GAT GGC        48
Met Ala Ala Gly Ser Ile Thr Thr Leu Pro Ala Leu Pro Glu Asp Gly
  1               5                  10                  15

GGC AGC GGC GCC TTC CCG CCC GGC CAC TTC AAG GAC CCC AAG CGG CTG        96
Gly Ser Gly Ala Phe Pro Pro Gly His Phe Lys Asp Pro Lys Arg Leu
             20                  25                  30

TAC TGC AAA AAC GGG GGC TTC TTC CTG CGC ATC CAC CCC GAC GGC CGA       144

Tyr Cys Lys Asn Gly Gly Phe Phe Leu Arg Ile His Pro Asp Gly Arg
        35                  40                  45

GTT GAC GGG GTC CGG GAG AAG AGC GAC CCT CAC ATC AAG CTT CAA CTT        192
Val Asp Gly Val Arg Glu Lys Ser Asp Pro His Ile Lys Leu Gln Leu
    50                  55                  60

CAA GCA GAA GAG AGA GGA GTT GTG TCT ATC AAA GGA GTG TGT GCT AAC        240
Gln Ala Glu Glu Arg Gly Val Val Ser Ile Lys Gly Val Cys Ala Asn
65                  70                  75                  80

CGT TAC CTG GCT ATG AAG GAA GAT GGA AGA TTA CTG GCT TCT AAA TGT        288
Arg Tyr Leu Ala Met Lys Glu Asp Gly Arg Leu Leu Ala Ser Lys Cys
                85                  90                  95

GTT ACG GAT GAG TGT TTC TTT TTT GAA CGA TTG GAA TCT AAT AAC TAC        336
Val Thr Asp Glu Cys Phe Phe Phe Glu Arg Leu Glu Ser Asn Asn Tyr
            100                 105                 110

AAT ACT TAC CGG TCA AGG AAA TAC ACC AGT TGG TAT GTG GCA TTG AAA        384
Asn Thr Tyr Arg Ser Arg Lys Tyr Thr Ser Trp Tyr Val Ala Leu Lys
        115                 120                 125

CGA ACT GGG CAG TAT AAA CTT GGA TCC AAA ACA GGA CCT GGG CAG AAA        432
Arg Thr Gly Gln Tyr Lys Leu Gly Ser Lys Thr Gly Pro Gly Gln Lys
    130                 135                 140

GCT ATA CTT TTT CTT CCA ATG TCT GCT AAG AGC GCC ATG GGC CGA TCG        480
Ala Ile Leu Phe Leu Pro Met Ser Ala Lys Ser Ala Met Gly Arg Ser
145                 150                 155                 160

GGC TTC CTG GGC TTC GGC TTC CTG GGC TTC GCC ATG GTC ACA TCA ATC        528
Gly Phe Leu Gly Phe GLy Phe Leu GLy Phe Ala Met Val Thr Ser Ile
                165                 170                 175
ACA TTA GAT CTA GTA AAT CCG ACC GCG GGT CAA TAC TCA TCT TTT GTG        576
Thr Leu Asp Leu Val Asn Pro Thr Ala Gly Gln Tyr Ser Ser Phe Val
            180                 185                 190

GAT AAA ATC CGA AAC AAC GTA AAG GAT CCA AAC CTG AAA TAC GGT GGT        624
Asp Lys Ile Arg Asn Asn Val Lys Asp Pro Asn Leu Lys Tyr Gly Gly
        195                 200                 205

ACC GAC ATA GCC GTG ATA GGC CCA CCT TCT AAA GAA AAA TTC CTT AGA        672
Thr Asp Ile Ala Val Ile Gly Pro Pro Ser Lys Glu Lys Phe Leu Arg
    210                 215                 220

ATT AAT TTC CAA AGT TCC CGA GGA ACG GTC TCA CTT GGC CTA AAA CGC        720
Ile Asn Phe Gln Ser Ser Arg Gly Thr Val Ser Leu Gly Leu Lys Arg
225                 230                 235                 240

GAT AAC TTG TAT GTG GTC GCG TAT CTT GCA ATG GAT AAC ACG AAT GTT        768
Asp Asn Leu Tyr Val Val Ala Tyr Leu Ala Met Asp Asn Thr Asn Val
                245                 250                 255

AAT CGG GCA TAT TAC TTC AAA TCA GAA ATT ACT TCC GCC GAG TTA ACC        816

Asn Arg Ala Tyr Tyr Phe Lys Ser Glu Ile Thr Ser Ala Glu Leu Thr
            260                 265                 270

GCC CTT TTC CCA GAG GCC ACA ACT GCA AAT CAG AAA GCT TTA GAA TAC 864
Ala Leu Phe Pro Glu Ala Thr Thr Ala Asn Gln Lys Ala Leu Glu Tyr
        275                 280                 285

ACA GAA GAT TAT CAG TCG ATC GAA AAG AAT GCC CAG ATA ACA CAG GGA 912
Thr Glu Asp Tyr Gln Ser Ile Glu Lys Asn Ala Gln Ile Thr Gln Gly
    290                 295                 300

GAT AAA AGT AGA AAA GAA CTC GGG TTG GGG ATC GAC TTA CTT TTG ACG 960
Asp Lys Ser Arg Lys Glu Leu Gly Leu Gly Ile Asp Leu Leu Leu Thr
305                 310                 315                 320

TTC ATG GAA GCA GTG AAC AAG AAG GCA CGT GTG GTT AAA AAC GAA GCT 1008
Phe Met Glu Ala Val Asn Lys Lys Ala Arg Val Val Lys Asn Glu Ala
                325                 330                 335

AGG TTT CTG CTT ATC GCT ATT CAA ATG ACA GCT GAG GTA GCA CGA TTT 1056
Arg Phe Leu Leu Ile Ala Ile Gln Met Thr Ala Glu Val Ala Arg Phe
            340                 345                 350

AGG TAC ATT CAA AAC TTG GTA ACT AAG AAC TTC CCC AAC AAG TTC GAC 1104
Arg Tyr Ile Gln Asn Leu Val Thr Lys Asn Phe Pro Asn Lys Phe Asp
        355                 360                 365

TCG GAT AAC AAG GTG ATT CAA TTT GAA GTC AGC TGG CGT AAG ATT TCT 1152
Ser Asp Asn Lys Val Ile Gln Phe Glu Val Ser Trp Arg Lys Ile Ser
    370                 375                 380

ACG GCA ATA TAC GGG GAT GCC AAA AAC GGC GTG TTT AAT AAA GAT TAT 1200
Thr Ala Ile Tyr Gly Asp Ala Lys Asn Gly Val Phe Asn Lys Asp Tyr
385                 390                 395                 400

GAT TTC GGG TTT GGA AAA GTG AGG CAG GTG AAG GAC TTG CAA ATG GGA 1248
Asp Phe Gly Phe Gly Lys Val Arg Gln Val Lys Asp Leu Gln Met Gly
                405                 410                 415

CTC CTT ATG TAT TTG GGC AAA CCA AAG 1275
Leu Leu Met Tyr Leu Gly Lys Pro Lys
            420                 425

(2) INFORMATION FOR SEQ ID NO:47:

(i) SEQUENCE CHARACTERISTICS:
(A) LENGTH: 1251 base pairs
(B) TYPE: nucleic acid
(C) STRANDEDNESS: double
(D) TOPOLOGY: unknown

(ii) MOLECULE TYPE: cDNA

(ix) FEATURE:
(A) NAME/KEY: CDS

(B) LOCATION: 1..1251

(ix) FEATURE:
(A) NAME/KEY: mat_peptide
(B) LOCATION: 1..465
(D) OTHER INFORMATION: /product= "bFGF"

(ix) FEATURE:
(A) NAME/KEY: mat_peptide
(B) LOCATION: 466..492
(D) OTHER INFORMATION: /product= " Gly4Ser linker"

(ix) FEATURE:
(A) NAME/KEY: mat_peptide
(B) LOCATION: 493..1251
(D) OTHER INFORMATION: /product= "Saporin"

(xi) SEQUENCE DESCRIPTION: SEQ ID NO:47:

ATG GCA GCA GGA TCA ATA ACA ACA TTA CCC GCC TTG CCC GAG GAT GGC 48
Met Ala Ala Gly Ser Ile Thr Thr Leu Pro Ala Leu Pro Glu Asp Gly
1               5                   10                  15

GGC AGC GGC GCC TTC CCG CCC GGC CAC TTC AAG GAC CCC AAG CGG CTG 96
Gly Ser Gly Ala Phe Pro Pro Gly His Phe Lys Asp Pro Lys Arg Leu
            20                  25                  30

TAC TGC AAA AAC GGG GGC TTC TTC CTG CGC ATC CAC CCC GAC GGC CGA 144
Tyr Cys Lys Asn Gly Gly Phe Phe Leu Arg Ile His Pro Asp Gly Arg
        35                  40                  45

GTT GAC GGG GTC CGG GAG AAG AGC GAC CCT CAC ATC AAG CTT CAA CTT 192
Val Asp Gly Val Arg Glu Lys Ser Asp Pro His Ile Lys Leu Gln Leu
    50                  55                  60

CAA GCA GAA GAG AGA GGA GTT GTG TCT ATC AAA GGA GTG TGT GCT AAC 240
Gln Ala Glu Glu Arg Gly Val Val Ser Ile Lys Gly Val Cys Ala Asn
65                  70                  75                  80

CGT TAC CTG GCT ATG AAG GAA GAT GGA AGA TTA CTG GCT TCT AAA TGT 288
Arg Tyr Leu Ala Met Lys Glu Asp Gly Arg Leu Leu Ala Ser Lys Cys
                85                  90                  95

GTT ACG GAT GAG TGT TTC TTT TTT GAA CGA TTG GAA TCT AAT AAC TAC 336
Val Thr Asp Glu Cys Phe Phe Phe Glu Arg Leu Glu Ser Asn Asn Tyr
            100                 105                 110

AAT ACT TAC CGG TCA AGG AAA TAC ACC AGT TGG TAT GTG GCA TTG AAA 384
Asn Thr Tyr Arg Ser Arg Lys Tyr Thr Ser Trp Tyr Val Ala Leu Lys
        115                 120                 125

CGA ACT GGG CAG TAT AAA CTT GGA TCC AAA ACA GGA CCT GGG CAG AAA 432
Arg Thr Gly Gln Tyr Lys Leu Gly Ser Lys Thr Gly Pro Gly Gln Lys
    130                 135                 140

GCT ATA CTT TTT CTT CCA ATG TCT GCT AAG AGC GCC ATG GGC GGC GGC 480
Ala Ile Leu Phe Leu Pro Met Ser Ala Lys Ser Ala Met Gly Gly Gly
145                 150                 155                 160

GGC TCT GCC ATG GTC ACA TCA ATC ACA TTA GAT CTA GTA AAT CCG ACC 528
Gly Ser Ala Met Val Thr Ser Ile Thr Leu Asp Leu Val Asn Pro Thr
                165                 170                 175
GCG GGT CAA TAC TCA TCT TTT GTG GAT AAA ATC CGA AAC AAC GTA AAG 576
Ala Gly Gln Tyr Ser Ser Phe Val Asp Lys Ile Arg Asn Asn Val Lys
            180                 185                 190

GAT CCA AAC CTG AAA TAC GGT GGT ACC GAC ATA GCC GTG ATA GGC CCA 624
Asp Pro Asn Leu Lys Tyr Gly Gly Thr Asp Ile Ala Val Ile Gly Pro
        195                 200                 205

CCT TCT AAA GAA AAA TTC CTT AGA ATT AAT TTC CAA AGT TCC CGA GGA 672
Pro Ser Lys Glu Lys Phe Leu Arg Ile Asn Phe Gln Ser Ser Arg Gly
    210                 215                 220

ACG GTC TCA CTT GGC CTA AAA CGC GAT AAC TTG TAT GTG GTC GCG TAT 720
Thr Val Ser Leu Gly Leu Lys Arg Asp Asn Leu Tyr Val Val Ala Tyr
225                 230                 235                 240

CTT GCA ATG GAT AAC ACG AAT GTT AAT CGG GCA TAT TAC TTC AAA TCA 768
Leu Ala Met Asp Asn Thr Asn Val Asn Arg Ala Tyr Tyr Phe Lys Ser
                245                 250                 255

GAA ATT ACT TCC GCC GAG TTA ACC GCC CTT TTC CCA GAG GCC ACA ACT 816
Glu Ile Thr Ser Ala Glu Leu Thr Ala Leu Phe Pro Glu Ala Thr Thr
            260                 265                 270

GCA AAT CAG AAA GCT TTA GAA TAC ACA GAA GAT TAT CAG TCG ATC GAA 864
Ala Asn Gln Lys Ala Leu Glu Tyr Thr Glu Asp Tyr Gln Ser Ile Glu
        275                 280                 285

AAG AAT GCC CAG ATA ACA CAG GGA GAT AAA AGT AGA AAA GAA CTC GGG 912
Lys Asn Ala Gln Ile Thr Gln Gly Asp Lys Ser Arg Lys Glu Leu Gly
    290                 295                 300

TTG GGG ATC GAC TTA CTT TTG ACG TTC ATG GAA GCA GTG AAC AAG AAG 960
Leu Gly Ile Asp Leu Leu Leu Thr Phe Met Glu Ala Val Asn Lys Lys
305                 310                 315                 320

GCA CGT GTG GTT AAA AAC GAA GCT AGG TTT CTG CTT ATC GCT ATT CAA 1008
Ala Arg Val Val Lys Asn Glu Ala Arg Phe Leu Leu Ile Ala Ile Gln
                325                 330                 335

ATG ACA GCT GAG GTA GCA CGA TTT AGG TAC ATT CAA AAC TTG GTA ACT 1056
Met Thr Ala Glu Val Ala Arg Phe Arg Tyr Ile Gln Asn Leu Val Thr
            340                 345                 350

AAG AAC TTC CCC AAC AAG TTC GAC TCG GAT AAC AAG GTG ATT CAA TTT 1104
Lys Asn Phe Pro Asn Lys Phe Asp Ser Asp Asn Lys Val Ile Gln Phe
        355                 360                 365

GAA GTC AGC TGG CGT AAG ATT TCT ACG GCA ATA TAC GGG GAT GCC AAA 1152
Glu Val Ser Trp Arg Lys Ile Ser Thr Ala Ile Tyr Gly Asp Ala Lys
    370                 375                 380

AAC GGC GTG TTT AAT AAA GAT TAT GAT TTC GGG TTT GGA AAA GTG AGG 1200
Asn Gly Val Phe Asn Lys Asp Tyr Asp Phe Gly Phe Gly Lys Val Arg
385                 390                 395                 400

CAG GTG AAG GAC TTG CAA ATG GGA CTC CTT ATG TAT TTG GGC AAA CCA 1248
Gln Val Lys Asp Leu Gln Met Gly Leu Leu Met Tyr Leu Gly Lys Pro
                405                 410                 415

AAG 1251
Lys

(2) INFORMATION FOR SEQ ID NO:48:

(i) SEQUENCE CHARACTERISTICS:
(A) LENGTH: 1266 base pairs
(B) TYPE: nucleic acid
(C) STRANDEDNESS: double
(D) TOPOLOGY: unknown

(ii) MOLECULE TYPE: cDNA

(ix) FEATURE:
(A) NAME/KEY: CDS
(B) LOCATION: 1..1266

(ix) FEATURE:
(A) NAME/KEY: mat_peptide
(B) LOCATION: 1..465
(D) OTHER INFORMATION: /product= "bFGF"

(ix) FEATURE:
(A) NAME/KEY: mat_peptide
(B) LOCATION: 466..507
(D) OTHER INFORMATION: /product= " (Gly4Ser)2 linker"

(ix) FEATURE:
(A) NAME/KEY: mat_peptide
(B) LOCATION: 508..1266
(D) OTHER INFORMATION: /product= "Saporin"

(xi) SEQUENCE DESCRIPTION: SEQ ID NO:48:

ATG GCA GCA GGA TCA ATA ACA ACA TTA CCC GCC TTG CCC GAG GAT GGC 48
Met Ala Ala Gly Ser Ile Thr Thr Leu Pro Ala Leu Pro Glu Asp Gly
1               5                   10                  15

GGC AGC GGC GCC TTC CCG CCC GGC CAC TTC AAG GAC CCC AAG CGG CTG 96
Gly Ser Gly Ala Phe Pro Pro Gly His Phe Lys Asp Pro Lys Arg Leu
            20                  25                  30

TAC TGC AAA AAC GGG GGC TTC TTC CTG CGC ATC CAC CCC GAC GGC CGA 144
Tyr Cys Lys Asn Gly Gly Phe Phe Leu Arg Ile His Pro Asp Gly Arg

        35                  40                  45

GTT GAC GGG GTC CGG GAG AAG AGC GAC CCT CAC ATC AAG CTT CAA CTT 192
Val Asp Gly Val Arg Glu Lys Ser Asp Pro His Ile Lys Leu Gln Leu
    50                  55                  60

CAA GCA GAA GAG AGA GGA GTT GTG TCT ATC AAA GGA GTG TGT GCT AAC 240
Gln Ala Glu Glu Arg Gly Val Val Ser Ile Lys Gly Val Cys Ala Asn
65                  70                  75                  80

CGT TAC CTG GCT ATG AAG GAA GAT GGA AGA TTA CTG GCT TCT AAA TGT 288
Arg Tyr Leu Ala Met Lys Glu Asp Gly Arg Leu Leu Ala Ser Lys Cys
                85                  90                  95

GTT ACG GAT GAG TGT TTC TTT TTT GAA CGA TTG GAA TCT AAT AAC TAC 336
Val Thr Asp Glu Cys Phe Phe Phe Glu Arg Leu Glu Ser Asn Asn Tyr
            100                 105                 110

AAT ACT TAC CGG TCA AGG AAA TAC ACC AGT TGG TAT GTG GCA TTG AAA 384
Asn Thr Tyr Arg Ser Arg Lys Tyr Thr Ser Trp Tyr Val Ala Leu Lys
        115                 120                 125

CGA ACT GGG CAG TAT AAA CTT GGA TCC AAA ACA GGA CCT GGG CAG AAA 432
Arg Thr Gly Gln Tyr Lys Leu Gly Ser Lys Thr Gly Pro Gly Gln Lys
    130                 135                 140

GCT ATA CTT TTT CTT CCA ATG TCT GCT AAG AGC GCC ATG GGC GGC GGC 480
Ala Ile Leu Phe Leu Pro Met Ser Ala Lys Ser Ala Met Gly Gly Gly
145                 150                 155                 160

GGC TCT GGC GGC GGC GGC TCT GCC ATG GTC ACA TCA ATC ACA TTA GAT 528
Gly Ser Gly Gly Gly Gly Ser Ala Met Val Thr Ser Ile Thr Leu Asp
                165                 170                 175

CTA GTA AAT CCG ACC GCG GGT CAA TAC TCA TCT TTT GTG GAT AAA ATC 576
Leu Val Asn Pro Thr Ala Gly Gln Tyr Ser Ser Phe Val Asp Lys Ile
            180                 185                 190

CGA AAC AAC GTA AAG GAT CCA AAC CTG AAA TAC GGT GGT ACC GAC ATA 624
Arg Asn Asn Val Lys Asp Pro Asn Leu Lys Tyr Gly Gly Thr Asp Ile
        195                 200                 205

GCC GTG ATA GGC CCA CCT TCT AAA GAA AAA TTC CTT AGA ATT AAT TTC 672
Ala Val Ile Gly Pro Pro Ser Lys Glu Lys Phe Leu Arg Ile Asn Phe
    210                 215                 220

CAA AGT TCC CGA GGA ACG GTC TCA CTT GGC CTA AAA CGC GAT AAC TTG 720
Gln Ser Ser Arg Gly Thr Val Ser Leu Gly Leu Lys Arg Asp Asn Leu
225                 230                 235                 240

TAT GTG GTC GCG TAT CTT GCA ATG GAT AAC ACG AAT GTT AAT CGG GCA 768
Tyr Val Val Ala Tyr Leu Ala Met Asp Asn Thr Asn Val Asn Arg Ala
                245                 250                 255

TAT TAC TTC AAA TCA GAA ATT ACT TCC GCC GAG TTA ACC GCC CTT TTC 816

Tyr Tyr Phe Lys Ser Glu Ile Thr Ser Ala Glu Leu Thr Ala Leu Phe
            260                 265                 270

CCA GAG GCC ACA ACT GCA AAT CAG AAA GCT TTA GAA TAC ACA GAA GAT        864
Pro Glu Ala Thr Thr Ala Asn Gln Lys Ala Leu Glu Tyr Thr Glu Asp
        275                 280                 285

TAT CAG TCG ATC GAA AAG AAT GCC CAG ATA ACA CAG GGA GAT AAA AGT        912
Tyr Gln Ser Ile Glu Lys Asn Ala Gln Ile Thr Gln Gly Asp Lys Ser
    290                 295                 300

AGA AAA GAA CTC GGG TTG GGG ATC GAC TTA CTT TTG ACG TTC ATG GAA        960
Arg Lys Glu Leu Gly Leu Gly Ile Asp Leu Leu Leu Thr Phe Met Glu
305                 310                 315                 320

GCA GTG AAC AAG AAG GCA CGT GTG GTT AAA AAC GAA GCT AGG TTT CTG       1008
Ala Val Asn Lys Lys Ala Arg Val Val Lys Asn Glu Ala Arg Phe Leu
                325                 330                 335

CTT ATC GCT ATT CAA ATG ACA GCT GAG GTA GCA CGA TTT AGG TAC ATT       1056
Leu Ile Ala Ile Gln Met Thr Ala Glu Val Ala Arg Phe Arg Tyr Ile
            340                 345                 350

CAA AAC TTG GTA ACT AAG AAC TTC CCC AAC AAG TTC GAC TCG GAT AAC       1104
Gln Asn Leu Val Thr Lys Asn Phe Pro Asn Lys Phe Asp Ser Asp Asn
        355                 360                 365

AAG GTG ATT CAA TTT GAA GTC AGC TGG CGT AAG ATT TCT ACG GCA ATA       1152
Lys Val Ile Gln Phe Glu Val Ser Trp Arg Lys Ile Ser Thr Ala Ile
    370                 375                 380

TAC GGG GAT GCC AAA AAC GGC GTG TTT AAT AAA GAT TAT GAT TTC GGG       1200
Tyr Gly Asp Ala Lys Asn Gly Val Phe Asn Lys Asp Tyr Asp Phe Gly
385                 390                 395                 400

TTT GGA AAA GTG AGG CAG GTG AAG GAC TTG CAA ATG GGA CTC CTT ATG       1248
Phe Gly Lys Val Arg Gln Val Lys Asp Leu Gln Met Gly Leu Leu Met
                405                 410                 415

TAT TTG GGC AAA CCA AAG                                               1266
Tyr Leu Gly Lys Pro Lys
            420

(2) INFORMATION FOR SEQ ID NO:49:

    (i) SEQUENCE CHARACTERISTICS:
        (A) LENGTH: 1320 base pairs
        (B) TYPE: nucleic acid
        (C) STRANDEDNESS: double
        (D) TOPOLOGY: unknown

    (ii) MOLECULE TYPE: cDNA

(ix) FEATURE:
(A) NAME/KEY: CDS
(B) LOCATION: 1..1320

(ix) FEATURE:
(A) NAME/KEY: mat_peptide
(B) LOCATION: 1..465
(D) OTHER INFORMATION: /product= "bFGF"

(ix) FEATURE:
(A) NAME/KEY: mat_peptide
(B) LOCATION: 466..561
(D) OTHER INFORMATION: /product= "Trypsin linker"

(ix) FEATURE:
(A) NAME/KEY: mat_peptide
(B) LOCATION: 562..1320
(D) OTHER INFORMATION: /product= "Saporin"

(xi) SEQUENCE DESCRIPTION: SEQ ID NO:49:

ATG GCA GCA GGA TCA ATA ACA ACA TTA CCC GCC TTG CCC GAG GAT GGC 48
Met Ala Ala Gly Ser Ile Thr Thr Leu Pro Ala Leu Pro Glu Asp Gly
1               5                   10                  15

GGC AGC GGC GCC TTC CCG CCC GGC CAC TTC AAG GAC CCC AAG CGG CTG 96
Gly Ser Gly Ala Phe Pro Pro Gly His Phe Lys Asp Pro Lys Arg Leu
            20                  25                  30

TAC TGC AAA AAC GGG GGC TTC TTC CTG CGC ATC CAC CCC GAC GGC CGA 144
Tyr Cys Lys Asn Gly Gly Phe Phe Leu Arg Ile His Pro Asp Gly Arg
        35                  40                  45

GTT GAC GGG GTC CGG GAG AAG AGC GAC CCT CAC ATC AAG CTT CAA CTT 192
Val Asp Gly Val Arg Glu Lys Ser Asp Pro His Ile Lys Leu Gln Leu
    50                  55                  60

CAA GCA GAA GAG AGA GGA GTT GTG TCT ATC AAA GGA GTG TGT GCT AAC 240
Gln Ala Glu Glu Arg Gly Val Val Ser Ile Lys Gly Val Cys Ala Asn
65                  70                  75                  80

CGT TAC CTG GCT ATG AAG GAA GAT GGA AGA TTA CTG GCT TCT AAA TGT 288
Arg Tyr Leu Ala Met Lys Glu Asp Gly Arg Leu Leu Ala Ser Lys Cys
                85                  90                  95

GTT ACG GAT GAG TGT TTC TTT TTT GAA CGA TTG GAA TCT AAT AAC TAC 336
Val Thr Asp Glu Cys Phe Phe Phe Glu Arg Leu Glu Ser Asn Asn Tyr
            100                 105                 110

AAT ACT TAC CGG TCA AGG AAA TAC ACC AGT TGG TAT GTG GCA TTG AAA 384
Asn Thr Tyr Arg Ser Arg Lys Tyr Thr Ser Trp Tyr Val Ala Leu Lys
        115                 120                 125

CGA ACT GGG CAG TAT AAA CTT GGA TCC AAA ACA GGA CCT GGG CAG AAA 432

Arg Thr Gly Gln Tyr Lys Leu Gly Ser Lys Thr Gly Pro Gly Gln Lys
    130                 135                 140

GCT ATA CTT TTT CTT CCA ATG TCT GCT AAG AGC GCC ATG GGC CGA TCG         480
Ala Ile Leu Phe Leu Pro Met Ser Ala Lys Ser Ala Met Gly Arg Ser
145                 150                 155                 160

GGC GGT GGG TGC GCT GGT AAT AGA GTC AGA AGA TCA GTC GGA AGC AGC         528
Gly Gly Gly Cys Ala Gly Asn Arg Val Arg Arg Ser Val Gly Ser Ser
                165                 170                 175

CTG TCT TGC GGT GGT CTC GAC CTG CAG GCC ATG GTC ACA TCA ATC ACA         576
Leu Ser Cys Gly Gly Leu Asp Leu Gln Ala Met Val Thr Ser Ile Thr
            180                 185                 190

TTA GAT CTA GTA AAT CCG ACC GCG GGT CAA TAC TCA TCT TTT GTG GAT         624
Leu Asp Leu Val Asn Pro Thr Ala Gly Gln Tyr Ser Ser Phe Val Asp
        195                 200                 205

AAA ATC CGA AAC AAC GTA AAG GAT CCA AAC CTG AAA TAC GGT GGT ACC         672
Lys Ile Arg Asn Asn Val Lys Asp Pro Asn Leu Lys Tyr Gly Gly Thr
    210                 215                 220

GAC ATA GCC GTG ATA GGC CCA CCT TCT AAA GAA AAA TTC CTT AGA ATT         720
Asp Ile Ala Val Ile Gly Pro Pro Ser Lys Glu Lys Phe Leu Arg Ile
225                 230                 235                 240

AAT TTC CAA AGT TCC CGA GGA ACG GTC TCA CTT GGC CTA AAA CGC GAT         768
Asn Phe Gln Ser Ser Arg Gly Thr Val Ser Leu Gly Leu Lys Arg Asp
                245                 250                 255

AAC TTG TAT GTG GTC GCG TAT CTT GCA ATG GAT AAC ACG AAT GTT AAT         816
Asn Leu Tyr Val Val Ala Tyr Leu Ala Met Asp Asn Thr Asn Val Asn
            260                 265                 270

CGG GCA TAT TAC TTC AAA TCA GAA ATT ACT TCC GCC GAG TTA ACC GCC         864
Arg Ala Tyr Tyr Phe Lys Ser Glu Ile Thr Ser Ala Glu Leu Thr Ala
        275                 280                 285

CTT TTC CCA GAG GCC ACA ACT GCA AAT CAG AAA GCT TTA GAA TAC ACA         912
Leu Phe Pro Glu Ala Thr Thr Ala Asn Gln Lys Ala Leu Glu Tyr Thr
    290                 295                 300

GAA GAT TAT CAG TCG ATC GAA AAG AAT GCC CAG ATA ACA CAG GGA GAT         960
Glu Asp Tyr Gln Ser Ile Glu Lys Asn Ala Gln Ile Thr Gln Gly Asp
305                 310                 315                 320

AAA AGT AGA AAA GAA CTC GGG TTG GGG ATC GAC TTA CTT TTG ACG TTC        1008
Lys Ser Arg Lys Glu Leu Gly Leu Gly Ile Asp Leu Leu Leu Thr Phe
                325                 330                 335

ATG GAA GCA GTG AAC AAG AAG GCA CGT GTG GTT AAA AAC GAA GCT AGG        1056
Met Glu Ala Val Asn Lys Lys Ala Arg Val Val Lys Asn Glu Ala Arg
            340                 345                 350

TTT CTG CTT ATC GCT ATT CAA ATG ACA GCT GAG GTA GCA CGA TTT AGG 1104
Phe Leu Leu Ile Ala Ile Gln Met Thr Ala Glu Val Ala Arg Phe Arg
        355                 360                 365

TAC ATT CAA AAC TTG GTA ACT AAG AAC TTC CCC AAC AAG TTC GAC TCG 1152
Tyr Ile Gln Asn Leu Val Thr Lys Asn Phe Pro Asn Lys Phe Asp Ser
    370                 375                 380

GAT AAC AAG GTG ATT CAA TTT GAA GTC AGC TGG CGT AAG ATT TCT ACG 1200
Asp Asn Lys Val Ile Gln Phe Glu Val Ser Trp Arg Lys Ile Ser Thr
385                 390                 395                 400

GCA ATA TAC GGG GAT GCC AAA AAC GGC GTG TTT AAT AAA GAT TAT GAT 1248
Ala Ile Tyr Gly Asp Ala Lys Asn Gly Val Phe Asn Lys Asp Tyr Asp
                405                 410                 415

TTC GGG TTT GGA AAA GTG AGG CAG GTG AAG GAC TTG CAA ATG GGA CTC 1296
Phe Gly Phe Gly Lys Val Arg Gln Val Lys Asp Leu Gln Met Gly Leu
            420                 425                 430

CTT ATG TAT TTG GGC AAA CCA AAG 1320
Leu Met Tyr Leu Gly Lys Pro Lys
        435                 440

(2) INFORMATION FOR SEQ ID NO:50:

(i) SEQUENCE CHARACTERISTICS:
(A) LENGTH: 1299 base pairs
(B) TYPE: nucleic acid
(C) STRANDEDNESS: double
(D) TOPOLOGY: unknown

(ii) MOLECULE TYPE: cDNA

(ix) FEATURE:
(A) NAME/KEY: CDS
(B) LOCATION: 1..1299

(ix) FEATURE:
(A) NAME/KEY: mat_peptide
(B) LOCATION: 1..465
(D) OTHER INFORMATION: /product= "bFGF"

(ix) FEATURE:
(A) NAME/KEY: mat_peptide
(B) LOCATION: 466..540
(D) OTHER INFORMATION: /product= "(Ser4Gly)4linker"

(ix) FEATURE:
(A) NAME/KEY: mat_peptide
(B) LOCATION: 541..1299
(D) OTHER INFORMATION: /product= "Saporin"

(xi) SEQUENCE DESCRIPTION: SEQ ID NO:50:

ATG GCA GCA GGA TCA ATA ACA ACA TTA CCC GCC TTG CCC GAG GAT GGC 48
Met Ala Ala Gly Ser Ile Thr Thr Leu Pro Ala Leu Pro Glu Asp Gly
1               5                   10                  15

GGC AGC GGC GCC TTC CCG CCC GGC CAC TTC AAG GAC CCC AAG CGG CTG 96
Gly Ser Gly Ala Phe Pro Pro Gly His Phe Lys Asp Pro Lys Arg Leu
            20                  25                  30

TAC TGC AAA AAC GGG GGC TTC TTC CTG CGC ATC CAC CCC GAC GGC CGA 144
Tyr Cys Lys Asn Gly Gly Phe Phe Leu Arg Ile His Pro Asp Gly Arg
        35                  40                  45

GTT GAC GGG GTC CGG GAG AAG AGC GAC CCT CAC ATC AAG CTT CAA CTT 192
Val Asp Gly Val Arg Glu Lys Ser Asp Pro His Ile Lys Leu Gln Leu
    50                  55                  60

CAA GCA GAA GAG AGA GGA GTT GTG TCT ATC AAA GGA GTG TGT GCT AAC 240
Gln Ala Glu Glu Arg Gly Val Val Ser Ile Lys Gly Val Cys Ala Asn
65                  70                  75                  80

CGT TAC CTG GCT ATG AAG GAA GAT GGA AGA TTA CTG GCT TCT AAA TGT 288
Arg Tyr Leu Ala Met Lys Glu Asp Gly Arg Leu Leu Ala Ser Lys Cys
                85                  90                  95

GTT ACG GAT GAG TGT TTC TTT TTT GAA CGA TTG GAA TCT AAT AAC TAC 336
Val Thr Asp Glu Cys Phe Phe Phe Glu Arg Leu Glu Ser Asn Asn Tyr
            100                 105                 110

AAT ACT TAC CGG TCA AGG AAA TAC ACC AGT TGG TAT GTG GCA TTG AAA 384
Asn Thr Tyr Arg Ser Arg Lys Tyr Thr Ser Trp Tyr Val Ala Leu Lys
        115                 120                 125

CGA ACT GGG CAG TAT AAA CTT GGA TCC AAA ACA GGA CCT GGG CAG AAA 432
Arg Thr Gly Gln Tyr Lys Leu Gly Ser Lys Thr Gly Pro Gly Gln Lys
    130                 135                 140

GCT ATA CTT TTT CTT CCA ATG TCT GCT AAG AGC GCC ATG GCC TCG TCG 480
Ala Ile Leu Phe Leu Pro Met Ser Ala Lys Ser Ala Met Ala Ser Ser
145                 150                 155                 160

TCG TCG GGC TCG TCG TCG TCG GGC TCG TCG TCG TCG GGC TCG TCG TCG 528
Ser Ser Gly Ser Ser Ser Ser Gly Ser Ser Ser Ser Gly Ser Ser Ser
                165                 170                 175

TCG GGC GCC ATG GTC ACA TCA ATC ACA TTA GAT CTA GTA AAT CCG ACC 576
Ser Gly Ala Met Val Thr Ser Ile Thr Leu Asp Leu Val Asn Pro Thr
            180                 185                 190
GCG GGT CAA TAC TCA TCT TTT GTG GAT AAA ATC CGA AAC AAC GTA AAG 624
Ala Gly Gln Tyr Ser Ser Phe Val Asp Lys Ile Arg Asn Asn Val Lys
        195                 200                 205

GAT CCA AAC CTG AAA TAC GGT GGT ACC GAC ATA GCC GTG ATA GGC CCA 672
Asp Pro Asn Leu Lys Tyr Gly Gly Thr Asp Ile Ala Val Ile Gly Pro
    210                 215                 220

CCT TCT AAA GAA AAA TTC CTT AGA ATT AAT TTC CAA AGT TCC CGA GGA 720
Pro Ser Lys Glu Lys Phe Leu Arg Ile Asn Phe Gln Ser Ser Arg Gly
225                 230                 235                 240

ACG GTC TCA CTT GGC CTA AAA CGC GAT AAC TTG TAT GTG GTC GCG TAT 768
Thr Val Ser Leu Gly Leu Lys Arg Asp Asn Leu Tyr Val Val Ala Tyr
                245                 250                 255

CTT GCA ATG GAT AAC ACG AAT GTT AAT CGG GCA TAT TAC TTC AAA TCA 816
Leu Ala Met Asp Asn Thr Asn Val Asn Arg Ala Tyr Tyr Phe Lys Ser
            260                 265                 270

GAA ATT ACT TCC GCC GAG TTA ACC GCC CTT TTC CCA GAG GCC ACA ACT 864
Glu Ile Thr Ser Ala Glu Leu Thr Ala Leu Phe Pro Glu Ala Thr Thr
        275                 280                 285

GCA AAT CAG AAA GCT TTA GAA TAC ACA GAA GAT TAT CAG TCG ATC GAA 912
Ala Asn Gln Lys Ala Leu Glu Tyr Thr Glu Asp Tyr Gln Ser Ile Glu
    290                 295                 300

AAG AAT GCC CAG ATA ACA CAG GGA GAT AAA AGT AGA AAA GAA CTC GGG 960
Lys Asn Ala Gln Ile Thr Gln Gly Asp Lys Ser Arg Lys Glu Leu Gly
305                 310                 315                 320

TTG GGG ATC GAC TTA CTT TTG ACG TTC ATG GAA GCA GTG AAC AAG AAG 1008
Leu Gly Ile Asp Leu Leu Leu Thr Phe Met Glu Ala Val Asn Lys Lys
                325                 330                 335

GCA CGT GTG GTT AAA AAC GAA GCT AGG TTT CTG CTT ATC GCT ATT CAA 1056
Ala Arg Val Val Lys Asn Glu Ala Arg Phe Leu Leu Ile Ala Ile Gln
            340                 345                 350

ATG ACA GCT GAG GTA GCA CGA TTT AGG TAC ATT CAA AAC TTG GTA ACT 1104
Met Thr Ala Glu Val Ala Arg Phe Arg Tyr Ile Gln Asn Leu Val Thr
        355                 360                 365

AAG AAC TTC CCC AAC AAG TTC GAC TCG GAT AAC AAG GTG ATT CAA TTT 1152
Lys Asn Phe Pro Asn Lys Phe Asp Ser Asp Asn Lys Val Ile Gln Phe
    370                 375                 380

GAA GTC AGC TGG CGT AAG ATT TCT ACG GCA ATA TAC GGG GAT GCC AAA 1200
Glu Val Ser Trp Arg Lys Ile Ser Thr Ala Ile Tyr Gly Asp Ala Lys
385                 390                 395                 400

AAC GGC GTG TTT AAT AAA GAT TAT GAT TTC GGG TTT GGA AAA GTG AGG 1248
Asn Gly Val Phe Asn Lys Asp Tyr Asp Phe Gly Phe Gly Lys Val Arg
                405                 410                 415

CAG GTG AAG GAC TTG CAA ATG GGA CTC CTT ATG TAT TTG GGC AAA CCA 1296
Gln Val Lys Asp Leu Gln Met Gly Leu Leu Met Tyr Leu Gly Lys Pro
            420                 425                 430

AAG 1299
Lys

(2) INFORMATION FOR SEQ ID NO:51:

(i) SEQUENCE CHARACTERISTICS:
(A) LENGTH: 1269 base pairs
(B) TYPE: nucleic acid
(C) STRANDEDNESS: double
(D) TOPOLOGY: unknown

(ii) MOLECULE TYPE: cDNA

(ix) FEATURE:
(A) NAME/KEY: CDS
(B) LOCATION: 1..1269

(ix) FEATURE:
(A) NAME/KEY: mat_peptide
(B) LOCATION: 1..465
(D) OTHER INFORMATION: /product= "bFGF"

(ix) FEATURE:
(A) NAME/KEY: mat_peptide
(B) LOCATION: 466..510
(D) OTHER INFORMATION: /product= "(Ser4Gly)2 linker"

(ix) FEATURE:
(A) NAME/KEY: mat_peptide
(B) LOCATION: 511..1269
(D) OTHER INFORMATION: /product= "Saporin"

(xi) SEQUENCE DESCRIPTION: SEQ ID NO:51

ATG GCA GCA GGA TCA ATA ACA ACA TTA CCC GCC TTG CCC GAG GAT GGC
Met Ala Ala Gly Ser Ile Thr Thr Leu Pro Ala Leu Pro Glu Asp Gly
1               5                   10                  15

GGC AGC GGC GCC TTC CCG CCC GGC CAC TTC AAG GAC CCC AAG CGG CTG
Gly Ser Gly Ala Phe Pro Pro Gly His Phe Lys Asp Pro Lys Arg Leu
            20                  25                  30

TAC TGC AAA AAC GGG GGC TTC TTC CTG CGC ATC CAC CCC GAC GGC CGA
Tyr Cys Lys Asn Gly Gly Phe Phe Leu Arg Ile His Pro Asp Gly Arg
        35                  40                  45

GTT GAC GGG GTC CGG GAG AAG AGC GAC CCT CAC ATC AAG CTT CAA CTT
Val Asp Gly Val Arg Glu Lys Ser Asp Pro His Ile Lys Leu Gln Leu
    50                  55                  60

CAA GCA GAA GAG AGA GGA GTT GTG TCT ATC AAA GGA GTG TGT GCT AAC
Gln Ala Glu Glu Arg Gly Val Val Ser Ile Lys Gly Val Cys Ala Asn
65                  70                  75                  80

CGT TAC CTG GCT ATG AAG GAA GAT GGA AGA TTA CTG GCT TCT AAA TGT
Arg Tyr Leu Ala Met Lys Glu Asp Gly Arg Leu Leu Ala Ser Lys Cys
                85                  90                  95

GTT ACG GAT GAG TGT TTC TTT TTT GAA CGA TTG GAA TCT AAT AAC TAC
Val Thr Asp Glu Cys Phe Phe Phe Glu Arg Leu Glu Ser Asn Asn Tyr
            100                 105                 110

AAT ACT TAC CGG TCA AGG AAA TAC ACC AGT TGG TAT GTG GCA TTG AAA
Asn Thr Tyr Arg Ser Arg Lys Tyr Thr Ser Trp Tyr Val Ala Leu Lys
        115                 120                 125

CGA ACT GGG CAG TAT AAA CTT GGA TCC AAA ACA GGA CCT GGG CAG AAA
Arg Thr Gly Gln Tyr Lys Leu Gly Ser Lys Thr Gly Pro Gly Gln Lys
    130                 135                 140

GCT ATA CTT TTT CTT CCA ATG TCT GCT AAG AGC GCC ATG GCC TCG TCG
Ala Ile Leu Phe Leu Pro Met Ser Ala Lys Ser Ala Met Ala Ser Ser
145                 150                 155                 160

TCG TCG GGC TCG TCG TCG TCG GGC GCC ATG GTC ACA TCA ATC ACA TTA
Ser Ser Gly Ser Ser Ser Ser Gly Ala Met Val Thr Ser Ile Thr Leu
                165                 170                 175
GAT CTA GTA AAT CCG ACC GCG GGT CAA TAC TCA TCT TTT GTG GAT AAA
Asp Leu Val Asn Pro Thr Ala Gly Gln Tyr Ser Ser Phe Val Asp Lys
            180                 185                 190
ATC CGA AAC AAC GTA AAG GAT CCA AAC CTG AAA TAC GGT GGT ACC GAC
Ile Arg Asn Asn Val Lys Asp Pro Asn Leu Lys Tyr Gly Gly Thr Asp
        195                 200                 205

ATA GCC GTG ATA GGC CCA CCT TCT AAA GAA AAA TTC CTT AGA ATT AAT
Ile Ala Val Ile Gly Pro Pro Ser Lys Glu Lys Phe Leu Arg Ile Asn
    210                 215                 220

TTC CAA AGT TCC CGA GGA ACG GTC TCA CTT GGC CTA AAA CGC GAT AAC
Phe Gln Ser Ser Arg Gly Thr Val Ser Leu Gly Leu Lys Arg Asp Asn
225                 230                 235                 240

TTG TAT GTG GTC GCG TAT CTT GCA ATG GAT AAC ACG AAT GTT AAT CGG
Leu Tyr Val Val Ala Tyr Leu Ala Met Asp Asn Thr Asn Val Asn Arg
                245                 250                 255

GCA TAT TAC TTC AAA TCA GAA ATT ACT TCC GCC GAG TTA ACC GCC CTT
Ala Tyr Tyr Phe Lys Ser Glu Ile Thr Ser Ala Glu Leu Thr Ala Leu
            260                 265                 270

TTC CCA GAG GCC ACA ACT GCA AAT CAG AAA GCT TTA GAA TAC ACA GAA
Phe Pro Glu Ala Thr Thr Ala Asn Gln Lys Ala Leu Glu Tyr Thr Glu
        275                 280                 285

GAT TAT CAG TCG ATC GAA AAG AAT GCC CAG ATA ACA CAG GGA GAT AAA
Asp Tyr Gln Ser Ile Glu Lys Asn Ala Gln Ile Thr Gln Gly Asp Lys
    290                 295                 300

AGT AGA AAA GAA CTC GGG TTG GGG ATC GAC TTA CTT TTG ACG TTC ATG
Ser Arg Lys Glu Leu Gly Leu Gly Ile Asp Leu Leu Leu Thr Phe Met
305                 310                 315                 320

GAA GCA GTG AAC AAG AAG GCA CGT GTG GTT AAA AAC GAA GCT AGG TTT

Glu Ala Val Asn Lys Lys Ala Arg Val Val Lys Asn Glu Ala Arg Phe
                325                 330                 335

CTG CTT ATC GCT ATT CAA ATG ACA GCT GAG GTA GCA CGA TTT AGG TAC
Leu Leu Ile Ala Ile Gln Met Thr Ala Glu Val Ala Arg Phe Arg Tyr
            340                 345                 350

ATT CAA AAC TTG GTA ACT AAG AAC TTC CCC AAC AAG TTC GAC TCG GAT
Ile Gln Asn Leu Val Thr Lys Asn Phe Pro Asn Lys Phe Asp Ser Asp
        355                 360                 365

AAC AAG GTG ATT CAA TTT GAA GTC AGC TGG CGT AAG ATT TCT ACG GCA
Asn Lys Val Ile Gln Phe Glu Val Ser Trp Arg Lys Ile Ser Thr Ala
    370                 375                 380

ATA TAC GGG GAT GCC AAA AAC GGC GTG TTT AAT AAA GAT TAT GAT TTC
Ile Tyr Gly Asp Ala Lys Asn Gly Val Phe Asn Lys Asp Tyr Asp Phe
385                 390                 395                 400

GGG TTT GGA AAA GTG AGG CAG GTG AAG GAC TTG CAA ATG GGA CTC CTT
Gly Phe Gly Lys Val Arg Gln Val Lys Asp Leu Gln Met Gly Leu Leu
                405                 410                 415

ATG TAT TTG GGC AAA CCA AAG
Met Tyr Leu Gly Lys Pro Lys
            420

(2) INFORMATION FOR SEQ ID NO:52:

(i) SEQUENCE CHARACTERISTICS:
(A) LENGTH: 18 base pairs
(B) TYPE: nucleic acid
(C) STRANDEDNESS: double
(D) TOPOLOGY: unknown

(ii) MOLECULE TYPE: cDNA

(ix) FEATURE:
(A) NAME/KEY: CDS
(B) LOCATION: 1..18
(D) OTHER INFORMATION: /product= Thrombin substrate linker

(xi) SEQUENCE DESCRIPTION: SEQ ID NO:52

CTG GTG CCG CGC GGC AGC
Leu Val Pro Arg Gly Ser
1               5

(2) INFORMATION FOR SEQ ID NO:53:

(i) SEQUENCE CHARACTERISTICS:
(A) LENGTH: 15 base pairs
(B) TYPE: nucleic acid

(C) STRANDEDNESS: double
(D) TOPOLOGY: unknown

(ii) MOLECULE TYPE: cDNA

(ix) FEATURE:
(A) NAME/KEY: CDS
(B) LOCATION: 1..15
(D) OTHER INFORMATION: /product= Enterokinase substrate linker

(xi) SEQUENCE DESCRIPTION: SEQ ID NO:53

GAC GAC GAC GAC CCA
Asp Asp Asp Asp Lys
1               5

(2) INFORMATION FOR SEQ ID NO:54:

(i) SEQUENCE CHARACTERISTICS:
(A) LENGTH: 12 base pairs
(B) TYPE: nucleic acid
(C) STRANDEDNESS: double
(D) TOPOLOGY: unknown

(ii) MOLECULE TYPE: cDNA

(ix) FEATURE:
(A) NAME/KEY: CDS
(B) LOCATION: 1..12
(D) OTHER INFORMATION: /product= Factor Xa [?]

(xi) SEQUENCE DESCRIPTION: SEQ ID NO:54

ATC GAA GGT CGT
Ile Glu Gly Arg
1

(2) INFORMATION FOR SEQ ID NO:55:

(i) SEQUENCE CHARACTERISTICS:
(A) LENGTH: 8 amino acids
(B) TYPE: amino acid
(C) STRANDEDNESS: single
(D) TOPOLOGY: unknown

(ii) MOLECULE TYPE: peptide

(ix) FEATURE:
(A) NAME/KEY: CDS
(B) LOCATION: 1..8
(D) OTHER INFORMATION: /product= Flexible linker

(xi) SEQUENCE DESCRIPTION: SEQ ID NO:55

Ala Ala Pro Ala Ala Ala Pro Ala

(2) INFORMATION FOR SEQ ID NO:56:

(i) SEQUENCE CHARACTERISTICS:
(A) LENGTH: 4 amino acids
(B) TYPE: amino acid
(C) STRANDEDNESS: single
(D) TOPOLOGY: unknown

(ii) MOLECULE TYPE: peptide

(ix) FEATURE:
(A) NAME/KEY: CDS
(B) LOCATION: 1..4
(D) OTHER INFORMATION: /product= subtilisin substrate linker

(xi) SEQUENCE DESCRIPTION: SEQ ID NO:56
Phe Ala His Tyr
1

(2) INFORMATION FOR SEQ ID NO:57:

(i) SEQUENCE CHARACTERISTICS:
(A) LENGTH: 4 amino acids
(B) TYPE: amino acid
(C) STRANDEDNESS: single
(D) TOPOLOGY: unknown

(ii) MOLECULE TYPE: peptide

(ix) FEATURE:
(A) NAME/KEY: CDS
(B) LOCATION: 1..4
(D) OTHER INFORMATION: /product= subtilisin substrate linker

(xi) SEQUENCE DESCRIPTION: SEQ ID NO:57

Xaa Asp Glu Leu
1

(2) INFORMATION FOR SEQ ID NO:58:

(i) SEQUENCE CHARACTERISTICS:
(A) LENGTH: 7 amino acids
(B) TYPE: amino acid
(C) STRANDEDNESS: single
(D) TOPOLOGY: unknown

(ii) MOLECULE TYPE: peptide

(ix) FEATURE:
(A) NAME/KEY: CDS
(B) LOCATION: 1..7
(D) OTHER INFORMATION: /product= nuclear translocation sequence

(xi) SEQUENCE DESCRIPTION: SEQ ID NO:58

Pro Lys Lys Arg Lys Val Glu
 1               5

(2) INFORMATION FOR SEQ ID NO:59:

     (i) SEQUENCE CHARACTERISTICS:
          (A) LENGTH: 8 amino acids
          (B) TYPE: amino acid
          (C) STRANDEDNESS: single
          (D) TOPOLOGY: unknown

    (ii) MOLECULE TYPE: peptide

    (ix) FEATURE:
          (A) NAME/KEY: CDS
          (B) LOCATION: 1..8
          (D) OTHER INFORMATION: /product= nuclear translocation sequence

    (xi) SEQUENCE DESCRIPTION: SEQ ID NO:59

Pro Pro Lys Lys Ala Arg Glu Val
 1               5

(2) INFORMATION FOR SEQ ID NO:60:

     (i) SEQUENCE CHARACTERISTICS:
          (A) LENGTH: 9 amino acids
          (B) TYPE: amino acid
          (C) STRANDEDNESS: single
          (D) TOPOLOGY: unknown

    (ii) MOLECULE TYPE: peptide

    (ix) FEATURE:
          (A) NAME/KEY: CDS
          (B) LOCATION: 1..9
          (D) OTHER INFORMATION: /product= nuclear translocation sequence

    (xi) SEQUENCE DESCRIPTION: SEQ ID NO:60

Pro Ala Ala Lys Arg Val Lys Leu Asp
 1               5

(2) INFORMATION FOR SEQ ID NO:61:

     (i) SEQUENCE CHARACTERISTICS:
          (A) LENGTH: 5 amino acids
          (B) TYPE: amino acid
          (C) STRANDEDNESS: single
          (D) TOPOLOGY: unknown

    (ii) MOLECULE TYPE: peptide

(ix) FEATURE:
(A) NAME/KEY: CDS
(B) LOCATION: 1..5
(D) OTHER INFORMATION: /product= nuclear translocation sequence

(xi) SEQUENCE DESCRIPTION: SEQ ID NO:61

Lys Arg Pro Arg Pro
1               5

(2) INFORMATION FOR SEQ ID NO:62:

(i) SEQUENCE CHARACTERISTICS:
(A) LENGTH: 5 amino acids
(B) TYPE: amino acid
(C) STRANDEDNESS: single
(D) TOPOLOGY: unknown

(ii) MOLECULE TYPE: peptide

(ix) FEATURE:
(A) NAME/KEY: CDS
(B) LOCATION: 1..5
(D) OTHER INFORMATION: /product= nuclear translocation sequence

(xi) SEQUENCE DESCRIPTION: SEQ ID NO:62

Lys Ile Pro Ile Lys
1               5

(2) INFORMATION FOR SEQ ID NO:63:

(i) SEQUENCE CHARACTERISTICS:
(A) LENGTH: 7 amino acids
(B) TYPE: amino acid
(C) STRANDEDNESS: single
(D) TOPOLOGY: unknown

(ii) MOLECULE TYPE: peptide

(ix) FEATURE:
(A) NAME/KEY: CDS
(B) LOCATION: 1..9
(D) OTHER INFORMATION: /product= nuclear translocation sequence

(xi) SEQUENCE DESCRIPTION: SEQ ID NO:63

Gly Lys Arg Lys Arg Lys Ser
1               5

(2) INFORMATION FOR SEQ ID NO:64:

(i) SEQUENCE CHARACTERISTICS:
(A) LENGTH: 9 amino acids

(B) TYPE: amino acid
(C) STRANDEDNESS: single
(D) TOPOLOGY: unknown

(ii) MOLECULE TYPE: peptide

(ix) FEATURE:
(A) NAME/KEY: CDS
(B) LOCATION: 1..9
(D) OTHER INFORMATION: /product= nuclear translocation sequence

(xi) SEQUENCE DESCRIPTION: SEQ ID NO:64

Ser Lys Arg Val Ala Lys Arg Lys leu
1               5

(2) INFORMATION FOR SEQ ID NO:65:

(i) SEQUENCE CHARACTERISTICS:
(A) LENGTH: 9 amino acids
(B) TYPE: amino acid
(C) STRANDEDNESS: single
(D) TOPOLOGY: unknown

(ii) MOLECULE TYPE: peptide

(ix) FEATURE:
(A) NAME/KEY: CDS
(B) LOCATION: 1..9
(D) OTHER INFORMATION: /product= nuclear translocation sequence

(xi) SEQUENCE DESCRIPTION: SEQ ID NO:65

Ser His Trp Lys Gln Lys Arg Lys Phe
1               5

(2) INFORMATION FOR SEQ ID NO:66:

(i) SEQUENCE CHARACTERISTICS:
(A) LENGTH: 8 amino acids
(B) TYPE: amino acid
(C) STRANDEDNESS: single
(D) TOPOLOGY: unknown

(ii) MOLECULE TYPE: peptide

(ix) FEATURE:
(A) NAME/KEY: CDS
(B) LOCATION: 1..8
(D) OTHER INFORMATION: /product= nuclear translocation sequence

(xi) SEQUENCE DESCRIPTION: SEQ ID NO:66

Pro Leu Leu Lys Lys Ile Lys Gln
1               5

(2) INFORMATION FOR SEQ ID NO:67:

(i) SEQUENCE CHARACTERISTICS:
(A) LENGTH: 7 amino acids
(B) TYPE: amino acid
(C) STRANDEDNESS: single
(D) TOPOLOGY: unknown

(ii) MOLECULE TYPE: peptide

(ix) FEATURE:
(A) NAME/KEY: CDS
(B) LOCATION: 1..7
(D) OTHER INFORMATION: /product= nuclear translocation sequence

(xi) SEQUENCE DESCRIPTION: SEQ ID NO:67

Pro Gln Pro Lys Lys Lys Pro
1               5

(2) INFORMATION FOR SEQ ID NO:68:

(i) SEQUENCE CHARACTERISTICS:
(A) LENGTH: 15 amino acids
(B) TYPE: amino acid
(C) STRANDEDNESS: single
(D) TOPOLOGY: unknown

(ii) MOLECULE TYPE: peptide

(ix) FEATURE:
(A) NAME/KEY: CDS
(B) LOCATION: 1..15
(D) OTHER INFORMATION: /product= nuclear translocation sequence

(xi) SEQUENCE DESCRIPTION: SEQ ID NO:68

Pro Gly Lys Arg Lys Lys Glu Met Thr Lys Gln Lys Glu Val Pro
1               5                   10                  15

(2) INFORMATION FOR SEQ ID NO:69:

(i) SEQUENCE CHARACTERISTICS:
(A) LENGTH: 12 amino acids
(B) TYPE: amino acid
(C) STRANDEDNESS: single
(D) TOPOLOGY: unknown

(ii) MOLECULE TYPE: peptide

(ix) FEATURE:
(A) NAME/KEY: CDS
(B) LOCATION: 1..12
(D) OTHER INFORMATION: /product= nuclear translocation sequence

(xi) SEQUENCE DESCRIPTION: SEQ ID NO:69

Gly Arg Lys Lys Arg Arg Gln Arg Arg Arg Ala Pro
1               5                   10

(2) INFORMATION FOR SEQ ID NO:70:

(i) SEQUENCE CHARACTERISTICS:
(A) LENGTH: 7 amino acids
(B) TYPE: amino acid
(C) STRANDEDNESS: single
(D) TOPOLOGY: unknown

(ii) MOLECULE TYPE: peptide

(ix) FEATURE:
(A) NAME/KEY: CDS
(B) LOCATION: 1..7
(D) OTHER INFORMATION: /product= nuclear translocation sequence

(xi) SEQUENCE DESCRIPTION: SEQ ID NO:70

Asn Tyr Lys Lys Pro Lys Leu
1               5

(2) INFORMATION FOR SEQ ID NO:71:

(i) SEQUENCE CHARACTERISTICS:
(A) LENGTH: 7 amino acids
(B) TYPE: amino acid
(C) STRANDEDNESS: single
(D) TOPOLOGY: unknown

(ii) MOLECULE TYPE: peptide

(ix) FEATURE:
(A) NAME/KEY: CDS
(B) LOCATION: 1..7
(D) OTHER INFORMATION: /product= nuclear translocation sequence

(xi) SEQUENCE DESCRIPTION: SEQ ID NO:71

His Phe Lys Asp Pro Lys Arg
1               5

(2) INFORMATION FOR SEQ ID NO:72:

(i) SEQUENCE CHARACTERISTICS:
(A) LENGTH: 7 amino acids
(B) TYPE: amino acid
(C) STRANDEDNESS: single
(D) TOPOLOGY: unknown

(ii) MOLECULE TYPE: peptide

(ix) FEATURE:
(A) NAME/KEY: CDS
(B) LOCATION: 1..7
(D) OTHER INFORMATION: /product= nuclear translocation sequence

(xi) SEQUENCE DESCRIPTION: SEQ ID NO:72

Ala Pro Arg Arg Arg Lys Leu
1               5

(2) INFORMATION FOR SEQ ID NO:73:

(i) SEQUENCE CHARACTERISTICS:
(A) LENGTH: 6 amino acids
(B) TYPE: amino acid
(C) STRANDEDNESS: single
(D) TOPOLOGY: unknown

(ii) MOLECULE TYPE: peptide

(ix) FEATURE:
(A) NAME/KEY: CDS
(B) LOCATION: 1..6
(D) OTHER INFORMATION: /product= nuclear translocation sequence

(xi) SEQUENCE DESCRIPTION: SEQ ID NO:73

Ile Lys Arg Leu Arg Arg
1               5

(2) INFORMATION FOR SEQ ID NO:74:

(i) SEQUENCE CHARACTERISTICS:
(A) LENGTH: 6 amino acids
(B) TYPE: amino acid
(C) STRANDEDNESS: single
(D) TOPOLOGY: unknown

(ii) MOLECULE TYPE: peptide

(ix) FEATURE:
(A) NAME/KEY: CDS
(B) LOCATION: 1..6
(D) OTHER INFORMATION: /product= nuclear translocation sequence

(xi) SEQUENCE DESCRIPTION: SEQ ID NO:74

Ile Lys Arg Gln Arg Arg
1               5

(2) INFORMATION FOR SEQ ID NO:75:

(i) SEQUENCE CHARACTERISTICS:

(A) LENGTH: 5 amino acids
(B) TYPE: amino acid
(C) STRANDEDNESS: single
(D) TOPOLOGY: unknown

(ii) MOLECULE TYPE: peptide

(ix) FEATURE:
(A) NAME/KEY: CDS
(B) LOCATION: 1..5
(D) OTHER INFORMATION: /product= nuclear translocation sequence

(xi) SEQUENCE DESCRIPTION: SEQ ID NO:75

Ile Arg Val Arg Arg
1               5

(2) INFORMATION FOR SEQ ID NO:76:

(i) SEQUENCE CHARACTERISTICS:
(A) LENGTH: 36 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:76

CATATGGTCA CATCATGTAC ATTAGATCTA GTAAAT                                36

(2) INFORMATION FOR SEQ ID NO:77:

(i) SEQUENCE CHARACTERISTICS:
(A) LENGTH: 50 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:77

CATATGGTCA CATCAATCAC ATTAGATCTA GTATGTCCGA CCGCGGGTCA                 50

(2) INFORMATION FOR SEQ ID NO:78

(i) SEQUENCE CHARACTERISTICS:
(A) LENGTH: 66 base pairs
(B) TYPE: nucleic acid
(C) STRANDEDNESS: double
(D) TOPOLOGY: both

(ii) MOLECULE TYPE: genomic

(ix) FEATURE:
(A) NAME/KEY: CDS
(B) LOCATION: 1..66
(D) OTHER INFORMATION: /product= VEGF gene EXON I (VEGF LEADER
SEQUENCE -26 - -5)

(xi) SEQUENCE DESCRIPTION: SEQ ID NO:78

ATG AAC TTT CTG CTG TCT TGG GTG CAT TGG AGC CTT GCC TTG CTG CTC 48
Met Asn Phe Leu Leu Ser Trp Val His Trp Ser Leu Ala Leu Leu Leu
1               5                   10                  15

TAC CTC CAC CAT GCC AAG 66
Tyr Leu His His Ala Lys
            20
(2) INFORMATION FOR SEQ ID NO:79

(i) SEQUENCE CHARACTERISTICS:
(A) LENGTH: 52 base pairs
(B) TYPE: nucleic acid
(C) STRANDEDNESS: double
(D) TOPOLOGY: both

(ii) MOLECULE TYPE: genomic

(ix) FEATURE:
(A) NAME/KEY: CDS
(B) LOCATION: 1..52
(D) OTHER INFORMATION: /product= VEGF gene EXON II

(xi) SEQUENCE DESCRIPTION: SEQ ID NO:79

TGG TCC CAG GCT GCA CCC ATG GCA GAA GGA GGA GGG CAG AAT CAT CAC 48
Trp Ser Gln Ala Ala Pro Met Ala Glu Gly Gly Gly Gln Asn His His
1               5                   10                  15

GAA G 52
Glu

(2) INFORMATION FOR SEQ ID NO:80

(i) SEQUENCE CHARACTERISTICS:
(A) LENGTH: 197 base pairs
(B) TYPE: nucleic acid
(C) STRANDEDNESS: double
(D) TOPOLOGY: both

(ii) MOLECULE TYPE: genomic

(ix) FEATURE:
(A) NAME/KEY: CDS
(B) LOCATION: 3..197
(D) OTHER INFORMATION: /product= VEGF gene EXON III

(xi) SEQUENCE DESCRIPTION: SEQ ID NO:80

TG GTG AAG TTC ATG GAT GTC TAT CAG CGC AGC TAC TGC CAT CCA ATC 47
Val Lys Phe Met Asp Val Tyr Gln Arg Ser Tyr Cys His Pro Ile
1               5                   10                  15

GAG ACC CTG GTG GAC ATC TTC CAG GAG TAC CCT GAT GAG ATC GAG TAC 95
Glu Thr Leu Val Asp Ile Phe Gln Glu Tyr Pro Asp Glu Ile Glu Tyr
                20                  25                  30

ATC TTC AAG CCA TCC TGT GTG CCC CTG ATG CGA TGC GGG GGC TGC TGC 143
Ile Phe Lys Pro Ser Cys Val Pro Leu Met Arg Cys Gly Gly Cys Cys
            35                  40                  45

AAT GAC GAG GGC CTG GAG TGT GTG CCC ACT GAG GAG TCC AAC ATC ACC 191
Asn Asp Glu Gly Leu Glu Cys Val Pro Thr Glu Glu Ser Asn Ile Thr
        50                  55                  60

ATG CAG 197
Met Gln
64

(2) INFORMATION FOR SEQ ID NO:81

(i) SEQUENCE CHARACTERISTICS:
(A) LENGTH: 77 base pairs
(B) TYPE: nucleic acid
(C) STRANDEDNESS: double
(D) TOPOLOGY: both

(ii) MOLECULE TYPE: genomic

(ix) FEATURE:
(A) NAME/KEY: CDS
(B) LOCATION: 1..75
(D) OTHER INFORMATION: /product= VEGF gene EXON IV

(xi) SEQUENCE DESCRIPTION: SEQ ID NO:81

ATT ATG CGG ATC AAA CCT CAC CAA GGC CAG CAC ATA GGA GAG ATG AGC 48
Ile Met Arg Ile Lys Pro His Gln Gly Gln His Ile Gly Glu Met Ser
110 115 120

TTC CTA CAG CAC AAC AAA TGT GAA TGC AG 77
Phe Leu Gln His Asn Lys Cys Glu Cys
125 130 135 140

(2) INFORMATION FOR SEQ ID NO:82

(i) SEQUENCE CHARACTERISTICS:
(A) LENGTH: 30 base pairs
(B) TYPE: nucleic acid
(C) STRANDEDNESS: double
(D) TOPOLOGY: both

(ii) MOLECULE TYPE: genomic

(ix) FEATURE:
(A) NAME/KEY: CDS
(B) LOCATION: 2..27
(D) OTHER INFORMATION: /product= VEGF gene EXON V

(xi) SEQUENCE DESCRIPTION: SEQ ID NO:82

A CCA AAG AAA GAT AGA GCA AGA CAA GAA AA 30
Pro Lys Lys Asp Arg Ala Arg Gln Glu
                5

(2) INFORMATION FOR SEQ ID NO:83

(i) SEQUENCE CHARACTERISTICS:
(A) LENGTH: 72 base pairs
(B) TYPE: nucleic acid
(C) STRANDEDNESS: double
(D) TOPOLOGY: both

(ii) MOLECULE TYPE: genomic

(ix) FEATURE:
(A) NAME/KEY: CDS
(B) LOCATION: 2..70
(D) OTHER INFORMATION: /product= VEGF gene EXON VI

(xi) SEQUENCE DESCRIPTION: SEQ ID NO:83

A AAA TCA GTT CGA GGA AAG GGA AAG GGG CAA AAA CGA AAG CGC AAG AAA 49
Lys Ser Val Arg Gly Lys Gly Lys Gly Gln Lys Arg Lys Arg Lys Lys
1               5                   10                  15

TCC CGG TAT AAG TCC TGG AGC GT 72
Ser Arg Tyr Lys Ser Trp Ser
            20

(2) INFORMATION FOR SEQ ID NO:84

(i) SEQUENCE CHARACTERISTICS:
(A) LENGTH: 51 base pairs
(B) TYPE: nucleic acid
(C) STRANDEDNESS: double
(D) TOPOLOGY: both

(ii) MOLECULE TYPE: genomic

(ix) FEATURE:
(A) NAME/KEY: CDS
(B) LOCATION: 1..51
(D) OTHER INFORMATION: /product= Insert between EXON VI & VII

(xi) SEQUENCE DESCRIPTION: SEQ ID NO:84

TAC GTT GGT GCC CGC TGC TGT CTA ATG CCC TGG AGC CTC CCT GGC CCC 48
Tyr Val Gly Ala Arg Cys Cys Leu Met Pro Trp Ser Leu Pro Gly Pro

1                5                   10                  15

CAT                                                                    51
His

(2) INFORMATION FOR SEQ ID NO:85

     (i) SEQUENCE CHARACTERISTICS:
          (A) LENGTH: 132 base pairs
          (B) TYPE: nucleic acid
          (C) STRANDEDNESS: double
          (D) TOPOLOGY: both

    (ii) MOLECULE TYPE: genomic

    (ix) FEATURE:
          (A) NAME/KEY: CDS
          (B) LOCATION: 2..130
          (D) OTHER INFORMATION: /product= EXON VII

    (xi) SEQUENCE DESCRIPTION: SEQ ID NO:85

T CCC TGT GGG CCT TGC TCA GAG CGG AGA AAG CAT TTG TTT GTA CAA GAT      49
  Pro Cys Gly Pro Cys Ser Glu Arg Arg Lys His Leu Phe Val Gln Asp
    1               5                  10                  15

CCG CAG ACG TGT AAA TGT TCC TGC AAA AAC ACA GAC TCG CGT TGC AAG        97
Pro Gln Thr Cys Lys Cys Ser Cys Lys Asn Thr Asp Ser Arg Cys Lys
            20                  25                  30

GCG AGG CAG CTT GAG TTA AAC GAA CGT ACT TGC AG                        132
Ala Arg Gln Leu Glu Leu Asn Glu Arg Thr Cys
        35                  40

(2) INFORMATION FOR SEQ ID NO:86

     (i) SEQUENCE CHARACTERISTICS:
          (A) LENGTH: 22 base pairs
          (B) TYPE: nucleic acid
          (C) STRANDEDNESS: double
          (D) TOPOLOGY: both

    (ii) MOLECULE TYPE: genomic

    (ix) FEATURE:
          (A) NAME/KEY: CDS
          (B) LOCATION: 2..19
          (D) OTHER INFORMATION: /product= EXON VIII

    (xi) SEQUENCE DESCRIPTION: SEQ ID NO:86

A TGT GAC AAG CCG AGG CGG TGA                                          22
  Cys Asp Lys Pro Arg Arg
    1               5

(2) INFORMATION FOR SEQ ID NO:87

(i) SEQUENCE CHARACTERISTICS:
(A) LENGTH: 473 base pairs
(B) TYPE: nucleic acid
(C) STRANDEDNESS: double
(D) TOPOLOGY: both

(ii) MOLECULE TYPE: cDNA

(ix) FEATURE:
(A) NAME/KEY: CDS
(B) LOCATION: 13..456
(D) OTHER INFORMATION: /product= "VEGF121-encoding DNA"

(ix) FEATURE:
(A) NAME/KEY: CDS
(B) LOCATION: 13..90
(D) OTHER INFORMATION: /product= leader-encoding sequence

(xi) SEQUENCE DESCRIPTION: SEQ ID NO:87

GGATCCGAAA CC ATG AAC TTT CTG CTG TCT TGG GTG CAT TGG AGC CTT 48
Met Asn Phe Leu Leu Ser Trp Val His Trp Ser Leu
1               5                   10

GCC TTG CTG CTC TAC CTC CAC CAT GCC AAG TGG TCC CAG GCT GCA CCC 96
Ala Leu Leu Leu Tyr Leu His His Ala Lys Trp Ser Gln Ala Ala Pro
        15                  20                  25

ATG GCA GAA GGA GGA GGG CAG AAT CAT CAC GAA GTG GTG AAG TTC ATG 144
Met Ala Glu Gly Gly Gly Gln Asn His His Glu Val Val Lys Phe Met
    30                  35                  40

GAT GTC TAT CAG CGC AGC TAC TGC CAT CCA ATC GAG ACC CTG GTG GAC 192
Asp Val Tyr Gln Arg Ser Tyr Cys His Pro Ile Glu Thr Leu Val Asp
45                  50                  55                  60

ATC TTC CAG GAG TAC CCT GAT GAG ATC GAG TAC ATC TTC AAG CCA TCC 240
Ile Phe Gln Glu Tyr Pro Asp Glu Ile Glu Tyr Ile Phe Lys Pro Ser
                65                  70                  75

TGT GTG CCC CTG ATG CGA TGC GGG GGC TGC TGC AAT GAC GAG GGC CTG 288
Cys Val Pro Leu Met Arg Cys Gly Gly Cys Cys Asn Asp Glu Gly Leu
            80                  85                  90

GAG TGT GTG CCC ACT GAG GAG TCC AAC ATC ACC ATG CAG ATT ATG CGG 336
Glu Cys Val Pro Thr Glu Glu Ser Asn Ile Thr Met Gln Ile Met Arg
        95                  100                 105

ATC AAA CCT CAC CAA GGC CAG CAC ATA GGA GAG ATG AGC TTC CTA CAG 384
Ile Lys Pro His Gln Gly Gln His Ile Gly Glu Met Ser Phe Leu Gln
    110                 115                 120

CAC AAC AAA TGT GAA TGC AGA CCA AAG AAA GAT AGA GCA AGA CAA GAA 432
His Asn Lys Cys Glu Cys Arg Pro Lys Lys Asp Arg Ala Arg Gln Glu
125                 130                 135                 140

AAA TGT GAC AAG CCG AGG CGG TGATGAATGA ATGAGGATCC 473
Lys Cys Asp Lys Pro Arg Arg
                145

(2) INFORMATION FOR SEQ ID NO:88

(i) SEQUENCE CHARACTERISTICS:
(A) LENGTH: 605 base pairs
(B) TYPE: nucleic acid
(C) STRANDEDNESS: double
(D) TOPOLOGY: both

(ii) MOLECULE TYPE: cDNA

(ix) FEATURE:
(A) NAME/KEY: CDS
(B) LOCATION: 13..588
(D) OTHER INFORMATION: /product= "VEGF165-encoding DNA"

(ix) FEATURE:
(A) NAME/KEY: CDS
(B) LOCATION: 13..90
(D) OTHER INFORMATION: /product= "leader sequence-encoding DNA"

(xi) SEQUENCE DESCRIPTION: SEQ ID NO:88

GGATCCGAAA CC ATG AAC TTT CTG CTG TCT TGG GTG CAT TGG AGC CTT 48
Met Asn Phe Leu Leu Ser Trp Val His Trp Ser Leu
1               5                   10

GCC TTG CTG CTC TAC CTC CAC CAT GCC AAG TGG TCC CAG GCT GCA CCC 96
Ala Leu Leu Leu Tyr Leu His His Ala Lys Trp Ser Gln Ala Ala Pro
        15                  20                  25

ATG GCA GAA GGA GGA GGG CAG AAT CAT CAC GAA GTG GTG AAG TTC ATG 144
Met Ala Glu Gly Gly Gly Gln Asn His His Glu Val Val Lys Phe Met
    30                  35                  40

GAT GTC TAT CAG CGC AGC TAC TGC CAT CCA ATC GAG ACC CTG GTG GAC 192
Asp Val Tyr Gln Arg Ser Tyr Cys His Pro Ile Glu Thr Leu Val Asp
45                  50                  55                  60

ATC TTC CAG GAG TAC CCT GAT GAG ATC GAG TAC ATC TTC AAG CCA TCC 240
Ile Phe Gln Glu Tyr Pro Asp Glu Ile Glu Tyr Ile Phe Lys Pro Ser
                65                  70                  75

TGT GTG CCC CTG ATG CGA TGC GGG GGC TGC TGC AAT GAC GAG GGC CTG 288
Cys Val Pro Leu Met Arg Cys Gly Gly Cys Cys Asn Asp Glu Gly Leu
            80                  85                  90

GAG TGT GTG CCC ACT GAG GAG TCC AAC ATC ACC ATG CAG ATT ATG CGG 336
Glu Cys Val Pro Thr Glu Glu Ser Asn Ile Thr Met Gln Ile Met Arg
        95                  100                 105

ATC AAA CCT CAC CAA GGC CAG CAC ATA GGA GAG ATG AGC TTC CTA CAG 384
Ile Lys Pro His Gln Gly Gln His Ile Gly Glu Met Ser Phe Leu Gln
    110                 115                 120

CAC AAC AAA TGT GAA TGC AGA CCA AAG AAA GAT AGA GCA AGA CAA GAA 432
His Asn Lys Cys Glu Cys Arg Pro Lys Lys Asp Arg Ala Arg Gln Glu
125                 130                 135                 140

AAT CCC TGT GGG CCT TGC TCA GAG CGG AGA AAG CAT TTG TTT GTA CAA 480
Asn Pro Cys Gly Pro Cys Ser Glu Arg Arg Lys His Leu Phe Val Gln
                145                 150                 155

GAT CCG CAG ACG TGT AAA TGT TCC TGC AAA AAC ACA GAC TCG CGT TGC 528
Asp Pro Gln Thr Cys Lys Cys Ser Cys Lys Asn Thr Asp Ser Arg Cys
            160                 165                 170

AAG GCG AGG CAG CTT GAG TTA AAC GAA CGT ACT TGC AGA TGT GAC AAG 576
Lys Ala Arg Gln Leu Glu Leu Asn Glu Arg Thr Cys Arg Cys Asp Lys
        175                 180                 185

CCG AGG CGG TGATGAATGA ATGAGGATCC 605
Pro Arg Arg
    190

(2) INFORMATION FOR SEQ ID NO:89

(i) SEQUENCE CHARACTERISTICS:
(A) LENGTH: 677 base pairs
(B) TYPE: nucleic acid
(C) STRANDEDNESS: double
(D) TOPOLOGY: both

(ii) MOLECULE TYPE: cDNA

(ix) FEATURE:
(A) NAME/KEY: CDS
(B) LOCATION: 13..657
(D) OTHER INFORMATION: /product= "VEGF189-encoding DNA"

(ix) FEATURE:
(A) NAME/KEY: CDS
(B) LOCATION: 13..90
(D) OTHER INFORMATION: /product= "leader sequence-encoding DNA"

(xi) SEQUENCE DESCRIPTION: SEQ ID NO:89

GGATCCGAAA CC ATG AAC TTT CTG CTG TCT TGG GTG CAT TGG AGC CTT 48
Met Asn Phe Leu Leu Ser Trp Val His Trp Ser Leu
1               5                   10

GCC TTG CTG CTC TAC CTC CAC CAT GCC AAG TGG TCC CAG GCT GCA CCC 96
Ala Leu Leu Leu Tyr Leu His His Ala Lys Trp Ser Gln Ala Ala Pro
        15                  20                  25

ATG GCA GAA GGA GGA GGG CAG AAT CAT CAC GAA GTG GTG AAG TTC ATG 144
Met Ala Glu Gly Gly Gly Gln Asn His His Glu Val Val Lys Phe Met
    30                  35                  40

GAT GTC TAT CAG CGC AGC TAC TGC CAT CCA ATC GAG ACC CTG GTG GAC 192
Asp Val Tyr Gln Arg Ser Tyr Cys His Pro Ile Glu Thr Leu Val Asp
45                  50                  55                  60

ATC TTC CAG GAG TAC CCT GAT GAG ATC GAG TAC ATC TTC AAG CCA TCC 240
Ile Phe Gln Glu Tyr Pro Asp Glu Ile Glu Tyr Ile Phe Lys Pro Ser
                65                  70                  75

TGT GTG CCC CTG ATG CGA TGC GGG GGC TGC TGC AAT GAC GAG GGC CTG 288
Cys Val Pro Leu Met Arg Cys Gly Gly Cys Cys Asn Asp Glu Gly Leu
            80                  85                  90

GAG TGT GTG CCC ACT GAG GAG TCC AAC ATC ACC ATG CAG ATT ATG CGG 336
Glu Cys Val Pro Thr Glu Glu Ser Asn Ile Thr Met Gln Ile Met Arg
        95                  100                 105

ATC AAA CCT CAC CAA GGC CAG CAC ATA GGA GAG ATG AGC TTC CTA CAG 384
Ile Lys Pro His Gln Gly Gln His Ile Gly Glu Met Ser Phe Leu Gln
    110                 115                 120

CAC AAC AAA TGT GAA TGC AGA CCA AAG AAG GAT AGA GCA AGA CAA GAA 432
His Asn Lys Cys Glu Cys Arg Pro Lys Lys Asp Arg Ala Arg Gln Glu
125                 130                 135                 140

AAA AAA TCA GTT CGA GGA AAG GGA AAG GGG CAA AAA CGA AAG CGC AAG 480
Lys Lys Ser Val Arg Gly Lys Gly Lys Gly Gln Lys Arg Lys Arg Lys
                145                 150                 155

AAA TCC CGG TAT AAG TCC TGG AGC GTT CCC TGT GGG CCT TGC TCA GAG 528
Lys Ser Arg Tyr Lys Ser Trp Ser Val Pro Cys Gly Pro Cys Ser Glu
            160                 165                 170

CGG AGA AAG CAT TTG TTT GTA CAA GAT CCG CAG ACG TGT AAA TGT TCC 576
Arg Arg Lys His Leu Phe Val Gln Asp Pro Gln Thr Cys Lys Cys Ser
        175                 180                 185

TGC AAA AAC ACA GAC TCG CGT TGC AAG GCG AGG CAG CTT GAG TTA AAC 624
Cys Lys Asn Thr Asp Ser Arg Cys Lys Ala Arg Gln Leu Glu Leu Asn
    190                 195                 200

GAA CGT ACT TGC AGA TGT GAC AAG CCG AGG CGG TGATGAATGA ATGAGGATCC 677
Glu Arg Thr Cys Arg Cys Asp Lys Pro Arg Arg
205                 210                 215

(2) INFORMATION FOR SEQ ID NO:90

(i) SEQUENCE CHARACTERISTICS:
(A) LENGTH: 728 base pairs
(B) TYPE: nucleic acid
(C) STRANDEDNESS: double
(D) TOPOLOGY: both

(ii) MOLECULE TYPE: cDNA

(ix) FEATURE:
(A) NAME/KEY: CDS
(B) LOCATION: 13..711
(D) OTHER INFORMATION: /product= "VEGF206-encoding DNA"
(ix) FEATURE:
(A) NAME/KEY: CDS
(B) LOCATION: 13..90
(D) OTHER INFORMATION: /product= leader sequence encoding DNA

(xi) SEQUENCE DESCRIPTION: SEQ ID NO:90

GGATCCGAAA CC ATG AAC TTT CTG CTG TCT TGG GTG CAT TGG AGC CTT 48
Met Asn Phe Leu Leu Ser Trp Val His Trp Ser Leu
1               5                   10

GCC TTG CTG CTC TAC CTC CAC CAT GCC AAG TGG TCC CAG GCT GCA CCC 96
Ala Leu Leu Leu Tyr Leu His His Ala Lys Trp Ser Gln Ala Ala Pro
        15                  20                  25

ATG GCA GAA GGA GGA GGG CAG AAT CAT CAC GAA GTG GTG AAG TTC ATG 144
Met Ala Glu Gly Gly Gly Gln Asn His His Glu Val Val Lys Phe Met
    30                  35                  40

GAT GTC TAT CAG CGC AGC TAC TGC CAT CCA ATC GAG ACC CTG GTG GAC 192
Asp Val Tyr Gln Arg Ser Tyr Cys His Pro Ile Glu Thr Leu Val Asp
45                  50                  55                  60

ATC TTC CAG GAG TAC CCT GAT GAG ATC GAG TAC ATC TTC AAG CCA TCC 240
Ile Phe Gln Glu Tyr Pro Asp Glu Ile Glu Tyr Ile Phe Lys Pro Ser
                65                  70                  75

TGT GTG CCC CTG ATG CGA TGC GGG GGC TGC TGC AAT GAC GAG GGC CTG 288
Cys Val Pro Leu Met Arg Cys Gly Gly Cys Cys Asn Asp Glu Gly Leu
            80                  85                  90

GAG TGT GTG CCC ACT GAG GAG TCC AAC ATC ACC ATG CAG ATT ATG CGG 336
Glu Cys Val Pro Thr Glu Glu Ser Asn Ile Thr Met Gln Ile Met Arg
        95                  100                 105

ATC AAA CCT CAC CAA GGC CAG CAC ATA GGA GAG ATG AGC TTC CTA CAG 384
Ile Lys Pro His Gln Gly Gln His Ile Gly Glu Met Ser Phe Leu Gln
    110                 115                 120

CAC AAC AAA TGT GAA TGC AGA CCA AAG AAG GAT AGA GCA AGA CAA GAA 432
His Asn Lys Cys Glu Cys Arg Pro Lys Lys Asp Arg Ala Arg Gln Glu
125                 130                 135                 140

AAA AAA TCA GTT CGA GGA AAG GGA AAG GGG CAA AAA CGA AAG CGC AAG 480
Lys Lys Ser Val Arg Gly Lys Gly Lys Gly Gln Lys Arg Lys Arg Lys
                145                 150                 155

AAA TCC CGG TAT AAG TCC TGG AGC GTT TAC GTT GGT GCC CGC TGC TGT 528
Lys Ser Arg Tyr Lys Ser Trp Ser Val Tyr Val Gly Ala Arg Cys Cys
            160                 165                 170

CTA ATG CCC TGG AGC CTC CCT GGC CCC CAT CCC TGT GGG CCT TGC TCA 576
Leu Met Pro Trp Ser Leu Pro Gly Pro His Pro Cys Gly Pro Cys Ser
        175                 180                 185

GAG CGG AGA AAG CAT TTG TTT GTA CAA GAT CCG CAG ACG TGT AAA TGT 624
Glu Arg Arg Lys His Leu Phe Val Gln Asp Pro Gln Thr Cys Lys Cys
    190                 195                 200

TCC TGC AAA AAC ACA GAC TCG CGT TGC AAG GCG AGG CAG CTT GAG TTA 672
Ser Cys Lys Asn Thr Asp Ser Arg Cys Lys Ala Arg Gln Leu Glu Leu
205                 210                 215                 220

AAC GAA CGT ACT TGC AGA TGT GAC AAG CCG AGG CGG TGATGAATGA 718
Asn Glu Arg Thr Cys Arg Cys Asp Lys Pro Arg Arg
225 230 235

ATGAGGATCC 728

(2) INFORMATION FOR SEQ ID NO: 91

(i) SEQUENCE CHARACTERISTICS:
(A) LENGTH: 627 base pairs
(B) TYPE: nucleic acid
(C) STRANDEDNESS: double
(D) TOPOLOGY: both

(ii) MOLECULE TYPE: cDNA

(ix) FEATURE:
(A) NAME/KEY: CDS
(B) LOCATION: 1..627
(D) OTHER INFORMATION: /note "human HBEGF precursor"

(xi) SEQUENCE DESCRIPTION: SEQ ID NO:91

ATG AAG CTG CTG CCG TCG GTG GTG CTG AAG CTC TTT CTG GCT GCA GTT 48
Met Lys Leu Leu Pro Ser Val Val Leu Lys Leu Phe Leu Ala Ala Val
1               5                   10                  15

CTC TCG GCA CTG GTG ACT GGC GAG AGC CTG GAG CGG CTT CGG AGA GGG 96
Leu Ser Ala Leu Val Thr Gly Glu Ser Leu Glu Arg Leu Arg Arg Gly
            20                  25                  30

CTA GCT GCT GGA ACC AGC AAC CCG GAC CCT CCC ACT GTA TCC ACG GAC 144
Leu Ala Ala Gly Thr Ser Asn Pro Asp Pro Pro Thr Val Ser Thr Asp
        35                  40                  45

Leu Ala Ala Gly Thr Ser Asn Pro Asp Pro Pro Thr Val Ser Thr Asp
        35                  40                  45

CAG CTG CTA CCC CTA GGA GGC GGC CGG GAC CGG AAA GTC CGT GAC TTG        192
Gln Leu Leu Pro Leu Gly Gly Gly Arg Asp Arg Lys Val Arg Asp Leu
    50                  55                  60

CAA GAG GCA GAT CTG GAC CTT TTG AGA GTC ACT TTA TCC TCC AAG CCA        240
Gln Glu Ala Asp Leu Asp Leu Leu Arg Val Thr Leu Ser Ser Lys Pro
 65                  70                  75                  80

CAA GCA CTG GCC ACA CCA AAC AAG GAG GAG CAC GGG AAA AGA AAG AAG        288
Gln Ala Leu Ala Thr Pro Asn Lys Glu Glu His Gly Lys Arg Lys Lys
                 85                  90                  95

AAA GGC AAG GGG CTA GGG AAG AAG AGG GAC CCA TGT CTT CGG AAA TAC        336
Lys Gly Lys Gly Leu Gly Lys Lys Arg Asp Pro Cys Leu Arg Lys Tyr
            100                 105                 110

AAG GAC TTC TGC ATC CAT GGA GAA TGC AAA TAT GTG AAG GAG CTC CGG        384
Lys Asp Phe Cys Ile His Gly Glu Cys Lys Tyr Val Lys Glu Leu Arg
        115                 120                 125

GCT CCC TCC TGC ATC TGC CAC CCG GGT TAC CAT GGA GAG AGG TGT CAT        432
Ala Pro Ser Cys Ile Cys His Pro Gly Tyr His Gly Glu Arg Cys His
    130                 135                 140

GGG CTG AGC CTC CCA GTG GAA AAT CGC TTA TAT ACC TAT GAC CAC ACA        480
Gly Leu Ser Leu Pro Val Glu Asn Arg Leu Tyr Thr Tyr Asp His Thr
145                 150                 155                 160

ACC ATC CTG GCC GTG GTG GCT GTG GTG CTG TCA TCT GTC TGT CTG CTG        528
Thr Ile Leu Ala Val Val Ala Val Val Leu Ser Ser Val Cys Leu Leu
                165                 170                 175

GTC ATC GTG GGG CTT CTC ATG TTT AGG TAC CAT AGG AGA GGA GGT TAT        576
Val Ile Val Gly Leu Leu Met Phe Arg Tyr His Arg Arg Gly Gly Tyr
            180                 185                 190

GAT GTG GAA AAT GAA GAG AAA GTG AAG TTG GGC ATG ACT AAT TCC CAC        624
Asp Val Glu Asn Glu Glu Lys Val Lys Leu Gly Met Thr Asn Ser His
        195                 200                 205

TGA                                                                    627

(2) INFORMATION FOR SEQ ID NO:92

     (i) SEQUENCE CHARACTERISTICS:
          (A) LENGTH: 208 amino acids
          (B) TYPE: amino acid
          (D) TOPOLOGY: linear

    (ii) MOLECULE TYPE: protein

(ix) FEATURE:
(A) NAME/KEY: CDS
(B) LOCATION: 1..627
(D) OTHER INFORMATION: /note "human HBEGF precursor"

(xi) SEQUENCE DESCRIPTION: SEQ ID NO:92

Met Lys Leu Leu Pro Ser Val Val Leu Lys Leu Phe Leu Ala Ala Val
1               5                   10                  15

Leu Ser Ala Leu Val Thr Gly Glu Ser Leu Glu Arg Leu Arg Arg Gly
            20                  25                  30

Leu Ala Ala Gly Thr Ser Asn Pro Asp Pro Pro Thr Val Ser Thr Asp
        35                  40                  45

Gln Leu Leu Pro Leu Gly Gly Gly Arg Asp Arg Lys Val Arg Asp Leu
    50                  55                  60

Gln Glu Ala Asp Leu Asp Leu Leu Arg Val Thr Leu Ser Ser Lys Pro
65                  70                  75                  80

Gln Ala Leu Ala Thr Pro Asn Lys Glu Glu His Gly Lys Arg Lys Lys
                85                  90                  95

Lys Gly Lys Gly Leu Gly Lys Lys Arg Asp Pro Cys Leu Arg Lys Tyr
            100                 105                 110

Lys Asp Phe Cys Ile His Gly Glu Cys Lys Tyr Val Lys Glu Leu Arg
        115                 120                 125

Ala Pro Ser Cys Ile Cys His Pro Gly Tyr His Gly Glu Arg Cys His
    130                 135                 140

Gly Leu Ser Leu Pro Val Glu Asn Arg Leu Tyr Thr Tyr Asp His Thr
145                 150                 155                 160

Thr Ile Leu Ala Val Val Ala Val Val Leu Ser Ser Val Cys Leu Leu
                165                 170                 175

Val Ile Val Gly Leu Leu Met Phe Arg Tyr His Arg Arg Gly Gly Tyr
            180                 185                 190

Asp Val Glu Asn Glu Glu Lys Val Lys Leu Gly Met Thr Asn Ser His
        195                 200                 205

(2) INFORMATION FOR SEQ ID NO:93

(i) SEQUENCE CHARACTERISTICS:
(A) LENGTH: 77 amino acids
(B) TYPE: amino acid
(C) STRANDEDNESS: unknown
(D) TOPOLOGY: unknown

(ii) MOLECULE TYPE: protein

(ix) FEATURE:
(D) OTHER INFORMATION: /note "human mature HBEGF"

(xi) SEQUENCE DESCRIPTION: SEQ ID NO:93

Arg Val Thr Leu Ser Ser Lys Pro Gln Ala Leu Ala Thr Pro Asn Lys
1               5                   10                  15

Glu Glu His Gly Lys Arg Lys Lys Lys Gly Lys Gly Leu Gly Lys Lys
            20                  25                  30

Arg Asp Pro Cys Leu Arg Lys Tyr Lys Asp Phe Cys Ile His Gly Glu
        35                  40                  45

Cys Lys Tyr Val Lys Glu Leu Arg Ala Pro Ser Cys Ile Cys His Pro
    50                  55                  60

Gly Tyr His Gly Glu Arg Cys His Gly Leu Ser Leu Pro
65                  70                  75

(2) INFORMATION FOR SEQ ID NO:94

(i) SEQUENCE CHARACTERISTICS:
(A) LENGTH: 208 amino acids
(B) TYPE: amino acid
(C) STRANDEDNESS: unknown
(D) TOPOLOGY: unknown

(ii) MOLECULE TYPE: protein

(ix) FEATURE:
(D) OTHER INFORMATION: /note "monkey HBEGF precursor"

(xi) SEQUENCE DESCRIPTION: SEQ ID NO:94

Met Lys Leu Leu Pro Ser Val Val Leu Lys Leu Leu Leu Ala Ala Val
1               5                   10                  15

Leu Ser Ala Leu Val Thr Gly Glu Ser Leu Glu Gln Leu Arg Arg Gly
            20                  25                  30

Leu Ala Ala Gly Thr Ser Asn Pro Asp Pro Ser Thr Gly Ser Thr Asp
        35                  40                  45

Gln Leu Leu Arg Leu Gly Gly Gly Arg Asp Arg Lys Val Arg Asp Leu
    50                  55                  60

Gln Glu Ala Asp Leu Asp Leu Leu Arg Val Thr Leu Ser Ser Lys Pro
65                  70                  75                  80

Gln Ala Leu Ala Thr Pro Ser Lys Glu Glu His Gly Lys Arg Lys Lys

85                  90                  95

Lys Gly Lys Gly Leu Gly Lys Lys Arg Asp Pro Cys Leu Arg Lys Tyr
            100                 105                 110

Lys Asp Phe Cys Ile His Gly Glu Cys Lys Tyr Val Lys Glu Leu Arg
        115                 120                 125

Ala Pro Ser Cys Ile Cys His Pro Gly Tyr His Gly Glu Arg Cys His
    130                 135                 140

Gly Leu Ser Leu Pro Val Glu Asn Arg Leu Tyr Thr Tyr Asp His Thr
145                 150                 155                 160

Thr Ile Leu Ala Val Val Ala Val Val Leu Ser Ser Val Cys Leu Leu
                165                 170                 175

Val Ile Val Gly Leu Leu Met Phe Arg Tyr His Arg Arg Gly Gly Tyr
            180                 185                 190

Asp Val Glu Asn Glu Glu Lys Val Lys Leu Gly Met Thr Asn Ser His
        195                 200                 205

(2) INFORMATION FOR SEQ ID NO:95

    (i) SEQUENCE CHARACTERISTICS:
        (A) LENGTH: 208 amino acids
        (B) TYPE: amino acid
        (C) STRANDEDNESS: unknown
        (D) TOPOLOGY: unknown

    (ii) MOLECULE TYPE: protein

    (ix) FEATURE:
            (D) OTHER INFORMATION: /note "rat HBEGF precursor"

    (xi) SEQUENCE DESCRIPTION: SEQ ID NO:95

Met Lys Leu Leu Pro Ser Val Val Leu Lys Leu Phe Leu Ala Ala Val
1               5                   10                  15

Leu Ser Ala Leu Val Thr Gly Glu Ser Leu Glu Arg Leu Arg Arg Gly
            20                  25                  30

Leu Ala Ala Ala Thr Ser Asn Pro Asp Pro Pro Thr Gly Thr Thr Asn
        35                  40                  45

Gln Leu Leu Pro Thr Gly Ala Asp Arg Ala Gln Glu Val Gln Asp Leu
    50                  55                  60

Glu Gly Thr Asp Leu Asp Leu Phe Lys Val Ala Phe Ser Ser Lys Pro
65                  70                  75                  80

Gln Ala Leu Ala Thr Pro Gly Lys Glu Lys Asn Gly Lys Lys Lys Arg
                85                  90                  95

Lys Gly Lys Gly Leu Gly Lys Lys Arg Asp Pro Cys Leu Lys Lys Tyr
            100                 105                 110

Lys Asp Tyr Cys Ile His Gly Glu Cys Arg Tyr Leu Lys Glu Leu Arg
        115                 120                 125

Ile Pro Ser Cys His Cys Leu Pro Gly Tyr His Gly Gln Arg Cys His
    130                 135                 140

Gly Leu Thr Leu Pro Val Glu Asn Pro Leu Tyr Thr Tyr Asp His Thr
145                 150                 155                 160

Thr Val Leu Ala Val Val Ala Val Val Leu Ser Ser Val Cys Leu Leu
                165                 170                 175

Val Ile Val Gly Leu Leu Met Phe Arg Tyr His Arg Arg Gly Gly Tyr
            180                 185                 190

Asp Leu Glu Ser Glu Glu Lys Val Lys Leu Gly Met Ala Ser Ser His
        195                 200                 205